# EUROPEAN PATENT SPECIFICATION

(11) **EP 1 562 902 B1**
(45) Date of publication and mention of the grant of the patent: **03.05.2006**
(21) Application number: 03811170.4
(22) Date of filing: 11.11.2003
(51) Int. Cl.: C07D 211/86, C07D 239/22, A61K 31/4412, A61K 31/505, A61P 29/00

(54) **2-PYRIDONE DERIVATIVES AS INHIBITORS OF NEUTROPHILE ELASTASE**
2-PYRIDONDERIVATE ALS INHIBITOREN VON NEUTROPHILER ELASTASE
DERIVES DE 2-PYRIDONE EN TANT QU'INHIBITEURS DE L'ELASTASE DE NEUTROPHILE

(30) Priority: 12.11.2002 SE 0203348; 12.02.2003 SE 0300388; 22.07.2003 SE 0302120
(43) Date of publication of application: 17.08.2005
(73) Proprietor: AstraZeneca AB, 151 85 Södertälje (SE)
(72) Inventor: BLADH, Hakan, S-221 87 Lund (SE); KLINGSTEDT, Tomas, S-221 87 Lund (SE); LARSSON, Joakim, S-221 87 Lund (SE); LAWITZ, Karolina, S-221 87 Lund (SE); LEPISTÖ, Matti, S-221 87 Lund (SE); LÖNN, Hans, S-221 87 Lund (SE); NIKITIDIS, Grigorios, S-221 87 Lund (SE)
(86) International application number: PCT/SE2003/001739
(87) International publication number: WO 2004/043924

(56) References cited:
- WO-A1-02/053543
- WO-A1-03/053543
- US-A- 541 960
- US-A- 4 181 658
- US-A- 5 441 960

## Description

### Field of the Invention

This invention relates to novel 2-pyridone derivatives, processes for their preparation, pharmaceutical compositions comprising them, and their use in therapy.

### Background of the Invention

Elastases are possibly the most destructive enzymes in the body, having the ability to degrade virtually all connective tissue components. The uncontrolled proteolytic degradation by elastases has been implicated in a number of pathological conditions. Human neutrophil elastase (hNE), a member of the chymotrypsin superfamily of serine proteases is a 33-KDa enzyme stored in the azurophilic granules of the neutrophils. In neutrophils the concentration of NE exceeded 5 mM and its total cellular amount has been estimated to be up to 3 pg. Upon activation, NE is rapidly released from the granules into the extracellular space with some portion remaining bound to neutrophil plasma membrane (See Kawabat et al. 2002, Eur. J. Pharmacol. 451,1-10). The main intracellular physiological function of NE is degradation of foreign organic molecules phagocytosed by neutrophils, whereas the main target for extracellular elastase is elastin (Janoff and Scherer, 1968, J. Exp. Med. 128, 1137-1155). NE is unique, as compared to other proteases (for example, proteinase 3) in that it has the ability to degrade almost all extracellular matrix and key plasma proteins (See Kawabat et al., 2002, Eur. J. Pharmacol. 451, 1-10). It degrades a wide range of extracellular matrix proteins such as elastin, Type 3 and type 4 collagens laminin, fibronectin, cytokines etc. (Ohbayashi, H., 2002, Expert Opin. Investig. Drugs, 11, 965-980). NE is a major common mediator of many pathological changes seen in chronic lung disease including epithelial damage (Stockley, R.A. 1994, Am. J. Resp. Crit. Care Med. 150, 109-113).

The destructive role of NE was solidified almost 40 years ago when Laurell and Eriksson reported an association of chronic airflow obstruction and emphysema with deficiency of serum α₁-antitrypsin (Laurell and Eriksson, 1963, Scand. J. Clin. Invest. 15, 132-140). Subsequently it was determined that α₁-antitrypsin is the most important endogenous inhibitor of human NE. The imbalance between human NE and endogenous antiprotease is believed to cause excess human NE in pulmonary tissues which is considered as a major pathogenic factor in chronic obstructive pulmonary disease (COPD). The excessive human NE shows a prominent destructive profile and actively takes part in destroying the normal pulmonary structures, followed by the irreversible enlargement of the respiratory airspaces, as seen mainly in emphysema. There is an increase in neutrophil recruitment into the lungs which is associated with increased lung elastase burden and emphysema in α₁-proteinase inhibitor-deficient mice (Cavarra et al., 1996, Lab. Invest. 75, 273-280). Individuals with higher levels of the NE-α₁ protease inhibitor complex in bronchoalveolar lavage fluid show significantly accelerated decline in lung functions compared to those with lower levels (Betsuyaku et al. 2000, Respiration, 67, 261-267). Instillation of human NE via the trachea in rats causes lung haemorrhage, neutrophil accumulation during acute phase and emphysematous changes during chronic phase (Karaki et al., 2002, Am. J. Resp. Crit. Care Med., 166, 496-500). Studies have shown that the acute phase of pulmonary emphysema and pulmonary haemorrhage caused by NE in hamsters can be inhibited by pre-treatment with inhibitors of NE (Fujie et al.,1999, Inflamm. Res. 48, 160-167).

Neutrophil-predominant airway inflammation and mucus obstruction of the airways are major pathologic features of COPD, including cystic fibrosis and chronic bronchitis. NE impairs mucin production, leading to mucus obstruction of the airways. NE is reported to increase the expression of major respiratory mucin gene, MUC5AC (Fischer, B.M & Voynow, 2002, Am. J. Respir. Cell Biol., 26, 447-452). Aerosol administration of NE to guinea pigs produces extensive epithelial damage within 20 minutes of contact (Suzuki et al., 1996, Am. J. Resp. Crit. Care Med., 153, 1405-1411). Furthermore NE reduces the ciliary beat frequency of human respiratory epithelium *in vitro* (Smallman et al., 1984, Thorax, 39, 663-667) which is consistent with the reduced mucociliary clearance that is seen in COPD patients (Currie et al., 1984, Thorax, 42, 126-130). The instillation of NE into the airways leads to mucus gland hyperplasia in hamsters (Lucey et al., 1985, Am. Resp. Crit. Care Med., 132, 362-366). A role for NE is also implicated in mucus hypersecretion in asthma. In an allergen sensitised guinea pig acute asthma model an inhibitor of NE prevented goblet cell degranulation and mucus hypersecretion (Nadel et al., 1999, Eur. Resp. J., 13, 190-196).

NE has been also shown to play a role in the pathogenesis of pulmonary fibrosis.
NE: α₁₋protenase inhibitor complex is increased in serum of patients with pulmonary fibrosis, which correlates with the clinical parameters in these patients (Yamanouchi et al., 1998, Eur. Resp. J. 11, 120-125). In a murine model of human pulmonary fibrosis, a NE inhibitor reduced bleomycin-induced pulmonary fibrosis (Taooka et al., 1997, Am. J. Resp. Crit. Care Med., 156,260-265). Furthermore investigators have shown that NE deficient mice are resistant to bleomycin-induced pulmonary fibrosis (Dunsmore et al., 2001, Chest, 120, 35S-36S). Plasma NE level was found to be elevated in patients who progressed to ARDS implicating the importance of NE in early ARDS disease pathogenesis. (Donnelly et al., 1995, Am. J. Res. Crit. Care Med., 151, 428-1433). The antiproteases and NE complexed with antiprotease are increased in lung cancer area (Marchandise et al., 1989, Eur. Resp. J. 2, 623-629). Recent studies have shown that polymorphism in the promoter region of the NE gene are associated with lung cancer development (Taniguchi et al., 2002, Clin. Cancer Res., 8, 1115-1120.

Acute lung injury caused by endotoxin in experimental animals is associated with elevated levels of NE (Kawabata, et al., 1999, Am. J. Resp. Crit. Care, 161, 2013-2018). Acute lung inflammation caused by intratracheal injection of lipopolysaccharide in mice has been shown to elevate the NE activity in bronchoalveolar lavage fluid which is significantly inhibited by a NE inhibitor (Fujie et al., 1999, Eur. J. Pharmacol., 374, 117-125; Yasui, et al., 1995, Eur. Resp. J., 8,1293-1299). NE also plays an important role in the neutrophil-induced increase of pulmonary microvascular permeability observed in a model of acute lung injury caused by tumor necrosis factor α (TNFα) and phorbol myristate acetate (PMA) in isolated perfused rabbit lungs (Miyazaki et al., 1998, Am. J. Respir. Crit. Care Med., 157, 89-94).

A role for NE has also been suggested in monocrotoline-induced pulmonary vascular wall thickening and cardiac hypertrophy (Molteni et al., 1989, Biochemical Pharmacol. 38, 2411-2419). Serine elastase inhibitor reverses the monocrotaline-induced pulmonary hypertension and remodelling in rat pulmonary arteries (Cowan et al., 2000, Nature Medicine, 6, 698-702). Recent studies have shown that serine elastase, that is, NE or vascular elastase are important in cigarette smoke-induced muscularisation of small pulmonary arteries in guinea pigs (Wright et al., 2002, Am. J. Respir. Crit. Care Med., 166, 954-960).

NE plays a key role in experimental cerebral ischemic damage (Shimakura et al., 2000, Brain Research, 858, 55-60), ischemia-reperfusion lung injury (Kishima et al., 1998, Ann. Thorac. Surg. 65, 913-918) and myocardial ischemia in rat heart (Tiefenbacher et al., 1997, Eur. J. Physiol., 433, 563-570). Human NE levels in plasma are significantly increased above normal in inflammatory bowel diseases, for example, Crohn's disease and ulcerative colitis (Adeyemi et al., 1985, Gut, 26, 1306-1311). In addition NE has also been assumed to be involved in the pathogenesis of rheumatoid arthritis (Adeyemi et al., 1986, Rheumatol. Int., 6, 57). The development of collegen induced arthritis in mice is suppressed by a NE inhibitor (Kakimoto et al., 1995, Cellular Immunol. 165, 26-32).

Thus, human NE is known as one of the most destructive serine proteases and has been implicated in a variety of inflammatory diseases. The important endogenous inhibitor of human NE is α₁-antitrypsin. The imbalance between human NE and antiprotease is believed to give rise to an excess of human NE resulting in uncontrolled tissue destruction. The protease/ antiprotease balance may be upset by a decreased availability of α₁-antitrypsin either through inactivation by oxidants such as cigarette smoke, or as a result of genetic inability to produce sufficient serum levels. Human NE has been implicated in the promotion or exacerbation of a number of diseases such as pulmonary emphysema, pulmonary fibrosis, adult respiratory distress syndrome (ARDS), ischemia reperfusion injury, rheumatoid arthritis and pulmonary hypertension.

WO 02/053543 discloses pyridone derivatives having affinity for cannabinoid 2-type receptor.

The present invention discloses novel 2-pyridone derivatives that are inhibitors of human neutrophil elastase and homologous serine proteases such as proteinase 3 and pancreatic elastase, and are thereby useful in therapy.

### Disclosure of the Invention

The present invention provides a compound of formula (I) 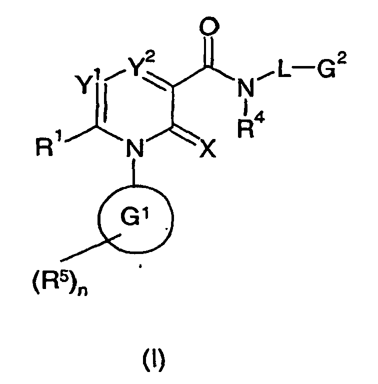 wherein
**X** represents O or S;
**Y**^{**1**} represents N or CR²; and when **R**^{**1**} represents OH, **Y**^{**1**} may also, in the tautomeric form, represent NR⁶;
**Y**^{**2**} represents CR³; and when **Y**^{**1**} represents CR², then **Y**^{**2**} may also represent N;
**R**^{**1**} represents H or C1 to 6 alkyl; said alkyl being optionally substituted by one or more substituents selected independently from halogen, CN, CHO, OR⁷, NR⁸R⁹, S(O)ₘR¹⁰ and SO₂NR¹¹R¹²;
and, when Y¹ represents N, **R**^{**1**} may also represent OH;
**R**^{**7**} represents H, C1 to 6 alkyl or phenyl; said phenyl being optionally further substituted by halogen, C1 to 6 alkyl and C1 to 6 alkoxy;
**R**^{**2**} represents H, halogen or C1 to 6 alkyl;
**R**^{**3**} represents H or F;
**G**^{**1**} represents phenyl or a five- or six-membered heteroaromatic ring containing 1 to 3 heteroatoms independently selected from O, S and N; or **G**^{**1**} represents a five- or six-membered saturated or partially unsaturated cycloalkyl ring; or **G**^{**1**} represents a five- or six-membered saturated or partially unsaturated heterocyclic ring containing one heteroatom selected from O, S and NR¹³ where R¹³ represents H or C1 to 6 alkyl;
**R**^{**5**} represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R**^{**14**} and **R**^{**15**} independently represent H or C1 to 3 alkyl; said alkyl being optionally further substituted by one or more F atoms;
**n** represents an integer 1, 2 or 3 and when n represents 2 or 3, each R⁵ group is selected independently;
**R**^{**4**} and **R**^{**6**} independently represent H or C1 to 6 alkyl; said alkyl being optionally further substituted by OH or C1 to 6 alkoxy;
or **R**^{**4**} and L are joined together such that the group **-NR**^{**4**}**L** represents a 5 to 7 membered azacyclic ring optionally incorporating one further heteroatom selected from O, S and NR¹⁶;
**L** represents a bond, O, NR²⁹ or C1 to 6 alkyl; said alkyl optionally incorporating a heteroatom selected from O, S and NR¹⁶; and said alkyl being optionally further substituted by OH or OMe;
**G**^{**2**} represents a monocyclic ring system selected from:
i) phenyl or phenoxy,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group; or
**G**^{**2**} represents a bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)p and NR¹⁷ and optionally further incorporating a carbonyl group;
and the two rings are either fused together, or are bonded directly together or are separated by a linker group selected from O, S(O)q or CH₂,
said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C1 to 3 alkyl substituted by SO₂R³⁹ or by one or more F atoms; or
when L does not represent a bond, **G**^{**2**} may also represent H;
**m, p, q,** s and t independently represent an integer 0, 1 or 2;
**R**^{**8**} and **R**^{**9**} independently represent H, C1 to 6 alkyl, formyl or C2 to 6 alkanoyl; said alkyl being optionally further substituted by phenyl optionally substituted by halogen, C1 to 6 alkyl, C1 to 6 alkoxy or SO₂R³⁰;
or the group **NR**^{**8**}**R**^{**9**} together represents a 5 to 7 membered azacyclic ring optionally incorporating one further heteroatom selected from O, S and NR²⁸;
**R**^{**18**} and **R**^{**19**} independently represent H, C1 to 6 alkyl, formyl, C2 to 6 alkanoyl, S(O)ₜR³² or SO₂NR³³R³⁴; said alkyl group being optionally further substituted by halogen, CN, C1
to 4 alkoxy or CONR⁴¹R⁴²;
**R**^{**25**} represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)ₚ and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C1 to 4 alkyl, C1 to 4 alkoxy, OH, CONR⁴⁴R⁴⁵, CO₂R⁴⁶, S(O)ₛR⁴⁷ and NHCOCH₃;
**R**^{**26**} and **R**^{**27**} independently represent H, C1 to 6 alkyl, formyl or C2 to 6 alkanoyl;
**R**^{**32**} represents H, C1 to 6 alkyl or C3 to 6 cycloalkyl;
**R**^{**38**} represents H, C1 to 6 alkyl or phenyl; said phenyl being optionally further substituted by halogen, C1 to 6 alkyl or C1 to 6 alkoxy;
**R**^{**10**}**,R**^{**11**}**,R**^{**12**}**R**^{**16**}**,R**^{**17**}**,R**^{**20**}**,R**^{**21**}**,R**^{**22**}**,R**^{**23**}**,R**^{**24**}**,R**^{**28**}**,R**^{**29**}**,R**^{**30**}**,R**^{**31**}**,R**^{**33**}**,R**^{**34**}**,R**^{**35**}**, R**^{**36**}**,R**^{**37**}**,R**^{**39**}**,R**^{**40**}**,R**^{**41**}**,R**^{**42**}**,R**^{**43**}**,R**^{**44**}**,R**^{**45**}**,R**^{**46**} and **R**^{**47**} independently represent H or C 1 to 6 alkyl;
and pharmaceutically acceptable salts thereof, with the proviso that the following compounds are disclaimed:
N-benzyl-5,6-dimethyl-2-oxo-1-phenyl-1,2-dihydropyridine-3-carboxamide;
N-(2-phenethyl)-5,6-dimethyl-2-oxo-1-phenyl-1,2-dihydropyxidine-3-carboxamide;
N-(2-hydroxyethyl)-2,4-dioxo-3-phenyl-1,2,3,4-tetrahydropyrimidine-5-carboxamide;
N-[2-(dimethylamino)ethyl]-2,4-dioxo-3-phenyl-1,2,3,4-tetrahydropyrimidine-5-carboxamide;
4-[2-[[[1,2-dihydro-1-(4-methylcyclohexyl)-2-oxo-3-pyridinyl]carbonyl]amino]ethyl]-benzoic acid; and
4-[2-[[(1-cyclohexyl-1,2-dihydro-2-oxo-3-pyridinyl]carbonyl)amino]ethyl]-benzoic acid.

The compounds of formula (I) may exist in enantiomeric forms. It is to be understood that all enantiomers, diastereomers, racemates and mixtures thereof are included within the scope of the invention.

Compounds of formula (I) may also exist in various tautomeric forms. Thus, for example, compounds of formula (I) wherein R¹ represents OH and Y¹ represents N, are tautomers of compounds of formula (Ia) wherein R⁶ represents H.

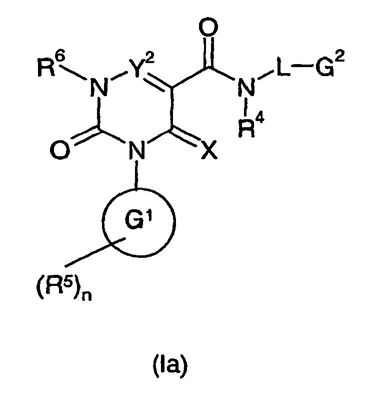

All possible tautomeric forms and mixtures thereof are included within the scope of the invention. Compounds of formula (Ia) wherein R⁶ represents H or optionally substituted C1 to 6 alkyl are thus specifically included within the scope of the invention.

Unless otherwise indicated, the term "C1 to 6 alkyl" referred to herein denotes a straight or branched chain alkyl group having from 1 to 6 carbon atoms. Examples of such groups include methyl, ethyl, n-propyl, i-propyl, n-butyl, i-butyl, t-butyl, pentyl and hexyl. The terms "C1 to 3 alkyl" and "C1 to 4 alkyl" are to be interpreted analogously.

Examples of "C1 to 3 alkyl substituted by one or more F atoms" include fluoromethyl, difluoromethyl, trifluoromethyl, 2,2,2-trifluoroethyl, 1,1-difluoroethyl, pentafluoroethyl and 3,3,3-trifluoropropyl.

Unless otherwise indicated, the term "C1 to 6 alkoxy " referred to herein denotes an oxygen substituent bonded to a straight or branched chain alkyl group having from 1 to 6 carbon atoms. Examples of such groups include methoxy, ethoxy, n-propoxy, i-propoxy, n-butoxy, i-butoxy and s-butoxy. The terms "C1 to 3 alkoxy" and "C1 to 4 alkoxy" are to be interpreted analogously.

Examples of "C1 to 3 alkoxy substituted by one or more F atoms" include fluoromethoxy, trifluoromethoxy, 2,2,2-trifluoroethoxy and 3,3,3-trifluoropropoxy.

Unless otherwise indicated, the term "C2 to 6 alkanoyl" referred to herein denotes a straight or branched chain alkyl group having from 1 to 5 carbon atoms bonded to the molecule via a carbonyl group. Examples of such groups include acetyl, propionyl and pivaloyl.

Unless otherwise indicated, the term "halogen" referred to herein denotes fluorine, chlorine, bromine and iodine.

Examples of a five or six membered heteroaromatic ring containing 1 to 3 heteroatoms independently selected from O, S and N include furan, thiophene, pyrrole, oxazole, oxadiazole, isoxazole, imidazole, thiazole, triazole, thiadiazole, pyridine, pyrimidine and pyrazine.

Unless otherwise indicated, the term "C3 to 6 saturated or partially unsaturated cycloalkyl" referred to herein denotes a 3 to 6 membered non-aromatic carbocyclic ring optionally incorporating one or more double bonds. Examples include cyclopropyl, cyclopentyl, cyclopentenyl, cyclohexyl and cyclohexenyl. The term "five- or six-membered saturated or partially unsaturated cycloalkyl ring" is to be interpreted analogously.

Unless otherwise indicated, the term "C4 to 7 saturated or partially unsaturated heterocyclic ring containing one or two heteroatoms independently selected from O, S(O)ₚ and NR¹⁷ and optionally further incorporating a carbonyl group" referred to herein denotes a 4 to 7 membered non-aromatic heterocyclic ring optionally incorporating one or more double bonds and optionally incorporating a carbonyl group. Examples include tetrahydrofuran, thiolane 1,1-dioxide, tetrahydropyran, 4-oxo-4H-pyran, pyrrolidine, pyrroline, imidazolidine, 1,3-dioxolane, piperidine, piperazine, morpholine, perhydroazepine, pyrrolidone and piperidone. The term "five- or six-membered saturated or partially unsaturated heterocyclic ring containing one heteroatom selected from O, S and NR¹³" is to be interpreted analogously.

Examples of a "5 to 7 membered azacyclic ring optionally incorporating one further heteroatom selected from O, S and NR¹⁶" include pyrrolidine, piperidine, morpholine, thiomorpholine and piperazine.

In the definition of L, "C1 to 6 alkyl; said alkyl optionally incorporating a heteroatom selected from O, S and NR¹⁶" embraces a straight or branched chain arrangement of 1 to 6 carbon atoms in which any two carbon atoms are optionally separated by O, S or NR¹⁶. The definition thus includes, for example, methylene, ethylene, propylene, hexamethylene, ethylethylene, -CH₂CH₂O-CH₂-, -CH₂CH₂O-CH₂-CH₂-, -CH₂CH₂S- and -CH₂CH₂NR¹⁶-.

Examples of bicyclic ring systems in which the two rings are either fused together, or are bonded directly together or are separated by a linker group selected from O, S(O)q or CH₂ include biphenyl, thienylphenyl, pyrazolylphenyl, phenoxyphenyl, naphthyl, indanyl, quinolyl, tetrahydroquinolyl, benzofuranyl, indolyl, isoindolyl, indolinyl, benzofuranyl, benzothienyl, indazolyl, benzimidazolyl, benzthiazolyl, purinyl, isoquinolyl, chromanyl, indenyl, quinazolyl, quinoxalyl, chromanyl, isocromanyl, 3H-indolyl, 1H-indazolyl, quinuclidyl, tetrahydronaphthyl, dihydrobenzofuranyl, morpholine-4-ylphenyl, 1,3-benzodioxolyl, 1,1-dioxido-2,3-dihydro-1-benzothienyl, 2,3-dihydro-1,4-benzodioxiny) and 3,4-dihydro-isochromenyl .

Examples of bicyclic ring systems in which the two rings are separated by a linker group S(O)q include 4-(piperazin-1-ylsulfonyl)phenyl, 4-(morpholin-4-ylsulfonyl)phenyl, 4-(piperidin-1-ylsulfonyl)phenyl, 4-(pyrrolidin-1-ylsulfonyl)phenyl, 4-(4-pyridinylsulfonyl)phenyl, 4-(phenylsulfonyl)phenyl, 4-(thiazolylsulfonyl)phenyl, 4-(pyrimidin-2-ylsulfonyl)phenyl, 4-(imidazolylsulfonyl)phenyl, 4-(triazolylsulfonyl)phenyl and 4-(oxazolylsulfonyl)phenyl.

In one embodiment, R⁵ represents H, halogen, C1 to 6 alkyl, CN, C1 to 6 alkoxy, C1 to 3 alkyl substituted by one or more F atoms or C1 to 3 alkoxy substituted by one or more F atoms. In another embodiment, R⁵ represents halogen, C1 to 6 alkyl, CN, C1 to 6 alkoxy or C1 to 3 alkyl substituted by one or more F atoms. In another embodiment, R⁵ represents halogen, CH₃, CN, OCH₃ or CF₃.

In one embodiment, n represents an integer I or 2. In another embodiment, n represents the integer 1.

In one embodiment, R⁵ represents halogen, CN or CF₃; n represents the integer 1; and G¹ represents phenyl.

In one aspect, the invention provides compounds of formula (I) wherein X represents O; Y¹ represents CR²; Y² represents CR³; R¹ represents optionally substituted C1 to 6 alkyl; G¹ represents phenyl or a five- or six-membered heteroaromatic ring containing 1 to 3 heteroatoms independently selected from O, S and N; R⁴ represents H; L represents C1 to 6 alkyl; and G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group.

In another aspect the invention provides compounds of formula (I) wherein X represents O; Y¹ represents CR²; Y² represents CR³; R¹ represents C1 to 6 alkyl; R² and R³ each represent H; G¹ represents phenyl or a five- or six-membered heteroaromatic ring containing 1 to 3 heteroatoms independently selected from O, S and N; R⁴ represents H; L represents C1 to 6 alkyl; and G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group.

In another aspect the invention provides compounds of formula (I) wherein X represents O; Y¹ represents N or NR⁶ and R represents OH or a tautomer thereof; Y² represents CR³; G¹ represents phenyl or a five- or six-membered heteroaromatic ring containing 1 to 3 heteroatoms independently selected from O, S and N; R⁴ represents H; L represents C1 to 6 alkyl; and G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C₄ to 7 saturated or partially unsaturated heterocyclic ring containing one or two heteroatoms independently selected from O, S(O)p and NR¹⁷ and optionally further incorporating a carbonyl group.

In another aspect the invention provides compounds of formula (I) wherein X represents O; Y¹ represents CR²; Y² represents CR³; R represents C1 to 6 alkyl; R² and R³ each represent H; G¹ represents phenyl or pyridyl; R⁴ represents H; L represents C1 to 6 alkyl; and G² represents optionally substituted phenyl.

In another aspect the invention provides compounds of formula (I) wherein X represents O; Y¹ represents CR²; Y² represents CR³; R¹ represents C1 to 6 alkyl; R² and R³ each represent H; G¹ represents phenyl or pyridyl; R⁴ represents H; L represents methylene; and G² represents optionally substituted phenyl.

In one embodiment, X in formula (I) represents O.

In one embodiment, the invention discloses compounds of formula (I) in which Y¹ represents CR² and Y² represents CR³. In another embodiment, the invention discloses compounds of formula (I) in which Y represents CR² and Y² represents CR³ and R² and R³ each represent H.

In another embodiment, Y¹ represents N. In another embodiment, R¹ represents OH in the tautomeric form and Y¹ represents NR⁶.

In one embodiment, R¹ represents optionally substituted C1 to 6 alkyl. In another embodiment, R¹ represents C1 to 6 alkyl, particularly methyl.

In one embodiment, G¹ represents phenyl or a five- or six-membered heteroaromatic ring containing 1 to 3 heteroatoms independently selected from O, S and N. In another embodiment, G¹ in formula (I) represents phenyl or pyridyl. In another embodiment, G¹ in formula (I) represents phenyl. In another embodiment, G¹ in formula (I) represents phenyl and (R⁵)ₙ represents a CF₃ group in the 3-position.

In one embodiment, R⁴ represents H.

In one embodiment, L represents C 1 to 6 alkyl. In another embodiment, L represents -CH₂-. In another embodiment, L represents NR²⁹ and R²⁹ represents H.

In one embodiment, G² represents an optionally substituted monocyclic ring system selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group.

In another embodiment, G² represents optionally substituted phenyl. In another embodiment, G² represents phenyl substituted by OSO₂R³⁸, S(O)ₛR²⁵, SO₂NR²⁶R²⁷, NR¹⁸R¹⁹ (wherein at least one of R¹⁸ and R¹⁹ represents S(O)ₜR³² or SO₂NR³³R³⁴) or Cl to 3 alkyl substituted by SO₂R³⁹.

In another aspect, the invention specifically provides one or more compounds as described in the Examples herein, or the non-salt form thereof or a pharmaceutically acceptable salt thereof.

Particular compounds include:
N-(4-chlorobenzyl)-1-(4-chlorophenyl)-6-methyl-2-oxo-1,2-dihydropyridine-3-carboxamide
6-methyl-N-[4-(methylsulfonyl)benzyl]-2-oxo-1-[3-(trifluoromethyl)phenyl]-1,2-dihydropyridine-3-carboxamide
6-methyl-N-(4-morpholin-4-ylbenzyl)-2-oxo-1-[3-(trifluoromethyl)phenyl]-1,2-dihydropyridine-3-carboxamide
6-methyl-N-[4-(methylsulfonyl)phenyl]-2-oxo-1-[3-(trifluoromethyl)phenyl]-1,2-dihydropyridine-3-carboxamide
N-[4-(dimethylamino)benzyl]-6-methyl-2-oxo-1-[3-(trifluoromethyl)phenyl]-1,2-dihydropyridine-3-carboxamide
N-[4-(aminosulfonyl)benzyl]-6-methyl-2-oxo-1-[3-(trifluoromethyl)phenyl]-1,2-dihydropyridine-3-carboxamide
N-(4-methoxybenzyl)-6-methyl-2-oxo-1-[3-(trifluoromethyl)phenyl]-1,2-dihydropyridine-3-carboxamide
N-benzyl-6-methyl-2-oxo-1-[3-(trifluoromethyl)phenyl]-1,2-dihydropyridine-3-carboxamide
N-(4-chlorobenzyl)-1-(2-fluoro-5-methylphenyl)-6-methyl-2-oxo-1,2-dihydropyridine-3-carboxamide
N-(3-chlorobenzyl)-1-(2-fluoro-5-methylphenyl)-6-methyl-2-oxo-1,2-dihydropyridine-3-carboxamide
1-(2-fluoro-5-methylphenyl)-N-(4-methoxybenzyl)-6-methyl-2-oxo-1,2-dihydropyridine-3-carboxamide
N-(4-methoxybenzyl)-1-(3-methoxyphenyl)-6-methyl-2-oxo-1,2-dihydropyridine-3-carboxamide
N-(3-chlorobenzyl)-1-(3-methoxyphenyl)-6-methyl-2-oxo-1,2-dihydropyridine-3-carboxamide
N-(4-chlorobenzyl)-1-(3-methoxyphenyl)-6-methyl-2-oxo-1,2-dihydropyridine-3-carboxamide
N-[4-(aminosulfonyl)benzyl]-1-(3-chlorophenyl)-6-methyl-2-oxo-1,2-dihydropyridine-3-carboxamide
N-(4-chlorobenzyl)-1-(3-chloro-4-methylphenyl)-6-methyl-2-oxo-1,2-dihydropyridine-3-carboxamide
1-(3-chloro-4-methylphenyl)-N-(4-methoxybenzyl)-6-methyl-2-oxo-1,2-dihydropyridine-3-carboxamide
N-(4-chlorobenzyl)-1-(2,3-dimethylphenyl)-6-methyl-2-oxo-1,2-dihydropyridine-3-carboxamide
N-(4-chlorobenzyl)-1-(3-chloro-4-fluorophenyl)-6-methyl-2-oxo-1,2-dihydropyridine-3-carboxamide
1-(3-chloro-4-fluorophenyl)-N-(4-methoxybenzyl)-6-methyl-2-oxo-1,2-dihydropyridine-3-carboxamide
N-(4-chlorobenzyl)-1-(3-ethylphenyl)-6-methyl-2-oxo-1,2-dihydropyridine-3-carboxamide
1-(3-bromophenyl)-N-(4-chlorobenzyl)-6-methyl-2-oxo-1,2-dihydropyridine-3-carboxamide
1-(3-bromophenyl)-N-(4-methoxybenzyl)-6-methyl-2-oxo-1,2-dihydropyridine-3-carboxamide
N-(2,3-dihydro-1-benzofuran-5-ylmethyl)-6-methyl-2-oxo-1-[3-(trifluoromethyl)phenyl]-1,2-dihydropyridine-3-carboxamide
6-methyl-2-oxo-N-[3-(2-oxopyrrolidin-1-yl)propyl]-1-[3-(trifluoromethyl)phenyl]-1,2-dihydropyridine-3-carboxamide
N-(4-bromobenzyl)-6-methyl-2-oxo-1-[3-(trifluoromethyl)phenyl]-1,2-dihydropyridine-3-carboxamide
N-(4-chlorophenyl)-6-methyl-2-oxo-1-[3-(trifluoromethyl)phenyl]-1,2-dihydropyridine-3-carboxamide
6-methyl-2-oxo-1-[3-(trifluoromethyl)phenyl]-1,2-dihydropyridine-3-carboxamide
N-(4-methoxybenzyl)-6-methyl-2-oxo-1-phenyl-1,2-dihydropyridine-3-carboxamide
N-(4-chlorobenzyl)-6-methyl-2-oxo-1-phenyl-1,2-dihydropyridine-3-carboxamide
N-(4-chlorobenzyl)-1-(3,5-dimethylphenyl)-6-methyl-2-oxo-1,2-dihydropyridine-3-carboxamide
N-[4-(aminosulfonyl)benzyl]-1-(3,5-dimethylphenyl)-6-methyl-2-oxo-1,2-dihydropyridine-3-carboxamide
1-(3,5-dimethylphenyl)-N-(4-methoxybenzyl)-6-methyl-2-oxo-1,2-dihydropyridine-3-carboxamide
N-benzyl-1-(3,5-dimethylphenyl)-6-methyl-2-oxo-1,2-dihydropyridine-3-carboxamide
N-(4-chlorobenzyl)-6-methyl-1-(3-methylphenyl)-2-oxo-1,2-dihydropyridine-3-carboxamide
N-(4-methoxybenzyl)-6-methyl-1-(3-methylphenyl)-2-oxo-1,2-dihydropyridine-3-carboxamide
N-(3-chlorobenzyl)-6-methyl-1-(3-methylphenyl)-2-oxo-1,2-dihydropyridine-3-carboxamide
N-(4-chlorobenzyl)-1-(3-chlorophenyl)-6-methyl-2-oxo-1,2-dihydropyridine-3-carboxamide
N-(3-chlorobenzyl)-1-(3-chlorophenyl)-6-methyl-2-oxo-1,2-dihydropyridine-3-carboxamide
1-(3-chlorophenyl)-N-(4-methoxybenzyl)-6-methyl-2-oxo-1,2-dihydropyridine-3-carboxamide
methyl 4-[({[1-(3-chlorophenyl)-6-methyl-2-oxo-1,2-dihydropyridin-3-yl]carbonyl}amino)methyl]benzoate
4-[({[1-(3-chlorophenyl)-6-methyl-2-oxo-1,2-dihydropyridin-3-yl]carbonyl}amino)methyl]benzoic acid
1-(3-cyanophenyl)-N-(cyclohexylmethyl)-6-methyl-2-oxo-1,2-dihydropyridine-3-carboxamide
1-(3-cyanophenyl)-N-(2-furylmethyl)-6-methyl-2-oxo-1,2-dihydropyridine-3-carboxamide
1-(3-cyanophenyl)-6-methyl-2-oxo-N-(pyridin-3-ylmethyl)-1,2-dihydropyridine-3-carboxamide
N-benzyl-1-(3-cyanophenyl)-6-methyl-2-oxo-1,2-dihydropyridine-3-carboxamide
1-(3-cyanophenyl)-N-2,3-dihydro-1H-inden-1-yl-6-methyl-2-oxo-1,2-dihydropyridine-3-carboxamide
1-(3-cyanophenyl)-N-(2-methoxybenzyl)-6-methyl-2-oxo-1,2-dihydropyridine-3-carboxamide
1-(3-cyanophenyl)-6-methyl-2-oxo-N-(3,4,5-trimethoxybenzyl)-1,2-dihydropyridine-3-carboxamide
1-(3-cyanophenyl)-N-(2,5-dimethoxybenzyl)-6-methyl-2-oxo-1,2-dihydropyridine-3-carboxamide
1-(3-cyanophenyl)-N-(3,4-dimethoxybenzyl)-6-methyl-2-oxo-1,2-dihydropyridine-3-carboxamide
1-(3-cyanophenyl)-N-[(1-ethylpyrrolidin-2-yl)methyl]-6-methyl-2-oxo-1,2-dihydropyridine-3-carboxamide
N-(4-chlorobenzyl)-1-(3-cyanophenyl)-6-methyl-2-oxo-1,2-dihydropyridine-3-carboxamide
1-(3-cyanophenyl)-N-(4-methoxybenzyl)-6-methyl-2-oxo-1,2-dihydropyridine-3-carboxamide
N-(3-chlorobenzyl)-1-(3-cyanophenyl)-6-methyl-2-oxo-1,2-dihydropyridine-3-carboxamide
1-(3-cyanophenyl)-6-methyl-2-oxo-N-(thien-2-ylmethyl)-1,2-dihydropyridine-3-carboxamide
1-(3-cyanophenyl)-N-(cyclopropylmethyl)-6-methyl-2-oxo-1,2-dihydropyridine-3-carboxamide
1-(3-cyanophenyl)-N-(3-methoxybenzyl)-6-methyl-2-oxo-1,2-dihydropyridine-3-carboxamide
1-(3-cyanophenyl)-6-methyl-2-oxo-N-(pyridin-4-ylmethyl)-1,2-dihydropyridine-3-carboxamide
1-(3-cyanophenyl)-N-[2-(3,4-dimethoxyphenyl)ethyl]-6-methyl-2-oxo-1,2-dihydropyridine-3-carboxamide
1-(3-cyanophenyl)-6-methyl-N-[2-(1-methylpyrrolidin-2-yl)ethyl]-2-oxo-1,2-dihydropyridine-3-carboxamide
N-[2-(3-chlorophenyl)ethyl]-1-(3-cyanophenyl)-6-methyl-2-oxo-1,2-dihydropyridine-3-carboxamide
1-(3-cyanophenyl)-6-methyl-2-oxo-N-(2-pyridin-2-ylethyl)-1,2-dihydropyridine-3-carboxamide
N-[2-(4-chlorophenyl)ethyl]-1-(3-cyanophenyl)-6-methyl-2-oxo-1,2-dihydropyridine-3-carboxamide
1-(3-cyanophenyl)-N-[2-(2-methoxyphenyl)ethyl]-6-methyl-2-oxo-1,2-dihydropyridine-3-carboxamide
N-[2-(2-chlorophenyl)ethyl]-1-(3-cyanophenyl)-6-methyl-2-oxo-1,2-dihydropyridine-3-carboxamide
1-(3-cyanophenyl)-N-[2-(3-methoxyphenyl)ethyl]-6-methyl-2-oxo-1,2-dihydropyridine-3-carboxamide
1-(3-cyanophenyl)-N-[2-(4-fluorophenyl)ethyl]-6-methyl-2-oxo-1,2-dihydropyridine-3-carboxamide
1-(3-cyanophenyl)-N-[2-(2,4-dichlorophenyl)ethyl]-6-methyl-2-oxo-1,2-dihydropyridine-3-carboxamide
1-(3-cyanophenyl)-N-[2-(3-fluorophenyl)ethyl]-6-methyl-2-oxo-1,2-dihydropyridine-3-carboxamide
1-(3-cyanophenyl)-N-[2-(2-fluorophenyl)ethyl]-6-methyl-2-oxo-1,2-dihydropyridine-3-carboxamide
1-(3-cyanophenyl)-N-(2-cyclohex-1-en-1-ylethyl)-6-methyl-2-oxo-1,2-dihydropyridine-3-carboxamide
N-[2-(4-bromophenyl)ethyl]-1-(3-cyanophenyl)-6-methyl-2-oxo-1,2-dihydropyridine-3-carboxamide
N-(3-bromobenzyl)-1-(3-cyanophenyl)-6-methyl-2-oxo-1,2-dihydropyridine-3-carboxamide
N-(4-bromobenzyl)-1-(3-cyanophenyl)-6-methyl-2-oxo-1,2-dihydropyridine-3-carboxamide
N-(2-bromobenzyl)-1-(3-cyanophenyl)-6-methyl-2-oxo-1,2-dihydropyridine-3-carboxamide
1-(3-cyanophenyl)-N-(3,4-dihydro-2H-pyran-2-ylmethyl)-6-methyl-2-oxo-1,2-dihydropyridine-3-carboxamide
1-(3-cyanophenyl)-6-methyl-N-(4-methylbenzyl)-2-oxo-1,2-dihydropyridine-3-carboxamide
1-(3-cyanophenyl)-6-methyl-N-(1-naphthylmethyl)-2-oxo-1,2-dihydropyridine-3-carboxamide
1-(3-cyanophenyl)-N-(2-ethoxybenzyl)-6-methyl-2-oxo-1,2-dihydropyridine-3-carboxamide
1-(3-cyanophenyl)-6-methyl-N-[4-(methylsulfonyl)benzyl]-2-oxo-1,2-dihydropyridine-3-carboxamide
1-(3-cyanophenyl)-6-methyl-N-(3-methylbenzyl)-2-oxo-1,2-dihydropyridine-3-carboxamide
1-(3-cyanophenyl)-N-(4-fluorobenzyl)-6-methyl-2-oxo-1,2-dihydropyridine-3-carboxamide
N-(1,3-benzodioxol-5-ylmethyl)-1-(3-cyanophenyl)-6-methyl-2-oxo-1,2-dihydropyridine-3-carboxamide
1-(3-cyanophenyl)-N-(2,4-dichlorobenzyl)-6-methyl-2-oxo-1,2-dihydropyridine-3-carboxamide
1-(3-cyanophenyl)-6-methyl-N-(2-methylbenzyl)-2-oxo-1,2-dihydropyridine-3-carboxamide
1-(3-cyanophenyl)-N-(3,4-difluorobenzyl)-6-methyl-2-oxo-1,2-dihydropyridine-3-carboxamide
1-(3-cyanophenyl)-N-(3,4-dichlorobenzyl)-6-methyl-2-oxo-1,2-dihydropyridine-3-carboxamide
1-(3-cyanophenyl)-6-methyl-N-[(5-methyl-2-furyl)methyl]-2-oxo-1,2-dihydropyridine-3-carboxamide
1-(3-cyanophenyl)-6-methyl-2-oxo-N-1,2,3,4-tetrahydronaphthalen-1-yl-1,2-dihydropyridine-3-carboxamide
1-(3-cyanophenyl)-N-(2,3-dimethoxybenzyl)-6-methyl-2-oxo-1,2-dihydropyridine-3-carboxamide
1-(3-cyanophenyl)-N-(3,5-dimethoxybenzyl)-6-methyl-2-oxo-1,2-dihydropyridine-3-carboxamide
1-(3-cyanophenyl)-N-[1-(4-fluorophenyl)ethyl]-6-methyl-2-oxo-1,2-dihydropyridine-3-carboxamide
N-[1-(4-chlorophenyl)ethyl]-1-(3-cyanophenyl)-6-methyl-2-oxo-1,2-dihydropyridine-3-carboxamide
1-(3-cyanophenyl)-N-(2,5-difluorobenzyl)-6-methyl-2-oxo-1,2-dihydropyridine-3-carboxamide
1-(3-cyanophenyl)-N-(2,3-dihydro-1-benzofuran-5-ylmethyl)-6-methyl-2-oxo-1,2-dihydropyridine-3-carboxamide
methyl 4-[({ [1-(3-cyanophenyl)-6-methyl-2-oxo-1,2-dihydropyridin-3-yl]carbonyl }amino)methyl]benzoate
1-(3-cyanophenyl)-6-methyl-2-oxo-N-(4-phenoxybenzyl)-1,2-dihydropyridine-3-carboxamide
1-(3-cyanophenyl)-N-[(1S)-2,3-dihydro-1H-inden-1-yl]-6-methyl-2-oxo-1,2-dihydropyridine-3-carboxamide
1-(3-cyanophenyl)-6-methyl-2-oxo-N-(thien-3-ylmethyl)-1,2-dihydropyridine-3-carboxamide
1-(3-cyanophenyl)-6-methyl-N-[(5-methylisoxazol-3-yl)methyl]-2-oxo-1,2-dihydropyridine-3-carboxamide
1-(3-cyanophenyl)-N-[(2,5-dimethyl-3-furyl)methyl]-6-methyl-2-oxo-1,2-dihydropyridine-3-carboxamide
1-(3-cyanophenyl)-N-(3-furylmethyl)-6-methyl-2-oxo-1,2-dihydropyridine-3-carboxamide
1-(3-cyanophenyl)-6-methyl-2-oxo-N-[4-(1H-pyrazol-1-yl)benzyl]-1,2-dihydropyridine-3-carboxamide
1-(3-cyanophenyl)-6-methyl-2-oxo-N-(4-thien-2-ylbenzyl)-1,2-dihydropyridine-3-carboxamide
N-[4-(anvnosulfonyl)benzyl)-1-(3-cyanophenyl)-6-methyl-2-oxo-1,2-dihyd,ropyridine-3-carboxamide
N-[2-(1,3-benzodioxol-5-yl)ethyl]-1-(3-cyanophenyl)-6-methyl-2-oxo-1,2-dihydropyridine-3-carboxamide
1-(3-cyanophenyl)-6-methyl-2-oxo-N-(2-thien-2-ylethyl)-1,2-dihydropyridine-3-carboxamide
1-(3-cyanophenyl)-N-[2-(2,4-dimethylphenyl)ethyl]-6-methyl-2-oxo-1,2-dihydropyridine-3-carboxamide
1-(3-cyanophenyl)-6-methyl-N-[2-(4-methylphenyl)ethyl]-2-oxo-1,2-dihydropyridine-3-carboxamide
N-{2-[4-(aminosulfonyl)phenyl]ethyl}-1-(3-cyanophenyl)-6-methyl-2-oxo-1,2-dihydropyridine-3-carboxamide
1-(3-cyanophenyl)-6-methyl-2-oxo-N-[(1S)-1-phenylethyl]-1,2-dihydropyridine-3-carboxamide
N-(cyclohexylmethyl)-6-methyl-2-oxo-1-[3-(trifluoromethyl)phenyl]-1,2-dihydropyridine-3-carboxamide
N-(2-furylmethyl)-6-methyl-2-oxo-1-[3-(trifluoromethyl)phenyl]-1,2-dihydropyridine-3-carboxamide
6-methyl-2-oxo-N-(pyridin-3-ylmethyl)-1-[3-(trifluoromethyl)phenyl]-1,2-dihydropyridine-3-carboxamide
N-2,3-dihydro-1H-inden-1-yl-6-methyl-2-oxo-1-[3-(trifluoromethyl)phenyl]-1,2-dihydropyridine-3-carboxamide
N-(2-methoxybenzyl)-6-methyl-2-oxo-1-[3-(trifluoromethyl)phenyl]-1,2-dihydropyridine-3-carboxamide
6-methyl-2-oxo-N-(tetrahydrofuran-2-ylmethyl)-1-[3-(trifluoromethyl)phenyl]-1,2-dihydropyridine-3-carboxamide
6-methyl-2-oxo-1-[3-(trifluoromethyl)phenyl]-N-(3,4,5-trimethoxybenzyl)-1,2-dihydropyridine-3-carboxamide
N-(3-fluorobenzyl)-6-methyl-2-oxo-1-[3-(trifluoromethyl)phenyl]-1,2-dihydropyridine-3-carboxamide
N-(2,5-dimethoxybenzyl)-6-methyl-2-oxo-1-[3-(trifluoromethyl)phenyl]-1,2-dihydropyridine-3-carboxamide
N-[(1-ethylpyrrolidin-2-yl)methyl]-6-methyl-2-oxo-1-[3-(trifluoromethyl)phenyl]-1,2-dihydropyridine-3-carboxamide
N-(2-chlorobenzyl)-6-methyl-2-oxo-1-[3-(trifluoromethyl)phenyl]-1,2-dihydmpyridine-3-carboxamide
N-(4-chlorobenzyl)-6-methyl-2-oxo-1-[3-(trifluoromethyl)phenyl]-1,2-dihydropyridine-3-carboxamide
N-(3-chlorobenzyl)-6-methyl-2-oxo-1-[3-(trifluoromethyl)phenyl]-1,2-dihydropyridine-3-carboxamide
6-methyl-2-oxo-N-(thien-2-ylmethyl)-1-[3-(trifluoromethyl)phenyl]-1,2-dihydropyridine-3-carboxamide
N-(cyclopropylmethyl)-6-methyl-2-oxo-1-[3-(trifluoromethyl)phenyl]-1,2-dihydropyridine-3-carboxamide
N-(3-methoxybenzyl)-6-methyl-2-oxo-1-[3-(trifluoromethyl)phenyl]-1,2-dihydropyridine-3-carboxamide
6-methyl-2-oxo-N-(pyridin-4-ylmethyl)-1-[3-(trifluoromethyl)phenyl]-1,2-dihydropyridine-3-carboxamide
N-[2-(3,4-dimethoxyphenyl)ethyl]-6-methyl-2-oxo-1-[3-(trifluoromethyl)phenyl]-1,2-dihydropyridine-3-carboxamide
N-[2-(4-methoxyphenyl)ethyl]-6-methyl-2-oxo-1-[3-(trifluoromethyl)phenyl]-1,2-dihydropyridine-3-carboxamide
6-methyl-2-oxo-N-(2-phenylethyl)-1-[3-(trifluoromethyl)phenyl]-1,2-dihydropyridine-3-carboxamide
6-methyl-N-[2-(1-methylpyrrolidin-2-yl)ethyl]-2-oxo-1-[3-(trifluoromethyl)phenyl]-1,2-dihydropyridine-3-carboxamide
N-[2-(3-chlorophenyl)ethyl]-6-methyl-2-oxo-1-[3-(trifluoromethyl)phenyl]-1,2-dihydropyridine-3-carboxamide
6-methyl-2-oxo-N-(2-pyridin-2-ylethyl)-1-[3-(trifluoromethyl)phenyl]-1,2-dihydropyridine-3-carboxamide
N-[2-(2-methoxyphenyl)ethyl]-6-methyl-2-oxo-1-[3-(trifluoromethyl)phenyl]-1,2-dihydropyridine-3-carboxamide
N-[2-(2-chlorophenyl)ethyl]-6-methyl-2-oxo-1-[3-(trifluoromethyl)phenyl]-1,2-dihydropyridine-3-carboxamide
N-[2-(3-methoxyphenyl)ethyl]-6-methyl-2-oxo-1-[3-(trifluoromethyl)phenyl]-1,2-dihydropyridine-3-carboxamide
N-[2-(4-fluorophenyl)ethyl]-6-methyl-2-oxo-1-[3-(trifluoromethyl)phenyl]-1,2-dihydropyridine-3-carboxamide
N-[2-(2,4-dichlorophenyl)ethyl]-6-methyl-2-oxo-1-[3-(trifluoromethyl)phenyl]-1,2-dihydropyridine-3-carboxamide
N-[2-(3-fluorophenyl)ethyl]-6-methyl-2-oxo-1-[3-(trifluoromethyl)phenyl]-1,2-dihydropyridine-3-carboxamide
N-[2-(2-fluorophenyl)ethyl]-6-methyl-2-oxo-1-[3-(trifluoromethyl)phenyl]-1,2-dihydmpyridine-3-carboxamide
N-(2-cyclohex-1-en-1-ylethyl)-6-methyl-2-oxo-1-[3-(trifluoromethyl)phenyl]-1,2-dihydropyridine-3-carboxamide
N-[2-(4-bromophenyl)ethyl]-6-methyl-2-oxo-1-[3-(trifluoromethyl)phenyl]-1,2-dihydropyridine-3-carboxamide
6-methyl-2-oxo-N-[(1S)-1-phenylethyl]-1-[3-(trifluoromethyl)phenyl]-1,2-dihydropyridine-3-carboxamide
N-(3-bromobenzyl)-6-methyl-2-oxo-1-[3-(trifluoromethyl)phenyl]-1,2-dihydropyridine-3-carboxamide
N-(4-bromobenzyl)-6-methyl-2-oxo-1-[3-(trifluoromethyl)phenyl]-1,2-dihydropyridine-3-carboxamide
N-(2-bromobenzyl)-6-methyl-2-oxo-1-[3-(trifluoromethyl)phenyl]-1,2-dihydropyridine-3-carboxamide
N-(3,4-dihydro-2H-pyran-2-ylmethyl)-6-methyl-2-oxo-l-[3-(trifluoromethyl)phenyl]-1,2-dihydropyridine-3-carboxamide
6-methyl-N-(4-methylbenzyl)-2-oxo-1-[3-(trifluoromethyl)phenyl]-1,2-dihydropyridine-3-carboxamide
6-methyl-N-(1-naphthylmethyl)-2-oxo-1-[3-(trifluoromethyl)phenyl]-1,2-dihydropyridine-3-carboxamide
N-(2-ethoxybenzyl)-6-methyl-2-oxo-1-[3-(trifluoromethyl)phenyl]-1,2-dihydropyridine-3-carboxamide
6-methyl-N-(3-methylbenzyl)-2-oxo-1-[3-(trifluoromethyl)phenyl)-1,2-dihydropyridine-3-carboxamide
N-(4-fluorobenzyl)-6-methyl-2-oxo-1-[3-(trifluoromethyl)phenyl]-1,2-dihydropyridine-3-carboxamide
N-(1,3-benzodioxol-5-ylmethyl)-6-methyl-2-oxo-1-[3-(trifluoromethyl)phenyl]-1,2-dihydropyridine-3-carboxamide
N-(2,4-dichlorobenzyl)-6-methyl-2-oxo-1-[3-(trifluoromethyl)phenyl]-1,2-dihydropyridine-3-carboxamide
6-methyl-N-(2-methylbenzyl)-2-oxo-1-[3-(trifluoromethyl)phenyl]-1,2-dihydropyridine-3-carboxamide
N-(3,4-difluorobenzyl)-6-methyl-2-oxo-1-[3-(trifluoromethyl)phenyl]-1,2-dihydropyridine-3-carboxamide
N-(2-fluorobenzyl)-6-methyl-2-oxo-1-[3-(trifluoromethyl)phenyl]-1,2-dihydropyridine-3-carboxamide
N-(2-chloro-4-fluorobenzyl)-6-methyl-2-oxo-1-[3-(trifluoromethyl)phenyl]-1,2-dihydropyridine-3-carboxamide
N-(3,4-dichlorobenzyl)-6-methyl-2-oxo-1-[3-(trifluoromethyl)phenyl]-1,2-dihydropyridine-3-carboxamide
6-methyl-N-[(5-methyl-2-furyl)methyl]-2-oxo-1-[3-(trifluoromethyl)phenyl]-1,2-dihydropyridine-3-carboxamide
6-methyl-2-oxo-N-1,2,3,4-tetrahydronaphthalen-1-yl-1-[3-(trifluoromethyl)phenyl]-1,2-dihydropyridine-3-carboxamide
N-(2,3-dimethoxybenzyl)-6-methyl-2-oxo-1-[3-(trifluoromethyl)phenyl]-1,2-dihydropyridine-3-carboxamide
N-[1-(4-chlorophenyl)ethyl]-6-methyl-2-oxo-1-[3-(trifluoromethyl)phenyl]-1,2-dihydropyridine-3-carboxamide
N-(2,5-difluorobenzyl)-6-methyl-2-oxo-1-[3-(trifluoromethyl)phenyl]-1,2-dihydropyridine-3-carboxamide
methyl 4-{[({6-methyl-2-oxo-1-[3-(trifluoromethyl)phenyl]-1,2-dihydropyridin-3-yl }carbonyl)amino]methyl}benzoate
6-methyl-2-oxo-N-(4-phenoxybenzyl)-1-[3-(trifluoromethyl)phenyl]-1,2-dihydropyridine-3-carboxamide
N-[(2,2-dimethyl-1,3-dioxolan-4-yl)methyl]-6-methyl-2-oxo-1-[3-(trifluoromethyl)phenyl]-1,2-dihydropyridine-3-carboxamide
6-methyl-N-[(5-methylisoxazol-3-yl)methyl]-2-oxo-1-[3-(trifluoromethyl)phenyl]-1,2-dihydropyridine-3-carboxamide
N-[(2,5-dimethyl-3-furyl)methyl]-6-methyl-2-oxo-1-[3-(trifluoromethyl)phenyl]-1,2-dihydropyridine-3-carboxamide
N-(3-furylmethyl)-6-methyl-2-oxo-1-[3-(trifluoromethyl)phenyl]-1,2-dihydropylidine-3-carboxamide
6-methyl-2-oxo-N-[4-(1H-pyrazol-1-yl)benzyl]-1-[3-(trifluoromethyl)phenyl]-1,2-dihydropyridine-3-carboxamide
6-methyl-2-oxo-N-(4-thien-2-ylbenzyl)-1-[3-(trifluoromethyl)phenyl]-1,2-dihydropyridine-3-carboxamide
N-[2-(1,3-benzodioxol-5-yl)ethyl]-6-methyl-2-oxo-1-[3-(trifluoromethyl)phenyl]-1,2-dihydropyridine-3-carboxamide
6-methyl-2-oxo-N-(2-thien-2-ylethyl)-1-[3-(trifluoromethyl)phenyl]-1,2-dihydropyridine-3-carboxamide
N-[2-(4-tert-butylphenyl)ethyl]-6-methyl-2-oxo-1-[3-(trifluoromethyl)phenyl]-1,2-dihydropyridine-3-carboxamide
6-methyl-N-[2-(4-methylphenyl)ethyl]-2-oxo-1-[3-(trifluoromethyl)phenyl]-1,2-dihydropyridine-3-carboxamide
N-{2-[4-(aminosulfonyl)phenyl]ethyl}-6-methyl-2-oxo-1-[3-(trifluoromethyl)phenyl]-1,2-dihydropyridine-3-carboxamide
6-methyl-2-oxo-N-[(1R)-1-phenylethyl]-1-[3-(trifluoromethyl)phenyl]-1,2-dihydropyridine-3-carboxamide
3-{[4-(2-methoxyphenyl)piperazin-1-yl]carbonyl}-6-methyl-1-[3-(trifluoromethyl)phenyl]pyridin-2(1H)-one
N-[(4-cyanocyclohexyl)methyl]-6-methyl-2-oxo-1-[3-(trifluoromethyl)phenyl]-1,2-dihydropyridine-3-carboxamide
3-{[4-(4-fluorophenyl)piperazin-1-yl]carbonyl}-6-methyl-1-[3-(trifluoromethyl)phenyl]pyridin-2(1H)-one
N-[2-(4'-fluoro-1,1'-biphenyl-4-yl)ethyl]-6-methyl-2-oxo-1-[3-(trifluoromethyl)phenyl]-1,2-dihydropyridine-3-carboxamide
N-(2-hydroxy-1-phenylethyl)-6-methyl-2-oxo-1-[3-(trifluoromethyl)phenyl]-1,2-dihydropyridine-3-carboxamide
6-methyl-2-oxo-N-[(2R)-2-phenylcyclopropyl]-1-[3-(trifluoromethyl)phenyl]-1,2-dihydropyridine-3-carboxamide
N-[1-(4-chlorobenzyl)piperidin-4-yl]-6-methyl-2-oxo-1-[3-(trifluoromethyl)phenyl]-1,2-dihydropyridine-3-carboxamide
6-methyl-N-(2-morpholin-4-ylethyl)-2-oxo-1-[3-(trifluoromethyl)phenyl]-1,2-dihydropyridine-3-carboxamide
N-[2-(4-chlorophenyl)ethyl]-6-methyl-2-oxo-l-[3-(trifluoromethyl)phenyl]-1,2-dihydropyridine-3-carboxamide
N-(2-hydroxy-2-phenylethyl)-6-methyl-2-oxo-1-[3-(trifluoromethyl)phenyl]-1,2-dihydropyridine-3-carboxamide
N-cyclopentyl-6-methyl-2-oxo-1-[3-(trifluoromethyl)phenyl]-1,2-dihydropyridine-3-carboxamide
N-[2-(1H-imidazol-4-yl)ethyl]-6-methyl-2-oxo-1-[3-(trifluoromethyl)phenyl]-1,2-dihydropyridine-3-carboxamide
N-(3,5-dimethoxybenzyl)-6-methyl-2-oxo-1-[3-(trifluoromethyl)phenyl]-1,2-dihydropyridine-3-carboxamide
N-(4-hydroxycyclohexyl)-6-methyl-2-oxo-1-[3-(trifluoromethyl)phenyl]-1,2-dihydropyridine-3-carboxamide
6-methyl-2-oxo-N-(2-pyridin-2-ylethyl)-1-[3-(trifluoromethyl)phenyl]-1,2-dihydropyridine-3-carboxamide
6-methyl-2-oxo-N-1H-1,2,4-triazol-3-yl-1-[3-(trifluoromethyl)phenyl]-1,2-dihydropyridine-3-carboxamide
N-[1-(hydroxymethyl)-2-methylpropyl]-6-methyl-2-oxo-1-[3-(trifluoromethyl)phenyl]-1,2-dihydropyridine-3-carboxamide
3-{[3-(3,4-dichlorophenoxy)pyrrolidin-1-yl]carbonyl}-6-methyl-1-[3-(trifluoromethyl)phenyl]pyridin-2(1H)-one
6-methyl-2-oxo-N-(pyridin-3-ylmethyl)-1-[3-(trifluoromethyl)phenyl]-1,2-dihydropyridine-3-carboxamide
N-(2-methoxyethyl)-6-methyl-2-oxo-1-[3-(trifluoromethyl)phenyl]-1,2-dihydropyridine-3-carboxamide
N-(2-hydroxypropyl)-6-methyl-2-oxo-1-[3-(trifluoromethyl)phenyl]-1,2-dihydropyridine-3-carboxamide
ethyl 4-[({6-methyl-2-oxo-1-[3-(trifluoromethyl)phenyl]-1,2-dihydropyridin-3-yl}carbonyl)amino]piperidine-1-carboxylate
N-[3-(1H-inudazol-1-yl)propyl]-6-methyl-2-oxo-1-[3-(trifluoromethyl)phenyl]-1,2-dihydropyridine-3-carboxamide
N-(4-chlorobenzyl)-1-(3-methylphenyl)-2-oxo-1,2-dihydropyridine-3-carboxamide
N-(4-chlorobenzyl)-6'-methyl-2-oxo-2H-1,2'-bipyridine-3-carboxamide
N-(4-methoxybenzyl)-1-(3-methylphenyl)-2-oxo-1,2-dihydropyridine-3-carboxamide
methyl 4-[({[1-(3-methylphenyl)-2-oxo-1,2-dihydropyridin-3-yl]carbonyl}amino)methyl]benzoate
4-[({[1-(3-methylphenyl)-2-oxo-1,2-dihydropyridin-3-yl]carbonyl}amino)methyl] benzoic acid
N-(4-chlorobenzyl)-1-(2-fluoro-5-methylphenyl)-2-oxo-1,2-dihydropyridine-3-carboxamide
1-(2-fluoro-5-methylphenyl)-N-(4-methoxybenzyl)-2-oxo-1,2-dihydropyridine-3-carboxamide
N-[4-(dimethylamino)benzyl]-1-(2-fluoro-5-methylphenyl)-2-oxo-1,2-dihydropyridine-3-carboxamide
N-[4-(aminosulfonyl)benzyl]-1-(2-fluoro-5-methylphenyl)-2-oxo-1,2-dihydropyridine-3-carboxamide
N-(4-chlorobenzyl)-4'-methyl-2-oxo-2H-1,2'-bipyridine-3-carboxamide
N-(4-chlorobenzyl)-1-(2,5-dimethylphenyl)-2-oxo-1,2-dihydropyridine-3-carboxamide
1-(2,5-dimethylphenyl)-N-(4-methoxybenzyl)-2-oxo-1,2-dihydropyridine-3-carboxamide
N-[4-(dimethylamino)benzyl]-1-(2,5-dimethylphenyl)-2-oxo-1,2-dihydropyridine-3-carboxamide
N-(4-chlorobenzyl)-1-[2-methyl-5-(trifluoromethyl)phenyl]-2-oxo-1,2-dihydropyridine-3-carboxamide
N-(4-methoxybenzyl)-1-[2-methyl-5-(trifluoromethyl)phenyl]-2-oxo-1,2-dihydropyridine-3-carboxamide
N-[4-(dimethylaniino)benzyl]-1-[2-methyl-5-(trifluoromethyl)phenyl]-2-oxo-1,2-dihydropyridine-3-carboxamide
N-benzyl-5-methyl-2-oxo-1-[3-(trifluoromethyl)phenyl]-1,2-dihydropyridine-3-carboxamide
N-(2-chlorobenzyl)-5-methyl-2-oxo-1-[3-(trifluoromethyl)phenyl]-1,2-dihydropyridine-3-carboxamide
5-methyl-2-oxo-N-(2-phenylethyl)-1-[3-(trifluoromethyl)phenyl]-1,2-dihydropyridine-3-carboxamide
N-(4-chlorophenyl)-5-methyl-2-oxo-1-[3-(trifluoromethyl)phenyl]-1,2-dihydropyridine-3-carboxamide
6-ethyl-N-[4-(methylsulfonyl)benzyl]-2-oxo-1-[3-(trifluoromethyl)phenyl]-1,2-dihydropyridine-3-carboxamide
N-[4-(methylsulfonyl)benzyl]-2-oxo-6-propyl-1-[3-(trifluoromethyl)phenyl]-1,2-dihydropyridine-3-carboxamide
6-butyl-N-[4-(methylsulfonyl)benzyl]-2-oxo-1-[3-(trifluoromethyl)phenyl]-1,2-dihydropyridine-3-carboxamide
6-(methoxymethyl)-N-[4-(methylsulfonyl)benzyl]-2-oxo-1-[3-(trifluoromethyl)phenyl]-1,2-dihydropyridine-3-carboxamide
6-(hydroxymethyl)-N-[4-(methylsulfonyl)benzyl]-2-oxo-1-[3-(trifluoromethyl)phenyl]-1,2-dihydropyridine-3-carboxamide
N-[4-(aminosulfonyl)benzyl]-2,4-dioxo-3-[3-(trifluoromethyl)phenyl]-1,2,3,4-tetrahydropyrimidine-5-carboxamide
N-[4-(dimethylamino)benzyl]-2,4-dioxo-3-[3-(trifluoromethyl)phenyl]-1,2,3,4-tetrahydropyrimidine-5-carboxamide
N-(4-chlorobenzyl)-2,4-dioxo-3-[3-(trifluoromethyl)phenyl]-1,2,3,4-tetrahydropyrimidine-5-carboxamide
N-(2,3-dihydro-1-benzofuran-5-ylmethyl)-2,4-dioxo-3-[3-(trifluoromethyl)phenyl]-1,2,3,4-tetrahydropyrimidine-5-carboxamide
N-[4-(methylsulfonyl)benzyl]-2,4-dioxo-3-[3-(trifluoromethyl)phenyl]-1,2,3,4-tetrahydropyrimidine-5-carboxamide
N-(4-bromobenzyl)-2,4-dioxo-3-[3-(trifluoromethyl)phenyl]-1,2,3,4-tetrahydropyrimidine-5-carboxamide
N-(4-methoxybenzyl)-2,4-dioxo-3-[3-(trifluoromethyl)phenyl]-1,2,3,4-tetrahydropyrimidine-5-carboxamide
N-(1,3-benzodioxol-5-ylmethyl)-2,4-dioxo-3-[3-(trifluoromethyl)phenyl]-1,2,3,4-tetrahydropyrimidine-5-carboxamide
N-(3-chlorobenzyl)-2,4-dioxo-3-[3-(trifluoromethyl)phenyl]-1,2,3,4-tetrahydropyrimidine-5-carboxamide
1-butyl-N-[4-(methylsulfonyl)benzyl]-2,4-dioxo-3-[3-(trifluoromethyl)phenyl]-1,2,3,4-tetrahydropyrimidine-5-carboxamide
1-(2-methoxyethyl)-N-[4-(methylsulfonyl)benzyl]-2,4-dioxo-3-[3-(trifluoromethyl)phenyl]-1,2,3,4-tetrahydropyrimidine-5-carboxamide
1-methyl-N-[4-(methylsulfonyl)benzyl]-2,4-dioxo-3-[3-(trifluoromethyl)phenyl]-1,2,3,4-tetrahydropyrimidine-5-carboxamide
1-ethyl-N-[4-(methylsulfonyl)benzyl]-2,4-dioxo-3-[3-(trifluoromethyl)phenyl]-1,2,3,4-tetrahydropyrimidine-5-carboxamide
N-(4-chlorobenzyl)-1-(2-methoxyethyl)-2,4-dioxo-3-[3-(trifluoromethyl)phenyl]-1,2,3,4-tetrahydropyrimidine-5-carboxamide
5-iodo-6-methyl-N-[4-(methylsulfonyl)benzyl]-2-oxo-1-[3-(trifluoromethyl)phenyl]-1,2-dihydropyridine-3-carboxamide
N-(4-chlorobenzyl)-1-(2-methoxyethyl)-2,4-dioxo-3-[3-(trifluoromethyl)phenyl]-1,2,3,4-tetrahydropyrimidine-5-carboxamide
N-(4-methoxybenzyl)-1-(2-methoxyethyl)-2,4-dioxo-3-[3-(trifluoromethyl)phenyl]-1,2,3,4-tetrahydropyrimidine-5-carboxamide
1-(2-methoxyethyl)-2,4-dioxo-N-(pyridin-4-ylmethyl)-3-[3-(trifluoromethyl)phenyl]-1,2,3,4-tetrahydropyrimidine-5-carboxamide
N-[2-(3,4-dimethoxyphenyl)ethyl]-1-(2-methoxyethyl)-2,4-dioxo-3-[3-(trifluoromethyl)phenyl]-1,2,3,4-tetrahydropyrimidine-5-carboxamide
1-(2-methoxyethyl)-N-[2-(3-methoxyphenyl)ethyl]-2,4-dioxo-3-[3-(trifluoromethyl)phenyl]-1,2,3,4-tetrahydropyrimidine-5-carboxamide
1-(2-methoxyethyl)-N-(4-methylbenzyl)-2,4-dioxo-3-[3-(trifluoromethyl)phenyl]-1,2,3,4-tetrahydropyrimidine-5-carboxamide
1-(2-methoxyethyl)-N-[4-(methylsulfonyl)benzyl]-2,4-dioxo-3-[3-(trifluoromethyl)phenyl]-1,2,3,4-tetrahydropyrimidine-5-carboxamide
N-(4-fluorobenzyl)-1-(2-methoxyethyl)-2,4-dioxo-3-[3-(trifluoromethyl)phenyl]-1,2,3,4-tetrahydropyrimidine-5-carboxamide
N-(1,3-benzodioxol-5-ylmethyl)-1-(2-methoxyethyl)-2,4-dioxo-3-[3-(trifluoromethyl)phenyl]-1,2,3,4-tetrahydropyrimidine-5-carboxamide
N-(2-chloro-4-fluorobenzyl)-1-(2-methoxyethyl)-2,4-dioxo-3-[3-(trifluoromethyl)phenyl]-1,2,3,4-tetrahydropyrimidine-5-carboxamide
N-(3,4-dichlorobenzyl)-1-(2-methoxyethyl)-2,4-dioxo-3-[3-(trifluoromethyl)phenyl]-1,2,3,4-tetrahydropyrimidine-5-carboxamide
methyl 4-{[({1-(2-methoxyethyl)-2,4-dioxo-3-[3-(trifluoromethyl)phenyl]-1,2,3,4-tetrahydropyrimidin-5-yl}carbonyl)amino]methyl}benzoate
1-(2-methoxyethyl)-N-[(5-methylisoxazol-3-yl)methyl]-2,4-dioxo-3-[3-(trifluoromethyl)phenyl]-1,2,3,4-tetrahydropyrimidine-5-carboxamide
1-(2-methoxyethyl)-2,4-dioxo-N-[4-(1H-pyrazol-1-yl)benzyl]-3-[3-(trifluoromethyl)phenyl]-1,2,3,4-tetrahydropyrimidine-5-carboxamide
N-(4-chlorobenzyl)-3-(3-chlorophenyl)-1-(2-methoxyethyl)-2,4-dioxo-1,2,3,4-tetrahydropyrimidine-5-carboxamide
3-(3-chlorophenyl)-N-(4-methoxybenzyl)-1-(2-methoxyethyl)-2,4-dioxo-1,2,3,4-tetrahydropyrimidine-5-carboxamide
3-(3-chlorophenyl)-1-(2-methoxyethyl)-2,4-dioxo-N-(pyridin-4-ylmethyl)-1,2,3,4-tetrahydropyrimidine-5-carboxamide
3-(3-chlorophenyl)-N-[2-(3,4-dimethoxyphenyl)ethyl]-1-(2-methoxyethyl)-2,4-dioxo-1,2,3,4-tetrahydropyrimidine-5-carboxamide
3-(3-chlorophenyl)-1-(2-methoxyethyl)-N-[2-(3-methoxyphenyl)ethyl]-2,4-dioxo-1,2,3,4-tetrahydropyrimidine-5-carboxamide
N-(3-bromobenzyl)-3-(3-chlorophenyl)-1-(2-methoxyethyl)-2,4-dioxo-1,2,3,4-tetrahydropyrimidine-5-carboxamide
3-(3-chlorophenyl)-1-(2-methoxyethyl)-N-(4-methylbenzyl)-2,4-dioxo-1,2,3,4-tetrahydropyrimidine-5-carboxamide
3-(3-chlorophenyl)-1-(2-methoxyethyl)-N-[4-(methylsulfonyl)benzyl]-2,4-dioxo-1,2,3,4-tetrahydropyrimidine-5-carboxamide
3-(3-chlorophenyl)-N-(4-fluorobenzyl)-1-(2-methoxyethyl)-2,4-dioxo-1,2,3,4-tetrahydropyrimidine-5-carboxamide
N-(1,3-benzodioxol-5-ylmethyl)-3-(3-chlorophenyl)-1-(2-methoxyethyl)-2,4-dioxo-1,2,3,4-tetrahydropyrimidine-5-carboxamide
3-(3-chlorophenyl)-N-(3,4-difluorobenzyl)-1-(2-methoxyethyl)-2,4-dioxo-1,2,3,4-tetrahydropyrimidine-5-carboxamide
N-(2-chloro-4-fluorobenzyl)-3-(3-chlorophenyl)-1-(2-methoxyethyl)-2,4-dioxo-1,2,3,4-tetrahydropyrimidine-5-carboxamide
3-(3-chlorophenyl)-N-(3,4-dichlorobenzyl)-1-(2-methoxyethyl)-2,4-dioxo-1,2,3,4-tetrahydropyrimidine-5-carboxamide
methyl 4-[({[3-(3-chlorophenyl)-1-(2-methoxyethyl)-2,4-dioxo-1,2,3,4-tetrahydropyrimidin-5-yl]carbonyl}amino)methyl]benzoate
3-(3-chlorophenyl)-1-(2-methoxyethyl)-N-[(5-methylisoxazol-3-yl)methyl]-2,4-dioxo-1,2,3,4-tetrahydropyrimidine-5-carboxamide
3-(3-chlorophenyl)-1-(2-methoxyethyl)-2,4-dioxo-N-[4-(1H-pyrazol-1-yl)benzyl]-1,2,3,4-tetrahydropyrimidine-5-carboxamide
1-butyl-N-(4-chlorobenzyl)-3-(3-methoxyphenyl)-2,4-dioxo-1,2,3,4-tetrahydropyrimidine-5-carboxamide
1-butyl-3-(3-methoxyphenyl)-N-[2-(3-methoxyphenyl)ethyl]-2,4-dioxo-1,2,3,4-tetrahydropyrimidine-5-carboxamide
N-(3-bromobenzyl)-1-butyl-3-(3-methoxyphenyl)-2,4-dioxo-1,2,3,4-tetrahydropyrimidine-5-carboxamide
1-butyl-N-(4-fluorobenzyl)-3-(3-methoxyphenyl)-2,4-dioxo-1,2,3,4-tetrahydropyrimidine-5-carboxamide
N-(1,3-benzodioxol-5-ylmethyl)-1-butyl-3-(3-methoxyphenyl)-2,4-dioxo-1,2,3,4-tetrahydropyrimidine-5-carboxamide
1-butyl-N-(2,4-dichlorobenzyl)-3-(3-methoxyphenyl)-2,4-dioxo-1,2,3,4-tetrahydropyrimidine-5-carboxamide
1-butyl-N-(3,4-difluorobenzyl)-3-(3-methoxyphenyl)-2,4-dioxo-1,2,3,4-tetrahydropyrimidine-5-carboxamide
1-butyl-N-(2-chloro-4-fluorobenzyl)-3-(3-methoxyphenyl)-2,4-dioxo-1,2,3,4-tetrahydropyrimidine-5-carboxamide
1-butyl-N-(2,3-dihydro-1-benzofuran-5-ylmethyl)-3-(3-methoxyphenyl)-2,4-dioxo-1,2,3,4-tetrahydropyrimidine-5-carboxamide
1-butyl-N-(4-chlorobenzyl)-3-(3-chlorophenyl)-2,4-dioxo-1,2,3,4-tetrahydropyrimidine-5-carboxamide
1-butyl-3-(3-chlorophenyl)-N-(4-methoxybenzyl)-2,4-dioxo-1,2,3,4-tetrahydropyrimidine-5-carboxamide
1-butyl-3-(3-chlorophenyl)-2,4-dioxo-N-(pyridin-4-ylmethyl)-1,2,3,4-tetrahydropyrimidine-5-carboxamide
1-butyl-3-(3-chlorophenyl)-N-[2-(3,4-dimethoxyphenyl)ethyl]-2,4-dioxo-1,2,3,4-tetrahydropyrimidine-5-carboxamide
1-butyl-3-(3-chlorophenyl)-N-[2-(3-methoxyphenyl)ethyl]-2,4-dioxo-1,2,3,4-tetrahydropyrimidine-5-carboxamide
N-(3-bromobenzyl)-1-butyl-3-(3-chlorophenyl)-2,4-dioxo-1,2,3,4-tetrahydropyrimidine-5-carboxamide
N-(4-bromobenzyl)-1-butyl-3-(3-chlorophenyl)-2,4-dioxo-1,2,3,4-tetrahydropynmidine-5-carboxamide
1-butyl-3-(3-chlorophenyl)-N-(4-methylbenzyl)-2,4-dioxo-1,2,3,4-tetrahydropyfimidine-5-carboxamide
1-butyl-3-(3-chlorophenyl)-N-[4-(methylsulfonyl)benzyl]-2,4-dioxo-1,2,3,4-tetrahydropyrimidine-5-carboxamide
1-butyl-3-(3-chlorophenyl)-N-(4-fluorobenzyl)-2,4-dioxo-1,2,3,4-tetrahydropyrimidine-5-carboxamide
N-(1,3-benzodioxol-5-ylmethyl)-1-butyl-3-(3-chlorophenyl)-2,4-dioxo-1,2,3,4-tetrahydropyrimidine-5-carboxamide
1-butyl-3-(3-chlorophenyl)-N-(2,4-dichlorobenzyl)-2,4-dioxo-1,2,3,4-tetrahydropyrimidine-5-carboxamide
1-butyl-3-(3-chlorophenyl)-N-(3,4-difluorobenzyl)-2,4-dioxo-1,2,3,4-tetrahydropyrimidine-5-carboxamide
1-butyl-N-(2-chloro-4-fluorobenzyl)-3-(3-chlorophenyl)-2,4-dioxo-1,2,3,4-tetrahydropyrimidine-5-carboxamide
1-butyl-3-(3-chlorophenyl)-N-(3,4-dichlorobenzyl)-2,4-dioxo-1,2,3,4-tetrahydropyrimidine-5-carboxamide
1-butyl-3-(3-chlorophenyl)-N-(2,3-dihydro-1-benzofuran-5-ylmethyl)-2,4-dioxo-1,2,3,4-tetrahydropyrimidine-5-carboxamide
1-butyl-3-(3-chlorophenyl)-N-[(4-cyanocyclohexyl)methyl]-2,4-dioxo-1,2,3,4-tetrahydropyrimidine-5-carboxamide
1-butyl-3-(3-chlorophenyl)-N-[(5-methylisoxazol-3-yl)methyl]-2,4-dioxo-1,2,3,4-tetrahydropyrimidine-5-carboxamide
1-butyl-3-(3-chlorophenyl)-2,4-dioxo-N-[4-(1H-pyrazol-1-yl)benzyl]-1,2,3,4-tetrahydropyrimidine-5-carboxamide
1-butyl-3-(3-chlorophenyl)-2,4-dioxo-N-[3-(2-oxopyrrolidin-1-yl)propyl]-1,2,3,4-tetrahydropyrimidine-5-carboxamide
1-butyl-N-(4-chlorobenzyl)-3-(3-cyanophenyl)-2,4-dioxo-1,2,3,4-tetrahydropyrimidine-5-carboxamide
1-butyl-3-(3-cyanophenyl)-N-(4-methoxybenzyl)-2,4-dioxo-1,2,3,4-tetrahydropyrimidine-5-carboxamide
1-butyl-3-(3-cyanophenyl)-2,4-dioxo-N-(pyridin-4-ylmethyl)-1,2,3,4-tetrahydropyrimidine-5-carboxamide
1-butyl-3-(3-cyanophenyl)-N-[2-(3,4-dimethoxyphenyl)ethyl]-2,4-dioxo-1,2,3,4-tetrahydropyrimidine-5-carboxamide
1-butyl-3-(3-cyanophenyl)-N-[2-(3-methoxyphenyl)ethyl]-2,4-dioxo-1,2,3,4-tetrahydropyrimidine-5-carboxamide
N-(3-bromobenzyl)-1-butyl-3-(3-cyanophenyl)-2,4-dioxo-1,2,3,4-tetrahydropyrimidine-5-carboxamide
N-(4-bromobenzyl)-1-butyl-3-(3-cyanophenyl)-2,4-dioxo-1,2,3,4-tetrahydropyrimidine-5-carboxamide
1-butyl-3-(3-cyanophenyl)-N-(4-methylbenzyl)-2,4-dioxo-1,2,3,4-tetrahydropyrimidine-5-carboxamide
1-butyl-3-(3-cyanophenyl)-N-[4-(methylsulfonyl)benzyl]-2,4-dioxo-1,2,3,4-tetrahydropyrimidine-5-carboxamide
1-butyl-3-(3-cyanophenyl)-N-(4-fluorobenzyl)-2,4-dioxo-1,2,3,4-tetrahydropyrimidine-5-carboxamide
N-(1,3-benzodioxol-5-ylmethyl)-1-butyl-3-(3-cyanophenyl)-2,4-dioxo-1,2,3,4-tetrahydropyrimidine-5-carboxamide
1-butyl-3-(3-cyanophenyl)-N-(2,4-dichlorobenzyl)-2,4-dioxo-1,2,3,4-tetrahydropyhmidine-5-carboxamide
1-butyl-3-(3-cyanophenyl)-N-(3,4-difluorobenzyl)-2,4-dioxo-1,2,3,4-tetrahydropyrimidine-5-carboxamide
1-butyl-N-(2-chloro-4-fluorobenzyl)-3-(3-cyanophenyl)-2,4-dioxo-1,2,3,4-tetrahydropyrimidine-5-carboxamide
1-butyl-3-(3-cyanophenyl)-N-(3,4-dichlorobenzyl)-2,4-dioxo-1,2,3,4-tetrahydropyrimidine-5-carboxamide
1-butyl-N-[(4-cyanocyclohexyl)methyl]-3-(3-cyanophenyl)-2,4-dioxo-1,2,3,4-tetrahydropyrimidine-5-carboxamide
1-butyl-3-(3-cyanophenyl)-N-[(5-methylisoxazol-3-yl)methyl]-2,4-dioxo-1,2,3,4-tetrahydropyfimidine-5-carboxamide
1-butyl-3-(3-cyanophenyl)-2,4-dioxo-N-[4-(1H-pyrazol-1-yl)benzyl]-1,2,3,4-tetrahydropyrimidine-5-carboxamide
1-butyl-3-(3-cyanophenyl)-2,4-dioxo-N-[3-(2-oxopyrrolidin-1-yl)propyl]-1,2,3,4-tetrahydropyrimidine-5-carboxamide
1-butyl-N-(4-chlorobenzyl)-2,4-dioxo-3-[3-(trifluoromethyl)phenyl]-1,2,3,4-tetrahydropyrimidine-5-carboxamide
1-butyl-N-(4-methoxybenzyl)-2,4-dioxo-3-[3-(trifluoromethyl)phenyl]-1,2,3,4-tetrahydropyrimidine-5-carboxamide
1-butyl-2,4-dioxo-N-(pyridin-4-ylmethyl)-3-[3-(trifluoromethyl)phenyl]-1,2,3,4-tetrahydropyrimidine-5-carboxamide
1-butyl-N-[2-(3,4-dimethoxyphenyl)ethyl]-2,4-dioxo-3-[3-(trifluoromethyl)phenyl]-1,2,3,4-tetrahydropyrimidine-5-carboxamide
1-butyl-N-[2-(3-methoxyphenyl)ethyl]-2,4-dioxo-3-[3-(trifluoromethyl)phenyl]-1,2,3,4-tetrahydropyrimidine-5-carboxamide
N-(3-bromobenzyl)-1-butyl-2,4-dioxo-3-[3-(tnfluoromethyl)phenyl]-1,2,3,4-tetrahydropyrimidine-5-carboxamide
N-(4-bromobenzyl)-1-butyl-2,4-dioxo-3-[3-(trifluoromethyl)phenyl]-1,2,3,4-tetrahydropyrimidine-5-carboxamide
1-butyl-N-(4-methylbenzyl)-2,4-dioxo-3-[3-(trifluoromethyl)phenyl]-1,2,3,4-tetrahydropyrimidine-5-carboxamide
1-butyl-N-[4-(methylsulfonyl)benzyl]-2,4-dioxo-3-[3-(trifluoromethyl)phenyl]-1,2,3,4-tetrahydropyrimidine-5-carboxamide
1-butyl-N-(4-fluorobenzyl)-2,4-dioxo-3-[3-(trifluoromethyl)phenyl)-1,2,3,4-tetrahydropynmidine-5-carboxamide
N-(1,3-benzodioxol-5-ylmethyl)-1-butyl-2,4-dioxo-3-[3-(trifluoromethyl)phenyl]-1,2,3,4-tetrahydropyrimidine-5-carboxamide
1-butyl-N-(2,4-dichlorobenzyl)-2,4-dioxo-3-[3-(trifluoromethyl)phenyl]-1,2,3,4-tetrahydropyrimidine-5-carboxamide
1-butyl-N-(3,4-difluorobenzyl)-2,4-dioxo-3-[3-(trifluoromethyl)phenyl]-1,2,3,4-tetrahydropyrimidine-5-carboxamide
1-butyl-N-(2-chloro-4-fluorobenzyl)-2,4-dioxo-3-[3-(trifluoromethyl)phenyl]-1,2,3,4-tetrahydropyrimidine-5-carboxamide
1-butyl-N-(3,4-dichlorobenzyl)-2,4-dioxo-3-[3-(trifluoromethyl)phenyl]-1,2,3,4-tetrahydropyrimidine-5-carboxamide
1-butyl-N-(2,3-dihydro-1-benzofuran-5-ylmethyl)-2,4-dioxo-3-[3-(trifluoromethyl)phenyl]-1,2,3,4-tetrahydropyrimidine-5-carboxamide
1-butyl-N-[(4-cyanocyclohexyl)methyl]-2,4-dioxo-3-[3-(trifluoromethyl)phenyl]-1,2,3,4-tetrahydropyrimidine-5-carboxamide
1-butyl-N-[(5-methylisoxazol-3-yl)methyl]-2,4-dioxo-3-[3-(trifluoromethyl)phenyl]-1,2,3,4-tetrahydropyrimidine-5-carboxamide
1-butyl-2,4-dioxo-N-[4-(1H-pyrazol-1-yl)benzyl]-3-[3-(trifluoromethyl)phenyl]-1,2,3,4-tetrahydropyrimidine-5-carboxamide
1-butyl-2,4-dioxo-N-[3-(2-oxopyrrolidin-1-yl)propyl]-3-[3-(trifluoromethyl)phenyl]-1,2,3,4-tetrahydropyrimidine-5-carboxamide
6-(chloromethyl)-N-[4-(methylsulfonyl)benzyl]-2-oxo-1-[3-(trifluoromethyl)phenyl]-1,2-dihydropyridine-3-carboxamide
N-[4-(methylsulfonyl)benzyl]-6-[(methylthio)methyl]-2-oxo-1-[3-(trifluoromethyl)phenyl]-1,2-dihydropyridine-3-carboxamide
N-[4-(methylsulfonyl)benzyl]-6-({[4-(methylsulfonyl)benzyl]amino}methyl)-2-oxo-1-[3-(trifluoromethyl)phenyl]-1,2-dihydropyridine-3-carboxamide
N-[4-(methylsulfonyl)benzyl]-6-(morpholin-4-ylmethyl)-2-oxo-1-[3-(trifluoromethyl)phenyl]-1,2-dihydropyridine-3-carboxamide
6-(cyanomethyl)-N-[4-(methylsulfonyl)benzyl]-2-oxo-1-[3-(trifluoromethyl)phenyl]-1,2-dihydropyridine-3-carboxamide
6-isopropyl-N-[4-(methylsulfonyl)benzyl]-2-oxo-1-[3-(trifluoromethyl)phenyl]-1,2-dihydropyridine-3-carboxamide
N-[4-(ethylsulfonyl)benzyl]-6-methyl-2-oxo-1-[3-(trifluoromethyl)phenyl]-1,2-dihydropyridine-3-carboxamide
N-[3-chloro-4-(methylsulfonyl)benzyl]-6-methyl-2-oxo-1-[3-(trifluoromethyl)phenyl]-1,2-dihydropyridine-3-carboxamide
6-methyl-2-oxo-1-(3-trifluoromethyl-phenyl)-1,2-dihydro-pyridine-3-carboxylic acid 4-cyclopropanesulfonyl-benzylamide
N-[3-methoxy-4-(methylsulfonyl)benzyl]-6-methyl-2-oxo-1-[3-(trifluoromethyl)phenyl]-1,2-dihydropyridine-3-carboxamide
N-[3-bromo-4-(methylsulfonyl)benzyl]-6-methyl-2-oxo-1-[3-(trifluoromethyl)phenyl]-1,2-dihydropyridine-3-carboxamide
N-[3-cyano-4-(methylsulfonyl)benzyl]-6-methyl-2-oxo-1-[3-(trifluoromethyl)-phenyl]-1,2-dihydropyridine-3-carboxamide
6-methyl-N-[3-methyl-4-(methylsulfonyl)benzyl]-2-oxo-1-[3-(trifluoromethyl)-phenyl]-1,2-dihydropyridine-3-carboxamide
6-methyl-N-[4-(methylthio)benzyl]-2-oxo-1-[3-(trifluoromethyl)phenyl]-1,2-dihydropyridine-3-carboxamide
6-methyl-N-[4-(methylsulfinyl)benzyl]-2-oxo-1-[3-(trifluoromethyl)phenyl]-1,2-dihydropyridine-3-carboxamide
N-[4-(benzylsulfonyl)benzyl]-6-methyl-2-oxo-1-[3-(trifluoromethyl)phenyl]-1,2-dihydropyridine-3-carboxamide
6-methyl-2-oxo-N-[4-(propylsulfonyl)benzyl]-1-[3-(trifluoromethyl)phenyl]-1,2-dihydropyridine-3-carboxamide
N-[4-(butylsulfonyl)benzyl]-6-methyl-2-oxo-1-[3-(trifluoromethyl)phenyl]-1,2-dihydropyridine-3-carboxamide
N-[4-(isobutylsulfonyl)benzyl]-6-methyl-2-oxo-1-[3-(trifluoromethyl)phenyl]-1,2-dihydropyridine-3-carboxamide
N-[4-(*sec*-butylsulfonyl)benzyl]-6-methyl-2-oxo-1-[3-(trifluoromethyl)phenyl]-1,2-dihydropyridine-3-carboxamide
N-[4-(isopropylsulfonyl)benzyl]-6-methyl-2-oxo-1-[3-(trifluoromethyl)phenyl]-1,2-dihydropyridine-3-carboxamide
6-methyl-N-{4-[(3-methylbutyl)sulfonyl]benzyl}-2-oxo-1-[3-(trifluoromethyl)phenyl]-1,2-dihydropyridine-3-carboxamide
N-{4-[(cyclopmpylmethyl)sulfonyl]benzyl}-6-methyl-2-oxo-1-[3-(trifluoromethyl)phenyl]-1,2-dihydropyridine-3-carboxamide
6-methyl-2-oxo-N-{4-[(tetrahydrofuran-2-ylmethyl)sulfonyl]-benzyl}-1-[3-(trifluoromethyl)phenyl]-1,2-dihydropyridine-3-carboxamide
N-{4-[(2-hydroxyethyl)sulfonyl]benzyl}-6-methyl-2-oxo-1-[3-(trifluoromethyl)phenyl]-1,2-dihydmpyridine-3-carboxamide
N-{4-[(cyanomethyl)sulfonyl]benzyl}-6-methyl-2-oxo-1-[3-(trifluoromethyl)phenyl]-1,2-dihydropyricline-3-carboxamide
N-{4-[(2-amino-2-oxoethyl)sulfonyl]benzyl}-6-methyl-2-oxo-1-[3-(trifluoromethyl)phenyl]-1,2-dihydropyridine-3-carboxamide
N-{4-[(4-cyanobenzyl)sulfonyl]benzyl}-6-methyl-2-oxo-1-[3-(trifluoromethyl)phenyl]-1,2-dihydropyridine-3-carboxamide
N-{4-[(2-cyanoethyl)sulfonyl]benzyl}-6-methyl-2-oxo-1-[3-(trifluoromethyl)phenyl]-1,2-dihydropyridine-3-carboxamide
N-{4-[(3-hydroxypropyl)sulfonyl]benzyl}-6-methyl-2-oxo-1-[3-(trifluoromethyl)phenyl]-1,2-dihydropyridine-3-carboxamide
N-(4-{[2-(dimethylamino)-2-oxoethyl]sulfonyl}benzyl)-6-methyl-2-oxo-1-[3-(trifluoromethyl)phenyl]-1,2-dihydropyridine-3-carboxamide
ethyl 3-[(4-{[({6-methyl-2-oxo-1-[3-(trifluoromethyl)phenyl]-1,2-dihydropyridin-3-yl }carbonyl)amino]methyl}phenyl)sulfonyl]propanoate
2-[(4-{[({6-methyl-2-oxo-1-[3-(trifluoromethyl)phenyl]-1,2-dihydropyridin-3-yl}carbonyl)amino]methyl}phenyl)sulfonyl]ethyl acetate
N-{4-[(3-cyanobenzyl)sulfonyl]benzyl}-6-methyl-2-oxo-1-[3-(trifluoromethyl)phenyl]-1,2-dihydropyridine-3-carboxamide
methyl 3-[(4-{[({6-methyl-2-oxo-1-[3-(trifluoromethyl)phenyl]-1,2-dihydropyridin-3-yl}carbonyl)amino]methyl}phenyl)sulfonyl]propanoate
6-methyl-N-(4-{[(2-methyl-1,3-thiazol-4-yl)methyl]sulfonyl}benzyl)-2-oxo-1-[3-(trifluoromethyl)phenyl]-1,2-dihydropyridine-3-carboxamide
6-methyl-2-oxo-N-{4-[(pyridin-4-ylmethyl)sulfonyl]benzyl}-1-[3-(trifluoromethyl)phenyl]-1,2-dihydropyridine-3-carboxamide
N-{4-[(3-cyanopropyl)sulfonyl]benzyl}-6-methyl-2-oxo-1-[3-(trifluoromethyl)phenyl]-1,2-dihydropyridine-3-carboxamide
N-(4-{[(3,5-dimethylisoxazol-4-yl)methyl]sulfonyl)benzyl)-6-methyl-2-oxo-1-[3-(trifluoromethyl)phenyl]-1,2-dihydropyridine-3-carboxamide
N-(4-{[4-(acetylamino)benzyl]sulfonyl}benzyl)-6-methyl-2-oxo-1-[3-(trifluoromethyl)phenyl]-1,2-dihydropyridine-3-carboxamide
6-methyl-N-[4-({2-[(5-methyl-1,3,4-thiadiazol-2-yl)amino]-2-oxoethyl}sulfonyl)benzyl]-2-oxo-1-[3-(trifluoromethyl)phenyl]-1,2-dihydropyridine-3-carboxamide
6-methyl-N-[4-(methylsulfonyl)phenoxy]-2-oxo-1-[3-(trifluoromethyl)phenyl]-1,2-dihydropyridine-3-carboxamide
6-methyl-2-oxo-1-(3-trifluoromethyl-phenyl)-1,2-dihydro-pyricline-3-carboxylic acid (4-bromo-phenoxy)-amide
6-methyl-2-oxo-N-phenoxy-1-[3-(trifluoromethyl)phenyl]-1,2-dihydropyridine-3-carboxamide
N-(4-aminobenzyl)-6-methyl-2-oxo-1-[3-(trifluoromethyl)phenyl]-1,2-dihydroproline-3-carboxamide
6-methyl-N-{4-[(methylsulfonyl)amino]benzyl}-2-oxo-1-[3-(trifluoromethyl)phenyl]-1,2-dihydropyridine-3-carboxamide
N-{4-[bis(methylsulfonyl)amino]benzyl}-6-methyl-2-oxo-1-[3-(trifluoromethyl)phenyl]-1,2-dihydropyridine-3-carboxamide
N-[[4-[[(dimethylamino)sulfonyl]amino]phenyl]methyl]-1,2-dihydro-6-methyl-2-oxo-1-[3-(trifluoromethyl)phenyl]-3-pyridinecarboxamide
6-methyl-N-{4-[methyl(methylsulfonyl)amino]benzyl}-2-oxo-1-[3-(trifluoromethyl)phenyl]-1,2-dihydropyridine-3-carboxamide
N-[[4-[butyl(methylsulfonyl)amino]phenyl]methyl]-1,2-dihydro-6-methyl-2-oxo-1-[3-(trifluoromethyl)phenyl]-3-pyridinecarboxamide
1,2-dihydro-6-methyl-N-[[4-[(1-methylethyl)(methylsulfonyl)-amino]phenyl]methyl]-2-oxo-1-[3-(trifluoromethyl)phenyl]-3-pyridinecarboxamide
N-{4-[(2-methoxyethyl)(methylsulfonyl)amino]benzyl}-6-methyl-2-oxo-1-[3-(trifluoromethyl)phenyl]-1,2-dihydropyridine-3-carboxamide
N-{4-[(2-cyanoethyl)(methylsulfonyl)amino]benzyl}-6-methyl-2-oxo-1-[3-(trifluoromethyl)phenyl]-1,2-dihydropyridine-3-carboxamide
N-{4-[ethyl(methylsulfonyl)amino]benzyl}-6-methyl-2-oxo-1-[3-(trifluoromethyl)phenyl]-1,2-dihydropyridine-3-carboxamide
1,2-dihydro-6-methyl-N-[[4-[(methylsulfonyl)propylamino]-phenyl]methyl]-2-oxo-1-[3-(trifluoromethyl)phenyl]-3-pyridinecarboxamide
N-[[4-[(3-amino-3-oxopropyl)(methylsulfonyl)amino]phenyl]-methyl]-1,2-dihydro-6-methyl-2-oxo-1-[3-(trifluoromethyl)phenyl]-3-pyridinecarboxamide
1,2-dihydro-6-methyl-N-[[4-[(methylsulfonyl)oxy]phenyl]methyl]-2-oxo-1-[3-(trifluoromethyl)phenyl]-3-pyridinecarboxamide
2-propanesulfonic acid, 4-[[[[1,2-dihydro-6-methyl-2-oxo-1-[3-(trifluoromethyl)phenyl]-3-pyridinyl]carbonyl]amino]methyl]phenyl ester
N-[(1,1-dioxido-2,3-dihydro-1-benzothien-5-yl)methyl]-6-methyl-2-oxo-1-[3-(trifluoromethyl)phenyl]-1,2-dihydropyridine-3-carboxamide
N-[(1,1-dioxido-2,3-dihydro-1-benzothien-5-yl)methyl]-5-iodo-6-methyl-2-oxo-1-[3-(trifluoromethyl)phenyl]-1,2-dihydropyridine-3-carboxamide
5-iodo-N-{4-[isopropyl(methylsulfonyl)amino]benzyl}-6-methyl-2-oxo-1-[3-(trifluoromethyl)phenyl]-1,2-dihydropyridine-3-carboxamide
1,2-dihydro-6-methyl-N-[[4-[(methylsulfonyl)methyl]phenyl]-methyl]-2-oxo-1-[3-(trifluoromethyl)phenyl]-3-pyridinecarboxamide
6-chloro-5-methyl-4-(3-methylphenyl-N-[4-(methylsulfonyl)benzyl]-3-oxo-3,4-dihydropyrazine-2-carboxamide
5-bromo-6-(difluoromethyl)-N-[4-(methylsulfonyl)benzyl]-2-oxo-1-[3-(trifluoromethyl)phenyl]-1,2-dihydropyridine-3-carboxamide
6-(difluoromethyl)-N-[4-(methylsulfonyl)benzyl]-2-oxo-1-[3-(trifluoromethyl)phenyl]-1,2-dihydropyridine-3-carboxamide
N-(2,3-dihydro-1,4-benzodioxin-6-ylmethyl)-6-methyl-2-oxo-1-[3-(trifluoromethyl)phenyl]-1,2-dihydropyridine-3-carboxamide
6-methyl-N-[3-(methylsulfonyl)benzyl]-2-oxo-1-[3-(trifluoromethyl)phenyl]-1,2-dihydropyridine-3-carboxamide
6-methyl-N'-[4-(methylsulfonyl)phenyl]-2-oxo-1-[3-(trifluoromethyl)phenyl]-1,2 dihydropyridine-3-carbohydrazide
N'-(4-bromophenyl)-6-methyl-2-oxo-1-[3-(trifluoromethyl)phenyl]-1,2-dihydropyridine-3-carbohydrazide
N-[(5-methoxy-4-oxo-4H-pyran-2-yl)methyl]-6-methyl-2-oxo-1-[3-(trifluoromethyl)phenyl]-1,2-dihydropyridine-3-carboxamide
N-(4-cyanobenzyl)-6-methyl-2-oxo-1-[3-(trifluoromethyl)phenyl]-1,2-dihydropyridine-3-carboxamide
N-{[3-(4-methoxyphenyl)isoxazol-5-yl]methyl}-6-methyl-2-oxo-1-[3-(trifluoromethyl)phenyl]-1,2-dihydropyridine-3-carboxamide
N'-(4-cyanophenyl)-6-methyl-2-oxo-1-[3-(trifluoromethyl)phenyl]-1,2-dihydropyridine-3-carbohydrazide
6-methyl-2-oxo-N-[(1-phenyl-1H-pyrazol-4-yl)methyl]-1-[3-(trifluoromethyl)phenyl]-1,2-dihydropyridine-3-carboxamide
N-(2,3-dihydro-1,4-benzodioxin-2-ylmethyl)-6-methyl-2-oxo-1-[3-(trifluoromethyl)phenyl]-1,2-dihydropyridine-3-carboxamide
6-methyl-N-{[1-(3-methylphenyl)-1H-pyrazol-4-yl]methyl}-2-oxo-1-[3-(trifluoromethyl)phenyl]-1,2-dihydropyridine-3-carboxamide
N'-(4-chlorophenyl)-6-methyl-2-oxo-1-[3-(trifluoromethyl)phenyl]-1,2-dihydropyridine-3-carbohydrazide
6-methyl-2-oxo-N-[2-(tetrahydro-2H-pyran-4-yl)ethyl]-1-[3-(trifluoromethyl)phenyl]-1,2-dihydropyridine-3-carboxamide
N-[(1-ethyl-1H-pyrazol-4-yl)methyl]-6-methyl-2-oxo-1-[3-(trifluoromethyl)phenyl]-1,2-dihydropyridine-3-carboxamide
N-[(4-benzylmorpholin-2-yl)methyl]-6-methyl-2-oxo-1-[3-(trifluoromethyl)phenyl]-1,2-dihydropyridine-3-carboxamide
6-methyl-N-[3-(2-methylpiperidin-1-yl)propyl]-2-oxo-1-[3-(trifluoromethyl)phenyl]-1,2-dihydropyridine-3-carboxamide
methyl 2-{[({6-methyl-2-oxo-1-[3-(trifluoromethyl)phenyl]-1,2-dihydropyidin-3-yl}carbonyl)amino]methyl}-3-furoate
6-methyl-N-[(1-methyl-1H-pyrazol-4-yl)methyl]-2-oxo-1-[3-(trifluoromethyl)phenyl]-1,2-dihydropyridine-3-carboxamide
N-(3-azepan-1-ylpropyl)-6-methyl-2-oxo-1-[3-(trifluoromethyl)phenyl]-1,2-dihydropyridine-3-carboxamide
6-methyl-N-(3-morpholin-4-ylpropyl)-2-oxo-1-[3-(trifluoromethyl)phenyl]-1,2-dihydropyridine-3-carboxamide
6-methyl-2-oxo-N-(3-piperidin-1-ylpropyl)-1-[3-(trifluoromethyl)phenyl]-1,2-dihydropyridine-3-carboxamide
N-[3-(3,5-dimethyl-1H-pyrazol-1-yl)propyl]-6-methyl-2-oxo-1-[3-(trifluoromethyl)phenyl]-1,2-dihydropyridine-3-carboxamide
N-[3-(2-ethylpiperidin-1-yl)propyl]-6-methyl-2-oxo-1-[3-(trifluoromethyl)phenyl]-1,2-dihydropyridine-3-carboxamide
6-methyl-N-[2-(1-methyl-1H-imidazol-5-yl)ethyl]-2-oxo-1-[3-(trifluoromethyl)phenyl]-1,2-dihydropyridine-3-carboxamide
N-[(1-ethyl-3-methyl-1H-pyrazol-4-yl)methyl]-6-methyl-2-oxo-1-[3-(trifluoromethyl)phenyl]-1,2-dihydropyridine-3-carboxamide
N-[4-(acetylamino)benzyl]-6-methyl-2-oxo-1-[3-(trifluoromethyl)phenyl]-1,2-dihydropyridine-3-carboxamide
6-methyl-2-oxo-N-[3-(1H-pyrazol-1-yl)propyl]-1-[3-(trifluoromethyl)phenyl]-1,2-dihydropyridine-3-carboxamide
6-methyl-2-oxo-N-(pyridin-2-ylmethyl)-1-[3-(trifluoromethyl)phenyl]-1,2-dihydropyridine-3-carboxamide
6-methyl-N-{[1-(4-methylphenyl)-1H-pyrazol-4-yl]methyl}-2-oxo-1-[3-(trifluoromethyl)phenyl]-1,2-dihydropyridine-3-carboxamide
6-methyl-N'-(4-methylphenyl)-2-oxo-1-[3-(trifluoromethyl)phenyl]-1,2-dihydropyridine-3-carbohydrazide
6-methyl-N-[3-(4-methylpiperidin-1-yl)propyl]-2-oxo-1-[3-(trifluoromethyl)phenyl]-1,2-dihydropyridine-3-carboxamide
6-methyl-2-oxo-N-[3-(5-oxo-4,5-dihydro-1H-pyrazol-4-yl)propyl]-1-[3-(trifluoromethyl)phenyl]-1,2-dihydropyridine-3-carboxamide
ethyl 5-methyl-4-{[({6-methyl-2-oxo-1-[3-(trifluoromethyl)phenyl]-1,2-dihydropyridin-3-yl}carbonyl)amino]methyl}-2-furoate
N-[(6-fluoro-4H-1,3-benzodioxin-8-yl)methyl]-6-methyl-2-oxo-1-[3-(trifluoromethyl)phenyl]-1,2-dihydropyridine-3-carboxamide
6-methyl-2-oxo-N-(2-pyridin-3-ylethyl)-1-[3-(trifluoromethyl)phenyl]-1,2-dihydropyridine-3-carboxamide
N-[(1,3-dimethyl-1H-pyrazol-4-yl)methyl]-6-methyl-2-oxo-1-[3-(trifluoromethyl)phenyl]-1,2-dihydropyridine-3-carboxamide
6-methyl-2-oxo-N-(2-pyridin-4-ylethyl)-1-[3-(trifluoromethyl)phenyl]-1,2-dihydropyridine-3-carboxamide
N'-(4-fluorophenyl)-6-methyl-2-oxo-1-[3-(trifluoromethyl)phenyl]-1,2-dihydropyridine-3-carbohydrazide
6-methyl-N-[(1-methyl-1H-pyrrol-2-yl)methyl]-2-oxo-1-[3-(trifluoromethyl)phenyl]-1,2-dihydropyridine-3-carboxamide
6-methyl-2-oxo-N'-phenyl-1-[3-(trifluoromethyl)phenyl]-1 ,2-dihydropyridine-3-carbohydrazide
N-[(1-ethyl-5-methyl-1H-pyrazol-4-yl)methyl]-6-methyl-2-oxo-1-[3-(trifluoromethyl)phenyl]-1,2-dihydropyridine-3-carboxamide
6-methyl-N-[2-(1-methyl-1H-imidazol-4-yl)ethyl]-2-oxo-1-[3-(trifluoromethyl)phenyl]-1,2-dihydropyridine-3-carboxamide
N-[2-(1,3-dioxolan-2-yl)ethyl]-6-methyl-2-oxo-1-[3-(trifluoromethyl)phenyl]-1,2-dihydropyridine-3-carboxamide
N-(1-benzothien-3-ylmethyl)-6-methyl-2-oxo-1-[3-(trifluoromethyl)phenyl]-1,2-dihydropyridine-3-carboxamide
N-[(1,5-dimethyl-1H-pyrazol-4-yl)methyl]-6-methyl-2-oxo-1-[3-(trifluoromethyl)phenyl]-1,2-dihydropyridine-3-carboxamide
N-[2-(3,5-dimethyl-1H-pyrazol-4-yl)ethyl]-6-methyl-2-oxo-1-[3-(trifluoromethyl)phenyl]-1,2-dihydropyridine-3-carboxamide
N-[2-(3,5-dimethylisoxazol-4-yl)ethyl]-6-methyl-2-oxo-1-[3-(trifluoromethyl)phenyl]-1,2-dihydropyridine-3-carboxamide
N-(3,4-dihydro-1H-isochromen-1-ylmethyl)-6-methyl-2-oxo-1-[3-(trifluoromethyl)phenyl]-1,2-dihydropyridine-3-carboxamide
N-{[(2R)-1-ethylpyrrolidin-2-yl]methyl}-6-methyl-2-oxo-1-[3-(trifluoromethyl)phenyl]-1,2-dihydropyridine-3-carboxamide
6-methyl-2-oxo-N-[(2R)-tetrahydrofuran-2-ylmethyl]-1-[3-(trifluoromethyl)phenyl]-1,2-dihydropyridine-3-carboxamide
5-chloro-N-{4-[(dimethylamino)sulfonyl]benzyl}-6-methyl-2-oxo-1-[3-(trifluoromethyl)phenyl]-1,2-dihydropyridine-3-carboxamide
N-{4-[(dimethylamino)sulfonyl]benzyl}-6-methyl-2-oxo-1-[3-(trifluoromethyl)-phenyl]-1,2-dihydropyridine-3-carboxamide
5-chloro-6-methyl-2-oxo-N-[4-(piperazin-1-ylsulfonyl)benzyl]-1-[3-(trifluoromethyl)phenyl]-1,2-dihydropyridine-3-carboxamide
6-methyl-2-oxo-N-[4-(piperazin-1-ylsulfonyl)benzyl]-1-[3-(trifluoromethyl)-phenyl]-1,2-dihydropyridine-3-carboxamide
6-methyl-N-[4-(morpholin-4-ylsulfonyl)benzyl]-2-oxo-1-[3-(trifluoromethyl)phenyl]-1,2-dihydropyridine-3-carboxamide
6-methyl-2-oxo-N-[4-(piperidin-1-ylsulfonyl)benzyl]-1-[3-(trifluoromethyl)-phenyl]-1,2-dihydropyridine-3-carboxamide
6-methyl-N-{4-[(methylamino)sulfonyl]benzyl}-2-oxo-1-[3-(trifluoromethyl)-phenyl]-1,2-dihydropyridine-3-carboxamide
6-methyl-2-oxo-N-[4-(pyrrolidin-1-ylsulfonyl)benzyl]-1-[3-(trifluoromethyl)phenyl]-1,2-dihydropyridine-3-carboxamide
5-chloro-6-methyl-2-oxo-N-[4-(pyrrolidin-1-ylsulfonyl)benzyl]-1-[3-(trifluoromethyl)phenyl]-1,2-dihydropyridine-3-carboxamide
5-chloro-6-rnethyl-N-[4-(methylsulfonyl)benzyl]-2-oxo-1-[3-(trifluoromethyl)-phenyl]-1,2-dihydropyridine-3-carboxamide
N-{4-[(acetylamino)sulfonyl]benzyl}-6-methyl-2-oxo-1-[3-(trifluoromethyl)-phenyl]-1,2-dihydropyridine-3-carboxamide
N-[4-(isopropylsulfonyl)benzyl]-5-iodo-6-methyl-2-oxo-1-[3-(trifluorornethyl)phenyl]-1,2-dihydropyridine-3-carboxamide
N-[4-(cyclopropylsulfonyl)benzyl]-5-iodo-6-methyl-2-oxo-1-[3-(trifluoromethyl)phenyl]-1,2-dihydropyridine-3-carboxamide
1,2-dahydro-6-methyl-N-[[4-[(methylsulfonyl)oxy]phenyl]methyl]-2-oxo-1-[3-(trifluoromethyl)phenyl]-3-pyridinecarboxamide
N-[4-(1,1-dioxidoisothiazolidin-2-yl)benzyl]-6-methyl-2-oxo-1-[3-(trifluoromethyl)phenyl]-1,2-dihydropyridine-3-carboxamide
6-methyl-2-oxo-N-[[4-(4-pyridinylsulfonyl)phenyl]methyl]-1-[3-(trifluoromethyl)phenyl]-1,2-dihydropyridine-3-carboxamide
6-methyl-2-oxo-N-[4-(phenylsulfonyl)benzyl]-1-[3-(trifluoromethyl)phenyl]-1,2-dihydropyridine-3-carboxamide
6-methyl-2-oxo-N-[4-(1,3-thiazol-2-ylsulfonyl)benzyl]-1-[3-(trifluoromethyl)phenyl]-1,2-dihydropyridine-3-carboxamide
6-methyl-2-oxo-N-[4-(pyrimidin-2-ylsulfonyl)benzyl]-1-[3-(trifluoromethyl)phenyl]-1,2-dihydropyridine-3-carboxamide
N-[4-(1*H*-imidazol-2-ylsulfonyl)benzyl]-6-methyl-2-oxo-1-[3-(trifluoromethyl)phenyl]-1,2-dihydropyridine-3-carboxamide
6-methyl-N-{4-[(1-methyl-1*H*-1,2,4-triazol-5-yl)sulfonyl]benzyl}-2-oxo-1-[3-(trifluoromethyl)phenyl]-1,2-dihydropyridine-3-carboxamide
6-methyl-N-{4-[(5-methyl-1,3-oxazol-4-yl)sulfonyl]benzyl}-2-oxo-1-[3-(trifluoromethyl)phenyl]-1,2-dihydropyridine-3-carboxamide
6-methyl-N-{[6-(methylsulfonyl)pyridin-3-yl]methyl}-2-oxo-1-[3-(trifluoromethyl)phenyl]-1,2-dihydropyridine-3-carboxamide
5-fluoro-6-methyl-N-[4-(methylsulfonyl)benzyl]-2-oxo-1-[3-(trifluoromethyl)phenyl]-1,2-dihydropyridine-3-carboxamide
N-[4-(methylsulfonyl)benzyl]-2-oxo-6-(2-oxoethyl)-1-[3-(trifluoromethyl)phenyl]-1,2-dihydropyridine-3-carboxamide
5-ethyl-6-methyl-N-[4-(methylsulfonyl)benzyl]-2-oxo-1-[3-(trifluoromethyl)phenyl]-1,2-dihydropyridine-3-carboxamide
and pharmaceutically acceptable salts thereof.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

In a further aspect the invention provides a process for the preparation of a compound of formula (I) which comprises:
reacting a compound of formula (II) 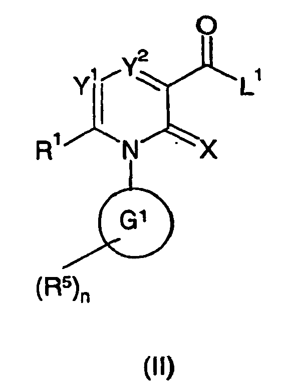
wherein R¹, R⁵, Y¹, Y², X, G¹ and n are as defined in formula (I) and L¹ represents a leaving group,
with an amine of formula (III) or a salt thereof 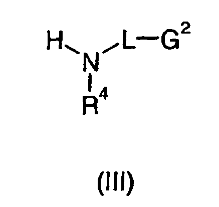 wherein R⁴, G² and L are as defined in formula (I),
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

The process is carried out at a suitable temperature, generally between 0 °C and the boiling point of the solvent, in a suitable solvent such as dichloromethane or N-methylpyrrolidinone. The process is optionally carried out in the presence of a base and/or a coupling reagent such as HATU, HOAT, HOBT or DIEA. Suitable leaving groups L¹ include OH and halogen, particularly OH.

Compounds of formula (II) wherein Y¹ is CR², Y² is CR³, L¹ is OH and R² and R³ are both hydrogen can be prepared by condensing a compound of formula (IV) 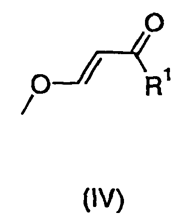 wherein R¹ is as defined in formula (I),
with a compound of formula (V) 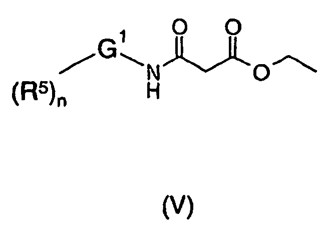 wherein G¹, R⁵ and n are as defined in formula (I), in the presence of a suitable base, such as sodium methoxide, in a suitable solvent, such as ethanol, followed by hydrolysis using a suitable base such as sodium hydroxide.

In general, compounds of formulae (IV) and (V) are either known or may be prepared using methods that will be readily apparent to the man skilled in the art. For example, compounds of formula (IV) can be prepared according to the methods of S.M Brombridge et al., *Synthetic Communications,* 1993, **23**, 487-494. And compounds of formula (V) can be prepared according to the methods of Igor V. Ukrainets et al., *Tetrahedron,* 1994, **50,** 10331-10338.

Compounds of formula (II) wherein Y¹ is CR², Y² is CR³, L¹ is OH and R¹ is hydrogen can be prepared by reacting a compound of formula (VI) 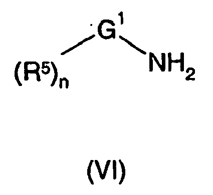 wherein G¹, R⁵ and n are as defined in formula (I), with a compound of formula (VII) 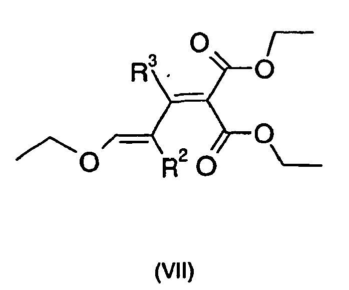 wherein R² or R³ are as defined in formula (I), at a suitable temperature, such as 160 °C, followed by base promoted cyclisation and acid hydrolysis. Compounds of formula (VII) can be prepared according to US 3,838,155.

Compounds of formula (II) wherein Y¹ is CR², Y² is CR³, L¹ is OH, R¹ is methyl and R² and R³ are both hydrogen can be prepared by condensing a compound of formula (VIII) 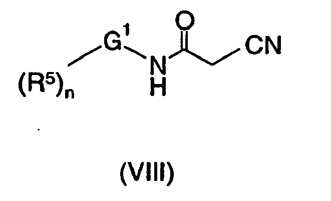 wherein G¹, R⁵ and n are as defined in formula (I), with 4-methoxy-3-buten-2-one in the presence of a suitable base, such as 1,4-diazabicyclo[2.2.2]octane, at a suitable temperature in a suitable solvent such as diethyleneglycol monomethyl ether, followed by acid hydrolysis.

Compounds of formula (II) wherein R¹ is OH, Y¹ is nitrogen and Y² is CR³ can be prepared by condensing a compound of formula (IX) 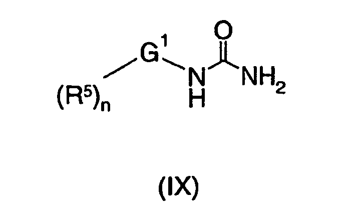 wherein G¹, R⁵ and n are as defined in formula (I), with a compound of formula (X) 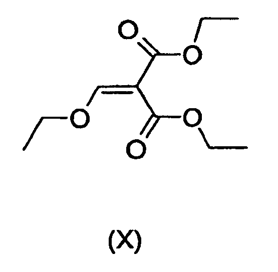 in the presence of a suitable base, such as sodium ethoxide, at a suitable temperature in a suitable solvent such as ethanol.

A compound of formula (IX) can be prepared from the corresponding isocyanate derivative by treatment with ammonia in acetonitrile.

Salts of compounds of formula (I) may be formed by reacting the free base or a salt, enantiomer, tautomer or protected derivative thereof, with one or more equivalents of the appropriate acid. The reaction may be carried out in a solvent or medium in which the salt is insoluble, or in a solvent in which the salt is soluble followed by subsequent removal of the solvent *in vacuo* or by freeze drying. Suitable solvents include, for example, water, dioxane, ethanol, 2-propanol, tetrahydrofuran or diethyl ether, or mixtures thereof. The reaction may be a metathetical process or it may be carried out on an ion exchange resin.

Compounds of formula (I) and intermediate compounds thereto may be prepared as such or in protected form. The protection and deprotection of functional groups is, for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

The compounds of the invention and intermediates may be isolated from their reaction mixtures, and if necessary further purified, by using standard techniques.

The compounds of formula (I) may exist in enantiomeric or diastereoisomeric forms or mixtures thereof, all of which are included within the scope of the invention. The various optical isomers may be isolated by separation of a racemic mixture of the compounds using conventional techniques, for example, fractional crystallisation or HPLC. Alternatively, the individual enantiomers may be made by reaction of the appropriate optically active starting materials under reaction conditions that will not cause racemisation.

Intermediate compounds may also exist in enantiomeric forms and may be used as purified enantiomers, diastereomers, racemates or mixtures thereof.

According to a further aspect of the invention we provide a compound of formula (I) or a pharmaceutically acceptable salt thereof, for use as a medicament.

The compounds of formula (I), and their pharmaceutically acceptable salts, are useful because they possess pharmacological activity in animals. The compounds of formula (I) have activity as pharmaceuticals, in particular as modulators of human neutrophil elastase and homologous serine proteases such as proteinase 3 and pancreatic elastase, and as such are predicted to be useful in therapy. The compounds of formula (I) are particularly useful as inhibitors of human neutrophil elastase. They may thus be used in the treatment or prophylaxis of inflammatory diseases and conditions.

Examples of these conditions are: adult respiratory distress syndrome (ARDS), cystic fibrosis, pulmonary emphysema, chronic obstructive pulmonary disease (COPD) and ischaemic-reperfusion injury. The compounds of this invention may also be useful in the modulation of endogenous and/or exogenous biological irritants which cause and/or propagate atherosclerosis, diabetes, myocardial infarction; hepatic disorders including but not limited to cirrhosis, systemic lupus erythematous, inflammatory disease of lymphoid origin, including but not limited to T lymphocytes, B lymphocytes, thymocytes; autoimmune diseases, bone marrow; inflammation of the joint (especially rheumatoid arthritis, osteoarthritis and gout); inflammation of the gastro-intestinal tract (especially inflammatory bowel disease, ulcerative colitis, pancreatitis and gastritis); inflammation of the skin (especially psoriasis, eczema, dermatitis); in tumour metastasis or invasion; in disease associated with uncontrolled degradation of the extracellular matrix such as osteoarthritis; in bone resorptive disease (such as osteoporosis and Paget's disease); diseases associated with aberrant angiogenesis; the enhanced collagen remodelling associated with diabetes, periodontal disease (such as gingivitis), corneal ulceration, ulceration of the skin, post-operative conditions (such as colonic anastomosis) and dermal wound healing; demyelinating diseases of the central and peripheral nervous systems (such as multiple sclerosis); age related illness such as dementia, inflammatory diseases of cardiovascular origins; granulomatous diseases; renal diseases including but not limited to nephritis and polyarteritis; cancer; pulmonary hypertension, ingested poisons, skin contacts, stings, bites; asthma; rhinitis; HIV disease progression; for minimising the effects of organ rejection in organ transplantation including but not limited to human organs; and replacement therapy of proteinase inhibitors.

Thus, another aspect of the invention provides the use of a compound of formula (I) or a pharmaceutically acceptable salt thereof, in the manufacture of a medicament for the treatment or prophylaxis of diseases or conditions in which inhibition of neutrophil elastase activity is beneficial; and a method of treating, or reducing the risk of, diseases or conditions in which inhibition of neutrophil elastase activity is beneficial which comprises administering to a person suffering from or at risk of, said disease or condition, a therapeutically effective amount of a compound of formula (I) or a pharmaceutically acceptable salt thereof.

In another aspect, the invention provides the use of a compound of formula (I) or a pharmaceutically acceptable salt thereof, in the manufacture of a medicament for the treatment or prophylaxis of inflammatory diseases or conditions; and a method of treating, or reducing the risk of, inflammatory diseases or conditions which comprises administering to a person suffering from or at risk of, said disease or condition, a therapeutically effective amount of a compound of formula (I) or a pharmaceutically acceptable salt thereof.

In particular, the compounds of this invention may be used in the treatment of adult respiratory distress syndrome (ARDS), cystic fibrosis, pulmonary emphysema, chronic obstructive pulmonary disease (COPD), pulmonary hypertension, asthma, rhinitis, ischemia-reperfusion injury, rheumatoid arthritis, osteoarthritis, cancer, atherosclerosis and gastric mucosal injury.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dose of the compound to be administered will depend on the compound employed, the disease being treated, the mode of administration, the age, weight and sex of the patient. Such factors may be determined by the attending physician. However, in general, satisfactory results are obtained when the compounds are administered to a human at a daily dosage of between 0.1 mg/kg to 100 mg/kg (measured as the active ingredient).

The compounds of formula (I) may be used on their own, or in the form of appropriate pharmaceutical formulations comprising the compound of the invention in combination with a pharmaceutically acceptable diluent, adjuvant or carrier. Particularly preferred are compositions not containing material capable of causing an adverse reaction, for example, an allergic reaction. Conventional procedures for the selection and preparation of suitable pharmaceutical formulations are described in, for example, "Pharmaceuticals - The Science of Dosage Form Designs", M. E. Aulton, Churchill Livingstone, 1988.

According to the invention, there is provided a pharmaceutical formulation comprising preferably less than 95% by weight and more preferably less than 50% by weight of a compound of formula (I) in admixture with a pharmaceutically acceptable diluent or carrier.

We also provide a method of preparation of such pharmaceutical formulations that comprises mixing the ingredients.

The compounds may be administered topically, for example, to the lungs and/or the airways, in the form of solutions, suspensions, HFA aerosols or dry powder formulations, for example, formulations in the inhaler device known as the Turbuhaler®; or systemically, for example, by oral administration in the form of tablets, pills, capsules, syrups, powders or granules; or by parenteral administration, for example, in the form of sterile parenteral solutions or suspensions; or by rectal administration, for example, in the form of suppositories.

Dry powder formulations and pressurized HFA aerosols of the compounds of the invention may be administered by oral or nasal inhalation. For inhalation, the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (for example, oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, for example, a mono-, di- or polysaccharide, a sugar alcohol, or an other polyol. Suitable carriers are sugars, for example, lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, for example, that known as the Turbuhaler® in which a dosing unit meters the desired dose which is then inhaled by the patient. With this system the active compound, with or without a carrier substance, is delivered to the patient.

For oral administration the active compound may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with, for example, a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example, solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

The following Examples are intended to illustrate, but in no way limit the scope of the invention.

### General procedures

¹H NMR and ¹³C NMR were recorded on a Varian *Inova* 400 MHz or a Varian *Mercury-*VX 300 MHz instrument. The central peaks of chlorofonn-*d* (δ_{H} 7.27 ppm), dimethylsulfoxide-*d*₆ (δ_{H} 2.50 ppm), acetonitrile-d₃ (δ_{H} 1.95 ppm) or methanol-*d*₄ (δ_{H} 3.31 ppm) where used as internal references. Low-resolution mass spectra were obtained on an Agilent 100 LC-MS system equipped with an APCI ionisation chamber. Column chromatography was carried out using silica gel (0.040-0.063 mm, Merck).
Unless stated otherwise, starting materials were commercially available. All solvents and commercial reagents were of laboratory grade and were used as received. Unless otherwise stated, organic solutions were dried using anhydrous Na₂SO₄.

### Unless otherwise stated, the following methods were used for HPLC and LC/MS analysis:

*LC*/*MS-Method A*
   Instrument Agilent 1100; Column Waters Symmetry 2.1 x 30 mm; Mass APCI; Flow rate 0.7 ml/min; Wavelength 254 nm; Solvent A: Water + 0.1% TFA; B: Acetonitrile + 0.1% TFA ; Gradient 15-95%/B 8 min, 95% B 1 min.
*LC-Method B*
   Instrument Agilent 1100; Column KR100-5C18 150 x 4.6 mm; Flow rate 1.0 ml/min; Wavelength 220 nm; Solvent A: Water + 0.1% TFA; B: Acetonitrile + 0.1 % TFA; Gradient 20-100%/B 8 min, 100% B 2 min.

The following abbreviations are used:
- HBTU: O-(Benzotriazol-1-yl)-N,N,N',N'-tetramethyluronium hexafluorophosphate
- HATU: O-(7-Azabenzotriazol-1-yl)-N,N,N',N'-tetramethyluronium hexafluorophosphate
- HOBT: 1-Hydroxybenzotriazole
- HOAT: 1-Hydroxy-7-azabenzotriazole
- DIEA: N,N-Diisopropylethylamine
- NMP: 1-N-Methyl-2-pyrrolidinone
- THF: Tetrahydrofuran
- TFA: Trifluoroacetic acid

### Example 1 N-(4-Chlorobenzyl)-1-(4-chlorophenyl)-6-methyl-2-oxo-1,2-dihydropyridine-3-carboxamide

### a) Ethyl 3-[(4-chlorophenyl)amino]-3-oxopropanoate

The title compound was prepared essentially as described by I. V. Ukrainets et al., Tetrahedron, 1994, **50,** 10331-10338.

### b) Ethyl 1-(4-chlorophenyl)-6-methyl-2-oxo-1,2-dihydropyridine-3-carboxylate

A mixture of ethyl 3-[(4-chlorophenyl)amino]-3-oxopropanoate (1 g, 4 mmol), 4-methoxy-3-buten-2-one (0.42 g, 4.2 mmol) and sodium methoxide (0.22 g, 4.1 mmol) in ethanol (10 ml) was heated to reflux for 5 h. After cooling, the solvent was evaporated off. The residue was chromatographed on silica using heptane/ethyl acetate (1:1 to 1:5) as eluent, affording the title compound (297 mg, 25%).
¹H NMR (CDCl₃): δ 8.17 (1H, d); 7.49 (2H, d); 7.13 (2H, d); 6.21 (1H, d); 4.34 (2H, q); 2.03 (3H, s); 1.35 (3H, t).

### c) 1-(4-Chlorophenyl)-6-methyl-2-oxo-1,2-dihydropyridine-3-carboxylic acid

Ethyl 1-(4-chlorophenyl)-6-methyl-2-oxo-1,2-dihydropyridine-3-carboxylate (297 mg, 1.0 mmol) was dissolved in a mixture of 1M sodium hydroxide solution (6 ml) and THF (5 ml). The reaction mixture was stirred for 2.5 h at room temperature, then acidified to pH 2 using 5M hydrochloric acid, and then extracted with dichloromethane. The combined organic phases were washed with water, dried, filtered and evaporated to give the title compound (268 mg, 100%).
¹H NMR (CDCl₃): δ 8.51 (1H, d); 7.59 (2H, d); 7.18 (2H, d); 6.53 (1H, d); 2.15 (3H, s).

### d) N-(4-Chlorobenzyl)-1-(4-chlorophenyl)-6-methyl-2-oxo-1,2-dihydropyridine-3-carboxamide

A mixture of 1-(4-chlorophenyl)-6-methyl-2-oxo-1,2-dihydropyridine-3-carboxylic acid (100 mg, 0.38 mmol), HBTU (59 mg, 0.42 mmol), HOBT (64 mg, 0.42 mmol) and DIEA (195 µl, 1.14 mmol) in NMP (1 ml) was added to 4-chlorobenzylamine (108 mg, 0.76 mmol) in NMP (0.5 ml). The reaction mixture was stirred for 18 h. The solvent was evaporated off and the residue was purified using preparative HPLC to give the title compound (60 mg, 41%).
¹H NMR (CDCl₃): δ 9.91 (1H, brs); 8.54 (1H, d); 7.53 (2H, d); 7.24 (4H, s); 7.13 (2H, d); 6.42 (1H, d); 4.53 (2H, d); 2.07 (3H, s).

Using the general method described in Example 1, the compounds of Examples 1.1 to 1.27 were prepared:

### Example 1.1 6-Methyl-N-[4-(methylsulfonyl)benzyl]-2-oxo-1-[3-(trifluoromethyl)phenyl]-1,2-dihydropyridine-3-carboxamide

¹H NMR (CDCl₃): δ 9.96 (1H, t); 8.57 (1H, d); 7.85 (2H, d); 7.80 (1H, d); 7.73 (1H, t); 7.50 (3H, brd); 7.42 (1H, d); 6.46 (1H, d); 4.65 (2H, d); 3.00 (3H, s); 2.07 (3H, s).

### Example 1.2 6-Methyl-N-(4-morpholin-4-ylbenzyl)-2-oxo-1-[3-(trifluoromethyl)phenyl]-1,2-dihydropyridine-3-carboxamide

¹H NMR (DMSO-d₆): 8 9.69 (1H, brt); 8.38 (1H, d); 7.89-7.87 (2H, m); 7.79 (1H, t); 7.70 (1H, d); 7.15 (2H, d); 6.87 (2H, d); 6.62 (1H, d); 4.36 (2H, d); 3.72-3.69 (4H, m) 3.05-3.03; (4H, m); 2.00 (3H, s).
APCI-MS m/z: 472 [MH⁺].

### Example 1.3 6-Methyl-N-[4-(methylsulfonyl)phenyl]-2-oxo-1-[3-(trifluoromethyl)phenyl]-1,2-dihydropyridine-3-carboxamide

¹H NMR (CDCl₃): 812.00 (1H, s); 8.66 (1H, d); 7.92-7.85 (5H, m); 7.79 (1H, t); 7.56 (1H, s); 7.49 (1H, d); 6.55 (1H, d); 3.04 (3H, s); 2.13 (3H, s).
APCI-MS m/z: 451[MH⁺].

### Example 1.4 N-[4-(Dimethylamino)benzyl]-6-methyl-2-oxo-1-[3-(trifluoromethyl)phenyl]-1,2-dihydropyridine-3-carboxamide

¹H NMR (CDCl₃): δ 9.67 (1H, brs); 8.57 (1H, d); 7.78 (1H, d); 7.71 (1H, t); 7.49 (1H, s); 7.41 (1H, d); 7.21 (2H, brd); 6.72 (2H, brs); 6.43 (1H, d); 4.50 (2H, d); 2.91 (6H, s); 2.05 (3H, s).

### Example 1.5 N-[4-(Aminosulfonyl)benzyl]-6-methyl-2-oxo-1-[3-(trifluoromethyl)phenyl]-1,2-dihydropyridine-3-carboxamide

¹H NMR (DMSO-d₆): δ 9.89 (1H, brs); 8.37 (1H, d); 7.91 (1H, s); 7.89 (1H, d); 7.80 (1H, t); 7.75 (2H, d); 7.72 (1H, d); 7.45 (2H, d); 7.27 (2H, s); 6.62 (1H, d); 4.54 (2H, s); 2.02 (3H, s).

### Example 1.6 N-(4-Methoxybenzyl)-6-methyl-2-oxo-1-[3-(trifluoromethyl)phenyl]-1,2-dihydropyridine-3-carboxamide

¹H NMR (CDCl₃): 8 9.72 (1H, brs); 8.59 (1H, d); 7.79 (1H, d); 7.72 (1H, t); 7.49 (1H, s); 7.42 (1H, d); 7.24 (2H, d); 6.82 (2H, d); 6.44 (1H, d); 4.52 (2H, d); 3.76 (3H, s); 2.05 (3H, s).

### Example 1.7 N-Benzyl-6-methyl-2-oxo-1-[3-(trifluoromethyl)phenyl]-1,2-dihydropyridine-3-carboxamide

¹H NMR (CDCl₃): δ 9.85 (1H, brs); 8.62 (1H, d); 7.81 (1H, d); 7.74 (1H, t); 7.52 (1H, s); 7.44 (1H, d); 7.36-7.21 (5H, m); 6.47 (1H, d); 4.61 (2H, d); 2.08 (3H, s).

### Example 1.8 N-(4-Chlorobenzyl)-1-(2-fluoro-5-methylphenyl)-6-methyl-2-oxo-1,2-dihydronyridine-3-carboxamide

¹H NMR (CD₃OD): δ 8.48 (1H, d); 7.40-7.36 (1H, m); 7.30 (4H, s); 7.25 (1H, t); 7.19 (1H, dd); 6.62 (1H, d); 4.56 (2H, q); 2.39 (3H, s); 2.13 (3H, s).

### Example 1.9 N-(3-Chlorobenzyl)-1-(2-fluoro-5-methylphenyl)-6-methyl-2-oxo-1,2-dihydropyridine-3-carboxamide

¹H NMR-(CD₃OD): δ 8.48 (1H, d); 7.40-7.36 (1H, m); 7.33-7.32 (1H, m); 7.29-7.22 (4H, m); 7.20 (1H, dd); 6.62 (1H, d); 4.57 (2H, q); 2.39 (3H, s); 2.13 (3H, s).

### Example 1.10 1-(2-Fluoro-5-methylphenyl)-N-(4-methoxybenzyl)-6-methyl-2-oxo-1,2-dihydropyridine-3-carboxamide

¹H NMR (CD₃OD): δ 8.48 (1H, d); 7.40-7.36 (1H, m); 7.27-7.21 (3H, m); 7.02 (1H, dd); 6.86 (2H, d); 6.62 (1H, d); 4.50 (2H, q); 3.75 (3H, s); 2.39 (3H, s); 2.12 (3H, s).
APCI-MS m/z: 381 [MH⁺].

### Example 1.11 N-(4-Methoxybenzyl)-1-(3-methoxyphenyl)-6-methyl-2-oxo-1,2-dihydropyridine-3-carboxamide

¹H NMR (CDCl₃): δ 9.86 (1H, brs); 8.54 (1H, d); 7.45 (1H, t); 7.23 (2H, d); 7.04-7.01 (1H, m); 6.80 (2H, d); 6.78-6.75 (1H, m); 6.70 (1H, t); 6.39 (1H, d); 4.51 (2H, d); 3.82 (3H, s); 3.76 (3H, s); 2.09 (3H, s).
APCI-MS m/z: 379 [MH⁺].

### Example 1.12 N-(3-Chlorobenzyl)-1-(3-methoxyphenyl)-6-methyl-2-oxo-1,2-dihydropyridine-3-carboxamide

¹H NMR (CDCl₃): δ 10.00 (1H, brs); 8.55 (1H, d); 7.47 (1H, t); 7.30 (1H, brs); 7.19 (3H, brs); 7.05-7.01 (1H, m); 6.80-6.75 (1H, m); 6.72 (1H, t); 6.41 (1H, d); 4.55 (2H, d); 3.83 (3H, s); 2.11 (3H, s).

### Example 1.13 N-(4-Chlorobenzyl)-1-(3-methoxyphenyl)-6-methyl-2-oxo-1,2-dihydropyridine-3-carboxamide

¹H NMR (CDCl₃): δ 10.00 (1H, brs); 8.56 (1H, d); 7.48 (1H, t); 7.28 (4H, s); 7.07-7.03 (1H, m); 6.81-6.77 (1H, m); 6.73 (1H, t); 6.41 (1H, d); 4.56 (2H, d); 3.85 (3H, s); 2.12 (3H, s).

### Example 1.14 N-[4-(Aminosulfonyl)benzyl]-1-(3-chlorophenyl)-6-methyl-2-oxo-1,2-dihydropyridine-3-carboxamide

¹H NMR (CDCl₃): δ 9.99 (1H, t); 8.55 (1H, d); 7.84 (2H, d); 7.53-7.49 (2H, m); 7.46 (1H, d); 7.25-7.24 (1H, m); 7.14-7.10 (1H, m); 6.44 (1H, d); 4.72 (2H, brs); 4.64 (2H, d); 2.10 (3H, s).

### Example 1.15 N-(4-Chlorobenzyl)-1-(3-chloro-4-methylphenyl)-6-methyl-2-oxo-1,2-dihydropyridine-3-carboxamide

¹H NMR (CDCl₃): δ 9.93 (1H, brs); 8.56 (1H, d); 7.44 (1H, d); 7.28 (4H, s); 7.24 (1H, d); 7.03 (1H, dd); 6.43 (1H, d); 4.56 (2H, d); 2.46 (3H, s); 2.12 (3H, s).

### Example 1.16 1-(3-Chloro-4-methylphenyl)-N-(4-methoxybenzyl)-6-methyl-2-oxo-1,2-dihydropyridine-3-carboxamide

¹H NMR (CDCl₃): δ 9.80 (1H, brs); 8.54 (1H, d); 7.40 (1H, d); 7.23 (2H, d); 7.20 (1H, d); 7.00 (1H, dd); 6.81 (2H, d); 6.39 (1H, d); 4.51 (2H, d); 3.76 (3H, s); 2.43 (3H, s); 2.09 (3H, s).

### Example 1.17 N-(4-Chlorobenzyl)-1-(2,3-dimethylphenyl)-6-methyl-2-oxo-1,2-dihydropyridine-3-carboxamide

¹H NMR (CDCl₃): δ 10.04 (1H, brs); 8.58 (1H, d); 7.31-7.24 (6H, m); 6.96-6.94 (1H, m); 6.45 (1H, d); 4.63-4.50 (2H, m); 2.38 (3H, s); 2.03 (3H, s); 1.95 (3H, s).

### Example 1.18 N-(4-Chlorobenzyl)-1-(3-chloro-4-fluorophenyl)-6-methyl-2-oxo-1,2-dihydropyridine-3-carboxamide

¹H NMR (CDCl₃): δ 9.83 (1H, brs); 8.57 (1H, d); 7.38-7.32 (2H, m); 7.27 (4H, s); 7.15-7.11 (1H, m); 6.45 (1H, d); 4.57 (2H, d); 2.12 (3H, s).
APCI-MS m/z: 405.1, 407 [MH⁺].

### Example 1.19 1-(3-Chloro-4-fluorophenyl)-N-(4-methoxybenzyl)-6-methyl-2-oxo-1,2-dihydropyridine-3-carboxamide

¹H NMR (CDCl₃): δ 9.72 (1H, brs); 8.58 (1H, d); 7.39-7.31 (2H, m); 7.26 (2H, d); 7.14-7.10 (1H, m); 6.84 (2H, d); 6.43 (1H, d); 4.54 (2H, d); 3.79 (3H, s); 2.11 (3H, s).

### Example 1.20 N-(4-Chlorobenzyl)-1-(3-ethylphenyl)-6-methyl-2-oxo-1,2-dihydropyridine-3-carboxamide

¹H NMR (CDCl₃): δ 10.03 (1H, brs); 8.56 (1H, d); 7.49 (1H, t); 7.36 (1H, d); 7.28 (4H, s); 7.02 (2H, d); 6.42 (1H, d); 4.61-4.50 (2H, m); 2.75 (2H, q); 2.09 (3H, s); 1.29 (3H, t).

### Example 1.21 1-(3-Bromophenyl)-N-(4-chlorobenzyl)-6-methyl-2-oxo-1,2-dihydropyridine-3-carboxamide

¹H NMR (CDCl₃): δ 9.86 (1H, brs); 8.55 (1H, d); 7.67-7.64 (1H, m); 7.45 (1H, t); 7.39 (1H, t); 7.25 (4H, s); 7.17-7.15 (1H, m); 6.42 (1H, d); 4.54 (2H, d); 2.09 (3H, s).
APCI-MS m/z: 431.1, 433 [MH⁺].

### Example 1.22 1-(3-Bromophenyl)-N-(4-methoxybenzyl)-6-methyl-2-oxo-1,2-dihydropyridine-3-carboxamide

¹H NMR (CDCl₃): δ 9.74 (1H, brs); 8.55 (1H, d); 7.65-7.63 (1H, m); 7.44 (1H, t); 7.38 (1H, t); 7.23 (2H, d); 7.16-7.14 (1H, m); 6.81 (2H, d); 6.40 (1H, d); 4.52 (2H, d); 3.76 (3H, s); 2.07 (3H, s).

### Example 1.23 N-(2,3-Dihydro-1-benzofuran-5-ylmethyl)-6-methyl-2-oxo-1-[3-(trifluoromethyl)phenyl]-1,2-dihydronyridine-3-carboxamide

¹H NMR (CDCl₃): δ 9.70 (1H, brs); 8.59 (1H, d); 7.79 (1H, d); 7.73 (1H, t); 7.50 (1H, s); 7.43 (1H, d); 7.17 (1H, s); 7.05 (1H, d); 6.69 (1H, d); 6.44 (1H, d); 4.56-4.50 (4H, m); 3.16 (2H, t); 2.06 (3H, s).
APCI-MS m/z: 429 [MH⁺].

### Example 1.24 6-Methyl-2-oxo-N-[3-(2-oxopyrrolidin-1-yl)propyl]-1-[3-(trifluoromethyl)phenyl]-1,2-dihydropyridine-3-carboxamide

¹H NMR (CDCl₃): δ 9.55 (1H, brs); 8.55 (1H, d); 7.82 (1H, d); 7.75 (1H, t); 7.52 (1H, s); 7.45 (1H, d); 6.45 (1H, d); 3.44-3.33 (6H, m); 2.38 (2H, t); 2.05-1.98 (2H, m); 2.08 (3H, s); 1.86-1.79 (2H, m).
APCI-MS m/z: 422 [MH⁺].

### Example 1.25 N-(4-Bromobenzyl)-6-methyl-2-oxo-1-[3-(trifluoromethyl)phenyl]-1,2-dihydropyridine-3-carboxamide

¹H NMR (CDCl₃): δ 9.84 (1H, brs); 8.58 (1H, d); 7.81 (1H, d); 7.73 (1H, t); 7.51 (1H, s); 7.43 (1H, d); 7.41 (2H, d); 7.20 (2H, d); 6.46 (1H, d); 4.59-4.49 (2H, m); 2.08 (3H, s).
APCI-MS m/z: 465.1,467 [MH⁺].

### Example 1.26 N-(4-Chlorophenyl)-6-methyl-2-oxo-1-[3-(trifluoromethyl)phenyl]-1,2-dihydropyridine-3-carboxamide

APCI-MS m/z: 407 [MH⁺].

### Example 1.27 6-Methyl-2-oxo-1-[3-(trifluoromethyl)phenyl]-1,2-dihydropyridine-3-carboxamide

APCI-MS m/z: 297 [MH⁺].

### Example 2 N-(4-Methoxybenzyl)-6-methyl-2-oxo-1-phenyl-1,2-dihydropyridine-3-carboxamide

### a) 6-Methyl-2-oxo-1-phenyl-1,2-dihydropyridine-3-carbonitrile

A mixture of cyanoacetanilide (0.80 g, 5 mmol), 4-methoxy-3-buten-2-one (1 g, 10 mmol) and 1,4-diazabicyclo[2,2,2]octane (0.55 g, 5 mmol) in diethyleneglycol monomethylether was heated to 125 °C for 5 h. The reaction mixture was partitioned between dichloromethane (100 ml) and 2M hydrochloric acid (100 ml). The organic layer was separated, washed with water, dried, filtered and evaporated. The residue was chromatographed on silica using heptane/ethyl acetate (1:1) as eluent, affording the title compound (660 mg, 63%).
¹H NMR (CDCl₃): δ 7.78 (1H, d); 7.52 (3H, m); 7.17 (2H, dd); 6.22 (1H, d); 2.06 (3H, s).

### b) 6-Methyl-2-oxo-1-phenyl-1,2-dihydropyridine-3-carboxylic acid

6-Methyl-2-oxo-1-phenyl-1,2-dihydropyridine-3-carbonitrile (300 mg, 1.4 mmol) was dissolved in 2.5M sulphuric acid (10 ml). The mixture was heated to 100 °C for 16 h. After cooling, the solution was poured into water and made alkaline with 5M sodium hydroxide solution. The water phase was washed with dichloromethane, then acidified to pH 2-3 using 2M hydrochloric acid. The acidified water phase was extracted with dichloromethane, dried, filtered and evaporated to give the title compound (300 mg, 92%).
¹H NMR (CDCl₃): δ 13.96 (1H, s); 8.50 (1H, d); 7.59 (3H, m); 7.23 (2H, dd); 6.53 (1H, d); 2.13 (3H, s).

### c) N-(4-Methoxybenzyl)-6-methyl-2-oxo-1-phenyl-1,2-dihydropyridine-3-carboxamide

The title compound was prepared from 6-methyl-2-oxo-1-phenyl-1,2-dihydropyridine-3-carboxylic acid and 4-methoxybenzylamine by a method analogous to that described in Example 1 step (d).
¹H NMR (CDCl₃): δ 9.87 (1H, brs); 8.56 (1H, brd); 7.52 (3H, m); 7.23 (2H, d); 7.18 (2H, d); 6.79 (2H, d); 6.40 (1H, d); 4.51 (2H, d); 3.75 (3H, s); 2.04 (3H, s).

The compounds of Examples 2.1 to 2.174 were prepared by a method analogous to that described in Example 1 or 2.

### Example 2.1 N-(4-Chlorobenzyl)-6-methyl-2-oxo-1-phenyl-1,2-dihydropyridine-3-carboxamide

¹H NMR (CDCl₃): δ 9.96 (1H, s); 8.54 (1H, d); 7.54 (3H, m); 7.23 (4H, s); 7.18 (2H, d); 6.41 (1H, d); 4.54 (2H, d); 2.06 (3H, s).

### Example 2.2 N-(4-Chlorobenzyl)-1-(3,5-dimethylphenyl)-6-methyl-2-oxo-1,2-dihydropyridine-3-carboxamide

¹H NMR (CDCl₃): δ 10.01 (1H, brs); 8.52 (1H, d); 7.23 (4H, s); 7.11 (1H, s); 6.78 (2H, s); 6.38 (1H, d); 4.53 (2H, d); 2.36 (6H, s); 2.07 (3H, s).

### Example 2.3 N-[4-(Aminosulfonyl)benzyl]-1-(3,5-dimethylphenyl)-6-methyl-2-oxo-1,2-dihydropyridine-3-carboxamide

¹H NMR (CDCl₃): δ 10.14 (1H, brs); 8.51 (1H, d); 7.82 (2H, d); 7.45 (2H, d); 7.12 (1H, s); 6.79 (2H, s); 6.41 (1H, d); 4.72 (2H, s); 4.62 (2H, d); 2.36 (6H, s); 2.09 (3H, s).

### Example 2.4 1-(3,5-Dimethylphenyl)-N-(4-methoxybenzyl)-6-methyl-2-oxo-1,2-dihydropyridine-3-carboxamide

¹H NMR (CDCl₃): δ 9.90 (1H, brs); 8.54 (1H, d); 7.24 (2H, s); 7.11 (1H, s); 6.81 (2H, d); 6.79 (2H, s); 6.38 (1H, d); 4.52 (2H, d); 3.77 (3H, s); 2.37 (6H, s); 2.07 (3H, s).

### Example 2.5 N-Benzyl-1-(3,5-dimethylphenyl)-6-methyl-2-oxo-1,2-dihydropyridine-3-carboxamide

¹H NMR (CDCl₃): δ 9.98 (1H, brs); 8.57 (1H, d); 7.36-7.19 (5H, m); 7.13 (1H, s); 6.82 (2H, s); 6.41 (1H, d); 4.61 (2H, d); 2.39 (6H, s); 2.10 (3H, s).

### Example 2.6 N-(4-Chlorobenzyl)-6-methyl-1-(3-methylphenyl)-2-oxo-1,2-dihydropyridine-3-carboxamide

¹H NMR (CD₃OD): δ 8.46 (1H, d); 7.47 (1H, t); 7.36 (1H, d); 7.30 (4H, s); 7.10 (1H, s); 7.06 (1H, d); 6.60 (1H, d); 4.56 (2H, s); 2.42 (3H, s); 2.09 (3H, s).
APCI-MS m/z: 367 [MH⁺].

### Example 2.7 N-(4-Methoxybenzyl)-6-methyl-1-(3-methylphenyl)-2-oxo-1,2-dihydropyridine-3-carboxamide

¹H NMR (CD₃OD): δ 8.45 (1H, d); 7.44 (1H, t); 7.33 (1H, d); 7.22 (2H, d); 7.07 (1H, s); 7.03 (1H, d); 6.84 (2H, d); 6.58 (1H, d); 4.49 (2H, s); 3.74 (3H, s); 2.41 (3H, s); 2.07 (3H, s).
APCI-MS m/z: 363 [MH⁺].

### Example 2.8 N-(3-Chlorobenzyl)-6-methyl-1-(3-methylphenyl)-2-oxo-1,2-dihydropyridine-3-carboxamide

¹H NMR (CD₃OD): δ 8.46 (1H, d); 7.46 (1H, t); 7.35 (1H, d); 7.32-7.21 (4H, m); 7.10 (1H, s); 7.06 (1H, d); 6.60 (1H, d); 4.56 (2H, s); 2.42 (3H, s); 2.09 (3H, s).

### Example 2.9 N-(4-Chlorobenzyl)-1-(3-chlorophenyl)-6-methyl-2-oxo-1,2-dihydropyridine-3-carboxamide

¹H NMR (DMSO-d₆): δ 9.86 (1H, t); 8.35 (1H, d); 7.59-7.58 (3H, m); 7.39-7.29 (5H, m); 6.60 (1H, d); 4.46 (2H, d); 2.04 (3H, s).
APCI-MS m/z: 387.1,389 [MH⁺].

### Example 2.10 N-(3-Chlorobenzyl)-1-(3-chlorophenyl)-6-methyl-2-oxo-1,2-dihydropyridine-3-carboxamide

¹H NMR (DMSO-d₆): δ 9.88 (1H, t); 8.36 (1H, d); 7.59-7.58 (3H, m); 7.39-7.32 (4H, m); 7.25 (1H, d); 6.60 (1H, d); 4.48 (2H, d); 2.04 (3H, s).
APCI-MS m/z: 387.1,389 [MH⁺].

### Example 2.11 1-(3-Chlorophenyl)-N-(4-methoxybenzyl)-6-methyl-2-oxo-1,2-dihydropyridine-3-carboxamide

¹H NMR (DMSO-d₆): δ 9.73 (1H, t); 8.34 (1H, d); 7.56 (2H, d); 7.34-7.31 (1H, m); 7.19 (2H, d); 6.85 (2H, d); 6.58 (1H, d); 6.60 (1H, d); 4.38 (2H, d); 3.69 (3H, s); 2.01 (3H, s). APCI-MS m/z: 383 [MH⁺].

### Example 2.12 Methyl 4-[({[1-(3-chlorophenyl)-6-methyl-2-oxo-1,2-dihydropyridin-3-yl]carbonyl}amino)methyl]benzoate

¹H NMR (DMSO-d₆): δ 9.92 (1H, t); 8.36 (1H, d); 7.91 (2H, d); 7.60-7.58 (3H, m); 7.41 (2H, d); 7.38-7.35 (1H, m); 6.60 (1H, d); 4.56 (2H, d); 3.83 (3H, s); 2.04 (3H, s).
APCI-MS m/z: 411 [MH⁺].

### Example 2.13 4-[({[1-(3-Chlorophenyl)-6-methyl-2-oxo-1,2-dihydropyridin-3-yl]carbonyl}amino)methyl]benzoic acid

¹H NMR (DMSO-d₆): δ 12.82 (1H, brs); 9.91 (1H, t); 8.36 (1H, d); 7.88 (2H, d); 7.59-7.58 (3H, m); 7.39 (2H, d); 7.38-7.35 (1H, m); 6.60 (1H, d); 4.55 (2H, d); 2.04 (3H, s).
APCI-MS m/z: 397 [MH⁺].

### Example 2.14 1-(3-Cyanophenyl)-N-(cyclohexylmethyl)-6-methyl-2-oxo-1,2-dihydropyridine-3-carboxamide

APCI-MS m/z: 350 [MH⁺].

### Example 2.15 1-(3-Cyanophenyl)-N-(2-furylmethyl)-6-methyl-2-oxo-1,2-dihydropyridine-3-carboxamide

APCI-MS m/z: 334 [MH⁺].

### Example 2.16 1-(3-Cyanophenyl)-6-methyl-2-oxo-N-(pyridin-3-ylmethyl)-1,2-dihydropyridine-3-carboxamide

APCI-MS m/z: 345 [MH⁺].

### Example 2.17 N-Benzyl-1-(3-cyanophenyl)-6-methyl-2-oxo-1,2-dihydropyridine-3-carboxamide

APCI-MS m/z: 344 [MH⁺].

### Example 2.18 1-(3-Cyanophenyl)-N-2,3-dihydro-1H-inden-1-yl-6-methyl-2-oxo-1,2-dihydropyridine-3-carboxamide

APCI-MS m/z: 370 [MH⁺].

### Example 2.19 1-(3-Cyanophenyl)-N-(2-methoxybenzyl)-6-methyl-2-oxo-1,2-dihydropyridine-3-carboxamide

APCI-MS m/z: 374 [MH⁺].

### Example 2.20 1-(3-Cyanophenyl)-6-methyl-2-oxo-N-(3,4,5-trimethoxybenzyl)-1,2-dihydropyridine-3-carboxamide

APCI-MS m/z: 434 [MH⁺].

### Example 2.21 1-(3-Cyanophenyl)-N-(2,5-dimethoxybenzyl)-6-methyl-2-oxo-1,2-dihydronyridine-3-carboxamide

APCI-MS m/z: 404 [MH⁺].

### Example 2.22 1-(3-Cyanophenyl)-N-(3,4-dimethoxybenzyl)-6-methyl-2-oxo-1,2-dihydropyridine-3-carboxamide

APCI-MS m/z: 404 [MH⁺].

### Example 2.23 1-(3-Cyanophenyl)-N-[(1-ethylpyrrolidin-2-yl)methyl]-6-methyl-2-oxo-1,2-dihydropyridine-3-carboxamide

APCI-MS m/z: 365 [MH⁺].

### Example 2.24 N-(4-Chlorobenzyl)-1-(3-cyanophenyl)-6-methyl-2-oxo-1,2-dihydropyridine-3-carboxamide

APCI-MS m/z: 378 [MH⁺].

### Example 2.25 1-(3-Cyanophenyl)-N-(4-methoxybenzyl)-6-methyl-2-oxo-1,2-dihydropyridine-3-carboxamide

APCI-MS m/z: 374 [MH⁺].

### Example 2.26 N-(3-Chlorobenzyl)-1-(3-cyanophenyl)-6-methyl-2-oxo-1,2-dihydronyridine-3-carboxamide

APCI-MS m/z: 378 [MH⁺].

### Example 2.27 1-(3-Cyanophenyl)-6-methyl-2-oxo-N-(thien-2-ylmethyl)-1,2-dihydropyridine-3-carboxamide

APCI-MS m/z: 350 [MH⁺].

### Example 2.28 1-(3-Cyanophenyl)-N-(cyclopropylmethyl)-6-methyl-2-oxo-1,2-dihydropyridine-3-carboxamide

APCI-MS m/z: 308 [MH⁺].

### Example 2.29 1-(3-Cyanonhenyl)-N-(3-methoxybenzyl)-6-methyl-2-oxo-1,2-dihydropyridine-3-carboxamide

APCI-MS m/z: 374 [MH⁺].

### Example 2.30 1-(3-Cyanophenyl)-6-methyl-2-oxo-N-(pyridin-4-ylmethyl)-1,2-dihydropyridine-3-carboxamide

APCI-MS m/z: 345 [MH⁺].

### Example 2.31 1-(3-Cyanophenyl)-N-[2-(3,4-dimethoxyphenyl)ethyl]-6-methyl-2-oxo-1,2-dihydropyridine-3-carboxamide

APCI-MS m/z: 417 [MH⁺].

### Example 2.32 1-(3-Cyanophenyl)-6-methyl-N-[2-(1-methylpyrrolidin-2-yl)ethyl]-2-oxo-1,2-dihydropyridine-3-carboxamide

APCI-MS m/z: 365 [MH⁺].

### Example 2.33 N-[2-(3-Chlorophenyl)ethyl]-1-(3-cyanophenyl)-6-methyl-2-oxo-1,2-dihydropyridine-3-carboxamide

APCI-MS m/z: 392 [MH⁺].

### Example 2.34 1-(3-Cyanophenyl)-6-methyl-2-oxo-N-(2-pyridin-2-ylethyl)-1,2-dihydropyridine-3-carboxamide

APCI-MS m/z: 359 [MH⁺].

### Example 2.35 N-[2-(4-Chlorophenyl)ethyl]-1-(3-cyanophenyl)-6-methyl-2-oxo-1,2-dihydropyridine-3-carboxamide

APCI-MS m/z: 392 [MH⁺].

### Example 2.36 1-(3-Cyanophenyl)-N-[2-(2-methoxyphenyl)ethyl]-6-methyl-2-oxo-1,2-dihydropyridine-3-carboxamide

APCI-MS m/z: 388 [MH⁺].

### Example 2.37 N-[2-(2-Chlorophenyl)ethyl]-1-(3-cyanophenyl)-6-methyl-2-oxo-1,2-dihydropyridine-3-carboxamide

APCI-MS m/z: 392 [MH⁺].

### Example 2.38 1-(3-Cyanophenyl)-N-[2-(3-methoxyphenyl)ethyl]-6-methyl-2-oxo-1,2-dihydropyridine-3-carboxamide

APCI-MS m/z: 388 [MH⁺].

### Example 2.39 1-(3-Cyanophenyl)-N-[2-(4-fluorophenyl)ethyl]-6-methyl-2-oxo-1,2-dihydropyridine-3-carboxamide

APCI-MS m/z: 376 [MH⁺].

### Example 2.40 1-(3-Cyanophenyl)-N-[2-(2,4-dichlorophenyl)ethyl]-6-methyl-2-oxo-1,2-dihydropyridine-3-carboxamide

APCI-MS m/z: 426 [MH⁺].

### Example 2.41 1-(3-Cyanophenyl)-N-[2-(3-fluorophenyl)ethyl]-6-methyl-2-oxo-1,2-dihydropyridine-3-carboxamide

APCI-MS m/z: 376 [MH⁺].

### Example 2.42 1-(3-Cyanophenyl)-N-[2-(2-fluorophenyl)ethyl]-6-methyl-2-oxo-1,2-dihydropyridine-3-carboxamide

APCI-MS m/z: 376 [MH⁺].

### Example 2.43 1-(3-Cyanophenyl)-N-(2-cyclohex-1-en-1-ylethyl)-6-methyl-2-oxo-1,2-dihydropyridine-3-carboxamide

APCI-MS m/z: 362 [MH⁺].

### Example 2.44 N-[2-(4-Bromophenyl)ethyl]-1-(3-cyanophenyl)-6-methyl-2-oxo-1,2-dihydropyridine-3-carboxamide

APCI-MS m/z: 438 [MH⁺].

### Example 2.45 N-(3-Bromobenzyl)-1-(3-cyanophenyl)-6-methyl-2-oxo-1,2-dihydropyridine-3-carboxamide

APCI-MS m/z: 424 [MH⁺].

### Example 2.46 N-(4-Bromobenzyl)-1-(3-cyanophenyl)-6-methyl-2-oxo-1,2-dihydropyridine-3-carboxamide

APCI-MS m/z: 424 [MH⁺].

### Example 2.47 N-(2-Bromobenzyl)-1-(3-cyanophenyl)-6-methyl-2-oxo-1,2-dihydropyridine-3-carboxamide

APCI-MS m/z: 424 [MH⁺].

### Example 2.48 1-(3-Cyanophenyl)-N-(3,4-dihydro-2H-pyran-2-ylmethyl)-6-methyl-2-oxo-1,2-dihydropyridine-3-carboxamide

APCI-MS m/z: 350 [MH⁺].

### Example 2.49 1-(3-Cyanophenyl)-6-methyl-N-(4-methylbenzyl)-2-oxo-1,2-dihydropyridine-3-carboxamide

APCI-MS m/z: 358 [MH⁺].

### Example 2.50 1-(3-Cyanophenyl)-6-methyl-N-(1-naphthylmethyl)-2-oxo-1,2-dihydropyridine-3-carboxamide

APCI-MS m/z: 394 [MH⁺].

### Example 2.51 1-(3-Cyanophenyl)-N-(2-ethoxybenzyl)-6-methyl-2-oxo-1,2-dihydropyridine-3-carboxamide

APCI-MS m/z: 388 [MH⁺].

### Example 2.52 1-(3-Cyanophenyl)-6-methyl-N-[4-(methylsulfonyl)benzyl]-2-oxo-1,2-dihydropyridine-3-carboxamide

APCI-MS m/z: 422 [MH⁺].

### Example 2.53 1-(3-Cyanophenyl)-6-methyl-N-(3-methylbenzyl)-2-oxo-1,2-dihydropyridine-3-carboxamide

APCI-MS m/z: 358 [MH⁺].

### Example 2.54 1-(3-Cyanophenyl)-N-(4-fluorobenzyl)-6-methyl-2-oxo-1,2-dihydropyridine-3-carboxamide

APCI-MS m/z: 362 [MH⁺].

### Example 2.55 N-(1,3-Benzodioxol-5-ylmethyl)-1-(3-cyanophenyl)-6-methyl-2-oxo-1 2-dihydropyridine-3-carboxamide

APCI-MS m/z: 388 [MH⁺].

### Example 2.56 1-(3-Cyanophenyl)-N-(2,4-dichlorobenzyl)-6-methyl-2-oxo-1,2-dihydropyridine-3-carboxamide

APCI-MS m/z: 412 [MH⁺].

### Example 2.57 1-(3-Cyanophenyl)-6-methyl-N-(2-methylbenzyl)-2-oxo-1,2-dihydropyridine-3-carboxamide

APCI-MS m/z: 358 [MH⁺].

### Example 2.58 1-(3-Cyanophenyl)-N-(3,4-difluorobenzyl)-6-methyl-2-oxo-1,2-dihydropyridine-3-carboxamide

APCI-MS m/z: 380 [MH⁺].

### Example 2.59 1-(3-Cyanophenyl)-N-(3,4-dichlorobenzyl)-6-methyl-2-oxo-1,2-dihydropyridine-3-carboxamide

APCI-MS m/z: 412 [MH⁺].

### Example 2.60 1-(3-Cyanophenyl)-6-methyl-N-[(5-methyl-2-furyl)methyl]-2-oxo-1,2-dihydropyridine-3-carboxamide

APCI-MS m/z: 348 [MH⁺].

### Example 2.61 1-(3-Cyanophenyl)-6-methyl-2-oxo-N-1,2,3,4-tetrahydronaphthalen-1-yl-1,2-dihydropyridine-3-carboxamide

APCI-MS m/z: 384 [MH⁺].

### Example 2.62 1-(3-Cyanophenyl-N-(2,3-dimethoxybenzyl)-6-methyl-2-oxo-1,2-dihydropyridine-3-carboxamide

APCI-MS m/z: 404 [MH⁺].

### Example 2.63 1-(3-Cyanophenyl)-N-(3,5-dimethoxybenzyl)-6-methyl-2-oxo-1,2-dihydropyridine-3-carboxamide

APCI-MS m/z: 404 [MH⁺].

### Example 2.64 1-(3-Cyanophenyl)-N-[1-(4-fluorophenyl)ethyl]-6-methyl-2-oxo-1,2-dihydropyridine-3-carboxamide

APCI-MS m/z: 376 [MH⁺].

### Example 2.65 N-[1-(4-Chlorophenyl)ethyl]-1-(3-cyanophenyl)-6-methyl-2-oxo-1,2-dihydropyridine-3-carboxamide

APCI-MS m/z: 392 [MH⁺].

### Example 2.66 1-(3-Cyanophenyl)-N-(2,5-difluorobenzyl)-6-methyl-2-oxo-1,2-dihydropyridine-3-carboxamide

APCI-MS m/z: 380 [MH⁺].

### Example 2.67 1-(3-Cyanophenyl)-N-(2,3-dihydro-1-benzofuran-5-ylmethyl)-6-methyl-2-oxo-1,2-dihydropyridine-3-carboxamide

APCI-MS m/z: 386 [MH⁺].

### Example 2.68 Methyl 4-[({[1-(3-cyanophenyl)-6-methyl-2-oxo-1,2-dihydropyridin-3-yl]carbonyl}amino)methyl]benzoate

APCI-MS m/z: 402 [MH⁺].

### Example 2.69 1-(3-Cyanophenyl)-6-methyl-2-oxo-N-(4-phenoxybenzyl)-1,2-dihydropyridine-3-carboxamide

APCI-MS m/z: 436 [MH⁺].

### Example 2.70 1-(3-Cyanophenyl)-N-[(1S)-2,3-dihydro-1H-inden-1-yl]-6-methyl-2-oxo-1,2-dihydropyridine-3-carboxamide

APCI-MS m/z: 370 [MH⁺].

### Example 2.71 1-(3-Cyanophenyl)-6-methyl-2-oxo-N-(thien-3-ylmethyl)-1,2-dihydropyridine-3-carboxamide

APCI-MS m/z: 350 [MH⁺].

### Example 2.72 1-(3-Cyanophenyl)-6-methyl-N-[(5-methylisoxazol-3-yl)methyl]-2-oxo-1,2-dihydropyridine-3-carboxamide

APCI-MS m/z: 349 [MH⁺].

### Example 2.73 1-(3-Cyanophenyl)-N-[(2,5-dimethyl-3-furyl)methyl]-6-methyl-2-oxo-1,2-dihydropyridine-3-carboxamide

APCI-MS m/z: 362 [MH⁺].

### Example 2.74 1-(3-Cyanophenyl)-N-(3-furylmethyl)-6-methyl-2-oxo-1,2-dihydropyridine-3-carboxamide

APCI-MS m/z: 334 [MH⁺].

### Example 2.75 1-(3-Cyanophenyl)-6-methyl-2-oxo-N-[4-(1H-pyrazol-1-yl)benzyl]-1,2-dihydropyridine-3-carboxamide

APCI-MS m/z: 410 [MH⁺].

### Example 2.76 1-(3-Cyanonhenyl)-6-methyl-2-oxo-N-(4-thien-2-ylbenzyl)-1,2-dihydropyridine-3-carboxamide

APCI-MS m/z: 426 [MH⁺].

### Example 2.77 N-[4-(Aminosulfonyl)benzyl]-1-(3-cyanophenyl)-6-methyl-2-oxo 1,2-dihydropyridine-3-carboxamide

APCI-MS m/z: 423 [MH⁺].

### Example 2.78 N-[2-(1,3-Benzodioxol-5-yl)ethyl]-1-(3-cyanophenyl)-6-methyl-2-oxo-1,2-dihydropyridine-3-carboxamide

APCI-MS m/z: 402 [MH⁺].

### Example 2.79 1-(3-Cyanophenyl)-6-methyl-2-oxo-N-(2-thien-2-ylethyl)-1,2-dihydropyridine-3-carboxamide

APCI-MS m/z: 364 [MH⁺].

### Example 2.80 1-(3-Cyanophenyl)-N-[2-(2,4-dimethylphenyl)ethyl]-6-methyl-2-oxo-1,2-dihydropyridine-3-carboxamide

APCI-MS m/z: 386 [MH⁺].

### Example 2.81 1-(3-Cyanophenyl)-6-methyl-N-[2-(4-methylphenyl)ethyl]-2-oxo-1,2-dihydropyridine-3-carboxamide

APCI-MS m/z: 372 [MH⁺].

### Example 2.82 N-{2-[4-(Aminosulfonyl)phenlyl]ethyl}-1-(3-cyanophenyl)-6-methyl-2-oxo-1,2-dihydropyridine-3-carboxamide

APCI-MS m/z: 437 [MH⁺].

### Example 2.83 1-(3-Cyanophenyl)-6-methyl-2-oxo-N-[(1S)-1-phenylethyl]-1,2-dihydropyridine-3-carboxamide

APCI-MS m/z: 358 [MH⁺].

### Example 2.84 N-(Cyclohexylmethyl)-6-methyl-2-oxo-1-[3-(trifluoromethyl)phenyl]-1,2-dihydropyridine-3-carboxamide

APCI-MS m/z: 393 [MH⁺].

### Example 2.85 N-(2-Furylmethyl)-6-methyl-2-oxo-1-[3-(trifluoromethyl)phenyl]-1,2-dihydropyridine-3-carboxamide

APCI-MS m/z: 377 [MH⁺].

### Example 2.86 6-Methyl-2-oxo-N-(pyridin-3-ylmethyl)-1-[3-(trifluoromethyl)phenyl]-1,2-dihydropyridine-3-carboxamide

APCI-MS m/z: 388 [MH⁺].

### Example 2.87 N-2,3-Dihydro-1H-inden-1-yl-6-methyl-2-oxo-1-[3-(trifluoromethyl)phenyl]-1,2-dihydropyridine-3-carboxamide

APCI-MS m/z: 413 [MH⁺].

### Example 2.88 N-(2-Methoxybenzyl)-6-methyl-2-oxo-1-[3-(trifluoromethyl)phenyl]-1,2-dihydropyridine-3-carboxamide

APCI-MS m/z: 417 [MH⁺].

### Example 2.89 6-Methyl-2-oxo-N-(tetrahydrofuran-2-ylmethyl)-1-[3-(trifluoromethyl)phenyl]-1,2-dihydropyridine-3-carboxamide

APCI-MS m/z: 381 [MH⁺].

### Example 2.90 6-Methyl-2-oxo-1-[3-(trifluoromethyl)phenyl]-N-(3,4,5-trimethoxybenzyl)-1,2-dihydropyridine-3-carboxamide

APCI-MS m/z: 477 [MH⁺].

### Example 2.91 N-(3-Fluorobenzyl)-6-methyl-2-oxo-1-[3-(trifluoromethyl)phenyl]-1,2-dihydropyridine-3-carboxamide

APCI-MS m/z: 405 [MH⁺].

### Example 2.92 N-(2,5-Dimethoxybenzyl)-6-methyl-2-oxo-1-[3-(trifluoromethyl)phenyl]-1,2-dihydropyridine-3-carboxamide

APCI-MS m/z: 447 [MH⁺].

### Example 2.93 N-[(1-Ethylpyrrolidin-2-yl)methyl]-6-methyl-2-oxo-1-[3-(trifluoromethyl)phenyl]-1,2-dihydropyridine-3-carboxamide

APCI-MS m/z: 408 [MH⁺].

### Example 2.94 N-(2-Chlorobenzyl)-6-methyl-2-oxo-1-[3-(trifluoromethyl)phenyl]-1,2-dihydropyridine-3-carboxamide

APCI-MS m/z: 421 [MH⁺].

### Example 2.95 N-(4-Chlorobenzyl)-6-methyl-2-oxo-1-[3-(trifluoromethyl)phenyl]-1,2-dihydropyridine-3-carboxamide

APCI-MS m/z: 421 [MH⁺].

### Example 2.96 N-(3-Chlorobenzyl)-6-methyl-2-oxo-1-[3-(trifluoromethyl)phenyl]-1,2-dihydropyridine-3-carboxamide

APCI-MS m/z: 421 [MH⁺].

### Example 2.97 6-Methyl-2-oxo-N-(thien-2-ylmethyl)-1-[3-(trifluoromethyl)phenyl]-1,2-dihydropyridine-3-carboxamide

APCI-MS m/z: 393 [MH⁺].

### Example 2.98 N-(Cyclopropylmethyl)-6-methyl-2-oxo-1-[3-(trifluoromethyl)phenyl]-1,2-dihydropyridine-3-carboxamide

APCI-MS m/z: 351 [MH⁺].

### Example 2.99 N-(3-Methoxybenzyl)-6-methyl-2-oxo-1-[3-(trifluoromethyl)phenyl]-1,2-dihydropyridine-3-carboxamide

APCI-MS m/z: 417 [MH⁺].

### Example 2.100 6-Methyl-2-oxo-N-(pyridin-4-ylmethyl)-1-[3-(trifluoromethyl)phenyl]-1,2-dihydropyridine-3-carboxamide

APCI-MS m/z: 388 [MH⁺].

### Example 2.101 N-[2-(3,4-Dimethoxyphenyl)ethyl]-6-methyl-2-oxo-1-[3-(trifluoromethyl)phenyl]-1,2-dihydropyridine-3-carboxamide

APCI-MS m/z: 461 [MH⁺].

### Example 2.102 N-[2-(4-Methoxyphenyl)ethyl]-6-methyl-2-oxo-1-[3-(trifluoromethyl)phenyl]-1,2-dihydropyridine-3-carboxamide

APCI-MS m/z: 431 [MH⁺].

### Example 2.103 6-Methyl-2-oxo-N-(2-phenylethyl)-1-[3-(trifluoromethyl)phenyl]-1,2-dihydropyridine-3-carboxamide

APCI-MS m/z: 401 [MH⁺].

### Example 2.104 6-Methyl-N-[2-(1-methylpyrrolidin-2-yl)ethyl]-2-oxo-1-[3-(trifluoromethyl)phenyl]-1,2-dihydropyridine-3-carboxamide

APCI-MS m/z: 408 [MH⁺].

### Example 2.105 N-[2-(3-Chlorophenyl)ethyl]-6-methyl-2-oxo-1-[3-(trifluoromethyl)phenyl]-1,2-dihydropyridine-3-carboxamide

APCI-MS m/z: 435 [MH⁺].

### Example 2.106 6-Methyl-2-oxo-N-(2-pyridin-2-ylethyl)-1-[3-(trifluoromethyl)phenyl]-1,2-dihydropyridine-3-carboxamide

APCI-MS m/z: 402 [MH⁺].

### Example 2.107 N-[2-(2-Methoxyphenyl)ethyl]-6-methyl-2-oxo-1-[3-(trifluoromethyl)phenyl]-1,2-dihydropyridine-3-carboxamide

APCI-MS m/z: 431 [MH⁺].

### Example 2.108 N-[2-(2-Chlorophenyl)ethyl]-6-methyl-2-oxo-1-[3-(trifluoromethyl)phenyl]-1,2-dihydropyridine-3-carboxamide

APCI-MS m/z: 435 [MH⁺].

### Example 2.109 N-[2-(3-Methoxyphenyl)ethyl]-6-methyl-2-oxo-1-[3-(trifluoromethyl)phenyl]-1,2-dihydropyridine-3-carboxamide

APCI-MS m/z: 431 [MH⁺].

### Example 2.110 N-[2-(4-Fluorophenyl)ethyl]-6-methyl-2-oxo-1-[3-(trifluoromethyl)phenyl]-1,2-dihydropyridine-3-carboxamide

APCI-MS m/z: 419 [MH⁺].

### Example 2.111 N-[2-(2,4-Dichlorophenyl)ethyl]-6-methyl-2-oxo-1-[3-(trifluoromethyl)phenyl]-1,2-dihydropyridine-3-carboxamide

APCI-MS m/z: 469 [MH⁺].

### Example 2.112 N-[2-(3-Fluorophenyl)ethyl]-6-methyl-2-oxo-1-[3-(trifluoromethyl)phenyl]-1,2-dihydropyridine-3-carboxamide

APCI-MS m/z:419 [MH⁺].

### Example 2.113 N-[2-(2-Fluorophenyl)ethyl]methyl-2-oxo-1-[3-(trifluoromethyl)phenyl]-1,2-dihydropyridine-3-carboxamide

APCI-MS m/z: 418 [MH⁺].

### Example 2.114 N-(2-Cyclohex-1-en-1-ylethyl)-6-methyl-2-oxo-1-[3-(trifluoromethyl)phenyl]-1,2-dihydropyridine-3-carboxamide

APCI-MS m/z: 405 [MH⁺].

### Example 2.115 N-[2-(4-Bromophenyl)ethyl]-6-methyl-2-oxo-1-[3-(trifluoromethyl)phenyl]-1,2-dihydropyridine-3-carboxamide

APCI-MS m/z: 481 [MH⁺].

### Example 2.116 6-Methyl-2-oxo-N-[(1S)-1-phenylethyl]-1-[3-(trifluoromethyl)phenyl]-1,2-dihydropyridine-3-carboxamide

APCI-MS m/z: 401 [MH⁺].

### Example 2.117 N-(3-Bromobenzyl)-6-methyl-2-oxo-1-[3-(trifluoromethyl)phenyl]-1,2-dihydropyridine-3-carboxamide

APCI-MS m/z: 467 [MH⁺].

### Example 2.118 N-(4-Bromobenzyl)-6-methyl-2-oxo-1-[3-(trifluoromethyl)phenyl]-1,2-dihydropyridine-3-carboxamide

APCI-MS m/z: 467 [MH⁺].

### Example 2.119 N-(2-Bromobenzyl)-6-methyl-2-oxo-1-[3-(trifluoromethyl)phenyl]-1,2-dihydropyridine-3-carboxamide

APCI-MS m/z: 467 [MH⁺].

### Example 2.120 N-(3,4-Dihydro-2H-pyran-2-ylmethyl)-6-methyl-2-oxo-1-[3-(trifluoromethyl)phenyl]-1,2-dihydropyridine-3-carboxamide

APCI-MS m/z: 393 [MH⁺].

### Example 2.121 6-Methyl-N-(4-methylbenzyl)-2-oxo-1-[3-(trifluoromethyl)phenyl]-1,2-dihydropyridine-3-carboxamide

APCI-MS m/z: 401 [MH⁺].

### Example 2.122 6-Methyl-N-(1-naphthylmethyl)-2-oxo-1-[3-(trifluoromethyl)phenyl]-1,2-dihydropyridine-3-carboxamide

APCI-MS m/z: 437 [MH⁺].

### Example 2.123 N-(2-Ethoxybenzyl)-6-methyl-2-oxo-1-[3-(trifluoromethyl)phenyl]-1,2-dihydropyridine-3-carboxamide

APCI-MS m/z: 431 [MH⁺].

### Example 2.124 6-Methyl-N-(3-methylbenzyl)-2-oxo-1-[3-(trifluoromethyl)phenyl]-1,2-dihydropyridine-3-carboxamide

APCI-MS m/z: 401 [MH⁺].

### Example 2.125 N-(4-Fluorobenzyl)-6-methyl-2-oxo-1-[3-(trifluoromethyl)phenyl]-1,2-dihydropyridine-3-carboxamide

APCI-MS m/z: 405 [MH⁺].

### Example 2.126 N-(1,3-Benzodioxol-5-ylmethyl)-6-methyl-2-oxo-1-[3-(trifluoromethyl)phenyl]-1,2-dihydropyridine-3-carboxamide

APCI-MS m/z: 431 [MH⁺].

### Example 2.127 N-(2,4-Dichlorobenzyl)-6-methyl-2-oxo-1-[3-(trifluoromethyl)phenyl]-1,2-dihydropyridine-3-carboxamide

APCI-MS m/z: 456 [MH⁺].

### Example 2.128 6-Methyl-N-(2-methylbenzyl)-2-oxo-1-[3-(trifluoromethyl)phenyl]-1,2-dihydropyridine-3-carboxamide

APCI-MS m/z: 401 [MH⁺].

### Example 2.129 N-(3,4-Difluorobenzyl)-6-methyl-2-oxo-1-[3-(trifluoromethyl)phenyl]-1,2-dihydropyridine-3-carboxamide

APCI-MS m/z: 423 [MH⁺].

### Example 2.130 N-(2-Fluorobenzyl)-6-methyl-2-oxo-1-[3-(trifluoromethyl)phenyl]-1,2-dihydropyridine-3-carboxamide

APCI-MS m/z: 405 [MH⁺].

### Example 2.131 N-(2-Chloro-4-fluorobenzyl)-6-methyl-2-oxo-1-[3-(trifluoromethyl)phenyl]-1,2-dihydropyridine-3-carboxamide

APCI-MS m/z: 439 [MH⁺].

### Example 2.132 N-(3,4-Dichlorobenzyl)-6-methyl-2-oxo-1-[3-(trifluoromethyl)phenyl]-1,2-dihydropyridine-3-carboxamide

APCI-MS m/z: 456 [MH⁺].

### Example 2.133 6-Methyl-N-[(5-methyl-2-furyl)methyl]-2-oxo-1-[3-(trifluoromethyl)phenyl]-1,2-dihydropyridine-3-carboxamide

APCI-MS m/z: 391 [MH⁺].

### Example 2.134 6-Methyl-2-oxo-N-1,2,3,4-tetrahydronaphthalen-1-yl-1-[3-(trifluoromethyl)phenyl]-1,2-dihydropyridine-3-carboxamide

APCI-MS m/z: 427 [MH⁺].

### Example 2.135 N-(2,3-Dimethoxybenzyl)-6-methyl-2-oxo-1-[3-(trifluoromethyl)phenyl]-1,2-dihydropyridine-3-carboxamide

APCI-MS m/z: 447 [MH⁺].

### Example 2.136 N-[1-(4-Chlorophenyl)ethyl]-6-methyl-2-oxo-1-[3-(trifluoromethyl)phenyl]-1,2-dihydropyridine-3-carboxamide

APCI-MS m/z: 434 [MH⁺].

### Example 2.137 N-(2,5-Difluorobenzyl)-6-methyl-2-oxo-1-[3-(trifluoromethyl)phenyl]-1,2-dihydropyridine-3-carboxamide

APCI-MS m/z: 423 [MH⁺].

### Example 2.138 Methyl 4-{[({6-methyl-2-oxo-1-[3-(trifluoromethyl)phenyl]-1,2-dihydropyridin-3-yl}carbonyl)amino]methyl}benzoate

APCI-MS m/z: 445 [MH⁺].

### Example 2.139 6-Methyl-2-oxo-N-(4-phenoxybenzyl)-1-[3-(trifluoromethyl)phenyl]-1,2-dihydropyridine-3-carboxamide

APCI-MS m/z: 479 [MH⁺].

### Example 2.140 N-[(2,2-Dimethyl-1,3-dioxolan-4-yl)methyl]-6-methyl-2-oxo-1-[3-(trifluoromethyl)phenyl]-1,2-dihydropyridine-3-carboxamide

APCI-MS m/z: 411 [MH⁺].

### Example 2.141 6-Methyl-N-[(5-methylisoxazol-3-yl)methyl]-2-oxo-1-[3-(trifluoromethyl)phenyl]-1,2-dihydropyridine-3-carboxamide

APCI-MS m/z: 392 [MH⁺].

### Example 2.142 N-[(2,5-Dimethyl-3-furyl)methyl]-6-methyl-2-oxo-1-[3-(trifluoromethyl)phenyl]-1,2-dihydropyridine-3-carboxamide

APCI-MS m/z: 405 [MH⁺].

### Example 2.143 N-(3-Furylmethyl)-6-methyl-2-oxo-1-[3-(trifluoromethyl)phenyl]-1,2-dihydropyridine-3-carboxamide

APCI-MS m/z: 377 [MH⁺].

### Example 2.144 6-Methyl-2-oxo-N-[4-(1H-pyrazol-1-yl)benzyl]-1-[3-(trifluoromethyl)phenyl]-1,2-dihydropyridine-3-carboxamide

APCI-MS m/z: 453 [MH⁺].

### Example 2.145 6-Methyl-2-oxo-N-(4-thien-2-ylbenzyl)-1-[3-(trifluoromethyl)phenyl]-1,2-dihydropyridine-3-carboxamide

APCI-MS m/z: 469 [MH⁺].

### Example 2.146 N-[2-(1,3-Benzodioxol-5-yl)ethyl]-6-methyl-2-oxo-1-[3-(trifluoromethyl)phenyl]-1,2-dihydropyridine-3-carboxamide

APCI-MS m/z: 445 [MH⁺].

### Example 2.147 6-Methyl-2-oxo-N-(2-thien-2-ylethyl)-1-[3-(trifluoromethyl)phenyl]-1,2-dihydropyridine-3-carboxamide

APCI-MS m/z: 407 [MH⁺].

### Example 2.148 N-[2-(4-Tert-butylphenyl)ethyl]-6-methyl-2-oxo-1-[3-(trifluoromethyl)phenyl]-1,2-dihydropyridine-3-carboxamide

APCI-MS m/z: 457 [MH⁺].

### Example 2.149 6-Methyl-N-[2-(4-methylphenyl)ethyl]-2-oxo-1-[3-(trifluoromethyl)phenyl]-1,2-dihydropyridine-3-carboxamide

APCI-MS m/z: 415 [MH⁺].

### Example 2.150 N-{2-[4-(Aminosulfonyl)phenyl]ethyl}-6-methyl-2-oxo-1-[3-(trifluoromethyl)phenyl]-1,2-dihydropyridine-3-carboxamide

APCI-MS m/z: 480 [MH⁺].

### Example 2.151 6-Methyl-2-oxo-N-[(1R)-1-phenylethyl]-1-[3-(trifluoromethyl)phenyl]-1,2-dihydropyridine-3-carboxamide

APCI-MS m/z: 401 [MH⁺].

### Example 2.152 3-{[4-(2-Methoxyphenyl)piperazin-1-yl]carbonyl}-6-methyl-1-[3-(trifluoromethyl)phenyl]pyridin-2(1H)-one

APCI-MS m/z: 472 [MH⁺].

### Example 2.153 N-[(4-Cyanocyclohexyl)methyl]-6-methyl-2-oxo-1-[3-(trifluoromethyl)phenyl]-1,2-dihydropyridine-3-carboxamide

APCI-MS m/z: 418 [MH⁺].

### Example 2.154 3-{[4-(4-Fluorophenyl)piperazin-1-yl]carbonyl}-6-methyl-1-[3-(trifluoromethyl)phenyl]pyridin-2(1H)-one

APCI-MS m/z: 460 [MH⁺].

### Example 2.155 N-[2-(4'-Fluoro-1,1'-biphenyl-4-yl)ethyl]-6-methyl-2-oxo-1-[3-(trifluoromethyl)phenyl]-1,2-dihydropyridine-3-carboxamide

APCI-MS m/z: 495 [MH⁺].

### Example 2.156 N-(2-Hydroxy-1-phenylethyl)-6-methyl-2-oxo-1-[3-(trifluoromethyl)phenyl]-1,2-dihydropyridine-3-carboxamide

APCI-MS m/z: 417 [MH⁺].

### Example 2.157 6-Methyl-2-oxo-N-[(2R)-2-phenylcyclopropyl]-1-[3-(trifluoromethyl)phenyl]-1,2-dihydropyridine-3-carboxamide

APCI-MS m/z: 413 [MH⁺].

### Example 2.158 N-[1-(4-Chlorobenzyl)piperidin-4-yl]-6-methyl-2-oxo-1-[3-(trifluoromethyl)phenyl]-1,2-dihydropyridine-3-carboxamide

APCI-MS m/z: 504 [MH⁺].

### Example 2.159 6-Methyl-N-(2-morpholin-4-ylethyl)-2-oxo-1-[3-(trifluoromethyl)phenyl]-1,2-dihydropyridine-3-carboxamide

APCI-MS m/z: 410 [MH⁺].

### Example 2.160 N-[2-(4-Chlorophenyl)ethyl]-6-methyl-2-oxo-1-[3-(trifluoromethyl)phenyl]-1,2-dihydropyridine-3-carboxamide

APCI-MS m/z: 435 [MH⁺].

### Example 2.161 N-(2-Hydroxy-2-phenylethyl)-6-methyl-2-oxo-1-[3-(trifluoromethyl)phenyl]-1,2-dihydropyridine-3-carboxamide

APCI-MS m/z: 417 [MH⁺].

### Example 2.162 N-Cyclopentyl-6-methyl-2-oxo-1-[3-(trifluoromethyl)phenyl]-1,2-dihydropyridine-3-carboxamide

APCI-MS m/z: 365 [MH⁺].

### Example 2.163 N-[2-(1H-Imidazol-4-yl)ethyl]-6-methyl-2-oxo-1-[3-(trifluoromethyl)phenyl]-1,2-dihydropyridine-3-carboxamide

APCI-MS m/z: 391 [MH⁺].

### Example 2.164 N-(3,5-Dimethoxybenzyl)-6-methyl-2-oxo-1-[3-(trifluoromethyl)phenyl]-1,2-dihydropyridine-3-carboxamide

APCI-MS m/z: 447 [MH⁺].

### Example 2.165 N-(4-Hydroxycyclohexyl)-6-methyl-2-oxo-1-[3-(trifluoromethyl)phenyl]-1,2-dihydropyridine-3-carboxamide

APCI-MS m/z: 395 [MH⁺].

### Example 2.166 6-Methyl-2-oxo-N-(2-pyridin-2-ylethyl)-1-[3-(trifluoromethyl)phenyl]-1,2-dihydropyridine-3-carboxamide

APCI-MS m/z: 402 [MH⁺].

### Example 2.167 6-Methyl-2-oxo-N-1H-1,2,4-triazol-3-yl-1-[3-(trifluoromethyl)phenyl]-1,2-dihydropyridine-3-carboxamide

APCI-MS m/z: 364 [MH⁺].

### Example 2.168 N-[1-(Hydroxymethyl)-2-methylpropyl]-6-methyl-2-oxo-1-[3-(trifluoromethyl)phenyl]-1,2-dihydropyridine-3-carboxamide

APCI-MS m/z: 383 [MH⁺].

### Example 2.169 3-{[3-(3,4-Dichlorophenoxy)pyrrolidin-1-yl]carbonyl}-6-methyl-1-[3-(trifluoromethyl)phenyl]pyridin-2(1H)-one

APCI-MS m/z: 512 [MH⁺].

### Example 2.170 6-Methyl-2-oxo-N-(pyridin-3-ylmethyl)-1-[3-(trifluoromethyl)phenyl]-1,2-dihydropyridine-3-carboxamide

APCI-MS m/z: 388 [MH⁺].

### Example 2.171 N-(2-Methoxyethyl)-6-methyl-2-oxo-1-[3-(trifluoromethyl)phenyl]-1,2-dihydropyridine-3-carboxamide

APCI-MS m/z: 355 [MH⁺].

### Example 2.172 N-(2-Hydroxypropyl)-6-methyl-2-oxo-1-[3-(trifluoromethyl)phenyl]-1,2-dihydropyridine-3-carboxamide

APCI-MS m/z: 355 [MH⁺].

### Example 2.173 Ethyl 4-[({6-methyl-2-oxo-1-[3-(trifluoromethyl)phenyl]-1,2-dihydropyridin-3-yl}carbonyl)amino]piperidine-1-carboxylate

APCI-MS m/z: 452 [MH⁺].

### Example 2.174 N-[3-(1H-Imidazol-1-yl)propyl]-6-methyl-2-oxo-1-[3-(trifluoromethyl)phenyl]-1,2-dihydropyridine-3-carboxamide

APCI-MS m/z: 405 [MH⁺].

### Example 3 N-(4-Chlorobenzyl)-1-(3-methylphenyl)-2-oxo-1,2-dihydropyridine-3-carboxamide

### a) Diethyl [3-ethoxyprop-2-enylidene]malonate

Diethyl malonate (160 g, 1.0 mole) was added dropwise to a stirred, refluxing solution of 1,1,3,3-tetraethoxypropane (330 g, 1.5 mol), acetic anhydride (306 g, 2.0 moles) and zinc chloride (10 g, 0.073 mole) over a period of 30 minutes. The mixture was heated for 1 h, and after that a Dean-Stark apparatus was connected and the lower boiling components were distilled off. Additional acetic anhydride (150 ml) was added and refluxing was continued for 1 h. The reaction mixture was distilled to give the title compound as a yellow oil (182 g, 75%), b.p. 139-143 °C at 0.8 mm Hg.
¹H NMR (CDCl₃): δ 7.38 (1H, d, J=12.1 Hz); 7.04 (1H, d, J=12.2 Hz); 6.19 (1H, t, J=12.1 Hz); 4.27 (2H, q); 4.21 (2H, q); 3.96 (2H, q); 1.36-1.24 (9H, m).

### b) Diethyl {3-[(3-methylphenyl)amino]prop-2-enylidene}malonate

Diethyl [3-ethoxypmp-2-enylidene]malonate (9.7 g, 40 mmol) and m-toluidine (4.3 g, 40 mmol) were dissolved in ethanol (150 ml) and stirred at room temperature for three days. The solvents were evaporated off. Column chromatography on silica using heptane/ethyl acetate (4:1) as eluent afforded the title compound as an oil, which solidified after standing for a couple of days (10 g, 83%).
¹H NMR (CDCl₃): δ 7.65 (1H, d, J=12.4 Hz); 7.39 (2H, brd, J=7.7 Hz); 7.19 (1H, t, J=7.7 Hz); 6.85 (1H, d, J=7.7 Hz); 6.75 (1H, s); 6.73 (1H, d, J=6.5 Hz); 6.46 (1H, m, J=12.4, 6.5 Hz); 4.32 (2H, q); 4.25 (2H, q); 2.35 (3H, s); 1.36 (3H, t) 1.33 (3H, t).
APCI-MS m/z: 304 [MH⁺].

### c) 1-(3-Methylphenyl)-2-oxo-1,2-dihydropyridine-3-carboxylic acid

Diethyl {3-[(3-methylphenyl)amino]prop-2-enylidene}malonate (10 g, 33 mmol) was mixed with 2M sodium hydroxide solution (100 ml) and stirred at room temperature for 30 minutes. The reaction mixture was extracted (washed) with ethyl acetate and the water phases were acidified with hydrochloric acid to pH 3-4. An orange coloured precipitate appeared and was filtered off, washed with water and dried to afford the title compound (7.3 g, 97%).
¹H NMR (CDCl₃): δ 14.08 (1H, s); 8.64 (1H, dd, J=7.2, 2.2 Hz); 7.72 (1H, dd, J=6.7, 2.2 Hz); 7.47 (1H, t, J=7.7 Hz); 7.37 (1H, d, J=7.7 Hz); 7.23 (1H, s); 7.21 (1H, brd); 6.67 (1H, t, J=7.2, 6.7 Hz); 2.47 (3H, s).
APCI-MS m/z: 230 [MH⁺].

### d) N-(4-Chlorobenzyl)-1-(3-methylphenyl)-2-oxo-1,2-dihydropyridine-3-carboxamide

To a mixture of 1-(3-methylphenyl)-2-oxo-1,2-dihydropyridine-3-carboxylic acid (115 mg, 0.5 mmol), HATU (209 mg, 0.55 mmol), HOAT (75 mg, 0.55 mmol) and DIEA (275 µl, 1.6 mmol) in dichloromethane (2.5 ml) was added 4-chlorobenzylamine (71 mg, 0.5 mmol) in dichloromethane (1 ml). The reaction was stirred for 1 h at room temperature. More dichloromethane was added and the crude product was washed twice with aqueous sodium hydrogencarbonate, 0.5M aqueous citric acid and water. The solvent was removed in vacuo and the residue was purified by column chromatography on silica using dichloromethane/ethyl acetate (4:1) as eluent to afford the title compound in almost quantitative yield.
¹H NMR (CDCl₃): δ 10.10 (1H, brt); 8.68 (1H, dd); 7.58 (1H, dd); 7.43 (1H, t); 7.33-7.25 (5H, m); 7.20-7.14 (2H, m); 6.53 (1H, t); 4.59 (2H, d); 2.45 (3H, s).
APCI-MS m/z: 353 [MH⁺].

### Example 4 N-(4-Chlorobenzyl)-6'-methyl-2-oxo-2H-1,2'-bipyridine-3-carboxamide

### a) Diethyl {3-[(6-methylpyridin-2-yl)amino]prop-2-enylidene}malonate

Diethyl [3-ethoxyprop-2-enylidene]malonate (1.7 g, 7 mmol) and 2-amino-6-methylpyridine (1.08 g, 10 mmol) were heated (without solvent) at 140 °C for 6 h. The reaction mixture was worked-up as described in Example 3 (b) to afford the title compound.
APCI-MS m/z: 305 [MH⁺].

### b) 6'-Methyl-2-oxo-2H-1,2'-bipyridine-3-carboxylic acid

The title compound was prepared from diethyl {3-[(6-methylpyridin-2-yl)amino]prop-2-enylidene}malonate using the method described in Example 3 (c).
¹H NMR (CDCl₃): δ 14.02 (1H, brs); 8.65 (1H, dd); 8.20 (1H, dd); 7.84 (1H, t); 7.68 (1H, d); 7.33 (1H, d); 6.72 (1H, t); 2.64 (3H, s).
APCI-MS m/z: 231 [MH⁺].

### c) N-(4-Chlorobenzyl)-6'-methyl-2-oxo-2H-1,2'-bipyridine-3-carboxamide

The title compound was prepared from 6'-methyl-2-oxo-2H-1,2'-bipyridine-3-carboxylic acid and 4-chlorobenzylamine using the method described in Example 3 (d).
¹H NMR (CDCl₃): δ 10.04 (1H, brt); 8.68 (1H, dd); 7.95 (1H, dd); 7.79 (1H, t); 7.55 (2H, d); 7.29 (5H, brd); 6.58 (1H, t); 4.61 (2H, d); 2.62 (3H, s).
APCI-MS m/z: 354 [MH⁺].

The compounds of Examples 4.1 to 4.18 were prepared by a method analogous to that described for Example 4.

### Example 4.1 N-(4-Methoxybenzyl)-1-(3-methylphenyl)-2-oxo-1,2-dihydropyridine-3-carboxamide

¹H NMR (CDCl₃): δ 9.98 (1H, brt); 8.68 (1H, dd); 7.56 (1H, dd); 7.42 (1H, t); 7.32-7.25 (3H, m); 7.18-7.13 (2H, m); 6.84 (2H, d); 6.52 (1H, t); 4.56 (2H, d); 3.79 (3H, s); 2.43 (3H, s).
APCI-MS m/z: 349 [MH⁺].

### Example 4.2 Methyl 4-[({[1-(3-methylphenyl)-2-oxo-1,2-dihydropyridin-3-yl]carbonyl}amino)methyl]benzoate

¹H NMR (CDCl₃): δ 10.17 (1H, brt); 8.69 (1H, dd); 7.98 (2H, d); 7.59 (1H, dd); 7.46-7.40 (3H, m); 7.32 (1H, d); 7.20-7.16 (2H, m); 6.54 (1H, t); 4.69 (2H, d); 3.92 (3H, s); 2.45 (3H, s).
APCI-MS m/z: 377 [MH⁺].

### Example 4.3 4-[({[1-(3-Methylphenyl)-2-oxo-1,2-dihydropyridin-3-yl]carbonyl}amino)methyl]benzoic acid

A suspension of methyl 4-[({[1-(3-methylphenyl)-2-oxo-1,2-dihydropyridin-3-yl]carbonyl}amino)methyl] benzoate (120 mg, 0.32 mmol) and 2M sodium hydroxide solution (0.5 ml) in methanol (20 ml) and water (10 ml) was stirred at 40 °C overnight. The methanol was evaporated off and the aqueous solution was acidified with 1M hydrochloric acid (1 ml). A beige coloured precipitate appeared which was filtered off, washed twice with water and dried to afford the title compound (110 mg, 95%).
¹H NMR (DMSO-d₆): δ 12.84 (1H, s); 10.05 (1H, t); 8.45 (1H, dd); 7.99 (1H, dd); 7.88 (2H, d); 7.43-7.38 (3H, m); 7.30 (1H, d); 7.27 (1H, s); 7.24 (1H, d); 6.61 (1H, t); 4.57 (2H, d); 2.35 (3H, s).
APCI-MS m/z: 363 [MH⁺].

### Example 4.4 N-(4-Chlorobenzyl)-1-(2-fluoro-5-methylphenyl)-2-oxo-1,2-dihydropyridine-3-carboxamide

¹H NMR (CDCl₃): δ 9.97 (1H, brt); 8.69 (1H, dd); 7.51 (1H, dd); 7.31-7.26 (5H, m); 7.21-7.15 (2H, m); 6.56 (1H, t); 4.59 (2H, brs); 2.40 (3H, s).
APCI-MS m/z: 371 [MH⁺].

### Example 4.5 1-(2-Fluoro-5-methylphenyl)-N-(4-methoxybenzyl)-2-oxo-1,2-dihydropyridine-3-carboxamide

¹H NMR (CDCl₃): δ 9.86 (1H, brt); 8.69 (1H, dd); 7.49 (1H, dd); 7.30-7.25 (3H, m); 7.20-7.14 (2H, m); 6.84 (2H, d); 6.54 (1H, t); 4.56 (2H, brd); 3.79 (3H, s); 2.39 (3H, s).
APCI-MS m/z: 367 [MH⁺].

### Example 4.6 N-[4-(Dimethylamino)benzyl]-1-(2-fluoro-5-methylphenyl)-2-oxo-1,2-dihydropyridine-3-carboxamide

¹H NMR (CDCl₃): δ 9.78 (1H, brt); 8.69 (1H, dd); 7.47 (1H, dd); 7.27 (1H, dd); 7.22 (2H, d); 7.19-7.13 (2H, m); 6.68 (2H, d); 6.53 (1H, t); 4.53 (2H, brd); 2.92 (6H, s); 2.39 (3H, s).
APCI-MS m/z: 380 [MH⁺].

### Example 4.7 N-[4-(Aminosulfonyl)benzyl]-1-(2-fluoro-5-methylphenyl)-2-oxo-1,2-dihydropyridine-3-carboxamide

¹H NMR (CDCl₃): δ 10.11 (1H, brt); 8.69 (1H, dd); 7.86 (2H, d); 7.54 (1H, dd); 7.48 (2H, d); 7.30 (1H, dd); 7.20 (2H, d); 7.17 (2H, brd); 6.58 (1H, t); 4.68 (2H, brd); 2.41 (3H, s).
APCI-MS m/z: 416 [MH⁺].

### Example 4.8 N-(4-Chlorobenzyl)-4'-methyl-2-oxo-2H-1,2'-bipyridine-3-carboxamide

¹H NMR (CDCl₃): δ 10.02 (1H, brt); 8.68 (1H, dd); 8.48 (1H, d); 7.94 (1H, dd); 7.58 (1H, s); 7.30-7.28 (4H, m); 7.23 (1H, d); 6.58 (1H, t); 4.61 (2H, d); 2.48 (3H, s).
APCI-MS m/z: 354 [MH⁺].

### Example 4.9 N-(4-Chlorobenzyl)-1-(2,5-dimethylphenyl)-2-oxo-1,2-dihydropyridine-3-carboxamide

¹H NMR (CDCl₃): δ 10.11 (1H, brt); 8.69 (1H, dd); 7.47 (1H, dd); 7.29-7.20 (6H, m); 7.02 (1H, s); 6.54 (1H, t); 4.59 (2H, m); 2.38 (3H, s); 2.09 (3H, s).
APCI-MS m/z: 367 [MH⁺].

### Example 4.10 1-(2,5-Dimethylphenyl)-N-(4-methoxybenzyl)-2-oxo-1,2-dihydropyridine-3-carboxamide

¹H NMR (CDCl₃): δ 9.97 (1H, brt); 8.67 (1H, dd); 7.43 (1H, dd); 7.27-7.16 (4H, m); 6.99 (1H, s); 6.81 (2H, d); 6.50 (1H, t); 4.53 (2H, m); 3.77 (3H, s); 2.35 (3H, s); 2.07 (3H, s).
APCI-MS m/z: 363 [MH⁺].

### Example 4.11 N-[4-(Dimethylamino)benzyl]-1-(2,5-dimethylphenyl)-2-oxo-1,2-dihydropyridine-3-carboxamide

¹H NMR (CDCl₃): δ 9.91 (1H, brt); 8.69 (1H, dd); 7.43 (1H, dd); 7.26-7.19 (4H, m); 7.01 (1H, s); 6.68 (2H, d); 6.52 (1H, t); 4.52 (2H, m); 2.92 (6H, s); 2.37 (3H, s); 2.08 (3H, s).
APCI-MS m/z: 376 [MH⁺].

### Example 4.12 N-(4-Chlorobenzyl)-1-[2-methyl-5-(trifluoromethyl)phenyl]-2-oxo-1,2-dihydropyridine-3-carboxamide

¹H NMR (CDCl₃): δ 9.94 (1H, brt); 8.72 (1H, dd); 7.69 (1H, dd); 7.53 (1H, d); 7.50 (1H, s); 7.46 (1H, dd); 7.28 (4H, s); 6.60 (1H, t); 4.59 (2H, m); 2.23 (3H, s).
APCI-MS m/z: 421 [MH⁺].

### Example 4.13 N-(4-Methoxvbenzyl)-1-[2-methyl-5-(trifluoromethyl)phenyl]-2-oxo-1,2-dihydropyridine-3-carboxamide

¹H NMR (CDCl₃): δ 9.80 (1H, brt); 8.71 (1H, dd); 7.65 (1H, dd); 7.50 (1H, d); 7.47 (1H, s); 7.42 (1H, dd); 7.27-7.24 (4H, m); 6.82 (2H, d); 6.56 (1H, t); 4.54 (2H, m); 3.76 (3H, s); 2.19 (3H, s).
APCI-MS m/z: 417 [MH⁺].

### Example 4.14 N-[4-(Dimethylamino)benzyl]-1-[2-methyl-5-(trifluoromethyl)phenyl]-2-oxo-1,2-dihydropyridine-3-carboxamide

¹H NMR (CDCl₃): δ 9.75 (1H, brt); 8.73 (1H, dd); 7.67 (1H, dd); 7.52 (1H, d); 7.49 (1H, s); 7.42 (1H, dd); 7.23 (2H, d); 6.69 (2H, d); 6.57 (1H, t); 4.53 (2H, m); 2.92 (6H, s); 2.21 (3H, s).
APCI-MS m/z: 430 [MH⁺].

### Example 4.15 N-Benzyl-5-methyl-2-oxo-1-[3-(trifluoromethyl)phenyl]-1,2-dihydropyridine-3-carboxamide

APCI-MS m/z: 387 [MH⁺].

### Example 4.16 N-(2-Chlorobenzyl)-5-methyl-2-oxo-1-[3-(trifluoromethyl)phenyl]-1,2-dihydropyridine-3-carboxamide

APCI-MS m/z: 421 [MH⁺].

### Example 4.17 5-Methyl-2-oxo-N-(2-phenylethyl)-1-[3-(trifluoromethyl)phenyl]-1,2-dihydropyridine-3-carboxamide

APCI-MS m/z: 401 [MH⁺].

### Example 4.18 N-(4-Chlorophenyl)-5-methyl-2-oxo-1-[3-(trifluoromethyl)phenyl]-1,2-dihydropyridine-3-carboxamide

APCI-MS m/z: 407 [MH⁺].

### Example 5 6-Ethyl-N-[4-(methylsulfonyl)benzyl]-2-oxo-1-[3-(trifluoromethyl)phenyl]-1,2-dihydropyridine-3-carboxamide

### a) 6-Ethyl-2-oxo-1-[3-(trifluoromethyl)phenyl]-1,2-dihydropyridine-3-carboxylic acid

The title compound was prepared from ethyl 3-oxo-3-{[3-(trifluoromethyl)phenyl] amino}propanoate and 1-methoxypent-1-en-3-one using the method described in Example 1 steps (a) and (b).
¹H NMR (CDCl₃): δ 13.75 (1H, brs); 8.59 (1H, d); 7.87 (1H, d); 7.79 (1H, t); 7.55 (1H, s); 7.49 (1H, d); 6.61 (1H, d); 2.37 (2H, q); 1.20 (3H, t).
APCI-MS m/z: 312 [MH⁺].

### b) 6-Ethyl-N-[4-(methylsulfonyl)benzyl]-2-oxo-1-[3-(trifluoromethyl)phenyl]-1,2-dihydropyridine-3-carboxamide

The title compound was prepared from 6-ethyl-2-oxo-1-[3-(trifluoromethyl)phenyl]-1,2-dihydropyridine-3-carboxylic acid and 4-(methylsulfonyl) benzylamine hydrochloride using the method described in Example 3 (d).
¹H NMR (CDCl₃): 8 10.00 (1H, brt); 8.64 (1H, d); 7.88 (2H, d); 7.82 (1H, d); 7.75 (1H, t); 7.53 (2H, d); 7.52 (1H, s); 7.45 (1H, d); 6.51 (1H, d); 4.68 (2H, m); 3.02 (3H, s); 2.31 (2H, q); 1.17 (3H, t).
APCI-MS m/z: 479 [MH⁺].

The compounds of Examples 5.1 and 5.2 were prepared by a method analogous to that described for Example 5.

### Example 5.1 N-[4-(Methylsulfonyl)benzyl]-2-oxo-6-propyl-1-[3-(trifluoromethyl)phenyl]-1,2-dihydropyridine-3-carboxamide

¹H NMR (CDCl₃): δ 10.00 (1H, brt); 8.62 (1H, d); 7.88 (2H, d); 7.83 (1H, d); 7.75 (1H, t); 7.53 (2H, d); 7.52 (1H, s); 7.45 (1H, d); 6.49 (1H, d); 4.68 (2H, m); 3.02 (3H, s); 2.26 (2H, t); 1.55 (2H, sxt); 0.87 (3H, t).
APCI-MS m/z: 493 [MH⁺].

### Example 5.2 6-Butyl-N-[4-(methylsulfonyl)benzyl]-2-oxo-1-[3-(trifluoromethyl)phenyl]-1,2-dihydropyridine-3-carboxamide

¹H NMR (CDCl₃): δ 10.00 (1H, brt); 8.62 (1H, d); 7.88 (2H, d); 7.83 (1H, d); 7.75 (1H, t); 7.54 (2H, d); 7.52 (1H, s); 7.45 (1H, d); 6.49 (1H, d); 4.68 (2H, m); 3.03 (3H, s); 2.29 (2H, t); 1.49 (2H, qv); 1.24 (2H, sxt); 0.80 (3H, t).
APCI-MS m/z: 507 [MH⁺].

### Example 6 6-(Methoxymethyl)-N-[4-(methylsulfonyl)benzyl]-2-oxo-1-[3-(trifluoromethyl)phenyl]-1,2-dihydropyridine-3-carboxamide

### a) 6-(Bromomethyl)-2-oxo-1-[3-(trifluoromethyl)phenyl]-1,2-dihydropyridine-3-carboxylic acid

The title compound was prepared by refluxing 6-methyl-2-oxo-1-[3-(trifluoromethyl) phenyl]-1,2-dihydropyridine-3-carboxylic acid (297 mg, 1 mmol), N-bromosuccinimide (240 mg, 1.3 mmol) and 2,2'-azobis-2-methylpropionitrile (AIBN) (15 mg) in carbon tetrachloride/chloroform (2:1, 5 ml) overnight. The solvent was evaporated to give the title compound.
APCI-MS m/z: 376/378 [MH⁺].

### b) 6-(Methoxymethyl)-2-oxo-1-[3-(trifluoromethyl)phenyl]-1,2-dihydropyridine-3-carboxylic acid

The title compound was prepared by heating crude 6-(bromomethyl)-2-oxo-1-[3-(trifluoromethyl)phenyl]-1,2-dihydropyridine-3-carboxylic acid with an excess of sodium methoxide in methanol at 40 °C for 15 minutes. The organic solvents were removed, water was added and the reaction mixture was washed with ethyl acetate. The water phases were acidified with hydrochloric acid to pH 3-4. A yellowish precipitate appeared which was filtered off, washed (water and water/methanol, 1:1) and dried to give the title compound.
¹H NMR (CDCl₃): δ 13.66 (1H, brs); 8.63 (1H, d); 7.87 (1H, d); 7.77 (1H, t); 7.58 (1H, s); 7.50 (1H, d); 6.84 (1H, d); 3.96 (2H, d); 3.27 (3H, s).
APCI-MS m/z: 328 [MH⁺].

### c) 6-(Methoxymethyl)-N-[4-(methylsulfonyl)benzyl]-2-oxo-1-[3-(trifluoromethyl)phenyl]-1,2-dihydropyridine-3-carboxamide

The title compound was prepared from 6-(methoxymethyl)-2-oxo-1-[3-(trifluoromethyl)phenyl]-1,2-dihydropyridine-3-carboxylic acid and 4-(methylsulfonyl) benzylamine hydrochloride using the method described in Example 3 (d).
¹H NMR (CDCl₃): δ 9.98 (1H, brt); 8.67 (1H, d); 7.87 (2H, d); 7.82 (1H, d); 7.73 (1H, t); 7.53 (3H, m); 7.47 (1H, d); 6.72 (1H, d); 4.67 (2H, m); 3.92 (2H, d); 3.23 (3H, s); 3.01 (3H, s).
APCI-MS m/z: 495 [MH⁺].

### Example 7 6-(Hydroxymethyl)-N-[4-(methylsulfonyl)benzyl]-2-oxo-1-[3-(trifluoromethyl)phenyl]-1,2-dihydropyridine-3-carboxamide

### a) 6-(Hydroxymethyl)-2-oxo-1-[3-(trifluoromethyl)phenyl]-1,2-dihydropyridine-3-carboxylic acid

The title compound was prepared by heating 6-(bromomethyl)-2-oxo-1-[3-(trifluoromethyl)phenyl]-1,2-dihydropyridine-3-carboxylic acid and dilute sodium hydroxide in methanol for a few minutes. The reaction mixture was washed with ethyl acetate. The water phases were acidified with hydrochloric acid. A precipitate appeared which was recrystallised several times from ethyl acetate/methanol to give the title compound.
¹H NMR (DMSO-d₆): δ 13.99 (1H, brs); 8.53 (1H, d); 8.00 (1H, s); 7.94 (1H, d); 7.83 (1H, t); 7.81 (1H, d); 6.94 (1H, d); 5.85 (1H, t); 3.99 (2H, d).
APCI-MS m/z: 314 [MH⁺].

### b) 6-(Hydroxymethyl)-N-[4-(methylsulfonyl)benzyl]-2-oxo-1-[3-(trifluoromethyl)phenyl]-1,2-dihydropyridine-3-carboxamide

The title compound was prepared from 6-(hydroxymethyl)-2-oxo-1-[3-(trifluoromethyl)phenyl]-1,2-dihydropyridine-3-carboxylic acid and 4-(methylsulfonyl) benzylamine hydrochloride using the method described in Example 3 (d).
¹H NMR (DMSO-d₆): δ 9.92 (1H, brt); 8.49 (1H, d); 7.92-7.81 (4H, m); 7.78 (1H, t); 7.71 (1H, d); 7.53 (2H, d); 6.78 (1H, d); 5.71 (1H, t); 4.57 (2H, brd); 3.95 (2H, brd); 3.16 (3H, s).
APCI-MS m/z: 481 [MH⁺].

### Example 8 N-[4-(Aminosulfonyl)benzyl]-2,4-dioxo-3-[3-(trifluoromethyl)phenyl]-1,2,3,4-tetrahydropyrimidine-5-carboxamide

### a) 2,4-Dioxo-3-[3-(trifluoromethyl)phenyl]-1,2,3,4-tetrahydropyrimidine-5-carboxylic acid

3-(Trifluoromethyl)phenyl isocyanate (3.52 g, 22 mmol) was added quickly to a vigorously stirred ice-cooled solution of aqueous ammonia (10 ml, 33%) in acetonitrile (40 ml). The mixture was heated at 40 °C for 10 minutes and then the solvent was evaporated. The resulting urea was redissolved in dry ethanol (15 ml) and diethyl ethoxymethylene-malonate (5 ml, 24.7 mmol) and finally sodium ethoxide solution (50 mmol in ethanol) was added, and the mixture was refluxed for 2 h. Water (10 ml) was added and the mixture was allowed to cool, then washed with ethyl acetate, acidified to pH~3 with conc. hydrochloric acid and extracted with ethyl acetate. The organic extracts were dried and evaporated to give a solid material. Recrystallisation from heptane/ethyl acetate afforded the title compound (0.5 g, 7%).
¹H NMR (CDCl₃): δ 12.41 (1H, brd, J=6.4Hz); 8.15 (1H, d, J=6.4Hz); 7.46 (1H, d, J=7.6Hz); 7.40 (1H, t, J=7.6Hz); 7.30 (brs, 1H); 7.22 (1H, d, J=7.6Hz).
APCI-MS m/z: 300 [MH⁺].

### b) N-[4-(Aminosulfonyl)benzyl]-2,4-dioxo-3-[3-(trifluoromethyl)phenyl]-1,2,3,4-tetrahydropyrimidine-5-carboxamide

The title compound was prepared from 2,4-dioxo-3-[3-(trifluoromethyl)phenyl]-1,2,3,4-tetrahydropyrimidine-5-carboxylic acid and N-[4-(aminosulfonyl)-benzylamine hydrochloride using the method described in Example 1 (d).
¹H NMR (DMSO-d₆): δ 12.21 (1H, brd); 9.11 (1H, t); 8.29 (1H, s); 7.81 (2H, d); 7.77-7.70 (3H, m); 7.65 (1H, d); 7.44 (1H, d); 7.28 (2H, s); 4.46 (2H, d).

The compounds of Examples 8.1 to 8.8 were prepared using the general method described for Example 8.

### Example 8.1 N-[4-(Dimethylamino)benzyl]-2,4-dioxo-3-[3-(trifluoromethyl)phenyl]-1,2,3,4-tetrahydropyrimidine-5-carboxamide trifluoroacetate

¹H NMR (CD₃CN): δ 9.73 (1H, brd); 8.96 (1H, brt); 8.35 (1H, d); 7.79 (1H, d); 7.72 (1H, t); 7.63 (1H, s); 7.55 (1H, d); 7.36 (2H, d); 7.23 (2H, d); 4.49 (2H, d); 3.04 (6H, s).

### Example 8.2 N-(4-Chlorobenzyl)-2,4-dioxo-3-[3-(trifluoromethyl)phenyl]-1,2,3,4-tetrahydropyrimidine-5-carboxamide

¹H NMR (CD₃CN): δ 9.65 (1H, brd); 8.96 (1H, brt); 8.39 (1H, d); 7.80 (1H, d); 7.72 (1H, t); 7.63 (1H, s); 7.56 (1H, d); 7.33 (2H, d); 7.25 (2H, d); 4.49 (2H, d).

### Example 8.3 N-(2,3-Dihydro-1-benzofuran-5-ylmethyl)-2,4-dioxo-3-[3-(trifluoromethyl)phenyl]-1,2,3,4-tetrahydropyrimidine-5-carboxamide

¹H NMR (CDCl₃): δ 8.53 (1H, d); 7.76 (1H, m); 7.67 (1H, m); 7.53 (1H, m); 7.44 (1H, m), 7.13 (1H, m); 7.04 (1H, m); 6.68 (1H, d); 5.28 (2H, d); 4.57 (2H, t); 3.19 (2H, t).
APCI-MS m/z: 432 [MH⁺].

### Example 8.4 N-[4-(Methylsulfonyl)benzyl]-2,4-dioxo-3-[3-(trifluoromethyl)phenyl]-1,2,3,4-tetrahydropyrimidine-5-carboxamide

¹H NMR (CDCl₃): δ 9.05 (1H, m); 8.52 (1H, d); 8.40 (1H, m); 7.88 (2H, d); 7.75 (1H, d); 7.66 (1H, t); 7.53 (1H, m); 7.49 (2H, d); 7.44 (1H, d); 4.65 (2H, d); 3.01 (3H, s).
APCI-MS m/z: 468 [MH⁺].

### Example 8.5 N-(4-Bromobenzyl)-2,4-dioxo-3-[3-(trifluoromethyl)phenyl]-1,2,3,4-tetrahydropyrimidine-5-carboxamide

¹H NMR (CDCl₃): 8 8.90 (1H, m); 8.50 (1H, d); 8.32 (1H, m); 7.74 (1H, d); 7.65 (1H, m); 7.52 (1H, m); 7.42 (3H, m); 7.16 (2H, d); 4.50 (2H, d).
APCI-MS m/z: 467[MH⁺].

### Example 8.6 N-(4-Methoxybenzyl)-2,4-dioxo-3-[3-(trifluoromethyl)phenyl]-1 2 3,4-tetrahydropyrimidine-5-carboxamide

¹H NMR (CDCl₃): δ 8.86 (1H, m); 8.62- 8.53 (2H, m); 7.77 (1H, m); 7.69 (1H, m); 7.55 (1H, m), 7.47 (1H, m), 7.24 (2H, m); 6.86 (1H, m); 4.52 (2H, d); 3.80 (3H, s).
APCI-MS m/z: 420 [MH⁺].

### Example 8.7 N-(1,3-Benzodioxol-5-ylmethyl)-2,4-dioxo-3-[3-(trifluoromethyl)phenyl]-1,2,3,4-tetrahydropyrimidine-5-carboxamide

¹H NMR (CDCl₃): δ 8.93 (1H, m); 8.71 (1H, m); 8.54 (1H, t); 7.76 (1H, d); 7.68 (1H, t); 7.54 (1H, m); 7.46 (1H, d); 7.27 (1H, s); 6.85 (1H, d); 6.76 (1H, d); 5.95 (2H, d); 4.56 (1H, d); 4.48 (1H, d).
APCI-MS m/z: 434 [MH⁺].

### Example 8.8 N-(3-Chlorobenzyl)-2,4-dioxo-3-[3-(trifluoromethyl)phenyl]-1,2,3,4-tetrahydropyrimidine-5-carboxamide

¹H NMR (CDCl₃): δ 10.07 (1H, d); 9.11 (1H, t); 8.54 (1H, d); 7.78 (1H, d); 7.69 (1H, t); 7.55 (1H, m); 7.47 (1H, d); 7.29 (1H, m); 7.26 (2H, m); 7.19 (1H, m); 4.58 (2H, d).
APCI-MS m/z: 424 [MH⁺].

### Example 9 1-Butyl-N-[4-(methylsulfonyl)benzyl]-2,4-dioxo-3-[3-(trifluoromethyl)phenyl]-1,2,3,4-tetrahydropyrimidine-5-carboxamide

### a) N-Butyl-N'-[3-(trifluoromethyl)phenyl]urea

1-Isocyanato-3-(trifluoromethyl)benzene (0.74 ml, 5.34 mmol) was added to an ice cooled solution of n-butylamine (1.06 ml, 10.68 mmol) in acetonitrile (10 ml). The mixture was stirred for 10 minutes and then the solvent was evaporated to give the title compound as a white solid (1.37 g, 99%).
APCI-MS m/z: 261 [MH⁺].

### b) Ethyl 1-butyl-2,4-dioxo-3-[3-(trifluoromethyl)phenyl]-1,2,3,4-tetrahydropyrimidine-5-carboxylate

To a solution of N-butyl-N'-[3-(trifluoromethyl)phenyl]urea (1.37 g, 5.26 mmol) and diethyl (ethoxymethylene)malonate (2.13 ml, 10.68 mmol) in NMP (6 ml) at 100 °C was added potassium tert-butoxide (0.10 g, 0.89 mmol) and the mixture was stirred for 1 h. Ethyl acetate was added and the mixture was washed with 1M hydrochloric acid, brine and water. The solvent was evaporated and the resulting oil was purified by HPLC to give the title compound (667 mg, 33%).
¹H NMR (CDCl₃): δ 8.33 (1H, s); 7.70 (1H, d); 7.62 (1H, t); 7.51 (1H, s); 7.41 (1H, d); 4.35 (2H, q); 3.89 (2H, t); 1.81-1.74 (2H, m); 1.45-1.33 (5H, m); 0.99 (3H, t).
APCI-MS m/z: 385 [MH⁺].

### c) 1-Butyl-2,4-dioxo-3-[3-(trifluoromethyl)phenyl]-1,2,3,4-tetrahydropyrimidine-5-carboxylic acid

A solution of ethyl 1-butyl-2,4-dioxo-3-[3-(trifluoromethyl)phenyl]-1,2,3,4-tetrahydropyrimidine-5-carboxylate (101 mg, 0.26 mmol) and 0.5M sodium hydroxide solution (700 µl, 0.35 mmol) in THF was stirred for 2 h. Water was added and the mixture was washed with ethyl acetate. Acidification of the aqueous phase, extraction with ethyl acetate and removal of the solvent yielded the title compound (65 mg, 70%).
¹H NMR (CDCl₃): δ 8.57 (1H, s); 7.79 (1H, d); 7.70 (1H, t); 7.55 (1H, s); 7.46 (1H, d); 3.96 (2H, t); 1.85-1.75 (2H, m); 1.50-1.37 (2H, m); 1.00 (3H, t).
APCI-MS m/z: 357 [MH⁺].

### d) 1-Butyl-N-[4-(methylsulfonl)benzyl]-2,4-dioxo-3-[3-(trifluoromethyl)phenyl]-1,2,3,4-tetrahydropyrimidine-5-carboxamide

A solution of 4-methylsulphonylbenzylamine hydrochloride (30 mg, 0.14 mmol) and DIEA (24 µl, 0.14 mmol) in dichloromethane (1 ml) was added to a stirred mixture of 1-butyl-2,4-dioxo-3-[3-(trifluoromethyl)phenyl]-1,2,3,4-tetrahydropyimidine-5-carboxylic acid (44 mg, 0.12 mmol), HATU (52 mg, 0.14 mmol), HOAT (19 mg, 0.14 mmol) and DIEA (63 µl, 0.37 mmol) in dichloromethane (1 ml). The resulting mixture was stirred for 2 h. The solvent was evaporated and the product was purified by HPLC and by flash chromatography to give the title compound (22 mg, 35%).
¹H NMR (CDCl₃): δ 9.14 (1H, t); 8.55 (1H, s); 7.89 (2H, d); 7.75 (1H, d); 7.67 (1H, t); 7.53 (1H, s); 7.50 (2H, d); 7.44 (1H, d); 4.66 (2H, d); 3.93 (2H, t); 3.03 (3H, s); 1.83-1.75 (2H, m); 1.47-1.38 (2H, m); 0.99 (3H, t).
APCI-MS m/z: 524 [MH⁺].

The compounds of Examples 9.1 to 9.4 were prepared using the general method described for Example 9:

### Example 9.1 1-(2-Methoxyethyl)-N-[4-(methylsulfonyl)benzyl]-2,4-dioxo-3-[3-(trifluoromethyl)phenyl]-1,2,3,4-tetrahydropyrimidine-5-carboxamide

¹H NMR (CDCl₃): δ 9.13 (1H, t); 8.61 (1H, s); 7.90 (2H, d); 7.75 (1H, d); 7.67 (1H, t); 7.54 (1H, s); 7.51 (2H, d); 7.45 (1H, d); 4.66 (2H, d); 4.12 (2H, t); 3.68 (2H, t); 3.40 (3H, s); 3.03 (3H, s).
APCI-MS m/z: 526 [MH⁺].

### Example 9.2 1-Methyl-N-[4-(methylsulfonyl)benzyl]-2,4-dioxo-3-[3-(trifluoromethyl)phenyl]-1,2,3,4-tetrahydropyrimidine-5-carboxamide

¹H NMR (CDCl₃): δ 9.10 (1H, t); 8.57 (1H, s); 7.90 (2H, d); 7.76 (1H, d); 7.67 (1H, t); 7.52 (1H, s); 7.50 (2H, d); 7.43 (1H, d); 4.66 (2H, d); 3.61 (3H, s); 3.03 (3H, s).
APCI-MS m/z: 482 [MH⁺].

### Example 9.3 1-Ethyl-N-[4-(methylsulfonyl)benzyl]-2,4-dioxo-3-[3-(trifluoromethyl)phenyl]-1,2,3,4-tetrahydropyrimidine-5-carboxamide

¹H NMR (CDCl₃): δ 9.13 (1H, t); 8.58 (1H, s); 7.90 (2H, d); 7.75 (1H, d); 7.67 (1H, t); 7.53 (1H, s); 7.50 (2H, d); 7.44 (1H, d); 4.66 (2H, d); 4.01 (2H, q); 3.03 (3H, s); 1.45 (3H, t).
APCI-MS m/z: 496 [MH⁺].

### Example 9.4 N-(4-Chlorobenzyl)-1-(2-methoxyethyl)-2,4-dioxo-3-[3-(trifluoromethyl)phenyl]-1,2,3,4-tetrahydropyrimidine-5-caboxamide

¹H NMR (CDCl₃): δ 8.99 (1H, t); 8.60 (1H, s); 7.74 (1H, d); 7.66 (1H, t); 7.53 (1H, s); 7.44 (1H, d); 7.30-7.22 (4H, m); 4.54 (2H, d); 4.11 (2H, t); 3.67 (2H, t); 3.40 (3H, s).
APCI-MS m/z: 482 [MH⁺].

### Example 10 5-Iodo-6-methyl-N-[4-(methylsulfonyl)benzyl]-2-oxo-1-[3-(trifluoromethyl)phenyl]-1,2-dihydropyridine-3-carboxamide

N-Iodosuccinimide (9.7mg, 0.043 mmol) was added to a stirred solution of 6-methyl-N-[4-(methylsulfonyl)benzyl]-2-oxo-1-[3-(trifluoromethyl)phenyl]-1,2-dihydropyridine-3-carboxamide (20 mg, 0.043 mmol) in trifluoromethanesulfonic acid (0.5 ml). The mixture was stirred for 10 minutes. Dichloromethane (10 ml) was added and the organic phase was washed with aqueous sodium hydrogencarbonate, aqueous sodium thiosulfate and water. The extracts were dried and evaporated to give the title compound (100%).
¹H NMR (CDCl₃): δ 9.81 (1H, brt); 8.86 (1H, s,); 7.88 (2H, d); 7.82-7.69 (3H, m); 7.48 (2H, d); 7.40 (1H, d); 4.65 (2H, m); 3.01 (3H, s); 2.28 (3H, s).

The following Intermediates were prepared using the procedure described in Example 9(c):

### 1-(2-Methoxyethyl)-2,4-dioxo-3-[3-(trifluoromethyl)phenyl]-1,2,3,4-tetrahydropyrimidine-5-carboxylic acid

APCI-MS m/z: 359 [MH⁺].

### 3-(3-Chlorophenyl)-1-(2-methoxyethyl)-2,4-dioxo-1,2,3,4-tetrahydropyrimidine-5-carboxylic acid

APCI-MS m/z: 325 [MH⁺].

### 1-Butyl-3-(3-methoxyphenyl)-2,4-dioxo-1,2,3,4-tetrahydropyrimidine-5-carboxylic acid

¹H NMR (DMSO-d6): δ 12.09 (1H, br s); 8.79 (1H, s); 7.39 (1H, t); 7.01 (1H, dd); 9.91 (1H, t); 6.85(1H,d); 3.89 (2H, t); 3.74 (3H, s); 1.61 (2H, pentet); 1.30 (2H, hextet); 0.89 (3H, t).

### 1-Butyl-3-(3-(trifluoromethyl)phenyl)-2,4-dioxo-1,2,3,4-tetrahydropyrimidine-5-carboxylic acid

¹H NMR (DMSO-d6): δ 12.55 (1H, br s); 8.76 (1H, s); 7.84-7.60 (4H, m); 3.89 (2H, t); 1.63 (2H, pentet); 1.30 (2H, hextet); 0.89 (3H, t).

### 1-Butyl-3-(3-chlorophenyl)-2,4-dioxo-1,2,3,4-tetrahydropyrimidine-5-carboxylic acid

APCI-MS m/z 323 [MH⁺].

### 1-Butyl-3-(3-cyanophenyl)-2,4-dioxo-1,2,3,4-tetrahydropyrimidine-5-carboxylic acid

¹H NMR (DMSO-d6): δ 12.30 (1H, br s); 8.74 (1H, s); 7.94-7.85 (2H, m); 7.75-7.69 (2H, m); 3.89 (2H, t); 1.63 (2H, pentet); 1.30 (2H, hextet); 0.89 (3H, t).

### Example 11 N-(4-Chlorobenzyl)-1-(2-methoxyethyl)-2,4-dioxo-3-[3-(trifluoromethyl)phenyl]-1,2,3,4-tetrahydropyrimidine-5-carboxamide

1-(2-Methoxyethyl)-2,4-dioxo-3-[3-(trifluoromethyl)phenyl]-1,2,3,4-tetrahydropyrimidine-5-carboxylic acid (0.016 mmol, 0.2M in NMP) was added to PS-Carbodiimide resin (60 mg), HOBT (0.032 mmol, 0.3M in NMP) and NMP (200 µl). The mixture was stirred for 15 minutes and 4-chlorobenzylamine (0.019 mmol, 0.3M in NMP) was added. After shaking overnight the excess HOBT was scavenged using PS-Trisamine resin (45 mg), shaking for 2 h before the resin reagents were filtered off. The title compound was obtained after purification using preparative HPLC.
APCI-MS m/z: 482.4 [MH⁺].

Examples 11.1 to 11.13 were prepared from 1-(2-methoxyethyl)-2,4-dioxo-3-[3-(trifluoromethyl)phenyl]-1,2,3,4-tetrahydropyrimidine-5-carboxylic acid and the appropriate amine using the general procedure described in Example 11:

### Example 11.1 N-(4-Methoxybenzyl)-1-(2-methoxyethyl)-2,4-dioxo-3-[3-(trifluoromethyl)phenyl]-1,2,3,4-tetrahydropyrimidine-5-carboxamide

APCI-MS m/z: 478.5 [MH⁺].

### Example 11.2 1-(2-Methoxyethyl)-2,4-dioxo-N-(pyridin-4-ylmethyl)-3-[3-(trifluoromethyl)phenyl]-1,2,3,4-tetrahydropyrimidine-5-carboxamide

APCI-MS m/z: 449.4 [MH⁺].

### Example 11.3 N-[2-(3,4-Dimethoxyphenyl)ethyl]-1-(2-methoxyethyl)-2,4-dioxo-3-[3-(trifluoromethyl)phenyl]-1,2,3,4-tetrahydropyrimidine-5-carboxamide

APCI-MS m/z: 522.5 [MH⁺].

### Example 11.4 1-(2-Methoxyethyl)-N-[2-(3-methoxyphenyl)ethyl]-2,4-dioxo-3-[3-(trifluoromethyl)phenyl]-1,2,3,4-tetrahydropyrimidine-5-carboxamide

APCI-MS m/z: 492.5 [MH⁺].

### Example 11.5 1-(2-Methoxyethyl)-N-(4-methylbenzyl)-2,4-dioxo-3-[3-(trifluoromethyl)phenyl]-1,2,3,4-tetrahydropyrimidine-5-carboxamide

APCI-MS m/z: 462.5 [MH⁺].

### Example 11.6 1-(2-Methoxyethyl)-N-[4-(methylsulfonyl)benzyl]-2,4-dioxo-3-[3-(trifluoromethyl)phenyl]-1,2,3,4-tetrahydropyrimidine-5-carboxamide

APCI-MS m/z: 526.5 [MH⁺].

### Example 11.7 N-(4-Fluorobenzyl)-1-(2-methoxyethyl)-2,4-dioxo-3-[3-(trifluoromethyl)phenyl]-1,2,3,4-tetrahydropyrimidine-5-carboxamide

APCI-MS m/z: 466.4 [MH⁺].

### Example 11.8 N-(1,3-Benzodioxol-5-ylmethyl)-1-(2-methoxyethyl)-2,4-dioxo-3-[3-(trifluoromethyl)phenyl]-1,2,3,4-tetrahydropyrimidine-5-carboxamide

APCI-MS m/z: 492.5 [MH⁺].

### Example 11.9 N-(2-Chloro-4-fluorobenzyl)-1-(2-methoxyethyl)-2,4-dioxo-3-[3-(trifluoromethyl)phenyl]-1,2,3,4-tetrahydropyrimidine-5-carboxamide

APCI-MS m/z: 500.4 [MH⁺].

### Example 11.10 N-(3,4-Dichlorobenzyl)-1-(2-methoxyethyl)-2,4-dioxo-3-[3-(trifluoromethyl)phenyl]-1,2,3,4-tetrahydropyrimidine-5-carboxamide

APCI-MS m/z: 516.4, 518.4 [MH⁺].

### Example 11.11 Methyl 4-{[({1-(2-methoxyethyl)-2,4-dioxo-3-[3-(trifluoromethyl)phenyl]-1,2,3,4-tetrahydropyrimidin-5-yl}carbonyl)amino]methyl}benzoate

APCI-MS m/z: 506.5 [MH⁺].

### Example 11.12 1-(2-Methoxyethyl)-N-[(5-methylisoxazol-3-yl)methyl]-2,4-dioxo-3-[3-(trifluoromethyl)phenyl]-1,2,3,4-tetrahydropyrimidine-5-carboxamide

APCI-MS m/z: 453.4 [MH⁺].

### Example 11.13 1-(2-Methoxyethyl)-2,4-dioxo-N-[4-(1H-pyrazol-1-yl)benzyl]-3-[3-(trifluoromethyl)phenyl]-1,2,3,4-tetrahydropyrimidine-5-carboxamide

APCI-MS m/z: 514.5 [MH⁺].

Examples 11.14 to 11.29 were prepared from 3-(3-chlomphenyl)-1-(2-methoxyethyl)-2,4-dioxo-1,2,3,4-tetrahydropyrimidine-5-carboxylic acid and the appropriate amine using the general procedure described in Example 11:

### Example 11.14 N-(4-Chlorobenzyl)-3-(3-chlorophenyl)-1-(2-methoxyethyl)-2,4-dioxo-1,2,3,4-tetrahydropyrimidine-5-carboxamide

APCI-MS m/z: 448.4, 450.4 [MH⁺].

### Example 11.15 3-(3-Chlorophenyl)-N-(4-methoxybenzyl)-1-(2-methoxyethyl)-2,4-dioxo-1,2,3,4-tetrahydropyrimidine-5-carboxamide

APCI-MS m/z: 444.4 [MH⁺].

### Example 11.16 3-(3-Chlorophenyl)-1-(2-methoxyethyl)-2,4-dioxo-N-(pyridin-4-ylmethyl)-1,2,3,4-tetrahydropyrimidine-5-carboxamide

APCI-MS m/z: 415.4 [MH⁺].

### Example 11.17 3-(3-Chlorophenyl)-N-[2-(3,4-dimethoxyphenyl)ethyl]-1-(2-methoxyethyl)-2,4-dioxo-1,2,3,4-tetrahydropyrimidine-5-carboxamide

APCI-MS m/z: 488.5 [MH⁺].

### Example 11.18 3-(3-Chlorophenyl)-1-(2-methoxyethyl)-N-[2-(3-methoxyphenyl)ethyl]-2,4-dioxo-1,2,3,4-tetrahydropyrimidine-5-carboxamide

APCI-MS m/z: 458.5 [MH⁺].

### Example 11.19 N-(3-Bromobenzyl)-3-(3-chlorophenyl)-1-(2-methoxyethyl)-2,4-dioxo-1,2,3,4-tetrahydronyrimidine-5-carboxamide

APCI-MS m/z: 492.4, 494.3 [MH⁺].

### Example 11.20 3-(3-Chlomphenyl)-1-(2-methoxyethyl)-N-(4-methylbenzyl)-2,4-dioxo-1,2,3,4-tetrahydronyrimidine-5-carboxamide

APCI-MS m/z: 428.4 [MH⁺].

### Example 11.21 3-(3-Chlorophenyl)-1-(2-methoxyethyl)-N-[4-(methylsulfonyl)benzyl]-2,4-dioxo-1,2,3,4-tetrahydropyrimidine-5-carboxamide

APCI-MS m/z: 492.4 [MH⁺].

### Example 11.22 3-(3-Chlorophenyl)-N-(4-fluorobenzyl)-1-(2-methoxyethyl)-2,4-dioxo-1,2,3,4-tetrahydropyrimidine-5-carboxamide

APCI-MS m/z: 432.4 [MH⁺].

### Example 11.23 N-(1,3-Benzodioxol-5-ylmethyl)-3-(3-chlorophenyl)-1-(2-methoxyethyl)-2,4-dioxo-1,2,3,4-tetrahydropyrimidine-5-carboxamide

APCI-MS m/z: 458.4 [MH⁺].

### Example 11.24 3-(3-Chlorophenyl)-N-(3,4-difluorobenzyl)-1-(2-methoxyethyl)-2,4-dioxo-1,2,3,4-tetrahydropyrimidine-5-carboxamide

APCI-MS m/z: 450.3 [MH⁺].

### Example 11.25 N-(2-Chloro-4-fluorobenzyl)-3-(3-chlorophenyl)-1-(2-methoxyethyl)-2,4-dioxo-1,2,3,4-tetrahydropyrimidine-5-carboxamide

APCI-MS m/z: 466.4, 468.4 [MH⁺].

### Example 11.26 3-(3-Chlorophenyl)-N-(3,4-dichlorobenzyl)-1-(2-methoxyethyl)-2,4-dioxo-1,2,3,4-tetrahydropyrimidine-5-carboxamide

APCI-MS m/z: 482.3, 484.3 [MH⁺].

### Example 11.27 Methyl 4-[({[3-(3-chlorophenyl)-1-(2-methoxyethyl)-2,4-dioxo-1,2,3,4-tetrahydropyrimidin-5-yl]carbonyl}amino)methyl]benzoate

APCI-MS m/z: 472.4 [MH⁺].

### Example 11.28 3-(3-Chlorophenyl)-1-(2-methoxyethyl)-N-[(5-methylisoxazol-3-yl)methyl]-2,4-dioxo-1,2,3,4-tetrahydropyrimidine-5-carboxamide

APCI-MS m/z: 419.4 [MH⁺].

### Example 11.29 3-(3-Chlorophenyl)-1-(2-methoxyethyl)-2,4-dioxo-N-[4-(1H-pyrazol-1-yl)benzyl]-1,2,3,4-tetrahydropyrimidine-5-carboxamide

APCI-MS m/z: 480.5 [MH⁺].

Examples 11.30 to 11.38 were prepared from 1-butyl-3-(3-methoxyphenyl)-2,4-dioxo-1,2,3,4-tetrahydropyrimidine-5-carboxylic acid and the appropriate amine using the general procedure described in Example 11:

### Example 11.30 1-Butyl-N-(4-chlorobenzyl)-3-(3-methoxyphenyl)-2,4-dioxo-1,2,3,4-tetrahydropyrimidine-5-carboxamide

APCI-MS m/z: 442.4 [MH⁺].

### Example 11.31 1-Butyl-3-(3-methoxyphenyl)-N-[2-(3-methoxyphenyl)ethyl]-2,4-dioxo-1,2,3,4-tetrahydropyrimidine-5-carboxamide

APCI-MS m/z: 452.3 [MH⁺].

### Example 11.32 N-(3-Bromobenzyl)-1-butyl-3-(3-methoxyphenyl)-2,4-dioxo-1,2,3,4-tetrahydropyrimidine-5-carboxamide

APCI-MS m/z: 486.4,488.4 [MH⁺].

### Example 11.33 1-Butyl-N-(4-fluorobenzyl)-3-(3-methoxyphenyl)-2,4-dioxo-1,2,3,4-tetrahydropyrimidine-5-carboxamide

APCI-MS m/z: 426.3 [MH⁺].

### Example 11.34 N-(1,3-Benzodioxol-5-ylmethyl)-1-butyl-3-(3-methoxyphenyl)-2,4-dioxo-1,2,3,4-tetrahydropyrimidine-5-carboxamide

APCI-MS m/z: 452.4 [MH⁺].

### Example 11.35 1-Butyl-N-(2,4-dichlorobenzyl)-3-(3-methoxyphenyl)-2,4-dioxo-1,2,3,4-tetrahydropyrimidine-5-carboxamide

APCI-MS m/z: 476.4, 478.4 [MH⁺].

### Example 11.36 1-Butyl-N-(3,4-difluorobenzyl)-3-(3-methoxyphenyl)-2,4-dioxo-1,2,3,4-tetrahydropyrimidine-5-carboxamide

APCI-MS m/z: 444.3 [MH⁺].

### Example 11.37 1-Butyl-N-(2-chloro-4-fluorobenzyl)-3-(3-methoxyphenyl)-2,4-dioxo-1,2,3,4-tetrahydropyrimidine-5-carboxamide

APCI-MS m/z: 460.3 [MH⁺].

### Example 11.38 1-Butyl-N-(2,3-dihydro-1-benzofuran-5-ylmethyl)-3-(3-methoxyphenyl)-2,4-dioxo-1,2,3,4-tetrahydropyrimidine-5-carboxamide

APCI-MS m/z: 450.5 [MH⁺].

Examples 11.39 to 11.58 were prepared from 1-butyl-3-(3-chlorophenyl)-2,4-dioxo-1,2,3,4-tetrahydropyrimidine-5-carboxylic acid and the appropriate amine using the general procedure described in Example 11:

### Example 11.39 1-Butyl-N-(4-chlorobenzyl)-3-(3-chlorophenyl)-2,4-dioxo-1,2,3,4-tetrahydropyrimidine-5-carboxamide

APCI-MS m/z: 446.4, 448.4 [MH⁺].

### Example 11.40 1-Butyl-3-(3-chlorophenyl)-N-(4-methoxybenzyl)-2,4-dioxo-1,2,3,4-tetrahydropyrimidine-5-carboxamide

APCI-MS m/z: 442.4 [MH⁺].

### Example 11.41 1-Butyl-3-(3-chlorophenyl)-2,4-dioxo-N-(pyridin-4-ylmethyl)-1,2,3,4-tetrahydropyrimidine-5-carboxamide

APCI-MS m/z: 413.3 [MH⁺].

### Example 11.42 1-Butyl-3-(3-chlorophenyl)-N-[2-(3,4-dimethoxyphenyl)ethyl]-2,4-dioxo-1,2,3,4-tetrahydronyrimidine-5-carboxamide

APCI-MS m/z: 486.4 [MH⁺].

### Example 11.43 1-Butyl-3-(3-chlorophenyl)-N-[2-(3-methoxyphenyl)ethyl]-2,4-dioxo-1,2,3,4-tetrahydropyrimidine-5-carboxamide

APCI-MS m/z: 456.4 [MH⁺].

### Example 11.44 N-(3-Bromobenzyl)-1-butyl-3-(3-chlorophenyl)-2,4-dioxo-1,2,3,4-tetrahydropyrimidine-5-carboxamide

APCI-MS m/z: 490.3,492.3 [MH⁺].

### Example 11.45 N-(4-Bromobenzyl)-1-butyl-3-(3-chlorophenyl)-2,4-dioxo-1,2,3,4-tetrahydropyrimidine-5-carboxamide

APCI-MS m/z: 490.3,492.3 [MH⁺].

### Example 11.46 1-Butyl-3-(3-chlorophenyl)-N-(4-methylbenzyl)-2,4-dioxo-1,2,3,4-tetrahydropyrimidine-5-carboxamide

APCI-MS m/z: 426.4 [MH⁺].

### Example 11.47 1-Butyl-3-(3-chlorophenyl)-N-[4-(methylsulfonyl)benzyl]-2,4-dioxo-1,2,3,4-tetrahydropyrimidine-5-carboxamide

APCI-MS m/z: 490.4 [MH⁺].

### Example 11.48 1-Butyl-3-(3-chlorophenyl)-N-(4-fluorobenzyl)-2,4-dioxo-1,2,3,4-tetrahydronyrimidine-5-carboxamide

APCI-MS m/z: 430.4 [MH⁺].

### Example 11.49 N-(1,3-Benzodioxol-5-ylmethyl)-1-butyl-3-(3-chlorophenyl)-2,4-dioxo-1,2,3,4-tetrahydropyrimidine-5-carboxamide

APCI-MS m/z: 456.4 [MH⁺].

### Example 11.50 1-Butyl-3-(3-chlorophenyl)-N-(2,4-dichlorobenzyl)-2,4-dioxo-1,2,3,4-tetrahydropyrimidine-5-carboxamide

APCI-MS m/z: 480.3, 482.3 [MH⁺].

### Example 11.51 1-Butyl-3-(3-chlorophenyl)-N-(3,4-difluorobenzyl)-2,4-dioxo-1,2,3,4-tetrahydropyrimidine-5-carboxamide

APCI-MS m/z: 448.4 [MH⁺].

### Example 11.52 1-Butyl-N-(2-chloro-4-fluorobenzyl)-3-(3-chlorophenyl)-2,4-dioxo-1,2,3,4-tetrahydropyrimidine-5-carboxamide

APCI-MS m/z: 464.4, 466.4 [MH⁺].

### Example 11.53 1-Butyl-3-(3-chlorophenyl)-N-(3,4-dichlorobenzyl)-2,4-dioxo-1,2,3,4-tetrahydropyrimidine-5-carboxamide

APCI-MS m/z: 480.3,482.4 [MH⁺].

### Example 11.54 1-Butyl-3-(3-chlorophenyl)-N-(2,3-dihydro-1-benzofuran-5-ylmethyl)-2,4-dioxo-1,2,3,4-tetrahydropyrimidine-5-carboxamide

APCI-MS m/z: 452.4 [MH⁺].

### Example 11.55 1-Butyl-3-(3-chlorophenyl)-N-[(4-cyanocyclohexyl)methyl]-2,4-dioxo-1,2,3,4-tetrahydropyrimidine-5-carboxamide

APCI-MS m/z: 443.3 [MH⁺].

### Example 11.56 1-Butyl-3-(3-chlorophenyl)-N-[(5-methylisoxazol-3-yl)methyl]-2,4-dioxo-1,2,3,4-tetrahydropydmidine-5-carboxamide

APCI-MS m/z: 417.3 [MH⁺].

### Example 11.57 1-Butyl-3-(3-chlorophenyl)-2,4-dioxo-N-[4-(1H-pyrazol-1-yl)benzyl]-1,2,3,4-tetrahydropyrimidine-5-carboxamide

APCI-MS m/z: 478.4 [MH⁺].

### Example 11.58 1-Butyl-3-(3-chlorophenyl)-2,4-dioxo-N-[3-(2-oxopyrrolidin-1-yl)propyl]-1,2,3,4-tetrahydropyrimidine-5-carboxamide

APCI-MS m/z: 447.3 [MH⁺].

Examples 11.59 to 11.77 were prepared from 1-butyl-3-(3-cyanophenyl)-2,4-dioxo-1,2,3,4-tetrahydropyrimidine-5-carboxylic acid and the appropriate amine using the general procedure described in Example 11:

### Example 11.59 1-Butyl-N-(4-chlorobenzyl)-3-(3-cyanophenyl)-2,4-dioxo-1,2,3,4-tetrahydropyrimidine-5-carboxamide

APCI-MS m/z: 437.4 [MH⁺].

### Example 11.60 1-Butyl-3-(3-cyanophenyl)-N-(4-methoxybenzyl)-2,4-dioxo-1,2,3,4-tetrahydropyrimidine-5-carboxamide

APCI-MS m/z: 433.5 [MH⁺].

### Example 11.61 1-Butyl-3-(3-cyanophenyl)-2,4-dioxo-N-(pyridin-4-ylmethyl)-1,2,3,4-tetrahydropyrimidine-5-carboxamide

APCI-MS m/z: 404.3 [MH⁺].

### Example 11.62 1-Butyl-3-(3-cyanophenyl)-N-[2-(3,4-dimethoxyphenyl)ethyl]-2,4-dioxo-1,2,3,4-tetrahydropyrimidine-5-carboxamide

APCI-MS m/z: 477.4 [MH⁺].

### Example 11.63 1-Butyl-3-(3-cyanophenyl)-N-[2-(3-methoxyphenyl)ethyl]-2,4-dioxo-1,2,3,4-tetrahydropyrimidine-5-carboxamide

APCI-MS m/z: 447.3 [MH⁺].

### Example 11.64 N-(3-Bromobenzyl)-1-butyl-3-(3-cyanophenyl)-2,4-dioxo-1,2,3,4-tetrahydropyrimidine-5-carboxamide

APCI-MS m/z: 481.4, 483.4 [MH⁺].

### Example 11.65 N-(4-Bromobenzyl)-1-butyl-3-(3-cyanophenyl)-2,4-dioxo-1,2,3,4-tetrahydropyrimidine-5-carboxamide

APCI-MS m/z: 481.4, 483.4 [MH⁺].

### Example 11.66 1-Butyl-3-(3-cyanophenyl)-N-(4-methylbenzyl)-2,4-dioxo-1,2,3,4-tetrahydropyrimidine-5-carboxamide

APCI-MS m/z: 417.4 [MH⁺].

### Example 11.67 1-Butyl-3-(3-cyanophenyl)-N-[4-(methylsulfonyl)benzyl]-2,4-dioxo-1,2,3,4-tetrahydropyrimidine-5-carboxamide

APCI-MS m/z: 481.4 [MH⁺].

### Example 11.68 1-Butyl-3-(3-cyanophenyl)-N-(4-fluorobenzyl)-2,4-dioxo-1,2,3,4-tetrahydropyrimidine-5-carboxamide

APCI-MS m/z: 421.3 [MH⁺].

### Example 11.69 N-(1,3-Benzodioxol-5-ylmethyl)-1-butyl-3-(3-cyanophenyl)-2,4-dioxo-1,2,3,4-tetrahydropyrimidine-5-carboxamide

APCI-MS m/z: 447.4 [MH⁺].

### Example 11.70 1-Butyl-3-(3-cyanophenyl)-N-(2,4-dichlorobenzyl)-2,4-dioxo-1,2,3,4-tetrahydropyrimidine-5-carboxamide

APCI-MS m/z: 471.4,473.4 [MH⁺].

### Example 11.71 1-Butyl-3-(3-cyanophenyl)-N-(3,4-difluorobenzyl)-2,4-dioxo-1,2,3,4-tetrahydropyrimidine-5-carboxamide

APCI-MS m/z: 439.4 [MH⁺].

### Example 11.72 1-Butyl-N-(2-chloro-4-fluorobenzyl)-3-(3-cyanophenyl)-2,4-dioxo-1,2,3,4-tetrahydropyrimidine-5-carboxamide

APCI-MS m/z: 455.4 [MH⁺].

### Example 11.73 1-Butyl-3-(3-cyanophenyl)-N-(3,4-dichlorobenzyl)-2,4-dioxo-1,2,3,4-tetrahydropyrimidine-5-carboxamide

APCI-MS m/z: 471.4, 473.5 [MH⁺].

### Example 11.74 1-Butyl-N-[(4-cyanocyclohexyl)methyl]-3-(3-cyanophenyl)-2,4-dioxo-1,2,3,4-tetrahydropyrimidine-5-carboxamide

APCI-MS m/z: 434.5 [MH⁺].

### Example 11.75 1-Butyl-3-(3-cyanophenyl)-N-[(5-methylisoxazol-3-yl)methyl]-2,4-dioxo-1,2,3,4-tetrahydropyrimidine-5-carboxamide

APCI-MS m/z: 408.4 [MH⁺].

### Example 11.76 1-Butyl-3-(3-cyanophenyl)-2,4-dioxo-N-[4-(1H-pyrazol-1-yl)benzyl]-1,2,3,4-tetrahydropyrimidine-5-carboxamide

APCI-MS m/z: 469.5 [MH⁺].

### Example 11.77 1-Butyl-3-(3-cyanophenyl)-2,4-dioxo-N-[3-(2-oxopyrrolidin-1-yl)propyl]-1,2,3,4-tetrahydropyrimidine-5-carboxamide

APCI-MS m/z: 438.4 [MH⁺].

Examples 11.78 to 11.97 were prepared from 1-butyl-3-(3-(trifluoromethyl)phenyl)-2,4-dioxo-1,2,3,4-tetrahydropyrimidine-5-carboxylic acid and the appropriate amine using the general procedure described in Example 11:

### Example 11.78 1-Butyl-N-(4-chlorobenzyl)-2,4-dioxo-3-[3-(trifluoromethyl)phenyl]-1,2,3,4-tetrahydropyrimidine-5-carboxamide

APCI-MS m/z: 480.4, 482.4 [MH⁺].

### Example 11.79 1-Butyl-N-(4-methoxybenzyl)-2,4-dioxo-3-[3-(trifluoromethyl)phenyl]-1,2,3,4-tetrahydropyrimidine-5-carboxamide

APCI-MS m/z: 476.5 [MH⁺].

### Example 11.80 1-Butyl-2,4-dioxo-N-(pyridin-4-ylmethyl)-3-[3-(trifluoromethyl)phenyl]-1,2,3,4-tetrahydropyrimidine-5-carboxamide

APCI-MS m/z: 447.4 [MH⁺].

### Example 11.81 1-Butyl-N-[2-(3,4-dimethoxyphenyl)ethyl]-2,4-dioxo-3-[3-(trifluoromethyl)phenyl]-1,2,3,4-tetrahydropyrimidine-5-carboxamide

APCI-MS m/z: 520.4 [MH⁺].

### Example 11.82 1-Butyl-N-[2-(3-methoxyphenyl)ethyl]-2,4-dioxo-3-[3-(trifluoromethyl)phenyl]-1,2,3,4-tetrahydropyrimidine-5-carboxamide

APCI-MS m/z: 490.4 [MH⁺].

### Example 11.83 N-(3-Bromobenzyl)-1-butyl-2,4-dioxo-3-[3-(trifluoromethyl)phenyl]-1,2,3,4-tetrahydropyrimidine-5-carboxamide

APCI-MS m/z: 524.4, 526.5 [MH⁺].

### Example 11.84 N-(4-Bromobenzyl)-1-butyl-2,4-dioxo-3-[3-(trifluoromethyl)phenyl]-1,2,3,4-tetrahydropyrimidine-5-carboxamide

APCI-MS m/z: 524.4, 526.5 [MH⁺].

### Example 11.85 1-Butyl-N-(4-methylbenzyl)-2,4-dioxo-3-[3-(trifluoromethyl)phenyl]-1,2,3,4-tetrahydropyrimidine-5-carboxamide

APCI-MS m/z: 460.4 [MH⁺].

### Example 11.86 1-Bulyl-N-[4-(methylsulfonyl)bonzyl]-2,4-dioxo-3-[3-(trifluoromethyl)phenyl]-1,2,3,4-tetrahydropyrimidine-5-carboxamide

APCI-MS m/z: 524.5 [MH⁺].

### Example 11.87 1-Butyl-N-(4-fluorobenzyl)-2,4-dioxo-3-[3-(trifluoromethyl)phenyl]-1,2,3,4-tetrahydropyrimidine-5-carboxamide

APCI-MS m/z: 464.4 [MH⁺].

### Example 11.88 N-(1,3-Benzodioxol-5-ylmethyl)-1-butyl-2,4-dioxo-3-[3-(trifluoromethyl)phenyl]-1,2,3,4-tetrahydropyrimidine-5-carboxamide

APCI-MS m/z: 490.5 [MH⁺].

### Example 11.89 1-Butyl-N-(2,4-dichlorobenzyl)-2,4-dioxo-3-[3-(trifluoromethyl)phenyl]-1,2,3,4-tetrahydropyrimidine-5-carboxamide

APCI-MS m/z: 514.4, 516.4 [MH⁺].

### Example 11.90 1-Butyl-N-(3,4-difluorobenzyl)-2,4-dioxo-3-[3-(trifluoromethyl)phenyl]-1,2,3,4-tetrahydropyrimidine-5-carboxamide

APCI-MS m/z: 482.5 [MH⁺].

### Example 11.91 1-Butyl-N-(2-chloro-4-fluorobenzyl)-2,4-dioxo-3-[3-(trifluoromethyl)phenyl]-1,2,3,4-tetrahydropyrimidine-5-carboxamide

APCI-MS m/z: 498.4, 500.4 [MH⁺].

### Example 11.92 1-Butyl-N-(3,4-dichlorobenzyl)-2,4-dioxo-3-[3-(trifluoromethyl)phenyl]-1,2,3,4-tetrahydropyrimidine-5-carboxamide

APCI-MS m/z: 514.4, 516.4 [MH⁺].

### Example 11.93 1-Butyl-N-(2,3-dihydro-1-benzofuran-5-ylmethyl)-2,4-dioxo-3-[3-(trifluoromethyl)phenyl]-1,2,3,4-tetrahydropyrimidine-5-carboxamide

APCI-MS m/z: 486.5 [MH⁺].

### Example 11.94 1-Butyl-N-[(4-cyanocyclohexyl)methyl]-2,4-dioxo-3-[3-(trifluoromethyl)phenyl]-1,2,3,4-tetrahydropyrimidine-5-carboxamide

APCI-MS m/z: 477.5 [MH⁺].

### Example 11.95 1-Butyl-N-[(5-methylisoxazol-3-yl)methyl]-2,4-dioxo-3-[3-(trifluoromethyl)phenyl]-1,2,3,4-tetrahydropyrimidine-5-carboxamide

APCI-MS m/z: 451.3 [MH⁺].

### Example 11.96 1-Butyl-2,4-dioxo-N-[4-(1H-pyrazol-1-yl)benzyl]-3-[3-(trifluoromethyl)phenyl]-1,2,3,4-tetrahydropyrimidine-5-carboxamide

APCI-MS m/z: 512.5 [MH⁺].

### Example 11.97 1-Butyl-2,4-dioxo-N-[3-(2-oxopyrrolidin-1-yl)propyl]-3-[3-(trifluoromethyl)phenyl]-1,2,3,4-tetrahydropyrimidine-5-carboxamide

APCI-MS m/z: 481.4 [MH⁺].

### Example 12 6-(Chloromethyl)-N-[4-(methylsulfonyl)benzyl]-2-oxo-1-[3-(trifluoromethyl)phenyl]-1,2-dihydropyridine-3-carboxamide

The title compound was prepared by heating 6-(hydroxymethyl)-N-[4-(methylsulfonyl)benzyl]-2-oxo-1-[3-(trifluoromethyl)phenyl]-1,2-dihydropyridine-3-carboxamide (48 mg, 0.1 mmol) and an excess of thionyl chloride (1 ml) in CH₂Cl₂ (10 ml) for 1 h. Removal of the solvents afforded the product (50 mg, 100%) as a white solid after trituration from diethyl ether.
¹H-NMR (DMSO-d₆): δ 9.93 (1H, brt); 8.67 (1H, d); 7.88 (2H, d); 7.86 (1H, d); 7.76 (1H, t); 7.61 (1H, s); 7.54 (1H, d); 7.52 (2H, d); 6.75 (1H, d); 4.68 (2H, m); 4.12 (2H, s); 3.02 (3H, s).
APCI-MS m/z: 499 [MH⁺].

### Example 13 N-[4-(Methylsulfonyl)benzyl]-6-[(methylthio)methyl]-2-oxo-1-[3-(trifluoromethyl)phenyl]-1,2-dihydropyridine-3-carboxamide

The title compound was prepared by stirring 6-(chloromethyl)-N-[4-(methylsulfonyl)benzyl]-2-oxo-1-[3-(trifluoromethyl)phenyl]-1,2-dihydropyridine-3-carboxamide (40 mg, 0.08 mmol) with an excess of sodium methylthiolate (28 mg, 0.4 mmol) in NMP (10 ml) at room temperature overnight. Water was added and the reaction mixture was extracted with EtOAc. The crude product was purified by column chromatography on silica using EtOAc/heptane (4:1) as eluent to afford 15 mg (37%) of the title compound.
¹H-NMR (CDCl₃): δ 9.97 (1H, brt); 8.63 (1H, d); 7.88 (2H, d); 7.81 (1H, d); 7.73 (1H, t); 7.62 (1H, s); 7.53 (3H, m); 6.51 (1H, d); 4.67 (2H, m); 3.26 (2H, d); 3.02 (3H, s); 2.03 (3H, s).
APCI-MS m/z: 511 [MH⁺].

### Example 14 N-[4-(Methylsulfonyl)benzyl]-6-({[4-(methylsulfonyl)benzyl]amino}methyl)-2-oxo-1-[3-(trifluoromethyl)phenyl]-1,2-dihydropyridine-3-carboxamide

The title compound was prepared by stirring 6-(chloromethyl)-N-[4-(methylsulfonyl)benzyl]-2-oxo-1-[3-(trifluoromethyl)phenyl]-1,2-dihydropyridine-3-carboxamide with an excess of 4-(methylsulfonyl) benzylamine hydrochloride and DIEA in NMP at room temperature overnight. The crude product was purified by preparative HPLC.
APCI-MS m/z: 648 [MH⁺].

### Example 15 N-[4-(Methylsulfonyl)benzyl]-6-(morpholin-4-ylmethyl)-2-oxo-1-[3-(trifluoromethyl)phenyl]-1,2-dihydropyridine-3-carboxamide

The title compound was prepared by stirring 6-(chloromethyl)-N-[4-(methylsulfonyl)benzyl]-2-oxo-1-[3-(trifluoromethyl)phenyl]-1,2-dihydropyridine-3-carboxamide with an excess of morpholine in THF at 50 °C overnight. The crude product was purified by preparative HPLC.
APCI-MS m/z: 550 [MH⁺].

### Example 16 6-(Cyanomethyl)-N-[4-(methylsulfonyl)benzyl]-2-oxo-1-[3-(trifluoromethyl)phenyl]-1,2-dihydropyridine-3-carboxamide

The title compound was prepared by stirring 6-(chloromethyl)-N-[4-(methylsulfonyl)benzyl]-2-oxo-1-[3-(trifluoromethyl)phenyl]-1,2-dihydropyridine-3-carboxamide with an excess of sodium cyanide in THF at 50 °C overnight. The crude product was purified by preparative HPLC.
APCI-MS m/z: 490 [MH⁺].

### Example 17 6-Isopropyl-N-[4-(methylsulfonyl)benzyl]-2-oxo-1-[3-(trifluoromethyl)phenyl]-1,2-dihydropyridine-3-carboxamide

### a) 6-Isonropyl-2-oxo-1-[3-(trifluoromethyl)phenyl]-1,2-dihydropyridine-3-carboxylic acid

A mixture of ethyl 3-oxo-3-{[3-(trifluoromethyl)phenyl]amino}propanoate (5.5 g, 20 mmol), 1-methoxy-4-methylpent-1-en-3-one (prepared using the method of S.M. Bromidge et al, *Synth. Commun*., **23(4),** 487-494 (1993) (2.7 g, 21 mmol) and sodium methoxide (2 g, 40 mmol) in ethanol (50 ml) was heated to reflux for 5 h and then cooled. Water (50 ml) and 2M NaOH were added and the mixture stirred at room temperature overnight. The organic solvents were removed and the reaction mixture extracted with EtOAc. The aqueous phases were acidified with 0.5M citric acid to pH 3-4, the precipitate formed was filtered off, washed with water and dried to afford 0.4 g (6%) of the title compound as a brownish powder.
¹H-NMR (CDCl₃): δ 13.74 (1H, s); 8.59 (1H, d); 7.87 (1H, d); 7.78 (1H, t); 7.54 (1H, brs); 7.48 (1H, d); 6.64 (1H, d); 2.54 (1H, m); 1.20 (6H, t).
APCI-MS m/z: 326 [MH⁺].

### b) 6-Isopropyl-N-[4-(methylsulfonyl)benzyl]-2-oxo-1-[3-(trifluoromethyl)phenyl]-1,2-dihydropyridine-3-carboxamide

To a mixture of 6-isopropyl-2-oxo-1-[3-(trifluoromethyl)phenyl]-1,2-dihydropyridine-3-carboxylic acid (98 mg, 0.3 mmol), HATU (126 mg, 0,33 mmol), HOAT (45 mg, 0,33 mmol) and DIEA (162 µl, 0.95 mmol) in NMP (3 ml) was added 4-(methylsulfonyl) benzylamine hydrochloride (69 mg, 0.31 mmol), the pH was adjusted to approx. pH 8-9 with DIEA.
The mixture was stirred for 1 h at room temperature. EtOAc was added and the organic phase washed twice with aqueous sodium hydrogen carbonate, 0.5M citric acid and water. The solvent was removed in vacuo and the residue was purified by column chromatography on silica using CH₂Cl₂/EtOAc (4:1) as eluent to afford the title compound in quantitative yield.
¹H-NMR (CDCl₃): δ 9.99 (1H, brt); 8.65 (1H, d); 7.87 (2H, d); 7.82 (1H, d); 7.74 (1H, t); 7.51 (3H, m); 7.44 (1H, d); 6.54 (1H, d); 4.67 (2H, m); 3.01 (3H, s); 2.49 (1H, m); 1.17 (6H, t).
APCI-MS m/z: 493 [MH⁺].

### Example 18 N-[4-(Ethylsulfonyl)benzyl]-6-methyl-2-oxo-1-[3-(trifluoromethyl)phenyl]-1,2-dihydropyridine-3-carboxamide

### a) tert-Butyl [4-(methylsulfonyl)benzyl]carbamate

To a solution of [4-(methylsulfonyl)benzyl]amine (600 mg, 2.7 mmol) in THF (9 ml), di-tert-butyldicarbonate (590 mg, 2.7 mmol) and DIEA (926 µl, 5.4 mmol) were added and the mixture was stirred overnight. After removal of the solvent the crude product was purified by flash chromatography to give the subtitle compound (650 mg, 84 %).
¹H-NMR (CDCl₃): δ 7.91 (2H, d); 7.49 (2H, d); 5.00 (1H, bs); 4.42 (2H, d); 3.05 (3H, s); 1.48 (9H, s).
APCI-MS m/z: 169.1, 186.1 [MH⁺].

### b) tert-Butyl [4-(ethylsulfonyl)benzyl]carbamate

To a solution of tert-butyl [4-(methylsulfonyl)benzyl]carbamate (400 mg, 1.4 mmol) in THF (5 ml) at -72 °C, n-butyl lithium (1750 µl, 2.8 mmol) was added dropwise while maintaining the temperature at -70 °C. After addition the temperature was allowed to rise to -40 °C and methyl iodide (105 µl, 1.7 mmol) added. The mixture was stirred for 1 h, an aqueous solution of NH₄Cl was added and then the mixture was extracted with EtOAc. The organic layer was washed with NH₄Cl solution and evaporated. The crude product was purified by preparative HPLC to give the subtitle compound (148 mg, 35%).
¹H-NMR (CDCl₃): δ 7.88 (2H, d); 7.49 (2H, d); 4.98 (1H, bs); 4.43 (2H, bs); 3.11 (2H, q); 1.48 (9H, s); 1.28 (3H, t).
APCI-MS m/z: 183.2, 200.2 [MH⁺].

### c) [4-(Ethylsulfonyl)benzyl]amine trifluoroacetate

*Tert*-butyl [4-(ethylsulfonyl)benzyl]carbamate (148 mg, 0.5 mmol) was stirred in 10% TFA in CH₂Cl₂ for 3 h. The solvent was evaporated leaving the title compound (191 mg).
APCI-MS m/z: 200.1 [MH⁺].

### d) N-[4-(Ethylsulfonyl)benzyl]-6-methyl-2-oxo-1-[3-(trifluoromethyl)phenyl]-1,2-dihydropyridine-3-carboxamide

The title compound was obtained from 4-(ethylsulfonyl)benzylamine trifluoroacetate and 1-(3-methylphenyl)-2-oxo-1,2-dihydropyridine-3-carboxylic acid using a procedure analogous to that described in Example 17.
¹H-NMR (CDCl₃): δ 9.98 (1H, t); 8.59 (1H, d); 7.84-7.81 (3H, m); 7.74 (1H, t); 7.52-7.50 (3H, m); 7.45 (1H, d); 6.48 (1H, d); 4.74-4.63 (2H, m); 3.08 (2H, q); 2.09 (3H, t); 1.26 (3H, t).
APCI-MS m/z: 479.3 [MH⁺].

### Example 19 N-[3-Chloro-4-(methylsulfonyl)benzyl]-6-methyl-2-oxo-1-[3-(trifluoromethyl)phenyl]-1,2-dihydropyridine-3-carboxamide

### a) 3-Chloro-4-(methylthio)benzaldehyde

To a stirred solution of 2-chlorothioanisole (1.25 ml, 9.5 mmol) in CH₂Cl₂ at -5 °C, titanium(IV) chloride (2076 µl, 18.9 mmol) was added dropwise over 10 min, while maintaining the temperature below 0 °C . After addition the mixture was stirred for 10 min before α,α-dichloromethyl methyl ether (0.94 ml, 10.4 mmol) was added dropwise maintaining the temperature below 0 °C. After addition, the mixture was allowed to reach ambient temperature and poured onto a saturated aqueous solution of sodium bicarbonate (70 ml). The mixture was filtered through a plug of celite, washing with CH₂Cl₂. The phases were separated and the aqueous phase was extracted with CH₂Cl₂. The combined organic phases were washed with brine and the solvent removed in vacuo. The crude product was purified by flash chromatography to yield the subtitle product (535 mg, 30%).
¹H-NMR (CDCl₃): δ 9.91 (1H, s); 7.84 (1H, d); 7.75 (1H, dd); 7.27 (1H, d); 2.56 (3H, s).
APCI-MS m/z: 187.1 [MH⁺].

### b) [3-Chloro-4-(methylthio)phenyl]methanol

To a solution of 3-chloro-4-(methylthio)benzaldehyde (332 mg, 1.8 mmol) in THF (6 ml) and water (0.6 ml), NaBH₄ (269 mg, 7.1 mmol) was added. The mixture was stirred for 2 h. The reaction was quenched with 1M aqueous HCl and EtOAc was added. After separating the phases, the organic layer was washed with water, dried and evaporated to afford the subtitle compound (350 mg, 91%).
¹H-NMR (CDCl₃): δ 7.39 (1H, d); 7.26 (1H, dd); 7.17 (1H, d); 4.66 (2H, s); 2.49 (3H, s).
APCI-MS m/z: 170.9 [MH⁺].

### c) [3-Chloro-4-(methylsulfonyl)phenyl]methanol

[3-Chloro-4-(methylthio)phenyl]methanol (350 mg, 1.9 mmol) was dissolved in aqueous sodium hydroxide (0.5M, 4.44 ml) and stirred for 20 min, sodium bicarbonate (1.23 g) and acetone (1.5 ml) were added followed by addition of Oxone® (1.85 g, 3.0 mmol) in EDTA (6.5 ml, 0.4 mM). After stirring for 2 h the reaction was quenched by addition of sodium bisulphite (0.9 g) in water. EtOAc was added and the solution was acidified with aqueous 2M HCl. Sodium chloride was added to the aqueous which was then extracted with EtOAc. The organic layers were combined and washed with water, brine and dried. Evaporation afforded the subtitle compound as a white solid (359 mg, 88%).
¹H-NMR (CDCl₃): δ 8.12 (1H, d); 7.60 (1H, d); 7.44 (1H, dd); 4.81 (2H, s); 3.27 (3H, s).

### d) 4-(Bromomethyl)-2-chloro-1-(methylsulfonyl)benzene

[3-Chloro-4-(methylsulfonyl)phenyl]methanol (239 mg, 1.1 mmol) was added to dioxane. The slurry was stirred and heated to 40 °C before addition of PBr₃ (71 µl, 0.8 mmol). The reaction was heated to 100 °C for 1 h and then allowed to cool. Water was added and the mixture extracted with EtOAc. The organic layer was washed with water followed by brine and dried. Evaporation afforded the subtitle compound (310 mg, 100%).
¹H-NMR (CDCl₃): δ 8.14 (1H, d); 7.61 (1H, d); 7.49 (1H, dd); 4.46 (2H, s); 3.28 (3H, s).

### e) [3-Chloro-4-(methylsulfonyl)benzyl]amine

4-(Bromomethyl)-2-chloro-1-(methylsulfonyl)benzene (160 mg, 0.56 mmol) was dissolved in methanol (3 ml) and added to an equal mixture of ammonia (28%) and methanol (10 ml). The reaction was stirred at 40 °C for 1 h and evaporated. The crude product was dissolved in EtOAc and aqueous 6M sulphuric acid. The aqueous phase was separated, adjusted to pH 14 using sodium hydroxide and extracted with CH₂Cl₂. The CH₂Cl₂ phase was dried and evaporated to afford the title compound (65 mg, 53%).
APCI-MS m/z: 219.9 [MH⁺].

### f) N-[3-Chloro-4-(methylsulfonyl)benzyl]-6-methyl-2-oxo-1-[3-(trifluoromethyl)phenyl]-1,2-dihydropyridine-3-carboxamide

The title compound was prepared as described in Example 17.
¹H-NMR (CDCl₃): δ 10.03 (1H, t); 8.58 (1H, d); 8.07 (1H, d); 7.82 (1H, d); 7.75 (1H, t); 7.52 (2H, d); 7.45 (1H, d); 7.41 (1H, d); 6.49 (1H, d); 4.69-4.57 (2H, m); 3.23 (3H, s); 2.10 (3H, s).
APCI-MS m/z: 499.0 [MH⁺].

### Example 20 6-Methyl-2-oxo-1-(3-trifluoromethyl-phenyl)-1,2-dihydro-pyridine-3-carboxylic acid 4-cyclopropanesulfonyl-benzylamide

### a) 4-Cyclopropanesulfonyl-benzylamine

1-Cyclopropanesulfonyl-4-methyl-benzene, prepared by the method of W. E. Truce, et al, *J. Org. Chem*., 1961, 26, 1463-1467, (190 mg, 0.969 mmol), N-bromosuccinimide (190 mg, 1.07 mmol) and benzoyl peroxide (12 mg) in carbon tetrachloride (4 ml) were heated under reflux for 24 h, cooled, filtered and concentrated. The residue in methanol (1 ml) was added in portions during 10 min to a solution of ammonium hydroxide (28 %, 2 ml) in methanol (2 ml). After 2 h the solution was partitioned between EtOAc (10 ml) and sulphuric acid (0.4M, 10 ml), the pH of the aqueous layer was adjusted to 14 using 2M aqueous KOH and then extracted three times with dichloromethane. The organic phase was dried and concentrated to give the subtitle compound (83 mg).
¹H-NMR (CDCl₃): δ 7.78 (2H, bd); 7.35 (2H, bd); 2.46 (3H, s); 2.46 (1H, m); 1.34 (2H, m); 1.02 (2H, m).
APCI-MS m/z: 212 [MH⁺].
The corresponding dibenzyl amine (12%) is also present.

### b) 6-Methyl-2-oxo-1-(3-trifluoromethyl-phenyl)-1,2-dihydro-pyridine-3-carboxylic acid 4-cyclopropanesulfonyl-benzylamide

The title compound was obtained from 4-cyclopropanesulfonyl-benzylamine and 1-(3-methylphenyl)-2-oxo-1,2-dihydropyridine-3-carboxylic acid by a method analogous to that described in Example 17.
¹H-NMR (CDCl₃): δ 9.93 (1H, brt); 8.38 (1H, d); 7.89 (2H, bd); 7.84-7.80 (3H, m); 7.73 (1H, bd); 7.53 (2H, d); 6.26 (1H, d); 4.58 (2H, d); 2.80 (1H, m); 2.02 (3H, s); 1.08 (2H, m); 1.01 (2H, m).
APCI-MS m/z: 491.1 [MH⁺].

### Example 21 N-[3-Methoxy-4-(methylsulfonyl)benzyl]-6-methyl-2-oxo-1-[3-(trifluoromethyl)phenyl]-1,2-dihydropyridine-3-carboxamide

### a) [3-Methoxy-4-(methylsulfonyl)phenyl]methanol

[3-Methoxy-4-(methylthio)phenyl]methanol, prepared as described by Garcia et al. *Supramolecular Chemistry*, 1992,1, 31-45, (75 mg, 0.4 mmol) was dissolved in aqueous sodium hydroxide (0.5M, 1.22 ml) and stirred for 30 min. Further reaction as in Example 19 (c) gave, after trituation with toluene, the subtitle compound as white crystals (47 mg, 54%).
¹H-NMR (CDCl₃): δ 7.97 (d, J 8.0 Hz, 1H); 7.15 (s, 1H); 7.08 (d, J 8.0 Hz, 1H); 4.81 (d, *J* 5.4 Hz, 2H); 4.04 (s, 3H); 3.32 (s, 3H).
APCI-MS m/z: 217 [MH⁺].

### b) 4-(Bromomethyl)-2-methoxy-1-(methylsulfonyl)benzene

To a slurry of [3-methoxy-4-(methylsulfonyl)phenyl]methanol (69 mg, 0.32 mmol) and toluene at 40 °C was added PBr₃ (30 µl, 0.32 mmol). The reaction was heated to 100 °C for 1 h, cooled and water added. The reaction mixture was diluted with EtOAc, washed with water and brine and dried. Evaporation afforded the subtitle compound as a pale yellow oil (62 mg, 69%).
¹H-NMR (CDCl₃): δ 7.97 (d, J 8.0 Hz, 1H); 7.14 (dd, J 8.0, 1.4 Hz, 1H); 7.10 (d, J 1.2 Hz, 1H); 4.50 (s, 2H); 4.05 (s, 3H); 3.24 (s, 3H).
APCI-MS m/z: 280 [MH⁺].

### c) 3-Methoxy-4-(methylsulfonyl)benzylamine

4-(Bromomethyl)-2-methoxy-1-(methylsulfonyl)benzene (62 mg, 0.22 mmol) in methanol was slowly added to an equal mixture of ammonia (28%) and methanol. The reaction was stirred at room temperature for 2 h and evaporated. The reaction mixture was partitioned was between EtOAc and aqueous 6M sulphuric acid The aqueous phase was made alkaline with sodium hydroxide and extracted with CH₂Cl₂. The CH₂Cl₂ phase was dried and evaporated to afford the title compound (36 mg, 76%).
APCI-MS m/z: 216 [MH⁺].

### d) N-[3-Methoxy-4-(methylsulfonyl)benzyl]-6-methyl-2-oxo-1-[3-(trifluoromethyl)phenyl]-1,2-dihydropyridine-3-carboxamide

The title compound was obtained from 3-methoxy-4-(methylsulfonyl)benzylamine and 1-(3-methylphenyl)-2-oxo-1,2-dihydropyridine-3-carboxylic acid by a method analogous to that described in Example 17.
¹H-NMR (CDCl₃): δ 10.03 (s, 1H); 8.60 (d, *J* 7.5 Hz, 1H); 7.91 (d, J 8.1 Hz, 1H); 7.83 (d, J 8.3 Hz, 1H); 7.76 (t, *J* 7.8 Hz, 1H); 7.54 (d, *J* 4.7 Hz, 1H); 7.46 (d, J 7.2 Hz, 1H); 6.50 (d, J 7.2 Hz, 1H); 7.05 (td, J 7.9, 5.3 Hz, 2H); 4.64 (t, J 6.3 Hz, 2H); 3.98 (s, 3H); 3.19 (s, 3H); 2.11 (s, 3H).
APCI-MS m/z: 495 [MH⁺].

### Example 22 N-[3-Bromo-4-(methylsulfonyl)benzyl]-6-methyl-2-oxo-1-[3-(trifluoromethyl)phenyl]-1,2-dihydropyridine-3-carboxamide

### a) 3-Bromo-4-(methylthio)benzaldehyde

To a solution of 3-bromo-4-fluorobenzaldehyde (0.5 g, 2.5 mmol) in NMP (10 ml), potassium carbonate (0.68 g, 4.92 mmol) and sodium metanethiolate (0.26 g, 3.69 mmol) were added. The mixture was heated to 70 °C for 7 h, cooled, and partitioned between EtOAc and water. The organic phase was dried, filtered, evaporated and purified by column chromatography on silica using heptane/EtOAc (4:1) as eluent to afford the title compound (0.25 g, 44%).
¹H-NMR (CDCl₃): δ 9.89 (1H, s); 8.00 (1H, d); 7.79 (1H, dd); 7.23 (1H, d); 2.54 (3H, s).
APCI-MS m/z: 465.3 [MH⁺].

### b) [3-Bromo-4-(methylthio)phenyl]methanol

To a solution of 3-bromo-4-(methylthio)benzaldehyde (0.25 g, 1.08 mmol) in methanol (15 ml) was added sodium borohydride (0.2 g, 5.4 mmol). The solution was stirred at room temperature for 4 h, water was added, and the mixture was extracted with CH₂Cl₂. The organic layer was dried, filtered and evaporated to give 0.24 g (95%) of the title compound.
¹H-NMR (CDCl₃): δ 7.55 (1H, d); 7.29 (1H, dd); 7.12 (1H, d); 4.64 (2H, s); 2.48 (3H, s).

### c) [3-Bromo-4-(methylsulfonyl)phenyl]methanol

A suspension of sodium hydroxide (2.5 ml, 1.25 mmol) and [3-bromo-4-(methylthio)phenyl]methanol (0.24 g, 1.03 mmol) was stirred at ambient temperature for 20 min, then sodium bicarbonate (0.69 g, 8.2 mmol) and acetone (1 ml) were added, followed by a Oxone® solution (1.6 g in 6 ml of 0.4 mM EDTA) added over 10 min. The suspension was stirred overnight at room temperature. EtOAc was added and the solution was acidified with 5M HCl. The organic phase was washed several times with water and then dried, filtered and evaporated to give the title compound 0.19 g (70%).
APCI-MS m/z: 249.1, 251 [MH⁺].

### d) 2-Bromo-4-(bromomethyl)-1-(methylsulfonyl)benzene

[3-Bromo-4-(methylsulfonyl)phenyl]methanol (0.19 g, 0.72 mmol) was mixed with toluene (5 ml) and phosphorus tribromide (30 µl, 0.32 mmol) at 40 °C, and the mixture was stirred at 100 °C for 20 min. EtOAc (100 ml) was added to the cooled solution and then washed with water. The organic phase was dried, filtered and evaporated to give 0.23 g (97%) of the title compound.
¹H-NMR (DMSO-d₆): δ 8.06 (1H, d); 8.02 (1H, d); 7.73 (1H, dd); 4.76 (2H, s); 3.38 (3H, s).

### e) [3-Bromo-4-(methylsulfonyl)benzyl]amine

A solution of 2-bromo-4-(bromomethyl)-1-(methylsulfonyl)benzene (230 mg, 0.70 mmol) in methanol (3 ml) and THF (1 ml) was added to NH₄OH (7 ml, 28%) during 30 min. After 4 h the solution was acidified with 0.5M HCl, washed twice with CH₂Cl₂ and then basified to pH 14 with 5M sodium hydroxide. The water phase was extracted with CH₂Cl₂, and the organic layer was dried, filtered and evaporated to give 80 mg (43%) of the title compound.
¹H-NMR (DMSO-d₆): δ 7.99 (1H, d); 7.89 (1H, d); 7.58 (1H, dd); 3.79 (2H, s); 3.31 (3H, s).
APCI-MS m/z: 264, 266 [MH⁺].

### f) N-[3-Bromo-4-(methylsulfonyl)benzyl]-6-methyl-2-oxo-1-[3-(trifluoromethyl)phenyl]-1,2-dihydropyridine-3-carboxamide

The title compound was obtained from [3-bromo-4-(methylsulfonyl)benzyl]amine and 1-(3-methylphenyl)-2-oxo-1,2-dihydropyridine-3-carboxylic acid by a method analogous to that described in Example 17.
¹H-NMR (DMSO-d₆): δ 9.94 (1H, t); 8.37 (1H, d); 8.02 (1H, d); 7.93-7.68 (5H, m); 7.54 (1H, d); 6.61 (1H, d); 4.55 (2H, d); 3.33 (3H, s).
APCI-MS m/z: 543.2,545.2 [MH⁺].

### Example 23 N-[3-Cyano-4-(methylsulfonyl)benzyl]-6-methyl-2-oxo-1-[3-(trifluoromethyl)-phenyl]-1,2-dihydropyridine-3-carboxamide

### a) 5-Formyl-2-(methylthio)benzonitrile

The subtitle compound was prepared analogously to Example 22a but at room temperature for 1 h instead of heating the reaction mixture.
¹H-NMR (400 MHz, CDCl₃): δ 9.94 (1H, s); 8.07 (1H, d, *J* 1.7 Hz); 8.01 (1H, dd, *J* 8.3, 1.8 Hz); 7.40 (1H, d, *J* 8.3 Hz); 2.64 (3H, s).
GC-MS m/z: 177 [M⁺].

### b) 5-(Hydroxymethyl)-2-(methylthio)benzonitrile

The subtitle compound was prepared analogously to Example 22b.
¹H-NMR (400 MHz, CDCl₃): δ 7.62 (1H, s); 7.53 (1H, d, J 8.3 Hz); 7.32 (1H, d, *J* 8.3 Hz); 4.70 (2H, s); 2.57 (3H, s).

### c) 5-(Hydroxymethyl)-2-(methylsulfonyl)benzonitrile

The subtitle compound was prepared analogously to Example 22c.
¹H-NMR (400 MHz, CDCl₃): δ 8.17 (1H, d, J 8.1 Hz); 7.93 (1H, s); 7.79 (1H, d, J 8.2 Hz); 4.88 (2H, s); 3.27 (3H, s).

### d) 5-(Bromomethyl)-2-(methylsulfonyl)benzonitrile

The subtitle compound was prepared analogously to Example 22d.
¹H-NMR (400 MHz, CDCl₃): δ 8.18 (1H, d, *J* 8.1 Hz); 7.93 (1H, d, *J* 1.7 Hz); 7.82 (1H, dd, *J* 8.2, 1.6 Hz); 4.51 (2H, s); 3.29 (3H, s).

### e) 5-(Aminomethyl)-2-(methylsulfonyl)benzonitrile

The subtitle compound was prepared analogously to Example 22e.
¹H-NMR (400 MHz, CD₃OD): δ 8.14 (1H, d, J 8.2 Hz); 8.04 (1H, s); 7.88 (1H, d, J 8.1 Hz); 3.97 (2H, s); 3.29 (3H, s)
APCI-MS m/z: 211 [MH⁺].

### f) N-[3-Cyano-4-(methylsulfonyl)benzyl]-6-methyl-2-oxo-1-[3-trifluoromethyl)phenyl]-1,2-dihydropyridine-3-carboxamide

The title compound was prepared analogously to Example 17.
¹H-NMR (400 MHz, CDCl₃): δ 10.19 (1H, t, *J* 5.7 Hz); 8.56 (1H, d, *J* 7.4 Hz); 8.11 (1H, d, J 8.1 Hz); 7.85 - 7.71 (4H, m); 7.53 (1H, s); 7.46 (1H, d, *J* 8.0Hz); 6.51 (1H, d, *J* 7.5 Hz); 4.71 (1H, dd, *J* 15.9, 6.2 Hz); 4.65 (1H, dd, *J* 15.9, 6.0 Hz); 3.23 (3H, s); 2.11 (3H, s).
APCI-MS m/z: 490 [MH⁺].

### Example 24 6-Methyl-N-[3-methyl-4-(methylsulfonyl)benzyl]-2-oxo-1-[3-(trifluoromethyl)-phenyl]-1,2-dihydropyridine-3-carboxamide

### a) 3-Methyl-4-(methylthio)benzaldehyde

The subtitle compound was prepared analogously to Example 22a.
GC-MS m/z: 166 [M⁺].

### b) [3-Methyl-4-(methylthio)phenyl]methanol

The subtitle compound was prepared analogously to Example 22b.
¹H-NMR (300 MHz, CDCl₃): δ 7.18 - 7.09 (3H, m); 4.57 (2H, s); 2.44 (3H, s); 2.32 (3H, s).

### c) [3-Methyl-4-(methylsulfonyl)phenyl]methanol

The subtitle compound was prepared analogously to Example 22c.
¹H-NMR (300 MHz, CDCl₃): δ 7.92 (1H, d, J 18.7 Hz); 7.35 - 7.29 (2H, m); 4.73 (2H, s); 3.05 (3H, s); 2.66 (3H, s).

### d) 4-(Bromomethyl)-2-methyl-1-(methylsulfonyl)benzene

The subtitle compound was prepared analogously to Example 22d.
¹H-NMR (300 MHz, CDCl₃): δ 8.00 (1H, d, *J* 8.1 Hz); 7.42 - 7.28 (2H, m); 4.46 (2H, s); 3.07 (3H, s); 2.70 (3H, s).

### e) [3-Methyl-4-(methylsulfonyl)benzyl]amine

The subtitle compound was prepared analogously to Example 22e.
¹H-NMR (400 MHz, CDCl₃): δ 7.98 (1H, d, *J* 8.2 Hz); 7.35 - 7.29 (2H, m); 3.93 (2H, s); 3.06 (3H, s); 2.70 (3H, s).
APCI-MS m/z: 200 [MH⁺].

### f) 6-Methyl-N-[3-methyl-4-(methylsulfonyl)benzyl]-2-oxo-1-[3-(trifluoromethyl)-phenyl]-1,2-dihydropyridine-3-carboxamide

The title compound was prepared analogously to Example 17.
¹H-NMR (300 MHz, CDCl₃): δ 9.91 (1H, bt, J 5.7Hz); 8.58 (1H, d, *J* 7.5 Hz); 7.95 (1H, d, *J* 8.1Hz); 7.83 - 7.27 (6H, m); 6.47 (1H, dd, *J* 7.5, 0.5 Hz); 4.68 - 4.53 (2H, m); 3.03 (3H, s); 2.66 (3H, s); 2.08 (3H, s).
APCI-MS m/z: 479 [MH⁺].

### Example 25 6-Methyl-N-[4-(methylthio)benzyl]-2-oxo-1-[3-(trifluoromethyl)phenyl]-1,2-dihydropyridine-3-carboxamide

6-Methyl-2-oxo-1-[3-(trifluoromethyl)phenyl]-1,2-dihydropyridine-3-carboxylic acid (1.29 g, 4.35 mmol) and [4-(methylthio)benzyl]amine (0.66 g, 4.35 mmol) were dissolved in NMP (18 ml). HBTU (1.81 g, 4.79 mmol) and DIEA (1.86 ml, 10.9 mmol) were added neat at room temperature and the mixture was stirred overnight. The reaction mixture was poured into EtOAc. The organic phase was washed with 2.5% aqueous sodium carbonate, then three times with water and dried. Purification by flash-chromatography (EtOAc: cyclohexane 9:1) gave the title compound (1.3 g, 69%).
¹H-NMR (400 MHz, DMSO-d₆) δ 9.78 (1H, t, *J* 5.8 Hz); 8.38 (1H, d, *J* 7.4 Hz); 7.91 - 7.69 (4H, m,); 7.22 (4H, m); 6.62 (1H, d, *J* 7.6 Hz); 4.42 (2H, d, *J* 5.9 Hz); 2.43 (3H, s,); 2.01 (3H, s).
APCI-MS m/z: 433.1 [MH⁺].

### Example 26 6-Methyl-N-[4-(methylsulfinyl)benzyl]-2-oxo-1-[3-(trifluoromethyl)phenyl]-1,2-dihydropyridine-3-carboxamide

6-Methyl-N-[4-(methylthio)benzyl]-2-oxo-1-[3-(trifluoromethyl)phenyl]-1,2-dihydropyridine-3-carboxamide (1.26 g, 2.93 mmol) was dissolved in CH₂Cl₂ (12 ml). The solution was cooled to -15 °C. To the stirred solution 3-chloroperoxybenzoic acid (672 mg, 2.93 mmol) was added portion wise. The reaction was stirred for 1 h. The cooling bath was removed and the reaction was allowed to reach room temperature. After 2 h, CH₂Cl₂ and diluted sodium thiosulphate solution were added. The mixture was shaken and the water phase was separated. The organic layer was washed twice with saturated NaHCO₃, once with brine and finally dried. Purification by flash-chromatography (EtOAc: MeOH 9:1) gave the title compound (1.1 g, 80%).
¹H-NMR (400 MHz, DMSO-d₆) δ 9.88 (1H, t, *J* 5.9 Hz); 8.38 (1H, d, *J*7.5 Hz); 7.93 - 7.69 (4H, m,); 7.62 (2H, d, *J* 8.2 Hz); 7.47 (2H, d, *J* 8.2 Hz); 6.62 (1H, d, *J* 7.7 Hz); 4.54 (2H, d, *J* 6.0 Hz); 2.70 (3H, s,); 2.02 (3H, s,).
APCI-MS m/z: 449.1 [MH⁺].

### Example 27 N-[4-(Benzylsulfonyl)benzyl]-6-methyl-2-oxo-1-[3-(trifluoromethyl)phenyl]-1,2-dihydropyridine-3-carboxamide

### a) N-(4-Mercaptobenzyl)-6-methyl-2-oxo-1-[3-(trifluoromethyl)phenyl]-1,2-dihydropyridine-3-carboxamide

6-Methyl-N-[4-(methylsulfinyl)benzyl]-2-oxo-1-[3-(trifluoromethyl)phenyl]-1,2-dihydropyridine-3-carboxamide (0.30 g, 0.67 mmol) was dissolved in dry acetonitrile (3 ml) and 2,6-lutidine (0.24 ml, 2.08 mmol) was added. The solution was cooled to -20 °C and trifluoroacetic anhydride (0.28 ml, 2.0 mmol) was added . The reaction was kept between -10 °C and 0 °C for 1 h, and then allowed to reach room temperature. All volatile materials were evaporated at 30 °C. The crude residue was cooled to 0 °C. A degassed, dry 1:1 mixture of triethylamine (1.1 ml) and methanol (1.1 ml), cooled to 0 °C, was added. The reaction was stirred at room temperature for 30 min and then evaporated. The residue was dissolved in a 1:1 mixture of methanol and 6M hydrochloric acid and stirred at 50 °C for 20 min. The major part of the solvent was evaporated. Ethyl acetate and water were added. The water phase was separated and washed again with ethyl acetate. The combined organic phase was washed with brine, dried, evaporated and used as crude product in the next step.
LC-MS; method B RT: 8.19 min, APCI-MS m/z: 419.3 [MH⁺].

### b) N-[4-(Benzylsulfonyl)benzyl]-6-methyl-2-oxo-1-[3-(trifluoromethyl)phenyl]-1,2-dihydropyridine-3-carboxamide

In an argon filled vial N-(4-mercaptobenzyl)-6-methyl-2-oxo-1-[3-(trifluoromethyl)phenyl]-1,2-dihydropyridine-3-carboxamide (29 mg, 0.07 mmol) was dissolved in dried, degassed ethanol (0.1 ml) and cooled to 0 °C. A solution of potassium tert-butoxide (7.8 mg, 0.7 mmol) in ethanol (0.2 ml) was added. The reaction was stirred for 45 min. (Bromomethyl)benzene (12 µl, 0.105 mmol) in ethanol (0.1 ml) was added. The ice bath was removed and the reaction was stirred overnight. A mixture of ethyl acetate and 1M aqueous NH₄Cl was added. The organic layer was separated, dried and evaporated. The residue was dissolved in CH₂Cl₂ (240 µl) and cooled with stirring to - 15 °C. 3-Chloroperoxybenzoic acid (35 mg, 0.154 mmol) in CH₂Cl₂ (200 µl) was added. The cooling bath was removed and the reaction was stirred overnight. EtOAc and 5% aqueous sodium thiosulfate were added. The organic layer was separated, washed with 5% aqueous sodium carbonate, brine and dried. Purification by preparative HPLC gave the title compound (11 mg, 30%).
¹H-NMR (400 MHz, DMSO-d₆) δ 9.91 (1H, t, *J* 6.1 Hz); 8.38 (1H, d, *J* 7.5 Hz); 7.81 (4H, m); 7.66 (2H, d, *J* 8.4 Hz); 7.46 (2H, d, *J* 8.2 Hz); 7.31 - 7.11 (5H, m,); 6.63 (1H, d, *J* 7.6 Hz); 4.62 (2H, s,); 4.57 (2H, d, *J* 6.1 Hz); 2.03 (3H, s,).
APCI-MS m/z: 541.4 [MH⁺].

Following the general method of Example 27 (b), the compounds of Examples 28 to 33 were prepared:

### Example 28 6-Methyl-2-oxo-N-[4-(propylsulfonyl)benzyl]-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 1-bromopropane (10 µl, 0.105 mmol). Yield: 12 mg (35%).
¹H-NMR (400 MHz, DMSO-d₆) δ 9.93 (1H, t, *J* 6.1 Hz); 8.38 (1H, d, *J* 7.5 Hz); 7.94 - 7.70 (6H, m); 7.53 (2H, d, *J* 8.2 Hz); 6.63 (1H, d, *J* 7.6 Hz); 4.58 (2H, d, *J* 6.1 Hz); 3.22 (2H, m); 2.02 (3H, s,); 1.52 (2H, q, *J* 7.6 Hz); 0.89 (3H, t, *J* 7.4 Hz).
APCI-MS m/z: 493.3 [MH⁺].

### Example 29 N-[4-Butylsulfonyl)benzyl]-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 1-bromobutane (11 µl, 0.105 mmol). Yield: 14 mg, 0.028 mmol (39%).
¹H-NMR (400 MHz, DMSO-d₆) δ 9.93 (1H, t, *J* 6.1 Hz); 8.38 (1H, d, *J*7.4 Hz); 7.93 - 7.70 (6H, m); 7.53 (2H, d, *J* 8.3 Hz); 6.63 (1H, d, *J* 7.7 Hz); 4.59 (2H, d, *J* 6.1 Hz); 3.24 (2H, m); 2.02 (3H, s,); 1.48 (2H, m); 1.31 (2H, m); 0.81 (3H, t, *J* 7.3 Hz).
APCI-MS m/z: 507.3 [MH⁺].

### Example 30 N-[4-(Isobutylsulfonyl)benzyl]-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 1-bromo-2-methylpropane (11 µl, 0.105 mmol). Yield: 10 mg, 0.020 mmol (28%).
¹H-NMR (400 MHz, DMSO-d₆) δ 9.93 (1H, t, *J* 6.0 Hz); 8.38 (1H, d, *J* 7.5 Hz); 7.94 - 7.69 (6H, m,); 7.53 (2H, d, *J* 8.3 Hz); 6.63 (1H, d, *J7.7* Hz); 4.58 (2H, d, *J* 6.2 Hz); 3.16 (2H, d, *J 6.5* Hz); 2.02 (3H, s,); 1.96 (1H, quintet, *J* 6.6 Hz); 0.95 (6H, d, *J* 6.7 Hz).
APCI-MS m/z: 507.3 [MH⁺].

### Example 31 N-[4-(sec-Butylsulfonyl)benzyl]-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-brombutane (11 µl, 0.105 mmol). Yield: 12 mg, 0.024 mmol (35%).
¹H-NMR (400 MHz, DMSO-d₆) δ 9.94 (1H, t, *J* 6.0 Hz); 8.38 (1H, d, *J*7.5 Hz); 7.93 - 7.69 (6H, m,); 7.53 (2H, d, *J* 8.2 Hz); 6.63 (1H, d, *J* 7.8 Hz); 4.59 (2H, d, *J* 6.1 Hz); 3.17 (1H, dq, *J* 13.4, 6.7 Hz); 2.02 (3H, s,); 1.77 (1H, m); 1.29 (1H, m); 1.11 (3H, d, *J* 6.9 Hz); 0.89 (3H, t, *J* 7.5 Hz).
APCI-MS m/z: 507.4 [MH⁺].

### Example 32 N-[4-(Isopropylsulfonyl)benzyl]-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-bromopropane (10 µl, 0.105 mmol). Yield: 11 mg, 0.022 mmol (33%).
¹H-NMR (400 MHz, DMSO-d₆) δ 9.94 (1H, t, *J* 6.1 Hz); 8.38 (1H, d, *J* 7.4 Hz); 7.94 - 7.69 (6H, m,); 7.54 (2H, d, *J* 8.3 Hz); 6.63 (1H, d, *J* 7.7 Hz); 4.60 (2H, d, *J* 6.1 Hz); 3.36 (1H, septet, *J* 6.2 Hz); 2.03 (3H, s,); 1.13 (6H, d, *J* 6.8 Hz).
APCI-MS m/z: 493.3 [MH⁺].

### Example 33 6-Methyl-N-{4-[(3-methylbutyl)sulfonyl]benzyl}-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 1-bromo-3-methylbutane (13 µl, 0.105 mmol). Yield: 11 mg, 0.021 mmol (31 %).
¹H-NMR (400 MHz, DMSO-d₆) δ 9.93 (1H, t, *J* 6.1 Hz); 8.38 (1H, d, *J* 7.4 Hz); 7.94 - 7.69 (6H, m,); 7.53 (2H, d, *J* 8.3 Hz); 6.63 (1H, d, *J* 7.5 Hz); 4.59 (2H, d, *J* 6.1 Hz); 3.23 (2H, m); 2.02 (3H, s,); 1.57 (1H, septet, *J* 6.8 Hz); 1.39 (2H, m); 0.80 (6H, d, *J* 6.6 Hz).
APCI-MS m/z: 521.4 [MH⁺].

Following the general method of Example 27, the compounds of Examples 34 to 52 were prepared:

### Example 34 N-{4-[(Cyclopropylmethyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and (bromomethyl)cyclopropane (10 µl, 0.105 mmol). Yield: 13 mg, 0.027 mmol (38%).
¹H-NMR (400 MHz, DMSO-d₆) δ 9.93 (1H, t, *J* 6.2 Hz); 8.38 (1H, d, *J* 7.5 Hz); 7.93 - 7.70 (6H, m,); 7.53 (2H, d, *J* 8.3 Hz); 6.63 (1H, d, *J* 7.6 Hz); 4.59 (2H, d, *J* 6.1 Hz); 3.21 (2H, d, *J* 7.2 Hz); 2.02 (3H, s,); 0.80 (1H, m); 0.43 (2H, m); 0.09 (2H, m).
APCI-MS m/z: 505.3 [MH⁺].

### Example 35 6-Methyl-2-oxo-N-{4-[(tetrahydrofuran-2-ylmethyl)sulfonyl]-benzyl}-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-(bromomethyl)tetrahydrofuran (12 µl, 0.105 mmol). Yield: 8 mg, 0.015 mmol (22%).
¹H-NMR (400 MHz, DMSO-d₆) δ 9.93 (1H, t, *J* 6.0 Hz); 8.38 (1H, d, *J* 7.5 Hz); 7.93 - 7.70 (6H, m,); 7.51 (2H, d, *J* 8.2 Hz); 6.63 (1H, d, *J* 7.6 Hz); 4.58 (2H, d, *J* 6.1 Hz); 4.05 (1H, quintet, *J* 6.5 Hz); 3.59 (1H, q, *J* 7.4 Hz); 3.50 (3H, m); 2.02 (3H, s,); 1.94 (1H, m); 1.75 (2H, m); 1.54 (1H, m).
APCI-MS m/z: 535.4 [MH⁺].

### Example 36 N-{4-[(2-Hydroxyethyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-bromoethanol (7 µl, 0.105 mmol). Yield: 12 mg, 0.025 mmol (36%).
¹H-NMR (400 MHz, DMSO-d₆) δ 9.93 (1H, t, *J* 6.1 Hz); 8.38 (1H, d, *J* 7.4 Hz); 7.93 - 7.70 (6H, m,); 7.52 (2H, d, *J* 8.3 Hz); 6.63 (1H, d, *J* 7.6 Hz); 4.58 (2H, d, *J* 6.1 Hz); 3.65 (2H, t, *J* 6.4 Hz); 3.40 (2H, t, *J* 6.5 Hz); 2.02 (3H, s,).
APCI-MS m/z: 495.3 [MH⁺].

### Example 37 N-{4-[(Cyanomethyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and chloroacetonitrile (7 µl, 0.105 mmol). Yield: 8 mg, 0.017 mmol (24%).
¹H-NMR (400 MHz, DMSO-d₆) δ 9.96 (1H, t, *J* 6.1 Hz); 8.37 (1H, d, *J7.5* Hz); 7.95 - 7.69 (6H, m,); 7.62 (2H, d, *J* 8.3 Hz); 6.63 (1H, d, *J* 7.6 Hz); 5.20 (2H, s,); 4.61 (2H, d, *J* 6.2 Hz); 2.03 (3H, s,).
APCI-MS m/z: 490.3 [MH⁺].

### Example 38 N-{4-[(2-Amino-2-oxoethyl)sulfonyl]benzyl]-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-bromoacetamide (14 mg, 0.105 mmol). Yield: 15 mg, 0.029 mmol (41%). ¹H-NMR (400 MHz, DMSO-d₆) δ 9.94 (1H, t, *J* 6.1 Hz); 8.38 (1H, d, *J* 7.4 Hz); 7.95 - 7.46 (10H, m,); 6.62 (1H, d, *J* 7.6 Hz); 4.58 (2H, d, *J* 6.0 Hz); 4.18 (2H, s,); 2.02 (3H, s,).
APCI-MS m/z: 508.3 [MH⁺].

### Example 39 N-{4-[(4-Cyanobenzyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 4-(bromomethyl)benzonitrile (21 mg, 0.105 mmol). Yield: 3 mg, 0.004 mmol (6%).
LC-MS; method B RT: 7.80 min, APCI-MS m/z: 566.4 [MH⁺].

### Example 40 N-{4-[(2-Cyanoethyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 3-chloropropanenitrile (8 µl, 0.105 mmol). Yield: 1 mg, 0.002 mmol (3%).
LC-MS; method B RT: 7.16 min, APCI-MS m/z: 504.3 [MH⁺].

### Example 41 N-{4-[(3-Hydroxypropyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-(3-(trifluoromethyl)phenyl]-1,2-dihydropyridine-3-carboxamide (29 mg, 0.07 mmol) and 3-bromopropan-1-ol (9 µl, 0.105 mmol). Yield: 6 mg, 0.012 mmol (18%).
LC-MS; method B RT: 6.40 min, APCI-MS m/z: 509.3 [MH⁺].

### Example 42 N-(4-{[2-(Dimethylamino)-2-oxoethyl]sulfonyl}benzyl)-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-chloro-N,N-dimethylacetamide (11 µl, 0.105 mmol). Yield: 10 mg, 0.019 mmol (27%).
LC-MS; method B RT: 6.61 min, APCI-MS m/z: 536.4 [MH⁺].

### Example 43 Ethyl 3-[(4-{[({6-methyl-2-oxo-1-[3-(trifluoromethyl)phenyl]-1,2-dihydropyridin-3-yl}carbonyl)amino]methyl}phenyl)sulfonyl]propanoate

From N-(4-mercaptobenzyl)-6-methyl-2-oxo-1-[3-(trifluoromethyl)phenyl]-1,2-dihydropyridine-3-carboxamide (29 mg, 0.07 mmol) and ethyl 3-bromopropanoate (13 µl, 0.105 mmol). Yield: 5 mg, 0.009 mmol (13%).
LC-MS; method B RT: 7.64 min, APCI-MS m/z: 551.4 [MH⁺].

### Example 44 2-[(4-{[([6-Methyl-2-oxo-1-[3-(trifluoromethyl)phenyl]-1,2-dihydropyridin-3-yl}carbonyl)amino]methyl}phenyl)sulfonyl]ethyl acetate

From N-(4-mercaptobenzyl)-6-methyl-2-oxo-1-[3-(trifluoromethyl)phenyl]-1,2-dihydropyridine-3-carboxamide (29 mg, 0.07 mmol) and 2-bromoethyl acetate (12 µl, 0.105 mmol). Yield: 7 mg, 0.014 mmol (20%).
LC-MS; method B RT: 7.19 min, APCI-MS m/z: 537.3 [MH⁺].

### Example 45 N-{4-[(3-Cyanobenzyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 3-(bromomethyl)benzonitrile (21 mg, 0.105 mmol). Yield: 2 mg, 0.004 mmol (6%).
LC-MS; method B RT: 7.77 min, APCI-MS m/z: 566.3 [MH⁺].

### Example 46 Methyl 3-[(4-{[({6-methyl-2-oxo-1-[3-(trifluoromethyl)phenyl]-1,2-dihydropyridin-3-yl}carbonyl)amino]methyl}phenyl)sulfonyl]propanoate

From N-(4-mercaptobenzyl)-6-methyl-2-oxo-1-[3-(trifluoromethyl)phenyl]-1,2-dihydropyridine-3-carboxamide (29 mg, 0.07 mmol) and methyl 3-bromopropanoate (11 µl, 0.105 mmol). Yield: 2 mg, 0.005 mmol (7%).
LC-MS; method B RT: 7.28 min, APCI-MS m/z: 537.3 [MH⁺].

### Example 47 6-Methyl-N-(4-{[(2-methyl-1,3-thiazol-4-yl)methyl]sulfonyl}benzyl)-2-oxo-1-[3-(trifluoromethyl)phenyl]-1,2-dihydropyridine-3-carboxamide trifluoroacetate

From N-(4-mercaptobenzyl)-6-methyl-2-oxo-1-[3-(trifluoromethyl)phenyl]-1,2-dihydropyridine-3-carboxamide (29 mg, 0.07 mmol) and 4-(chloromethyl)-2-methyl-1,3-thiazole hydrochloride (19 mg, 0.105 mmol). Yield: 11 mg, 0.017 mmol (24%).
LC-MS; method B RT: 7.21 min, APCI-MS m/z: 562.3 [MH⁺].

### Example 48 6-Methyl-2-oxo-N-{4-[(pyridin-4-ylmethyl)sulfonyl]benzyl}-1-[3-(trifluoromethyl)phenyl]-1,2-dihydropyridine-3-carboxamide trifluoroacetate

From N-(4-mercaptobenzyl)-6-methyl-2-oxo-1-[3-(trifluoromethyl)phenyl]-1,2-dihydropyridine-3-carboxamide (29 mg, 0.07 mmol) and 4-(chloromethyl)pyridine hydrochloride (17 mg, 0.105 mmol). Yield: 5 mg, 0.008 mmol (11%).
LC-MS; method B RT: 5.79 min, APCI-MS m/z: 542.3 [MH⁺].

### Example 49 N-{4-[(3-Cyanopropyl)sulfonyl]benzyl}-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 4-bromobutanenitrile (10 µl, 0.105 mmol). Yield: 11 mg, 0.022 mmol (31 %).
LC-MS; method B RT: 7.19 min, APCI-MS m/z: 518.3 [MH⁺].

### Example 50 N-(4-{[(3,5-Dimethylisoxazol-4-yl)methyl]sulfonyl}benzyl)-6-methyl-2-oxo-1-[3-(trifluoromethyl)phenyl]-1,2-dihydropyridine-3-carboxamide trifluoroacetate

From N-(4-mercaptobenzyl)-6-methyl-2-oxo-1-[3-(trifluoromethyl)phenyl]-1,2-dihydropyridine-3-carboxamide (29 mg, 0.07 mmol) and 4-(chloromethyl)-3,5-dimethylisoxazole (13 µl, 0.105 mmol). Yield: 9 mg, 0.013 mmol (19%).
LC-MS; method B RT: 7.50 min, APCI-MS m/z: 560.4 [MH⁺].

### Example 51 N-(4-{[4-(Acetylamino)benzyl]sulfonyl}benzyl)-6-methyl-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and N-[4-(chloromethyl)phenyl]-acetamide (19 mg, 0.105 mmol). Yield: 7 mg, 0.013 mmol (18%).
LC-MS; method B RT: 6.98 min, APCI-MS m/z: 598.4 [MH⁺].

### Example 52 6-Methyl-N-[4-({2-[(5-methyl-1,3,4-thiadiazol-2-yl)amino]-2-oxoethyl}sulfonyl)benzyl]-2-oxo-1-[3-(trifluoromethyl)phenyl]-1,2-dihydropyridine-3-carboxamide

From N-(4-mercaptobenzyl)-6-methyl-2-oxo-1-[3-(trifluoromethyl)phenyl]-1,2-dihydropyridine-3-carboxamide (29 mg, 0.07 mmol) and 2-bromo-N-(5-methyl-1,3,4-thiadiazol-2-yl)acetamide (25 mg, 0.105 mmol). Yield: 4 mg, 0.006 mmol (9%).
LC-MS; method B RT: 6.62 min, APCI-MS m/z: 606.2 [MH⁺].

### Example 53 6-Methyl-N-[4-(methylsulfonyl)phenoxy]-2-oxo-1-[3-(trifluoromethyl)phenyl]-1,2-dihydropyridine-3-carboxamide

To a mixture of 6-methyl-2-oxo-1-[3-(trifluoromethyl)phenyl]-1,2-dihydropyridine-3-carboxylic acid (67 mg, 0.22 mmol), HATU (93.7 mg, 0.25 mmol), HOAT (34 mg, 0.25 mmol), DIEA (150 µl, .87 mmol) and NMP (2 ml) was added O-[4-(methylsulfonyl)phenyl]hydroxylamine, prepared according to the procedure in *J. Med. Chem*., **1967,** 512 (41 mg, 0.22 mmol). After stirring at room temperature for 3 h the mixture was partitioned between EtOAc and water. The organic extract was washed with brine, dried, filtered and evaporated to dryness. The crude product was further purified by preparative HPLC to give the title compound as a white solid (41 mg, 40%).
¹H-NMR (CDCl₃): δ 12.19 (1H, s); 8.58 (1H, d, *J* 7.4 Hz); 7.92 - 7.86 (3H, m); 7.80 (1H, t, *J* 7.9 Hz); 7.57 (1H, s); 7.50 (1H, d, *J* 7.9 Hz); 7.30 - 7.26 (5H, m); 6.56 (1H, d, *J* 7.5 Hz); 3.03 (3H, s); 2.16 (3H, s).
APCI-MS m/z: 567 [MH⁺].

### Example 54 6-Methyl-2-oxo-1-(3-trifluoromethyl-phenyl)-1,2-dihydro-pyridine-3-carboxylic acid (4-bromo-phenoxy)-amide

A solution of 6-methyl-2-oxo-1-(3-trifluoromethyl-phenyl)-1,2-dihydro-pyridine-3-carboxylic acid (0.060 g, 0.20 mmol) in CH₂Cl₂ (5 ml) and SOCl₂ (5 ml) was stirred for 3 h at room temperature and then concentrated to give the crude intermediate acid chloride as a solid. The solid was dissolved in 1,4-dioxane and O-(4-bromo-phenyl)-hydroxylamine, prepared according to the procedure in *J. Med. Chem*., **1967**, 512, (0.10 g, 0.53 mmol) was added and the mixture was stirred at room temperature for 10 min. The volatiles were removed and the residue was purified on preparative HPLC, giving 0.055 g (58%) of the title compound as an off-white solid.
¹H-NMR (CDCl₃): δ 12.08 (1H, s); 8.57 (1H, d, *J* 7.42 Hz); 7.84 (1H, d, *J* 8.01 Hz); 7.77 (1H, t, *J* 8.01 Hz); 7.55 (1H, s); 7.47 (1H, d, *J* 8.01 Hz); 7.38 (2H, d, *J* 8.87 Hz); 7.00 (2H, 8.90 Hz); 6.52 (1H, d, *J* 7.48 Hz); 2.13 (3H, s)
APCI-MS m/z: 467.1 and 469.0 [MH⁺].

### Example 55 6-Methyl-2-oxo-N-phenoxy-1-[3-(trifluoromethyl)phenyl]-1,2-dihydropyridine-3-carboxamide

The title compound was prepared as described in Example 53 starting form O-phenyl hydroxylamine.
¹H-NMR (CDCl₃): δ 12.04 (1H,s); 8.58 (1H,d); 7.85-7.74 (2H,m); 7.55 (1H,brs); 7.48 (1H,d); 7.32-7.26 (2H,m); 7.12 (2H,d); 7.02 (1H,t); 6.51 (1H,d); 2.12 (3H,s).
APCI-MS m/z: 389[MH⁺].

### Example 56 N-(4-Aminobenzyl)-6-methyl-2-oxo-1-[3-(trifluoromethyl)phenyl]-1,2-dihydroproline-3-carboxamide

To a mixture of 1-(3-methylphenyl)-2-oxo-1,2-dihydropyridine-3-carboxylic acid (212 mg, 0.7 mmol), HATU (272 mg, 0.7 mmol), HOAT (97 mg, 0.7 mmol) and DIEA (275 mg, 2.13 mmol) in NMP (3 ml) was added (4-aminobenzyl)amine (87 mg, 0.7 mmol) in NMP (1 ml). The reaction was stirred for 12 h at room temperature. The reaction mixture was diluted with water (1.0 ml) and purified on a Xterra@Prep MS C8 column (19 x 50 mm) using a gradient of CH₃CN/water at a flow rate of 20 ml/min. Freeze drying of the mixture afforded the title compound (140 mg, 49%).
¹H-NMR (400 MHz, CDCl₃), δ 9.74 (1H, t, J 5.4 Hz), 8.56 (1H, d, J 7.5 Hz ), 7.78 (1H, m), 7.50 (1H, s), 7.43 (1H, d, *J* 7.8 Hz), 7.27 (1H, s), 7.15 (2H, d, *J* 8.1 Hz), 6.81 (2H, d, *J* 8.2 Hz), 6.43 (1H, d, *J* 7.4 Hz), 4.49 (2H, m), 2.06 (3H, s, *J* 9.1 Hz)
APCI-MS m/z: 402.2 [MH⁺].

### Example 57 6-Methyl-N-{4-[(methylsulfonyl)amino]benzyl}-2-oxo-1-[3-(trifluoromethyl)phenyl]-1,2-dihydropyridine-3-carboxamide

To a mixture of N-(4-aminobenzyl)-6-methyl-2-oxo-1-[3-(trifluoromethyl)phenyl]-1,2-dihydroproline-3-carboxamide (80 mg, 0.2 mmol) in CH₂Cl₂, methanesulfonyl chloride (23 mg, 0.2 mmol) and DIEA (26 mg, 0.2 mmol) were added. The reaction mixture was stirred for 0.5 h at room temperature. The CH₂Cl₂ was evaporated off and the residue dissolved in CH₃CN/water and purified on a Xterra@Prep MS C8 column (19 x 50 mm) using a gradient of CH₃CN/water at a flow rate of 20 ml/min. Freeze drying of the mixture afforded the title compound (67 mg, 70%).
¹H-NMR (400 MHz, CDCl₃) δ 9.83 (1H, s ), 8.59 (1H, d, *J* 7.4 Hz ), 7.80 (1H, d, *J* 7.9 Hz), 7.73 (1H, t, *J* 7.8 Hz), 7.51 (1H, s), 7.43 (1H, d, *J* 7.9 Hz), 7.31 (2H, d, *J* 8.4 Hz), 7.14 (2H, d, *J* 8.4 Hz), 6.46 (1H, d, *J* 7.4 Hz), 6.38 (1H, s), 4.57 (m, 1H), 2.97 (3H, s, *J* 3.9 Hz), 2.08 (3H, s, *J* 4.3 Hz ).
APCI-MS m/z: 480.1 [MH⁺].

### Example 58 N-{4-[Bis(methylsulfonyl)amino]benzyl}-6-methyl-2-oxo-1-[3-(trifluoromethyl)phenyl]-1,2-dihydropyridine-3-carboxamide

The title compound was isolated as a by-product using the method described in Example 57.
¹H-NMR (400 MHz, CDCl₃): δ 9.94 (t, *J* 5.6 Hz, 1H), 8.59 (d, *J* 7.4 Hz, 1H), 7.82 (d, *J* 7.9 Hz, 1H), 7.75 (t, *J* 7.8 Hz, 2H), 7.53 (s, 1H), 7.45 (t, *J* 5.6 Hz, 3H), 7.29 (d, *J* 9.7 Hz, 2H); 6.47 (d, *J* 7.5 Hz, 1H), 4.64 (t, *J* 5.1 Hz, 2H), 3.39 (s, 6H), 2.09 (s, 3H).
APCI-MS m/z: 558.4 [MH⁺].

### Example 59 N-[[4-[[(Dimethylamino)sulfonyl]amino]phenyl]methyl]-1,2-dihydro-6-methyl-2-oxo-1-[3-(trifluoromethyl)phenyl]-3-pyridinecarboxamide

The title compound was prepared from N-(4-aminobenzyl)-6-methyl-2-oxo-1-[3-(trifluoromethyl)phenyl]-1,2-dihydroproline-3-carboxamide and dimethylsulfamoyl chloride (2 mg, 10%) following the method outlined in Example 57.
¹H-NMR (400 MHz, CD₃OD) δ 8.48 (1H, d, *J* 7.5 Hz); 7.86 (1H, d, *J7.4* Hz); 7.79 (2H, t, *J* 7.9 Hz); 7.73 (1H, s, ); 7.59 (1H, d, *J* 8.5 Hz); 7.25 (2H, d, *J* 8.6 Hz); 7.17 (2H, d, *J* 8.5 Hz); 6.63 (1H, d, *J* 7.6 Hz); 4.52 (2H, s,); 2.74 (6H, s,); 2.09 (3H, s).
APCI-MS m/z: 509.3 [MH⁺].

### Example 60 6-Methyl-N-{4-[methyl(methylsulfonyl)amino]benzyl}-2-oxo-1-[3-(trifluoromethyl)phenyl]-1,2-dihydropyridine-3-carboxamide

To a mixture of 6-methyl-N-{4-[(methylsulfonyl)amino]benzyl}-2-oxo-1-[3-(trifluoromethyl)phenyl]-1,2-dihydropyridine-3-carboxamide (10 mg, 0.02 mmol) in dichloromethane, iodomethane (4 mg, 0.03 mmol) and DIEA (3.9 mg, 0.03 mmol) were added. The reaction was stirred for 10 min at 60 °C in a microwave oven. After evaporation of the solvent, the residue was dissolved in CH₃CN/water and purified on a Xterra@Prep MS C8 column (19 x 50 mm) using a gradient of CH₃CN/water at a flow rate of 20 ml/min. Freeze drying of the mixture afforded the title compound (6 mg, 60%).
¹H-NMR (400 MHz, DMSO-d₆) δ 9.83 (1H, t, *J* 5.9 Hz), 8.38 (1H, d, *J* 7.5 Hz), 7.89 (2H, d, *J* 8.7 Hz), 7.80 (1H, t, *J* 7.7 Hz), 7.71 (1H, d, *J* 8.0 Hz), 7.32 (4H, m), 6.62 (1H, d, *J* 7.5 Hz), 4.48 (2H, d, *J* 5.9 Hz), 3.20 (3H, s), 2.91 (3H, s), 2.01 (3H, s)
APCI-MS m/z: 494.1 [MH⁺].

Following the general method of Example 60, the compounds of Examples 60.1 to 60.7 were prepared:

### Example 60.1

### N-[[4-[Butyl(methylsulfonyl)amino]phenyl]methyl]-1,2-dihydro-6-methyl-2-oxo-1-[3-(trifluoromethyl)phenyl]-3-pyridinecarboxamide

Using 1-iodobutane. Yield (7 mg, 43%).
¹H-NMR (400 MHz, DMSO-d₆) δ 9.86 (1H, s,); 8.38 (1H, d, *J* 7.3 Hz); 7.89 (2H, d, *J*9.5 Hz); 7.80 (1H, t, *J7.4* Hz); 7.72 (1H, d, *J* 8.2 Hz); 7.32 (4H, s,); 6.63 (1H, d, *J*7.2 Hz); 4.49 (2H, d, *J* 5.8 Hz); 3.58 (2H, s,); 2.91 (3H, s,); 2.02 (3H, s,); 1.27 (4H, s,); 0.81 (3H, t, *J* 6.2 Hz).
APCI-MS m/z: 536.4 [MH⁺].

### Example 60.2 1,2-Dihydro-6-methyl-N-[[4-[(1-methylethyl)(methylsulfonyl)-amino]phenyl]methyl]-2-oxo-1-[3-(trifluoromethyl)phenyl]-3-pyridinecarboxamide

Using 2-iodopropane. Yield (10 mg, 38%).
¹H-NMR (400 MHz, DMSO-d₆) δ 9.88 (2H, t, *J* 6.0 Hz); 8.39 (2H, d, *J* 7.4 Hz); 7.89 (4H, d, *J* 11.6 Hz); 7.80 (3H, t, *J* 7.8 Hz); 7.72 (2H, d, *J* 8.1 Hz); 7.33 (4H, d, *J* 8.3 Hz); 7.22 (4H, d, J 8.3 Hz); 6.63 (2H, d, *J 7.6* Hz); 4.52 (4H, d, *J* 6.1 Hz); 4.30 (2H, quintet, *J* 6.7 Hz); 3.02 (7H, s,); 2.02 (6H, s,); 1.04 (12H, d, *J* 6.8 Hz).
APCI-MS m/z: 522.4 [MH⁺].

### Example 60.3 N-{4-[(2-Methoxyethyl)(methylsulfonyl)amino]benzyl}-6-methyl-2-oxo-1-[3-(trifluoromethyl)phenyl]-1,2-dihydropyridine-3-carboxamide

Using 2-bromoethyl methyl ether. Yield (15 mg, 33%).
¹H-NMR (400 MHz, DMSO-d₆) δ 9.86 (1H, t, *J*5.9 Hz); 8.38 (1H, d, *J7.4* Hz); 7.89 (2H, d, *J* 9.3 Hz); 7.80 (1H, t, *J* 7.8 Hz); 7.72 (1H, d, *J* 7.9 Hz); 7.32 (4H, d,); 6.62 (1H, d, *J* 7.5 Hz); 4.49 (2H, d, *J* 6.0 Hz); 3.73 (2H, t, *J* 5.8 Hz); 3.29 (2H, t, *J* 5.7 Hz); 3.17 (3H, s, ); 2.98 (3H, s,); 2.02 (3H, s).
APCI-MS m/z: 538.4 [MH⁺].

### Example 60.4 N-{4-[(2-Cyanoethyl)(methylsulfonyl)amino]benzyl}-6-methyl-2-oxo-1-[3-(trifluoromethyl)phenyl]-1,2-dihydronyridine-3-carboxamide

Using 3-bromopropionitrile. Yield (3 mg, 19%).
¹H-NMR (400 MHz, DMSO-d₆) δ 9.86 (1H, t,); 8.39 (1H, d, *J* 7.4 Hz); 7.89 (2H, d, *J* 9.3 Hz); 7.80 (1H, t, *J* 0.0 Hz); 7.72 (1H, d, *J7.5* Hz); 7.36 (4H, s,); 6.63 (1H, d, *J*7.5 Hz); 4.50 (2H, d, *J* 6.2 Hz); 3.86 (2H, t, *J* 6.4 Hz); 3.00 (3H, s,); 2.60 (2H, t, *J* 6.3 Hz); 2.02 (3H, s).
APCI-MS m/z: 533.1 [MH⁺].

### Example 60.5 N-{4-[Ethyl(methylsulfonyl)amino]benzyl}-6-methyl-2-oxo-1-[3-(trifluoromethyl)phenyl]-1,2-dihydropyridine-3-carboxamide

Using iodoethane. Yield: (6 mg, 59%).
¹H-NMR (400 MHz, DMSO-d₆) δ 9.86 (1H, t, *J* 5.9 Hz); 8.38 (1H, d, *J* 7.4 Hz); 7.89 (2H, d, *J* 9.3 Hz); 7.80 (1H, t, *J* 7.8 Hz); 7.72 (1H, d, *J* 8.1 Hz); 7.32 (4H, dd, *J* 11.6, 8.7 Hz); 6.62 (1H, d, *J* 7.5 Hz); 4.49 (2H, d, *J* 6.0 Hz); 3.62 (2H, q, *J* 7.1 Hz); 2.93 (3H, s,); 2.02 (3H, s,); 0.96 (3H, t, *J* 7.1 Hz);
APCI-MS m/z: 508.4 [MH⁺].

### Example 60.6 1,2-Dihydro-6-methyl-N-[[4-[(methylsulfonyl)propylamino]-phenyl]methyl]-2-oxo-1-[3-(trifluoromethyl)phenyl]-3-pyridinecarboxamide

Using 1-iodopropane. Yield (6 mg, 57%).
¹H-NMR (400 MHz, DMSO-d₆) δ 9.86 (1H, t, *J* 6.0 Hz); 8.38 (1H, d, *J* 7.5 Hz); 7.89 (2H, d, *J* 8.8 Hz); 7.80 (1H, t, *J* 7.8 Hz); 7.72 (1H, d, *J* 8.2 Hz); 7.32 (4H, s,); 6.62 (1H, d, *J* 7.7 Hz); 4.49 (2H, d, *J* 6.0 Hz); 3.54 (2H, t, *J* 7.1 Hz); 2.92 (3H, s,); 2.01 (3H, s,); 1.31 (2H, q, *J* 7.2 Hz); 0.80 (3H, t, *J* 7.3 Hz).
APCI-MS m/z: 522.4 [MH⁺].

### Example 60.7 N-[[4-[(3-Amino-3-oxopropyl)(methylsulfonyl)amino]phenyl]-methyl]-1,2-dihydro-6-methyl-2-oxo-1-[3-(trifluoromethyl)phenyl]-3-pyridinecarboxamide

Using 3-bromopropionamide. Yield (7 mg, 30%).
¹H-NMR (400 MHz, DMSO-d₆) δ 9.86 (1H, t, *J* 6.0 Hz); 8.39 (1H, d, *J* 7.5 Hz); ∂7.89 (2H, d, *J* 10.5 Hz); 7.80 (1H, t, *J7.7* Hz); 7.72 (1H, d, *J* 7.8 Hz); 7.32 (5H, dd, *J* 11.0, 8.8 Hz); 6.81 (1H, s,); 6.63 (1H, d, *J* 7.6 Hz); 4.50 (2H, d, *J* 6.0 Hz); 3.80 (2H, t, *J* 7.5 Hz); 2.96 (3H, s,); 2.17 (2H, t, *J* 7.6 Hz); 2.02 (3H, s).
APCI-MS m/z: 551.4 [MH⁺].

### Example 61 1,2-Dihydro-6-methyl-N-[[4-[(methylsulfonyl)oxy]phenyl]methyl]-2-oxo-1-[3-(trifluoromethyl)phenyl]-3-pyridinecarboxamide

### a) N-(4-Hydroxybenzyl)-6-methyl-2-oxo-1-[3-(trifluoromethyl)phenyl]-1,2-dihydropyridine-3-carboxamide

To a mixture of 1-(3-methylphenyl)-2-oxo-1,2-dihydropyridine-3-carboxylic acid (1 g, 3.36 mmol), HATU (1.28 g, 3.36 mmol), HOAT (457 mg, 3.36 mmol) and DIEA (1.7 g, 13.36 mmol) in NMP (10 ml) was added (4-hydroxybenzyl)amine hydrobromide (686 mg, 3.36 mmol) in NMP (5 ml). The reaction was stirred for 12 h at room temperature. The reaction mixture was diluted with water (75.0 ml) and extracted with EtOAc. The organic phase was dried (MaSO₄), filtered and evaporated affording the title compound (1.2 g, 89%).
¹H-NMR (400 MHz, DMSO-d₆) δ 9.66 (1H, t, *J* 5.7 Hz); 9.27 (1H, s,); 8.36 (1H, d, *J* 7.4 Hz); 7.86 (2H, d, *J* 4.5 Hz); 7.78 (1H, t, *J* 8.0 Hz); 7.68 (1H, d, *J* 7.9 Hz); 7.06 (2H, d, *J* 8.3 Hz); 6.67 (2H, d, *J* 8.5 Hz); 6.60 (1H, d, *J* 7.4 Hz); 4.33 (2H, d, *J* 5.8 Hz); 1.99 (3H, s).
APCI-MS m/z: 403.3 [MH⁺].

### b) 1,2-Dihydro-6-methyl-N-[[4-[(methylsulfonyl)oxy]phenyl]methyl]-2-oxo-1-[3-(trifluoromethyl)phenyl]-3-pyridinecarboxamide

The title compound was prepared from N-(4-hydroxybenzyl)-6-methyl-2-oxo-1-[3-(trifluoromethyl)phenyl]-1,2-dihydropyridine-3-carboxamide and methansulfonyl chloride as in Example 57 (200 mg, 84%).
¹H-NMR (400 MHz, DMSO-d₆) δ 9.87 (1H, t, *J* 6.0 Hz); 8.38 (1H, d, *J* 7.5 Hz); 7.89 (2H, d, *J* 8.9 Hz); 7.80 (1H, t, *J* 7.8 Hz); 7.72 (1H, d, *J* 7.9 Hz); 7.39 (2H, d, *J* 8.6 Hz); 7.29 (2H, d, *J* 8.6 Hz); 6.62 (1H, d, *J* 7.5 Hz); 4.50 (2H, d, *J* 6.0 Hz); 3.35 (3H, s); 2.02 (3H, s).
APCI-MS m/z: 481.3 [MH⁺].

### Example 62 2-Propanesulfonic acid, 4-[[[[1,2-dihydro-6-methyl-2-oxo-1-[3-(trifluoromethyl)phenyl]-3-pyridinyl]carbonyl]amino]methyl]phenyl ester

The title compound was prepared from N-(4-hydroxybenzyl)-6-methyl-2-oxo-1-[3-(trifluoromethyl)phenyl]-1,2-dihydropyridine-3-carboxamide and isopropylsulfonyl chloride as in Example 57.
¹H-NMR (400 MHz, DMSO-d₆) δ 9.83 (1H, t,); 8.35 (1H, d, *J* 7.5 Hz); 7.86 (2H, d, *J* 8.7 Hz); 7.78 (1H, t, *J* 7.7 Hz); 7.69 (1H, d, *J* 7.3 Hz); 7.35 (2H, d, *J* 8.5 Hz); 7.22 (2H, d, *J* 8.6 Hz); 6.60 (1H, d, *J* 7.3 Hz); 4.46 (2H, d, *J* 6.2 Hz); 3.66 (1H, m); 1.99 (3H, s); 1.38 (6H, d, *J* 6.9 Hz).
APCI-MS m/z 509.4 [MH⁺].

### Example 63 N-[(1,1-Dioxido-2,3-dihydro-1-benzothien-5-yl)methyl]-6-methyl-2-oxo-1-[3-(trifluoromethyl)phenyl]-1,2-dihydropyridine-3-carboxamide

### a) 2,3-Dihydro-1-benzothiophene-5-carbaldehyde and 2,3-Dihydro-1-benzothiophene-7-carbaldehyde

The subtitle compounds were prepared according to the procedure described in WO 01/12602.

### b) 2,3-Dihydro-1-benzothien-5-ylmethanol and 2,3-Dihydro-1-benzothien-7-ylmethanol

The title compounds were prepared by stirring a mixture of 2,3-dihydro-1-benzothiophene-5-carbaldehyde and 2,3-dihydro-1-benzothiophene-7-carbaldehyde (4.3 g, 26 mmol) with sodium borohydride (3.78 g, 100 mmol) in THF (100 ml) and water (10 ml) at room temperature overnight. 1M Hydrochloric acid was added slowly to quench the excess of borohydride. The mixture was extracted with EtOAc and washed with water. The solvents were removed in vacuo and the residue purified by column chromatography on silica using heptane/EtOAc (4:1) as eluent to afford 2,3-dihydro-1-benzothien-5-ylmethanol (1.84 g):
¹H-NMR (CDCl₃): δ 7.22 (1H, brs); 7.20 (1H, d, *J* 8.3 Hz); 7.11 (1H, brd, *J* 8.3 Hz); 4.61 (2H, s); 3.41-3.25 (4H, m);
and 2,3-dihydro-1-benzothien-7-ylmethanol (1.18 g) (total yield 70%):
¹H-NMR (CDCl₃): δ 7.18 (1H, d, *J* 7.5 Hz); 7.15 (1H, d, *J* 7.5 Hz); 7.05 (1H, t, *J* 7.5 Hz); 4.63 (2H, s); 3.41-3.28 (4H, m).

### c) 1,1-Dioxido-2,3-dihydro-1-benzothien-5-yl)methanol

The title compound was prepared by stirring 2,3-dihydro-1-benzothien-5-ylmethanol (1.08 g, 6.38 mmol), oxone (5.8 g, 9.4 mmol), aqueous EDTA (22 ml, 0.4 mM), and sodium hydrogen carbonate (4.8 g) in a mixture of acetone and water at pH 7.5 at room temperature overnight. The reaction mixture was extracted with EtOAc and washed with water. The solvents were removed in vacuo and the residue purified by column chromatography on silica using heptane/EtOAc (4:1) as eluent to afford the subtitle compound (1.0 g, 79%).
¹H-NMR (CDCl₃): δ 7.70 (1H, d, *J* 7.9 Hz); 7.43 (1H, d, *J* 7.9 Hz); 7.42 (1H, s); 4.79 (2H, s); 3.53-3.36 (4H, m).

### d) 5-(Bromomethyl)-2,3-dihydro-1-benzothiophene 1,1-dioxide

The title compound was prepared by refluxing (1,1-dioxido-2,3-dihydro-1-benzothien-5-yl)methanol (1.0 g, 5 mmol) with phosphorus tribromide (0.524 g, 0.188 ml, 2 mmol) in dry toluene (20 ml) for 1 h. Water was added and the crude mixture was extracted with I EtOAc, washed with water and brine and dried. The solvents were removed in vacuo to afford the subtitle compound (1.15 g, 88%).
¹H-NMR (CDCl₃): δ 7.73 (1H, d, *J* 8.0 Hz); 7.51 (1H, d, *J* 8.0 Hz); 7.43 (1H, s); 4.51 (2H, s); 3.53 (2H, t, *J* 6.8 Hz); 3.43 (2H, t, *J* 6.8 Hz).

### e)(1,1-Dioxido-2,3-dihydro-1-benzothien-5-yl)methylamine

The title compound was prepared by stirring 5-(bromomethyl)-2,3-dihydro-1-benzothiophene 1,1-dioxide (1.14 g, 4.36 mmol) with aqueous ammonia (43 ml) in methanol/THF 1:1 (30 ml) overnight. The solvents were removed in vacuo to afford the subtitle compound (700 mg, 81%).
¹H-NMR (CDCl₃): δ 8.05 (2H, brs); 7.82 (1H, d, *J* 8.3 Hz); 7.63 (1H, s); 7.61 (1H, d, *J* 8.3 Hz); 4.15 (2H, s); 3.62 (2H, t, *J* 6.9 Hz); 3.36 (2H, t, *J* 6.9 Hz).
APCI-MS m/z: 198 [MH⁺].

### f) N-[(1,1-Dioxido-2,3-dihydro-1-benzothien-5-yl)methyl]-6-methyl-2-oxo-1-[3-(trifluoromethyl)phenyl]-1,2-dihydropyridine-3-carboxamide

Starting from 6-methyl-2-oxo-1-[3-(trifluoromethyl)phenyl]-1,2-dihydropyridine-3-carboxylic acid (743 mg, 2.5 mmol) and (1,1-dioxido-2,3-dihydro-1-benzothien-5-yl)methylamine (500 mg, 2.5 mmol) using the method described in Example 17, the title compound (1.05 g, 88%) was obtained.
¹H-NMR (CDCl₃): δ 9.94 (1H, brt, *J* 6.0 Hz); 8.39 (1H, d, *J* 7.5 Hz); 7.93 (1H, s); 7.91(1H, d, *J* 7.7 Hz); 7.83 (1H, t, *J* 7.7 Hz); 7.74 (1H, d, *J* 7.7 Hz); 7.70 (1H, d, *J* 8.0 Hz); 7.45(1H, d, *J* 8.0 Hz); 7.43 (1H, s); 6.64 (1H, d, *J*7.5 Hz); 4.58 (2H, d, *J* 6.0 Hz); 3.57 (2H, t, *J* 6.9 Hz); 3.34 (2H, t, *J* 6.9 Hz); 2.04 (3H, s).
APCI-MS m/z: 477 [MH⁺].

### Example 64 N-[(1,1-Dioxido-2,3-dihydro-1-benzothien-5-yl)methyl]-5-iodo-6-methyl-2-oxo-1-[3-(trifluoromethyl)phenyl]-1,2-dihydropyridine-3-carboxamide

To a solution of N-[(1,1-dioxido-2,3-dihydro-1-benzothien-5-yl)methyl]-6-methyl-2-oxo-1-[3-(trifluoromethyl)phenyl]-1,2-dihydropyridine-3-carboxamide (102 mg, 0.21 mmol), dichloromethane (1.8 ml) and TFA (0.9 ml) was added N-iodosuccinimide (47 mg, 0.21 mmol). The mixture was stirred at room temperature for 4 h and the solvent was then removed in vacuo. The residue was partitioned between EtOAc and aqueous NaHCO₃ and the organic extract was washed with water, dried, filtered and evaporated. The crude product was purified by preparative HPLC to give the title compound as a white solid (87 mg, 69%).
¹H-NMR (CDCl₃): δ 9.85 (1H, t, *J* 5.7 Hz); 8.91 (1H, s); 7.83 (1H, d, *J* 8.1 Hz); 7.76 (1H, t, *J* 8.0 Hz); 7.68 (1H, d, *J* 8.0 Hz); 7.49 (1H, s); 7.41 (2H, d, *J* 8.0 Hz); 7.34 (1H, s); 4.64 (2H, t, *J* 6.5 Hz); 3.48 (2H, t, *J* 6.9 Hz); 3.35 (2H, t, *J* 7.0 Hz); 2.32 (3H, s).
APCI-MS m/z: 603 [MH⁺].

### Example 65 5-Iodo-N-{4-[isopropyl(methylsulfonyl)amino]benzyl}-6-methyl-2-oxo-1-[3-(trifluoromethyl)phenyl]-1,2-dihydropyridine-3-carboxamide

The title product was prepared as described for Example 64 but starting from N-{4-[isopropyl(methylsulfonyl)-amino]benzyl}-6-methyl-2-oxo-1-[3-(trifluoromethyl)phenyl]-1,2-dihydropyridine-3-carboxamide. White powder (4 mg, 68%).
¹H-NMR (400 MHz, DMSO-d₆) d 9.77 (1H, t, *J* 6.1 Hz); 8.61 (1H, s); 7.90 (2H, t, *J* 8.2 Hz); 7.81 (1H, t, *J* 7.9 Hz); 7.72 (1H, d, *J* 7.9 Hz); 7.33 (2H, d, *J* 8.2 Hz); 7.21 (2H, d, *J* 8.3 Hz); 4.51 (2H, d, *J* 6.0 Hz); 4.30 (1H, quintet, *J* 6.7 Hz); 3.02 (3H, s); 2.20 (3H, s); 1.04 (6H, d, *J* 6.7 Hz).
APCI-MS m/z: 648 [MH⁺].

### Example 66 1,2-Dihydro-6-methyl-N-[[4-[(methylsulfonyl)methyl]phenyl]-methyl]-2-oxo-1-[3-(trifluoromethyl)phenyl]-3-pyridinecarboxamide

### a) 6-Methyl-N-{4-[(methylthio)methyl]benzyl}-2-oxo-1-[3-(trifluoromethyl)phenyl]-1,2-dihydropyridine-3-carboxamide

To a mixture of 1-(3-methylphenyl)-2-oxo-1,2-dihydropyridine-3-carboxylic acid (412 mg, 1.39 mmol), TBTU (527 mg, 1.39 mmol) and DIEA (719 mg, 5.56 mmol) in NMP was added [(methylthio)methyl]benzene (232 mg, 1.39 mmol) in NMP (1 ml). The reaction was stirred for 1 h at room temperature, then diluted with water (15 ml) and extracted with EtOAc. The organic phase was dried (MgSO₄), filtered and evaporated affording the crude title compound (620 mg), which was used directly in the next step.

### b) 1,2-Dihydro-6-methyl-N-[[4-[(methylsulfonyl)methyl]phenyl]methyl]-2-oxo-1-[3-(trifluoromethyl)phenyl]-3-pyridinecarboxamide.

To crude 6-methyl-N-{4-[(methylthio)methyl]benzyl}-2-oxo-1-[3-(trifluoromethyl)phenyl]-1,2-dihydropyridine-3-carboxamide (620 mg 1.39 mmol) in CH₂Cl₂ (10 ml) cooled to -150 °C was added m-chloroperoxybenzoic acid (483 mg, 2.8 mmol). The mixture was stirred for 30 min and then overnight at room temperature. The reaction mixture was diluted with more CH₂Cl₂ and water, washed with sodium thiosulfate, sodium bicarbonate and brine. The solvent was removed in vacuum and 25 mg of the residue was dissolved in CH₃CN/water (2.0 ml) and purified on a Xterra@Prep MS C8 column (19 x 50 mm) using a gradient of CH₃CN/water at a flow rate of 20 ml/min. Freeze drying of the mixture afforded the title compound (15 mg).
¹H-NMR (400 MHz, DMSO-d₆) δ 9.66 (1H, t, *J* 5.7 Hz); 9.27 (1H, s,); 8.36 (1H, d, *J7.4* Hz); 7.86 (2H, d, *J* 4.5 Hz); 7.78 (1H, t, *J* 8.0 Hz); 7.68 (1H, d, *J* 7.9 Hz); 7.06 (2H, d, *J* 8.3 Hz); 6.67 (2H, d, *J* 8.5 Hz); 6.60 (1H, d, *J* 7.4 Hz); 4.33 (2H, d, *J* 5.8 Hz); 1.99 (3H, s).
APCI-MS m/z: 479.3 [MH⁺].

### Example 67 6-Chloro-5-methyl-4-(3-methylphenyl-N-[4-(methylsulfonyl)benzyl]-3-oxo-3,4-dihydropyrazine-2-carboxamide

### a) 6-Chloro-5-methyl-4-(3-methylphenyl)-3-oxo-3,4-dihydropyrazine-2-carbonitrile

The title compound was prepared essentially as described by Gibson. et al. *J. Org. Chem.* **1994**, 59, 1072-1077 and Hoornaert et al. Tetrahedron, **1990**, 46, 5715-5732.

### b) 6-Chloro-5-methyl-4-(3-methylphenyl)-3-oxo-3,4-dihydropyrazine-2-carboxylic acid

A solution of 6-chloro-5-methyl-4-(3-methylphenyl)-3-oxo-3,4-dihydropyrazine-2-carbonitrile (100 mg, 0.38 mmol) in 11M sulphuric acid (10 ml) was heated at 90 °C for 16 h. Water (200 ml) was added. The water phase was extracted with dichloromethane.
The organic layer was dried, filtered and evaporated to give the subtitle compound (20 mg, 19%).
APCI-MS m/z: 279.2 [MH⁺], HPLC Chromolith speedROD RP 18e 50-4.6 mm, flow 2.5 ml/min, wavelength 254 nm, time 1.93 min.

### c) 6-Chloro-5-methyl-4-(3-methylphenyl-N-[4-(methylsulfonyl)benzyl]-3-oxo-3,4-dihydropyrazine-2-carboxamide

The title compound was prepared starting from 6-chloro-5-methyl-4-(3-methylphenyl)-3-oxo-3,4-dihydropyrazine-2-carboxylic acid and (4-methylsulfonyl)benzyl amine as described in Example 17.
¹H-NMR (DMSO-d₆): δ 9.68 (1H, t); 7.87 (2H, d); 7.57 (2H, d); 7.49 (1H, t); 7.36 (1H, d); 7.19 (2H, d); 4.59 (2H, d); 3.18 (3H, s); 2.36 (3H, s); 2.11 (3H, s).
APCI-MS m/z: 446.3 [MH⁺].

### Example 68 5-Bromo-6-(difluoromethyl)-N-[4-(methylsulfonyl)benzyl]-2-oxo-1-[3-(trifluoromethyl)phenyl]-1,2-dihydropyridine-3-carboxamide

### a) Ethyl 6-methyl-2-oxo-1-[3-(trifluoromethyl)phenyl]-1,2-dihydropyridine-3-carboxylate

The title compound was prepared by stirring a mixture of 6-methyl-2-oxo-1-[3-(trifluoromethyl)phenyl]-1,2-dihydropyridine-3-carboxylic acid (20.8 g, 70 mmol) with sodium carbonate (8.16 g, 77 mmol) in NMP (150 ml). Ethyl iodide (15.6 g, 100 mmol) was added slowly (about 10-15 minutes) and the mixture stirred at room temperature for 4 h. Water was added and the crude product was extracted with EtOAc, washed with water and dried and filtered. The solvent was removed in vacuo and the *residue* triturated with diethyl ether (100 ml), filtered, washed with diethyl ether and dried to afford the subtitle compound (18 g, 79%) as a white solid.
¹H-NMR (CDCl₃): δ 8.21 (1H, d, *J* 7.4 Hz); 7.75 (1H, d, *J* 7.9 Hz); 7.68 (1H, t, *J* 7.9 Hz); 7.49 (1H, s); 7.42 (1H, d, *J* 7.9 Hz); 6.25 (1H, d, *J*7.4 Hz); 4.36 (2H, q, *J*7.2 Hz); 2.03 (3H, s); 1.37 (3H, t, *J* 7.2 Hz).
APCI-MS m/z: 326 [MH⁺].

### b) Ethyl 5-bromo-6-(bromomethyl)-2-oxo-1-[3-(trifluoromethyl)phenyl]-1,2-dihydropyridine-3-carboxylate

The subtitle compound (3.25 g, 98%) was prepared by stirring ethyl 6-methyl-2-oxo-1-[3-(trifluoromethyl)phenyl]-1,2-dihydropyridine-3-carboxylate (2.25 g, 6.9 mmol) with N-bromosuccinimide (2.45 g, 13.8 mmol) and benzoyl peroxide (35 mg, 0.14 mmol) in carbon tetrachloride (40 ml) at 70 °C for 4 h.
¹H-NMR (CDCl₃): δ 8.33 (1H, s); 7.82 (1H, d, *J* 7.9 Hz); 7.72 (1H, t, *J* 7.9 Hz); 7.62 (1H, s); 7.56 (1H, d, *J* 7.9 Hz); 4.38 (2H, q, *J* 7.1 Hz); 4.16-4.08 (2H, m); 1.37 (3H, t, *J* 7.1 Hz).
APCI-MS m/z: 482/484/486 [MH⁺].

### c) Ethyl 5-bromo-6-(hydroxymethyl)-2-oxo-1-[3-(trifluoromethyl)phenyl]-1,2-dihydropyridine-3-carboxylate

The subtitle compound was prepared in quantitative yield by stirring ethyl 5-bromo-6-(bromomethyl)-2-oxo-1-[3-(trifluoromethyl)phenyl]-1,2-dihydropyridine-3-carboxylate with aqueous sodium bicarbonate in aqueous THF at 60 °C overnight.
¹H-NMR (CDCl₃): δ 8.35 (1H, s); 7.78 (1H, d, *J* 7.9 Hz); 7.68 (1H, t, *J7.9* Hz); 7.57 (1H, s); 7.50 (1H, d, *J* 7.9 Hz); 4.45-4.33 (4H, m); 1.37 (3H, t, *J* 7.1 Hz).
APCI-MS m/z: 420/422 [MH⁺].

### d) Ethyl 5-bromo-6-formyl-2-oxo-1-[3-(trifluoromethyl)phenyl]-1,2-dihydropyridine-3-carboxylate

Dimethyl sulphoxide (1.14 g, 1.036 ml, 14.6 mmol) was added dropwise to a solution of oxalyl chloride (0.93 g, 0.64 ml, 7.3 mmol) in dry CH₂Cl₂ (40 ml) at -70 °C under an argon atmosphere. After 10 minutes stirring, ethyl 5-bromo-6-(hydroxymethyl)-2-oxo-1-[3-(trifluoromethyl)phenyl]-1,2-dihydropyridine-3-carboxylate (2.8 g, 6.66 mmol) in CH₂Cl₂ (10 ml) was added and stirring was continued for 20 minutes followed by the addition of triethylamine (3.34 g, 4.6 ml, 33 mmol). After a further 15 minutes at low temperature, the reaction mixture was allowed to reach -15 °C and water (20 ml) was added. Stirring was continued until the reaction mixture reached room temperature. It was then extracted with water and CH₂Cl₂, washed with brine, dried and filtered. The solvents were removed in vacuo and the residue purified by column chromatography on silica using CH₂Cl₂ as eluent to afford the title compound (1.46 g, 52%).
¹H-NMR (CDCl₃): δ 9.74 (1H, s); 8.31 (1H, s); 7.75 (1H, d, *J* 7.9 Hz); 7.65 (1H, t, *J* 7.9 Hz); 7.46 (1H, s); 7.41 (1H, d, *J* 7.9 Hz); 4.41 (2H, q, *J* 7.1 Hz); 1.39 (3H, t, *J* 7.1 Hz).
APCI-MS m/z: 418/420 [MH⁺].

### e) Ethyl 5-bromo-6-(difluoromethyl)-2-oxo-1-[3-(trifluoromethyl)phenyl]-1,2-dihydropyridine-3-carboxylate.

The subtitle compound was prepared by stirring ethyl 5-bromo-6-formyl-2-oxo-1-[3-(trifluoromethyl)phenyl]-1,2-dihydropyridine-3-carboxylate (0.98 g, 2.34 mmol) with (diethylamino)sulfur trifluoride (DAST) (378 mg, 2.34 mmol) in dry CH₂Cl₂ (20 ml) overnight. Water was added and the reaction mixture was extracted with CH₂Cl₂. The solvents were removed in vacuo to afford 1.06 g (100%) of the subtitle compound.
¹H-NMR (CDCl₃): δ 8.29 (1H, brt, *J* 1.1 Hz); 7.77 (1H, d, *J* 8.1 Hz); 7.65 (1H, t, *J* 8.1 Hz); 7.53 (1H, s); 7.46 (1H, t, *J* 8.1 Hz); 6.82 (1H, t, *J* 55.1 Hz); 4.39 (2H, q, *J* 7.1 Hz); 1.38 (2H, q, *J* 7.1 Hz).
APCI-MS m/z: 440/442 [MH⁺].

### f) 5-Bromo-6-(difluoromethyl)-N-[4-(methylsulfonyl)benzyl]-2-oxo-1-[3-(trifluoromethyl)phenyl]-1,2-dihydropyridine-3-carboxamide

The title compound was prepared from 5-bromo-6-(difluoromethyl)-2-oxo-1-[3-(trifluoromethyl)phenyl]-1,2-dihydropyridine-3-carboxylic acid [obtained from ethyl 5-bromo-6-(difluoromethyl)-2-oxo-1-[3-(trifluoromethyl)phenyl]-1,2-dihydropyridine-3-carboxylate by alkaline hydrolysis] and 4-(methylsulfonyl) benzylamine hydrochloride using the method described in Example 17.
¹H-NMR (CDCl₃): δ 9.79 (1H, brt, *J* 5.2 Hz); 8.78 (1H, s); 7.89 (2H, d, *J* 8.4 Hz); 7.82 (1H, d, *J* 7.8 Hz); 7.70 (1H, t, *J* 7.8 Hz); 7.55 (1H, s); 7.50 (2H, d, *J* 8.4 Hz); 7.47 (1H, d, J7.8 Hz); 6.92 (1H, t, *J* 51.9 Hz); 4.67 (2H, m); 3.02 (3H, s).
APCI-MS m/z: 579/581 [MH⁺].

### Example 69 6-(Difluoromethyl)-N-[4-(methylsulfonyl)benzyl]-2-oxo-1-[3-(trifluoromethyl)phenyl]-1,2-dihydropyridine-3-carboxamide

The title compound was prepared by hydrogenation of 5-bromo-6-(difluoromethyl)-N-[4-(methylsulfonyl)benzyl]-2-oxo-1-[3-(trifluoromethyl)phenyl]-1,2-dihydropyridine-3-carboxamide with palladium on charcoal (Pd/C) and ammonium formate in methanol.
¹H-NMR (CDCl₃): δ 10.00 (1H, brt, *J* 5.8 Hz); 8.76 (1H, d, *J* 7.5 Hz); 7.89 (2H, d, *J* 8.2 Hz); 7.87 (1H, d, *J* 7.9 Hz); 7.77 (1H, t, *J* 7.9 Hz); 7.58 (1H, s); 7.52 (1H, d, *J* 7.9 Hz); 7.51 (2H, d, *J* 8.2 Hz); 6.98 (1H, d, *J* 7.5 Hz); 6.10 (1H, t, *J* 53.0 Hz); 4.70 (2H, m); 3.03 (3H, s).
APCI-MS m/z: 501 [MH⁺].

The compounds described in Examples 70.1 to 70.50 were prepared by a method analogous to that described in Examples 1 and 2:

### Example 70.1 N-(2,3-Dihydro-1,4-benzodioxin-6-ylmethyl)-6-methyl-2-oxo-1-[3-(trifluoromethyl)phenyl]-1,2-dihydropyridine-3-carboxamide

¹H NMR (DMSO-d₆): δ 9.93 (1H, t); 8.38 (1H, d); 7.88 (2H, d); 7.80 (1H, t); 7.70 (1H, d); 6.79-6.70 (3H, m); 6.62 (1H, d); 4.34 (2H, d); 4.18 (4H, s).
APCI-MS m/z: 466.3 [MH⁺].

### Example 70.2 6-Methyl-N-[3-(methylsulfonyl)benzyl]-2-oxo-1-[3-(trifluoromethyl)phenyl]-1,2-dihydropyridine-3-carboxamide

¹H NMR (DMSO-d₆): δ 9.93 (1H, t); 8.38 (1H, d); 7.95-7.45 (8H, m); 6.62 (1H, d); 4.58 (2H, d); 3.18 (3H, s).

### Example 70.3 6-Methyl-N'-[4-(methylsulfonyl)phenyl]-2-oxo-1-[3-(trifluoromethyl)phenyl]-1,2 dihydropyridine-3-carbohydrazide

¹H NMR (DMSO-d₆): δ 10.80 (1H, s); 9.62 (1H, d); 7.99-7.74 (4H, m); 7.65 (2H, d); 6.80 (2H, d); 6.67 (1H, d); 3.07 (3H, s).

### Example 70.4 N'-(4-Bromophenyl)-6-methyl-2-oxo-1-[3-(trifluoromethyl)phenyl]-1,2-dihydropyridine-3-carohydrazide

¹H NMR (DMSO-d₆): δ 10.69 (1H, s); 8.35 (1H, d); 7.99-7.73 (4H, m); 7.29 (2H, d); 6.66 (3H, dd).

### Example 70.5 N-[(5-Methoxy-4-oxo-4H-pyran-2-yl)methyl]-6-methyl-2-oxo-1-[3-(trifluoromethyl)phenyl]-1,2-dihydropyridine-3-carboxamide

APCI-MS m/z: 435.2 [MH⁺].

### Example 70.6 N-(4-Cyanobenzyl)-6-methyl-2-oxo-1-[3-(trifluoromethyl)phenyl]-1,2-dihydropyridine-3-carboxamide

APCI-MS m/z: 412.3 [MH⁺].

### Example 70.7 N-{[3-(4-Methoxyphenyl)isoxazol-5-yl]methyl}-6-methyl-2-oxo-1-[3-(trifluoromethyl)phenyl]-1,2-dihydropyridine-3-carboxamide

APCI-MS m/z: 484.4 [MH⁺].

### Example 70.8 N'-(4-Cyanophenyl)-6-methyl-2-oxo-1-[3-(trifluoromethyl)phenyl]-1,2-dihydropyridine-3-carbohydrazide

APCI-MS m/z: 413.3 [MH⁺].

### Example 70.9 6-Methyl-2-oxo-N-[(1-phenyl-1H-pyrazol-4-yl)methyl]-1-[3-(trifluoromethyl)phenyl]-1,2-dihydropyridine-3-carboxamide

APCI-MS m/z: 467.3 [MH⁺].

### Example 70.10 N-(2,3-Dihydro-1,4-benzodioxin-2-ylmethyl-6-methyl-2-oxo-1-[3-(trifluoromethyl)phenyl]-1,2-dihydropyridine-3-carboxamide

APCI-MS m/z: 445.2 [MH⁺].

### Example 70.11 6-Methyl-N-{[1-(3-methylphenyl)-1H-pyrazol-4-yl]methyl}-2-oxo-1-[3-(trifluoromethyl)phenyl]-1,2-dihydropyridine-3-carboxamide

APCI-MS m/z: 467.3 [MH⁺].

### Example 70.12 N'-(4-Chlorophenyl)-6-methyl-2-oxo-1-[3-(trifluoromethyl)phenyl]-1,2-dihydropyridine-3-carbohydrazide

APCI-MS m/z: 422.2 [MH⁺].

### Example 70.13 6-Methyl-2-oxo-N-[2-(tetrahydro-2H-pyran-4-yl)ethyl]-1-[3-(trifluoromethyl)phenyl]-1,2-dihydropyridine-3-carboxamide

APCI-MS m/z: 409.4 [MH⁺].

### Example 70.14 N-[(1-Ethyl-1H-pyrazol-4-yl)methyl]-6-methyl-2-oxa-1-[3-(trifluoromethyl)phenyl]-1,2-dihydropyridine-3-carboxamide

APCI-MS m/z: 405.2 [MH⁺].

### Example 70.15 N-[(4-Benzylmorpholin-2-yl)methyl]-6-methyl-2-oxo-1-[3-(trifluoromethyl)phenyl]-1,2-dihydropyridine-3-carboxamide

APCI-MS m/z: 486.3 [MH⁺].

### Example 70.16 6-Methy-N-[3-(2-methylpiperidin-1-yl)propyl]-2-oxo-1-[3-(trifluoromethyl)phenyl]-1,2-dihydropyridine-3-carboxamide

APCI-MS m/z: 436.3 [MH⁺].

### Example 70.17 Methyl 2-{[({6-methyl-2-oxo-1-[3-trifluoromethyl)phenyl]-1,2-dihydropyridin-3-yl}carbonyl)amino]methyl}-3-furoate

APCI-MS m/z: 558.3 [MH⁺].

### Example 70.18 6-Methyl-N-[(1-methyl-1H-pyrazol-4-yl)methyl]-2-oxo-1-[3-(trifluoromethyl)phenyl]-1,2-dihydropyridine-3-carboxamide

APCI-MS m/z: 391.2 [MH⁺].

### Example 70.19 N-(3-Azepan-1-ylpropyl)-6-methyl-2-oxo-1-[3-(trifluoromethyl)phenyl]-1,2-dihydropyridine-3-carboxamide

APCI-MS m/z: 436.3 [MH⁺].

### Example 70.20 6-Methyl-N-(3-morpholin-4-ylpropyl)-2-oxo-1-[3-(trifluoromethyl)phenyl]-1,2-dihydropyridine-3-carboxamide

APCI-MS m/z: 424.3 [MH⁺].

### Example 70.21 6-Methyl-2-oxo-N-(3-piperidin-1-ylpropyl)-1-[3-(trifluoromethyl)phenyl]-1,2-dihydropyridine-3-carboxamide

APCI-MS m/z: 422.3 [MH⁺].

### Example 70.22 N-[3-(3,5-Dimethyl-1H-pyrazol-1-yl)propyl]-6-methyl-2-oxo-1-[3-(trifluoromethyl)phenyl]-1,2-dihydropyridine-3-carboxamide

APCI-MS m/z: 433.3 [MH⁺].

### Example 70.23 N-[3-(2-Ethylpiperidin-1-yl)propyl]-6-methyl-2-oxo-1-[3-(trifluoromethyl)phenyl]-1,2-dihydropyridine-3-carboxamide

APCI-MS m/z: 450.4 [MH⁺].

### Example 70.24 6-Methyl-N-[2-(1-methyl-1H-imidazol-5-yl)ethyl]-2-oxo-1-[3-(trifluoromethyl)phenyl]-1,2-dihydropyridine-3-carboxamide

APCI-MS m/z: 405.2 [MH⁺].

### Example 70.25 N-[(1-Ethyl-3-methyl-1H-pyrazol-4-yl)methyl]-6-methyl-2-oxo-1-[3-(trifluoromethyl)phenyl]-1,2-dihydropyridine-3-carboxamide

APCI-MS m/z: 419.2 [MH⁺].

### Example 70.26 N-[4-(Acetylamino)benzyl]-6-methyl-2-oxo-1-[3-(trifluoromethyl)phenyl]-1,2-dihydropyridine-3-carboxamide

APCI-MS m/z: 444.2 [MH⁺].

### Example 70.27 6-Methyl-2-oxo-N-[3-(1H-pyazol-1-yl)propyl]-1-[3-(trifluoromethyl)phenyl]-1,2-dihydropyridine-3-carboxamide

APCI-MS m/z: 405.2 [MH⁺].

### Example 70.28 6-Methyl-2-oxo-N-(pyridin-2-ylmethyl)-1-[3-(trifluoromethyl)phenyl]-1,2-dihydropyridine-3-carboxamide

APCI-MS m/z: 388.3 [MH⁺].

### Example 70.29 6-Methyl-N-{[1-(4-methylphenyl)-1H-pyrazol-4-yl]methyl}-2-oxo-1-[3-(trifluoromethyl)phenyl]-1,2-dihydropyridine-3-carboxamide

APCI-MS m/z: 467.3 [MH⁺].

### Example 70.30 6-Methyl-N'-(4-methylphenyl)-2-oxo-1-[3-(trifluoromethyl)phenyl]-1,2-dihydropyridine-3-carbohydrazide

APCI-MS m/z: 402.2 [MH⁺].

### Example 70.31 6-Methyl-N-[3-(4-methylpiperidin-1-yl)propyl]-2-oxo-1-[3-(trifluoromethyl)phenyl]-1,2-dihydropyridine-3-carboxamide

APCI-MS m/z: 436.3 [MH⁺].

### Example 70.32 6-Methyl-2-oxo-N-[3-(5-oxo-4,5-dihydro-1H-pyrazol-4-yl)propyl]-1-[3-(trifluoromethyl)phenyl]-1,2-dihydropyridine-3-carboxamide

APCI-MS m/z: 421.3 [MH⁺].

### Example 70.33 Ethyl 5-methyl-4-{[({6-methyl-2-oxo-1-[3-(trifluoromethyl)phenyl]-1,2-dihydropyridin-3-yl}carbonyl)amino]methyl}-2-furoate

APCI-MS m/z: 463.3 [MH⁺].

### Example 70.34 N-[(6-Fluoro-4H-1,3-benzodioxin-8-yl)methyl]-6-methyl-2-oxo-1-[3-(trifluoromethyl)phenyl]-1,2-dihydropyridine-3-carboxamide

APCI-MS m/z: 463.3 [MH⁺].

### Example 70.35 6-Methyl-2-oxo-N-(2-pyridin-3-ylethyl)-1-[3-(trifluoromethyl)phenyl]-1,2-dihydropyridine-3-carboxamide

APCI-MS m/z: 402.2 [MH⁺].

### Example 70.36 N-[(1,3-Dimethyl-1H-pyrazol-4-yl)methyl]-6-methyl-2-oxo-1-[3-(trifluoromethyl)phenyl]-1,2-dihydropyridine-3-carboxamide

APCI-MS m/z: 405.2 [MH⁺].

### Example 70.37 6-Methyl-2-oxo-N-(2-pyridin-4-ylethyl)-1-[3-(trifluoromethyl)phenyl]-1,2-dihydropyridine-3-carboxamide

APCI-MS m/z: 402.2 [MH⁺].

### Example 70.38 N'-(4-Fluorophenyl)-6-methyl-2-oxo-1-[3-(trifluoromethyl)phenyl]-1,2-dihydropyridine-3-carbohydrazide

APCI-MS m/z: 406.2 [MH⁺].

### Example 70.39 6-Methyl-N-[(1-methyl-1H-pyrrol-2-yl)methyl]-2-oxo-1-[3-(trifluoromethyl)phenyl]-1,2-dihydropyridine-3-carboxamide

APCI-MS m/z: 390.3 [MH⁺].

### Example 70.40 6-Methyl-2-oxo-N'-phenyl-1-[3-(trifluoromethyl)phenyl]-1,2-dihydropyridine-3-carbohydrazide

APCI-MS m/z: 388.3 [MH⁺].

### Example 70.41 N-[(1-Ethyl-5-methyl-1H-pyrazol-4-yl)methyl]-6-methyl-2-oxo-1-[3-(trifluoromethyl)phenyl]-1,2-dihydropyridine-3-carboxamide

APCI-MS m/z: 419.2 [MH⁺].

### Example 70.42 6-Methyl-N-[2-(1-methyl-1H-imidazol-4-yl)ethyl]-2-oxo-1-[3-(trifluoromethyl)phenyl]-1,2-dihydropyridine-3-carboxamide

APCI-MS m/z: 405.2 [MH⁺].

### Example 70.43 N-[2-(1,3-Dioxolan-2-yl)ethyl]-6-methyl-2-oxo-1-[3-(trifluoromethyl)phenyl]-1,2-dihydropyridine-3-carboxamide

APCI-MS m/z: 397.3 [MH⁺].

### Example 70.44 N-(1-Benzothien-3-ylmethyl)-6-methyl-2-oxo-1-[3-(trifluoromethyl)phenyl]-1,2-dihydropyridine-3-carboxamide

APCI-MS m/z: 443.3 [MH⁺].

### Example 70.45 N-[(1,5-Dimethyl-1H-pyrazol-4-yl)methyl]-6-methyl-2-oxo-1-[3-(trifluoromethyl)phenyl]-1,2-dihydropyridine-3-carboxamide

APCI-MS m/z: 405.2 [MH⁺].

### Example 70.46 N-[2-(3,5-Dimethyl-1H-pyrazol-4-yl)ethyl]-6-methyl-2-oxo-1-[3-(trifluoromethyl)phenyl]-1,2-dihydropyridine-3-carboxamide

APCI-MS m/z: 419.2 [MH⁺].

### Example 70.47 N-[2-(3,5-Dimethylisoxazol-4-yl)ethyl]-6-methyl-2-oxo-1-[3-(trifluoromethyl)phenyl]-1,2-dihydropyridine-3-carboxamide

APCI-MS m/z: 420.3 [MH⁺].

### Example 70.48 N-(3,4-Dihydro-1H-isochromen-1-ylmethyl)-6-methyl-2-oxo-1-[3-(trifluoromethyl)phenyl]-1,2-dihydropyridine-3-carboxamide

APCI-MS m/z: 443.2 [MH⁺].

### Example 70.49 N-{[(2R)-1-Ethylpyrrolidin-2-yl]methyl}-6-methyl-2-oxo-1-[3-(trifluoromethyl)phenyl]-1,2-dihydropyridine-3-carboxamide

APCI-MS m/z: 408.3 [MH⁺].

### Example 70.50 6-Methyl-2-oxo-N-[(2R)-tetrahydrofuran-2-ylmethyl]-1-[3-(trifluoromethyl)phenyl]-1,2-dihydropyridine-3-carboxamide

APCI-MS m/z: 381.2 [MH⁺].

### Example 71 5-Chloro-N-{4-[(dimethylamino)sulfonyl]benzyl}-6-methyl-2-oxo-1-[3-(trifluoromethyl)phenyl]-1,2-dihydropyridine-3-carboxamide

### a) N-[4-(Benzylthio)benzyl]-6-methyl-2-oxo-1-[3-(trifluoromethyl)phenyl]-1,2-dihydropyridine-3-carboxamide

The subtitle compound was prepared as described for N-[4-(benzylsulfonyl)benzyl]-6-methyl-2-oxo-1-[3-(trifluoromethyl)phenyl]-1,2-dihydropyridine-3-carboxamide [Example 27 (b)] but excluding the oxidation step. The sub title product was purified by preparative HPLC (x-terra column, 0.2% ammonia, acetonitrile) to afford the title compound.
¹H NMR (DMSO): δ 9.78 (1H, t, *J* 6.0 Hz); 8.37 (1H, d, *J* 7.5 Hz); 7.91 - 7.68 (4H, m); 7.35 - 7.16 (9H, m); 6.62 (1H, d, *J* 7.6 Hz); 4.47 - 4.37 (2H, m); 4.20 (2H, s); 2.01 (3H, s).
APCI-MS m/z: 509 [MH⁺].

### b) 5-Chloro-N-{4-[(dimethylamino)sulfonyl]benzyl}-6-methyl-2-oxo-1-[3-(trifluoromethyl)phenyl]-1,2-dihydropyridine-3-carboxamide

To N-[4-(benzylthio)benzyl]-6-methyl-2-oxo-1-[3-(trifluoromethyl)phenyl]-1,2-dihydropyridine-3-carboxamide (92 mg, 0.18 mmol) was added 50% formic acid/water (18 ml) and CH₂Cl₂ (9 ml). The reaction mixture was cooled to -20 °C and chlorine gas was bubbled through for 1 min. After evaporation of the excess chlorine the reaction mixture was partitioned between CH₂Cl₂ and water. The organic phase was washed with 0.5M aqueous NaHCO₃ and brine, and then dried. After filtration, the solvent was removed in vacuo and the residue was dissolved in ethanol (10 ml). 5.6M Dimethylamine in ethanol (250 µl) was added and the mixture was stirred at room temperature overnight. After removal of the solvent the residue was purified by preparative HPLC (x-terra, 0.2% ammonia, acetonitrile) to afford the title compound (41 mg, 43%).
¹H NMR (CDCl₃): δ 9.88-9.81 (1H, m); 8.65 (1H, s); 7.85 - 7.67 (4H, m); 7.51 - 7.39 (4H, m); 4.73 - 4.59 (2H, m); 2.68 (6H, s); 2.19 (3H, s).
APCI-MS m/z: 528 [MH⁺].

### Example 72 N-{4-[(Dimethylamino)sulfonyl]benzyl}-6-methyl-2-oxo-1-[3-(trifluoromethyl)-phenyl]-1,2-dihydropyridine-3-carboxamide

5-Chloro-N-{4-[(dimethylamino)sulfonyl]benzyl}-6-methyl-2-oxo-1-[3-(trifluoromethyl)phenyl]-1,2-dihydropyridine-3-carboxamide (15 mg, 0.028 mmol) was dissolved in hot methanol (1 ml). After cooling to room temperature, ammonium formate (6 mg, 0.1 mmol) and 10% palladium on carbon (3 mg) were added. The reaction mixture was stirred in a sealed vial at room temperature for 4 h. After filtration through Celite, the solvent was evaporated and the residue was purified by column chromatography on silica using CH₂Cl₂/methanol (98:2) as eluent to afford the title compound (6 mg, 43%).
¹H NMR (CDCl₃): δ 10.01-9.92 (1H, m); 8.59 (1H, d, *J* 7.5 Hz); 7.85 - 7.66 (4H, m); 7.55 - 7.41 (4H, m); 6.48 (1H, d, *J* 7.5 Hz); 4.75 - 4.59 (2H, m); 2.68 (6H, s); 2.09 (3H, s).
APCI-MS m/z: 494 [MH⁺].

### Example 73 5-Chloro-6-methyl-2-oxo-N-[4-(piperazin-1-ylsulfonyl)benzyl]-1-[3-(trifluoro-methyl)phenyl]-1,2-dihydropyridine-3-carboxamide

The title compound was prepared using the general method of Example 71.
¹H NMR (CDCl₃): δ 9.99 - 9.82 (2H, m); 8.64 (1H, d, *J* 8.2 Hz); 7.86 - 7.62 (4H, m); 7.53 - 7.39 (4H, m); 4.76 - 4.55 (2H, m); 3.86 - 2.92 (8H, m); 2.19 (3H, s). APCI-MS m/z: 569 [MH⁺].

Using the general method of Example 72, the compounds of Examples 74 to 78 were prepared:

### Example 74 6-Methyl-2-oxo-N-[4-(piperazin-1-ylsulfonyl)benzyl]-1-[3-(trifluoromethyl)-phenyl]-1,2-dihydropyridine-3-carboxamide

¹H NMR (CDCl₃): δ 10.11- 9.79 (2H, m); 8.67 - 8.50 (1H, m); 7.89 - 7.38 (8H, m); 6.52 - 6.43 (1H, m); 4.78 - 4.52 (2H, m); 3.89 - 3.62 (2H, m); 3.49 - 2.94 (6H, m); 2.09 (3H, s).
APCI-MS m/z: 535 [MH⁺].

### Example 75 6-Methyl-N-[4-(morpholin-4-ylsulfonyl)benzyl]-2-oxo-1-[3-(trifluoromethyl)phenyl]-1,2-dihydropyridine-3-carboxamide

¹H NMR (CDCl₃): δ 10.05-9.95 (1H, m); 8.58 (1H, d, *J* 7.5 Hz); 7.84 - 7.64 (4H, m); 7.54 - 7.41 (4H, m); 6.48 (1H, dd, *J* 7.4, 0.8 Hz); 4.75 - 4.59 (2H, m); 3.76 - 3.69 (4H, m); 3.01 - 2.94 (4H, m); 2.09 (3H, s).
APCI-MS m/z: 536 [MH⁺].

### Example 76 6-Methyl-2-oxo-N-[4-(piperidin-1-ylsulfonyl)benzyl]-1-[3-(trifluoromethyl)-phenyl]-1,2-dihydropyridine-3-carboxamide

¹H NMR (CDCl₃): δ 10.02-9.92 (1H, m); 8.60 (1H, d, *J* 7.3 Hz); 7.84 - 7.64 (4H, m); 7.54 - 7.41 (4H, m); 6.48 (1H, dd, *J* 7.5, 0.5 Hz); 4.74 - 4.59 (2H, m); 2.99 - 2.91 (4H, m); 2.09 (3H, s); 1.68 - 1.35 (6H, m).
APCI-MS m/z: 534 [MH⁺].

### Example 77 6-Methyl-N-{4-[(methylamino)sulfonyl]benzyl}-2-oxo-1-[3-(trifluoromethyl)-phenyl]-1,2-dihydropyridine-3-carboxamide

¹H NMR (CDCl₃): δ 10.02-9.92 (1H, m); 8.59 (1H, d, *J* 7.3 Hz); 7.86 - 7.70 (4H, m); 7.54 - 7.41 (4H, m); 6.48 (1H, d, *J* 7.3 Hz); 4.73 - 4.58 (2H, m); 4.29 (1H, s); 2.63 (3H, s); 2.09 (3H, s).
APCI-MS m/z: 480 [MH⁺].

### Example 78 6-Methyl-2-oxo-N-[4-(pyrrolidin-1-ylsulfonyl)benzyl]-1-[3-(trifluoromethyl)phenyl]-1,2-dihydropyridine-3-carboxamide

¹H NMR (CDCl₃): δ 10.00 - 9.91 (1H, m); 8.60 (1H, d, *J* 7.5 Hz); 7.85 - 7.68 (4H, m); 7.53 - 7.40 (4H, m); 6.48 (1H, dd, *J* 7.5, 0.7 Hz); 4.76 - 4.58 (2H, m); 3.26 - 3.16 (4H, m); 2.09 (3H, s); 1.80 - 1.70 (4H, m).
APCI-MS m/z: 520 [MH⁺].

### Example 79 5-Chloro-6-methyl-2-oxo-N-[4-(pyrrolidin-1-ylsulfonyl)benzyl]-1-[3-(trifluoromethyl)phenyl]-1,2-dihydropyridine-3-carboxamide

The title compound was prepared using the general method of Example 71.
¹H NMR (CDCl₃): δ 9.89 - 9.79 (1H, m); 8.66 (1H, s); 7.86 - 7.72 (4H, m); 7.52 - 7.40 (4H, m); 4.74 - 4.59 (2H, m); 3.25 - 3.17 (4H, m); 2.19 (3H, s); 1.78 - 1.72 (4H, m).
APCI-MS m/z: 554 [MH⁺].

### Example 80 5-Chloro-6-methyl-N-[4-(methylsulfonyl)benzyl]-2-oxo-1-[3-(trifluoromethyl)-phenyl]-1,2-dihydropyridine-3-carboxamide

6-Methyl-N-[4-(methylsulfonyl)benzyl]-2-oxo-1-[3-(trifluoromethyl)phenyl]-1,2-dihydropyridine-3-carboxamide [Example 1.1] (50 mg, 0.108 mmol) was dissolved in acetonitrile (1 ml) and cooled to 0 °C. KNO₃ (16 mg, 0.16 mmol) and sulfuryl chloride (13 µl, 0.16 mmol) were added. The reaction mixture was stirred for 1 h at 0 °C, followed by the addition of saturated aqueous Na₂CO₃ and diethyl ether. The aqueous phase was extracted with diethyl ether and the combined organic phase was washed with brine and dried. After filtration, the solvent was removed in vacuo, the residue was dissolved in methanol, a white precipitate appeared and was filtered off and dried to afford the title compound (22 mg, 41 %).

¹H NMR (CDCl₃): δ 9.93 - 9.80 (1H, m); 8.64 (1H, s); 7.94 - 7.69 (4H, m); 7.57 - 7.37 (4H, m); 4.75 - 4.57 (2H, m); 3.01 (3H, s); 2.19 (3H, s).
APCI-MS m/z: 499 [MH⁺].

### Example 81 N-{4-[(Acetylamino)sulfonyl]benzyl}-6-methyl-2-oxo-1-[3-(trifluoromethyl)-phenyl]-1,2-dihydropyridine-3-carboxamide

N-[4-(Aminosulfonyl)benzyl]-6-methyl-2-oxo-1-[3-(trifluoromethyl)phenyl]-1,2-dihydropyridine-3-carboxamide [Example 1.5] (16 mg, 0.034 mmol) was dissolved in CH₂Cl₂ (1 ml). Powdered KOH (6 mg, 0.11 mmol) and 10% acetyl chloride in CH₂Cl₂ (25 µl, 0.035 mmol) were added and the mixture was stirred at room temperature for 1.5 h. Water and 1M aqueous HCl were added. The reaction mixture was extracted with CH₂Cl₂. The organic phase was washed with water, brine and dried. The solvent was removed in vacuo and the residue was purified by preparative HPLC (x-terra, 0.2% ammonia, acetonitrile) to afford the title compound (7 mg, 41%).
¹H NMR (CDCl₃): δ 10.03-9.96 (1H, m); 8.58 (1H, d, *J* 7.4 Hz); 8.16 (1H, s); 7.98 - 7.93 (2H, m); 7.83 - 7.71 (2H, m); 7.53 - 7.42 (4H, m); 6.47 (1H, d, *J* 7.4 Hz); 4.72 - 4.59 (2H, m); 2.08 (3H, s); 2.02 (3H, s).
APCI-MS m/z: 508 [MH⁺].

Using the general method of Example 10, the compounds of Examples 82 and 83 were prepared:

### Example 82 N-[4-(Isopropylsulfonyl)benzyl]-5-iodo-6-methyl-2-oxo-1-[3-(trifluoromethyl)phenyl]-1,2-dihydropyridine-3-carboxamide

¹H NMR (CDCl₃): δ 9.90 (1H, t, *J* 5.7 Hz); 8.91 (1H, s); 7.83-7.80 (3H, m); 7.76 (1H, t, *J* 7.9 Hz); 7.50-7.48 (3H, m); 7.41 (1H, d, *J* 7.8 Hz); 4.68 (2H, t, *J* 6.2 Hz); 3.15 (1H, m); 2.31 (3H, s); 1.28 (6H, d, *J* 6.89 Hz).
APCI-MS m/z: 619 [MH⁺].

### Example 83 N-[4-(Cyclopropylsulfonyl)benzyl]-5-iodo-6-methyl-2-oxo-1-[3-(trifluoromethyl)phenyl]-1,2-dihydropyridine-3-carboxamide

¹H NMR (CDCl₃): δ 9.86 (1H, t, *J* 5.8 Hz); 8.90 (1H, s); 7.83-7.80 (3H, m); 7.75 (1H, t, *J* 7.8 Hz); 7.49-7.47 (3H, m); 7.40 (1H, d, *J* 7.8 Hz); 4.66 (2H, t, *J* 5.7 Hz); 2.42 (1H, m); 2.31 (3H, s); 1.32 (2H, m); 1.01 (2H, m).
APCI-MS m/z: 617 [MH⁺].

### Example 84 1,2-Dihydro-6-methyl-N-[[4-[(methylsulfonyl)oxy]phenyl]methyl]-2-oxo-1-[3-(trifluoromethyl)phenyl]-3-pyridinecarboxamide

The title compound (31 mg, 46%) was prepared from N-(4-hydroxybenzyl)-6-methyl-2-oxo-1-[3-(trifluoromethyl)phenyl]-1,2-dihydropyridine-3-carboxamide and benzensulfonyl chloride using the general method of Example 61.
¹H NMR (DMSO-d₆) δ 9.87 (1H, t, *J* 6.0 Hz); 8.38 (1H, d, *J* 7.5 Hz); 7.89 (2H, d, *J* 8.9 Hz); 7.80 (1H, t, *J* 7.8 Hz); 7.72 (1H, d, *J* 7.9 Hz); 7.39 (2H, d, *J* 8.6 Hz); 7.29 (2H, d, *J* 8.6 Hz); 6.62 (1H, d, *J* 7.5 Hz); 4.50 (2H, d, *J* 6.0 Hz); 3.35 (3H, s,); 2.02 (3H, s).
APCI-MS m/z: 543.3 [MH⁺].

### Example 85 N-[4-(1,1-Dioxidoisothiazolidin-2-yl)benzyl]-6-methyl-2-oxo-1-[3-(trifluoromethyl)phenyl]-1,2-dihydropyridine-3-carboxamide

The title compound (12 mg, 9.5%) was prepared from N-(4-aminobenzyl)-6-methyl-2-oxo-1-[3-(trifluoromethyl)phenyl]-1,2-dihydroproline-3-carboxamide and 3-chloropropanesulfonyl chloride using the general method of Example 56.
¹H NMR (DMSO-d₆) δ 9.78 (1H, t, *J* 5.8 Hz); 8.37 (1H, d, *J* 7.4 Hz); 7.88 (2H, d, *J* 7.6 Hz); 7.79 (1H, t, *J* 7.8 Hz); 7.70 (1H, d, *J* 8.2 Hz); 7.28 (2H, d, *J* 8.6 Hz); 7.14 (2H, d, *J* 8.5 Hz); 6.61 (1H, d, *J* 7.5 Hz); 4.43 (2H, d, *J* 5.8 Hz); 3.69 (2H, t, *J* 6.5 Hz); 3.47 (2H, t, *J* 7.4 Hz); 2.38 (2H, quintet, *J* 7.0 Hz); 2.01 (3H, s).
APCI-MS m/z 506.3 [MH⁺].

### Example 86 6-Methyl-2-oxo-N-[[4-(4-pyridinylsulfonyl)phenyl]methyl]-1-[3-(trifluoromethyl)phenyl]-1,2-dihydropyridine-3-carboxamide

### a) 1-[4-(Pyridin-4-ylsulfonyl)phenyl]methanamine

To a mixture of 4-mercaptopyridine (0.8 g, 7.2 mmol) and K₂CO₃ (2.0 g, 14.4 mmol) in NMP, 4-fluorobenzaldehyde (0.99 g, 8.0 mmol) was added. The mixture was then stirred at 70 °C for 3 h. After cooling, the reaction mixture was diluted with water (5.0 ml) and extracted with EtOAc. The organic solvent was evaporated and the residue dissolved in methanol. Sodium borohydride (0.57 g, 15 mmol) was added and the mixture stirred for 3 h at room temperature. After addition of water, the methanol was removed in vacuo and the residue extracted with CH₂Cl₂. The organic phase was dried over MgSO₄, filtered, and evaporated and the residue was dissolved in toluene (10 ml). The toluene solution was heated to 40 °C, phosphorus tribromide (0.25 g, 0.92 mmol) was added and the temperature increased to 100 °C for 30 minutes. After cooling, water (50 ml) was added and the mixture extracted with EtOAc. The organic phase was evaporated, the residue dissolved in methanol and slowly added to a mixture of 25% ammonia (15 ml) in methanol (10 ml). After stirring for 3 h at room temperature the subtitle compound was obtained. (0.30 g, 37%) as a white solid.
APCI-MS m/z: 217.2 [MH⁺].

### b) 6-Methyl-2-oxo-N-[[4-(4-pyridinylsulfonyl)phenyl]methyl]-1-[3-(trifluoromethyl)phenyl]-1,2-dihydropyridine-3-carboxamide

To a mixture of 6-methyl-2-oxo-1-[3-(trifluoromethyl)phenyl]-1,2-dihydropyridine-3-carboxylic acid (0.28 g, 0.92 mmol), HBTU (0.35 g, 0.92 mmol) and DIEA (0.24 g, 1.84 mmol) in NMP was added 1-[4-(pyridin-4-ylsulfonyl)phenyl]methanamine (0.20 g, 0.92 mmol) in NMP (1 ml). The reaction mixture was stirred for 1 h at room temperature, then diluted with water (15 ml) and extracted with EtOAc. The organic phase was dried (MgSO₄), filtered and evaporated. To the residue dissolved in CH₂Cl₂ (10 ml) cooled to - 15 °C was added m-chloroperoxybenzoic acid (0.48 g, 2.76 mmol). The mixture was stirred for 30 min and then overnight at room temperature. The reaction mixture was diluted with more CH₂Cl₂ and water, washed with Na₂S₂O₃, NaHCO₃ and brine. The solvent was removed in vacuo and 25 mg of the residue was dissolved in acetonitrile/water (2.0 ml) and purified on a Xterra@Prep MS C8 column (19 x 50 mm) using a gradient of acetonitrile/water at a flow rate of 20 ml/min. Freeze drying of the mixture afforded the title compound (12 mg, 49%).
¹H NMR (DMSO-d₆) δ 9.92 (1H, t, *J* 6.0 Hz); 8.85 (2H, dd, *J* 4.4, 1.6 Hz); 8.34 (1H, d, *J* 7.5 Hz); 7.96 (2H, dd, *J* 6.7, 1.7 Hz); 7.90 (4H, m,); 7.87 (1H, t, *J* 4.4,1.6 Hz); 7.71 (1H, d, *J* 7.9 Hz); 7.54 (2H, d, *J* 8.4 Hz); 6.61 (2H, d, *J* 8.2 Hz); 4.55 (2H, d, *J* 6.0 Hz); 2.01 (3H, s).
APCI-MS m/z: 528.4 [MH⁺].

Starting from 6-methyl-2-oxo-1-[3-(trifluoxomethyl)phenyl]-1,2-dihydropyridine-3-carboxylic acid and the appropriate methanamine derivative and using the general method of Example 86, the compounds of Examples 87 to 91, 92(b) and 93(b) were prepared:

### Example 87 6-Methyl-2-oxo-N-[4-(phenylsulfonyl)benzyl]-1-[3-(trifluoromethyl)phenyl]-1,2-dihydropyridine-3-carboxamide

The title compound (34 mg, 21%) was prepared using 1-[4-(phenylthio)phenyl]methanamine (from thiophenol and 4-fluorobenzaldehyde).
¹H NMR (CDCl₃) δ 9.90 (1H, t, *J* 6.0 Hz); 8.34 (1H, d, *J* 7.5 Hz); 7.90 (6H, m); 7.80 (1H, t, *J* 8.1 Hz); 7.78 (2H, m); 7.59 (2H, t, *J* 7.5 Hz); 7.49 (2H, d, *J* 8.4 Hz); 6.60 (1H, d, *J* 7.7 Hz); 4.53 (2H, d, *J* 6.2 Hz); 2.01 (3H, s).
APCI-MS m/z: 527.4 [MH⁺].

### Example 88 6-Methyl-2-oxo-N-[4-(1,3-thiazol-2-ylsulfonyl)benzyl]-1-[3-(trifluoromethyl)phenyl]-1,2-dihydropyridine-3-carboxamide

The title compound (50 mg, 21%) was prepared using 1-[4-(1,3-thiazol-2-ylsulfonyl)phenyl]methanamine (from 2-mercaptothiazole and 4-fluorobenzaldehyde).
¹H NMR (400 MHz, DMSO-d₆) δ 9.91 (1H, t, *J* 6.1 Hz); 8.32 (1H, d, *J* 11.8 Hz); 8.23 (1H, d, *J* 7.9 Hz); 8.06 (1H, d, *J* 3.1 Hz); 7.95 (2H, d, *J* 8.4 Hz); 7.87 (2H, d, *J* 7.2 Hz); 7.78 (1H, t, *J* 7.9 Hz); 7.68 (1H, t, *J* 7.5 Hz); 7.54 (2H, d, *J* 8.4 Hz); 6.59 (1H, d, *J* 7.7 Hz); 4.54 (2H, d, *J* 6.4 Hz); 1.99 (3H, s).
APCI-MS m/z: 534.3 [MH⁺].

### Example 89 6-Methyl-2-oxo-N-[4-(pyrimidin-2-ylsulfonyl)benzyl]-1-[3-(trifluoromethyl)phenyl]-1,2-dihydropyridine-3-carboxamide

The title compound (10 mg, 23%) was prepared using 1-[4-(pyrimidin-2-ylsulfonyl)phenyl]methanamine (from 2-mercaptopyrimidine and 4-fluorobenzaldehyde).
¹H NMR (DMSO-d₆): δ 9.63 (1H, t, *J* 6.1 Hz); 8.69 (1H, d, *J* 4.9 Hz); 8.05 (1H, d, *J* 7.5 Hz); 7.60 (4H, q, *J* 8.6 Hz); 7.45 (3H, m); 7.23 (2H, d, *J* 8.3 Hz); 6.30 (1H, d, *J* 7.4 Hz); 4.27 (2H, d, *J* 6.6 Hz); 1.70 (3H, s).
A.PCI-MS m/z: 529.3 [MH⁺].

### Example 90 N-[4-(1H-Imidazol-2-ylsulfonyl)benzyl]-6-methyl-2-oxo-1-[3-(trifluoromethyl)phenyl]-1,2-dihydropyridine-3-carboxamide

The title compound (8 mg, 16%) was prepared using 1-[4-(1*H*-imidazol-2-ylsulfonyl)phenyl]methanamine (from 2-mercaptoimidazole and 4-fluorobenzaldehyde).
¹H NMR (DMSO-d₆) δ 9.91 (1H, t, *J* 6.2 Hz); 8.35 (2H, d, *J* 7.4 Hz); 7.88 (4H, t, *J* 8.4 Hz); 7.80 (1H, t, *J* 7.7 Hz); 7.72 (1H, d, *J* 7.9 Hz); 7.52 (2H, d, *J* 8.3 Hz); 7.27 (1H, s); 6.61 (1H, d, *J* 7.5 Hz); 5.75 (1H, s); 4.54 (2H, d, *J* 6.0 Hz); 2.02 (3H, s).
APCI-MS m/z: 517.3 [MH⁺].

### Example 91 6-Methyl-N-{4-[(1-methyl-1H-1,2,4-triazol-5-yl)sulfonyl]benzyl}-2-oxo-1-[3-(trifluoromethyl)phenyl]-1,2-dihydropyridine-3-carboxamide

The title compound (6 mg, 15%) was prepared using 1-{4-[(1-methyl-1*H*-1,2,4-triazol-5-yl)sulfonyl]phenyl}methanamine (from 3-mercapto-4-methyl-4H-1,2,4-triazole and 4-fluorobenzaldehyde).
¹H NMR (DMSO-d₆) δ 9.95 (1H, t, *J* 6.0 Hz); 8.74 (1H, s); 8.36 (1H, d, *J* 7.5 Hz); 7.99 (2H, d, *J* 8.4 Hz); 7.89 (2H, d, *J* 8.5 Hz); 7.80 (2H, t, *J* 7.8 Hz); 7.72 (1H, d, *J* 7.8 Hz); 7.59 (2H, d, *J* 8.4 Hz); 6.62 (1H, d, *J* 7.3 Hz); 4.59 (2H, d, *J* 6.3 Hz); 3.86 (3H, s); 2.02 (3H, s).
APCI-MS m/z: 532.3 [MH⁺].

### Example 92 6-Methyl-N-{4-[(5-methyl-1,3-oxazol-4-yl)sulfonyl]benzyl}-2-oxo-1-[3-(trifluoromethyl)phenyl]-1,2-dihydropyridine-3-carboxamide

### a) 1-{4-[(5-Methyl-1,3-oxazol-4-yl)sulfonyl]phenyl}methanamine

To 5-methyl-4-[(4-methylphenyl)sulfonyl]-1,3-oxazole (prepared according to *J*. Chem. Soc., Perkin Trans. 1, 2000, 527-531) (3.7 g, 15.6 mmol) dissolved in chlorobenzene, N-bromosuccinimide (3.5 g, 19.6 mmol) and 2,2'-azobis(2-methylpropinitrile) (0.27 g, 1.6 mmol) were added. The reaction mixture was then stirred and heated to 60 °C. Bromine (0.104 g, 0.65 mmol) was added and the mixture heated to 90 °C for an additional 2 h. After cooling, 2 % aqueous NaHSO₃ (10 ml) and water (40 ml) were added and the mixture extracted with EtOAc. The organic phase was evaporated, the residue dissolved in methanol and slowly added to a mixture of 25% ammonia (150 ml) in methanol (100 ml). After stirring for 3 h at room temperature the ammonia was removed in vacuo and the water phase extracted with EtOAc. The organic phase was dried (MgSO₄), filtered and evaporated affording the subtitle compound (2 g, 51 %).

### b) 6-Methyl-N-{4-[(5-methyl-1,3-oxazol-4-yl)sulfonyl]benzyl}-2-oxo-1-[3-(trifluoromethyl)phenyl]-1,2-dihydropyridine-3-carboxamide

The title compound (7 mg, 21%) was prepared using 1-{4-[(5-methyl-1,3-oxazol-4-yl)sulfonyl]phenyl }methanamine.
¹H NMR (DMSO-d₆) δ 9.92 (1H, t, *J* 6.0 Hz); 8.39 (1H, s); 8.35 (1H, d, *J* 7.4 Hz); 7.88 (4H, t, *J* 5.4 Hz); 7.80 (1H, t, *J* 8.2 Hz); 7.71 (1H, d, *J* 8.2 Hz); 7.52 (2H, d, *J* 8.1 Hz); 6.61 (1H, d, *J* 7.5 Hz); 4.55 (2H, d, *J* 6.1 Hz); 2.64 (3H, s); 2.02 (3H, s); 1.91 (2H, s).
APCI-MS m/z: 532.3 [MH⁺].

### Example 93 6-Methyl-N-{[6-(methylsulfonyl)pyridin-3-yl]methyl}-2-oxo-1-[3-(trifluoromethyl)phenyl]-1,2-dihydropyridine-3-carboxamide

### a) 1-[6-(Methylsulfonyl)pyridin-3-yl]methanamine

To 2-chloropyridine-5-carboxyaldehyde (0.50 g, 3.5 mmol) in THF (5 ml), sodium methanethiolate (0.50 g, 7.0 mmol) was added. The mixture was then stirred at 70 °C overnight. After cooling, the reaction mixture was diluted with water (15 ml) and extracted with EtOAc. The organic phase was evaporated and the residue dissolved in methanol. Sodium borohydride (0.26 g, 7.0 mmol) was added and the mixture stirred for 3 h at room temperature. After addition of water, the methanol was removed in vacuo and the residue extracted with CH₂Cl₂. The organic phase was dried (MgSO₄), filtered, evaporated and treated with CH₂Cl₂ (50 ml). To the CH₂Cl₂ mixture, phosphorus tribromide (0.25 g, 0.92 mmol) was added and the mixture stirred overnight at room temperature. Water (50 ml) was then added and the mixture extracted with EtOAc. Finally, the organic phase was evaporated, the residue dissolved in methanol and slowly added to a mixture of 25% ammonia (20 ml) in methanol (20 ml). After stirring for 3 h at room temperature the subtitle compound was obtained.

### b) 6-Methyl-N-{[6-(methylsulfonyl)pyridin-3-yl]methyl}-2-oxo-1-[3-(trifluoromethyl)phenyl]-1,2-dihydropyridine-3-carboxamide

The title compound (12 mg, 24%) was prepared using [6-(methylsulfonyl)pyridin-3-yl]methylamine 1-[6-(methylsulfonyl)pyridin-3-yl]methanamine.
¹H-NMR (DMSO-d₆) δ 9.98 (1H, t, *J* 6.1 Hz); 8.71 (1H, s); 8.36 (1H, d, *J* 7.5 Hz); 8.01 (2H, s); 7.90 (2H, d, *J* 8.3 Hz); 7.81 (1H, t, *J* 7.7 Hz); 7.72 (1H, d, *J* 7.9 Hz); 6.62 (1H, d, *J* 7.5 Hz); 4.61 (2H, d, *J* 6.1 Hz); 3.25 (3H, s); 2.03 (3H, s).
APCI-MS m/z: 466.3 [MH⁺].

### Example 94 5-Fluoro-6-methyl-N-[4-(methylsulfonyl)benzyl]-2-oxo-1-[3-(trifluoromethyl)phenyl]-1,2-dihydropyridine-3-carboxamide

To 6-methyl-N-[4-(methylsulfonyl)benzyl]-2-oxo-1-[3-(trifluoromethyl)phenyl]-1,2-dihydropyridine-3-carboxamide [Example 1.1] (0.25 g, 0.54 mmol) in acetonitrile (4.5 ml) under argon, 1-chloromethyl-4-fluoro-1,4-diazoniabicyclo[2.2.2]octane-bis(tetrafluoroborate) (0.45 g, 1.27 mmol) was added. The reaction mixture was heated at 80 °C for 2 h. Water was then added and the product purified on a Xterra@Prep MS C8 column (19 x 50 mm) using a gradient of acetonitrile/water at a flow rate of 20 ml/min. Freeze drying of the mixture afforded the title compound (75 mg, 29%).
¹H NMR (CDCl₃) δ 10.02 (1H, t, *J* 5.4 Hz); 8.57 (1H, d, *J* 9.0 Hz); 7.88 (2H, d, *J* 8.4 Hz); 7.84 (1H, d, *J* 8.1 Hz); 7.76 (1H, t, *J* 7.9 Hz); 7.51 (4H, d, *J* 8.4 Hz); 7.44 (1H, d, *J* 8.0 Hz); 4.67 (2H, t, *J* 5.7 Hz); 3.05 (3H, s); 2.08 (3H, d, *J* 3.3 Hz).
APCI-MS m/z: 483.3 [MH⁺].

### Example 95 N-[4-(methylsulfonyl)benzyl]-2-oxo-6-(2-oxoethyl)-1-[3-(trifluoromethyl)phenyl]-1,2-dihydropyridine-3-carboxamide

Phosphorus oxychloride (1.8 ml, 19.7 mmol) was added dropwise under argon to a stirred ice-cooled solution of dry N,N-dimethylformamide (2.8 ml). After the addition, the cooling was stopped and the mixture was stirred at room temperature for 30 min. Dry dichloromethane (10 ml) was added and the solution was cooled to -20 °C. 6-Methyl-N-[4-(methylsulfonyl)benzyl]-2-oxo-1-[3-(trifluoromethyl)phenyl]-1,2-dihydropyridine-3-carboxamide (1.1 g, 2.4 mmol) was added in small portions at such a rate that the temperature did not rise above 1 °C. After 15 min. at 0 °C, potassium carbonate (3.4 g, 24.6 mmol) was added and the mixture was heated to reflux for 20 min. The reaction mixture was cooled and poured into a cooled solution of 50% aqueous sodium carbonate (200 ml) and stirred at room temperature for 5 h. The mixture was extracted with ethyl acetate. The organic layers were washed with water, brine, dried, filtered and concentrated at reduced pressure to give a dark oil. A part of the oil was purified by preparative HPLC to give the title compound as a yellow solid.
¹H NMR (CDCl₃): δ 10.06 (1H, t, *J* 5.3 Hz); 9.47 (1H, s); 8.66 (1H, d, *J* 7.4 Hz); 7.89 (2H, d, *J* 8.2 Hz); 7.82 (1H, d, *J* 8.1 Hz); 7.71 (1H, t, *J* 7.9 Hz); 7.53 - 7.49 (3H, m); 7.41 (1H, d, *J* 8.2 Hz); 6.50 (1H, d, *J* 7.4 Hz); 4.69 (2H, t, *J* 5.6 Hz); 3.60 (2H, s); 3.02 (3H, s).
APCI-MS m/z: 493 [MH⁺].

### Example 96 5-Ethyl-6-methyl-N-[4-(methylsulfonyl)benzyl]-2-oxo-1-[3-(trifluoromethyl)phenyl]-1,2-dihydropyridine-3-carboxamide

A mixture of 6-methyl-N-[4-(methylsulfonyl)benzyl]-2-oxo-1-[3-(trifluoromethyl) phenyl]-5-vinyl-1,2-dihydropyridine-3-carboxamide (patent application SE 0302487-4) (58 mg, 0.12 mmol), 5% palladium on carbon (11 mg) in ethanol (15 ml) and EtOAc (15 ml) was stirred vigorously under a hydrogen atmosphere for 16 h. The mixture was filtered through Celite, the filtrate was evaporated to dryness and the residue was purified by preparative HPLC to give the title compound as a white solid (33 mg, 56%).
¹H-NMR (CDCl₃): δ 10.09 (1H, t, *J* 5.7 Hz); 8.55 (1H, s); 7.87 (2H, d, *J* 8.3 Hz); 7.80 (1H, d, *J* 7.8 Hz); 7.74 (1H, t, *J* 7.9 Hz); 7.52 (2H, d, *J* 8.4 Hz); 7.50 (1H, s); 7.43 (1H, d, *J* 7.6 Hz); 4.67 (2H, t, *J* 6.4 Hz); 3.01 (3H, s); 2.59 (2H, q, *J* 7.5 Hz); 2.04 (3H, s); 1.23 (3H, t, *J* 7.5 Hz).
APCI-MS m/z: 493 [MH⁺].

### Screen

### Human Neutrophil Elastase Quenched-FRET Assay

The assay uses Human Neutrophil Elastase (HNE) purified from serum (Calbiochem art. 324681; Ref. Baugh, R.J. et al., 1976, Biochemistry. 15, 836-841). HNE was stored in 50 mM NaOAc, 200 mM NaCl, pH 5.5 with added 30% glycerol at -20 °C. The protease substrate used was Elastase Substrate V Fluorogenic, MeOSuc-AAPV-AMC (Calbiochem art. 324740; Ref. Castillo, M.J. et al., 1979, Anal. Biochem. 99, 53-64). The substrate was stored in DMSO at -20°C. The assay additions were as follows: Test compounds and controls were added to black 96-well flat-bottom plates (Greiner 655076), 1 µL in 100% DMSO, followed by 30 µL HNE in assay buffer with 0.01% TritonX-100. The assay buffer constitution was: 100 mM Tris (pH 7.5) and 500 mM NaCl. The enzyme and the compounds were incubated at room temperature for 15 minutes. Then 30 µl substrate in assay buffer was added. The assay was stopped after 30 minutes incubation at room temperature by adding 60 µl stop solution (140 mM acetic acid, 200 mM sodium monochloroacetate, 60 mM sodium acetate, pH 4.3). Fluorescence was measured on a Wallac 1420 Victor 2 instrument at settings: Exitation 380 nm, Emission 460 nm. IC₅₀ values were determined using Xlfit curve fitting using model 205.

When tested in the above screen, the compounds of the Examples gave IC₅₀ values for inhibition of human neutrophil elastase activity of less than 30 µM, indicating that the compounds of the invention are expected to possess useful therapeutic properties. Specimen results are shown in the following Table:

| Compound | Inhibition of Human Neutrophil Elastase IC₅₀ (nM) |
|---|---|
| 1-(3-Bromophenyl)-N-(4-methoxybenzyl)-6-methyl-2-oxo-1,2-dihydropyridine-3-carboxamide | 353 |
| 6-Methyl-N-[(5-methylisoxazol-3-yl)methyl]-2-oxo-1-[3-(trifluoromethyl)phenyl]-1,2-dihydropyridine-3-carboxamide | 318 |
| N-(2,3-Dimethoxybenzyl)-6-methyl-2-oxo-1-[3-(trifluoromethyl)phenyl)-1,2-dihydropyridine-3-carboxamide | 701 |
| N-(2,3-Dihydro-1-benzofuran-5-ylmethyl)-2,4-dioxo-3-[3-(trifluoromethyl)phenyl]-1,2,3,4-tetrahydropyrimidine-5-carboxamide | 2025 |

## Claims

1. A compound of formula (I) 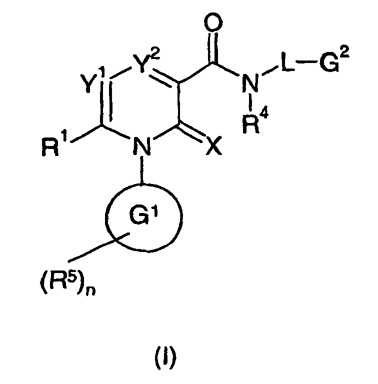 wherein:
**X** represents O or S;
**Y**^{**1**} represents N or CR²; and when R¹ represents OH, Y¹ may also, in the tautomeric form, represent NR⁶;
**Y**^{**2**} represents CR³; and when Y¹ represents CR², then **Y**^{**2**} may also represent N;
**R**^{**1**} represents H or C1 to 6 alkyl; said alkyl being optionally substituted by one or more substituents selected independently from halogen, CN, CHO, OR⁷, NR⁸R⁹, S(O)ₘR¹⁰ and SO₂NR¹¹R¹²;
and, when Y¹ represents N, **R**^{**1**} may also represent OH;
**R**^{**7**} represents H, C1 to 6 alkyl or phenyl; said phenyl being optionally further substituted by halogen, C1 to 6 alkyl and C1 to 6 alkoxy;
**R**^{**2**} represents H, halogen or C1 to 6 alkyl;
**R**^{**3**} represents H or F;
**G**^{**1**} represents phenyl or a five- or six-membered heteroaromatic ring containing 1 to 3 heteroatoms independently selected from O, S and N; or **G**^{**1**} represents a five- or six-membered saturated or partially unsaturated cycloalkyl ring; or **G**^{**1**} represents a five- or six-membered saturated or partially unsaturated heterocyclic ring containing one heteroatom selected from O, S and NR¹³ where R¹³ represents H or C1 to 6 alkyl;
**R**^{**5**} represents H, halogen, C1 to 6 alkyl, CN, C1 to 6 alkoxy, NO₂, NR¹⁴R¹⁵, C1 to 3 alkyl substituted by one or more F atoms or C1 to 3 alkoxy substituted by one or more F atoms;
**R**^{**14**} and **R**^{**15**} independently represent H or C1 to 3 alkyl; said alkyl being optionally further substituted by one or more F atoms;
**n** represents an integer 1, 2 or 3 and when n represents 2 or 3, each R⁵ group is selected independently;
**R**^{**4**} and **R**^{**6**} independently represent H or C1 to 6 alkyl; said alkyl being optionally further substituted by OH or C1 to 6 alkoxy;
or **R**^{**4**} and L are joined together such that the group **-NR**^{**4**}**L** represents a 5 to 7 membered azacyclic ring optionally incorporating one further heteroatom selected from O, S and NR¹⁶;
**L** represents a bond, O, NR²⁹ or C1 to 6 alkyl; said alkyl optionally incorporating a heteroatom selected from O, S and NR¹⁶; and said alkyl being optionally further substituted by OH or OMe;
**G**^{**2**} represents a monocyclic ring system selected from:
i) phenyl or phenoxy,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group; or
**G**^{**2**} represents a bicyclic ring system in which each of the two rings is independently selected from:
i) phenyl,
ii) a 5 or 6 membered heteroaromatic ring containing one to three heteroatoms independently selected from O, S and N,
iii) a C3 to 6 saturated or partially unsaturated cycloalkyl, or
iv) a C4 to 7 saturated or partially unsaturated heterocyclic ring containing one or two heteroatoms independently selected from O, S(O)ₚ and NR¹⁷ and optionally further incorporating a carbonyl group;
and the two rings are either fused together, or are bonded directly together or are separated by a linker group selected from O, S(O)q or CH₂,
said monocyclic or bicyclic ring system being optionally further substituted by one to three substituents independently selected from CN, OH, C1 to 6 alkyl, C1 to 6 alkoxy, halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, C1 to 3 alkoxy substituted by one or more F atoms and C 1 to 3 alkyl substituted by SO₂R³⁹ or by one or more F atoms; or
when L does not represent a bond, **G**^{**2**} may also represent H;
**m, p, q**, s and t independently represent an integer 0, 1 or 2;
**R**^{**8**} and **R**^{**9**} independently represent H, C 1 to 6 alkyl, formyl or C2 to 6 alkanoyl; said alkyl being optionally further substituted by phenyl optionally substituted by halogen, C1 to 6 alkyl, C1 to 6 alkoxy or SO₂R³⁰;
or the group **NR**^{**8**}**R**^{**9**} together represents a 5 to 7 membered azacyclic ring optionally incorporating one further heteroatom selected from O, S and NR²⁸;
**R**^{**18**} and **R**^{**19**} independently represent H, C1 to 6 alkyl, formyl, C2 to 6 alkanoyl, S(O)ₜR³² or SO₂NR³³R³⁴; said alkyl group being optionally further substituted by halogen, CN, C1 to 4 alkoxy or CONR⁴¹R⁴²;
**R**^{**25**} represents H, C1 to 6 alkyl or C3 to 6 cycloalkyl; said alkyl group being optionally further substituted by one or more substituents selected independently from OH, CN, CONR³⁵R³⁶, CO₂R³⁷, OCOR⁴⁰, C3 to 6 cycloalkyl, a C4 to 7 saturated heterocyclic ring containing one or two heteroatoms independently selected from O, S(O)ₚ and NR⁴³ and phenyl or a 5 or 6 membered heteroaromatic ring containing one to three heteroatoms independently selected from O, S and N; said aromatic ring being optionally further substituted by one or more substituents selected independently from halogen, CN, C 1 to 4 alkyl, C1 to 4 alkoxy, OH, CONR⁴⁴R⁴⁵, CO₂R⁴⁶, S(O)ₛR⁴⁷ and NHCOCH₃;
**R**^{**26**} and **R**^{**27**} independently represent H, C1 to 6 alkyl, formyl or C2 to 6 alkanoyl;
**R**^{**32**} represents H, C1 to 6 alkyl or C3 to 6 cycloalkyl;
**R**^{**38**} represents H, C1 to 6 alkyl or phenyl; said phenyl being optionally further substituted by halogen, C 1 to 6 alkyl or C 1 to 6 alkoxy;
**R**^{**10**}**, R**^{**11**}**, R**^{**12**}**, R**^{**16**}**, R**^{**17**}**, R**^{**20**}**, R**^{**21**}**, R**^{**22**}**, R**^{**23**}**, R**^{**24**}**, R**^{**28**}**, R**^{**29**}**, R**^{**30**}**, R**^{**31**}**, R**^{**33**}**, R**^{**34**}**, R**^{**35**}**, R**^{**36**}**, R**^{**37**}**, R**^{**39**}**, R**^{**40**}**, R**^{**41**}**, R**^{**42**}**, R**^{**43**}**, R**^{**44**}**, R**^{**45**}**, R**^{**46**} and **R**^{**47**} independently represent H or C 1 to 6 alkyl;
and pharmaceutically acceptable salts thereof, with the proviso that the following compounds are disclaimed:
N-benzyl-5,6-dimethyl-2-oxo-1-phenyl-1,2-dihydropyridine-3-carboxamide;
N-(2-phenethyl)-5,6-dimethyl-2-oxo-1-phenyl-1,2-dihydropyridine-3-carboxamide;
N-(2-hydroxyethyl)-2,4-dioxo-3-phenyl-1,2,3,4-tetrahydropyrimidine-5-carboxamide;
N-[2-(dimethylamino)ethyl]-2,4-dioxo-3-phenyl-1,2,3,4-tetrahydropyrimidine-5-carboxamide;
4-[2-[[[1,2-dihydro-1-(4-methylcyclohexyl)-2-oxo-3-pyridinyl]carbonyl]amino]ethyl]-benzoic acid;
4-[2-[[(1-cyclohexyl-1,2-dihydro-2-oxo-3-pyridinyl]carbonyl]amino]ethyl]-benzoic acid;
N-benzyl-1-cyclohexyl-5,6-dimethyl-2-oxo-1,2-dihydropyridine-3-carboxamide; and
N-(2-phenethyl)-1-cyclohexyl-5,6-dimethyl-2-oxo-1,2-dihydropyridine-3-carboxamide.

2. A compound according to Claim 1 wherein X represents O.

3. A compound according to Claim 1 or Claim 2 wherein R² and R³ each represent H.

4. A compound of formula (I), according to any one of Claims 1 to 3, wherein G¹ represents phenyl or pyridyl.

5. A compound of formula (I), according to Claim 1, or a pharmaceutically acceptable salt thereof, for use as a medicament.

6. A pharmaceutical formulation comprising a compound of formula (I), as defined in any one of Claims 1 to 4, or a pharmaceutically acceptable salt thereof, optionally in admixture with a pharmaceutically acceptable diluent or carrier.

7. The use of a compound of formula (I) as defined in any one of Claims 1 to 4, or a pharmaceutically acceptable salt thereof, in the manufacture of a medicament for the treatment or prophylaxis of human diseases or conditions in which inhibition of neutrophil elastase activity is beneficial.

8. The use of a compound of formula (I) as defined in any one of Claims 1 to 4, or a pharmaceutically acceptable salt thereof, in the manufacture of a medicament for the treatment or prophylaxis of inflammatory diseases or conditions.

9. A process for the preparation of a compound of formula (I), as defined in any one of Claims 1 to 4, and optical isomers, racemates and tautomers thereof and pharmaceutically acceptable salts thereof, which comprises:
reacting a compound of formula (II) 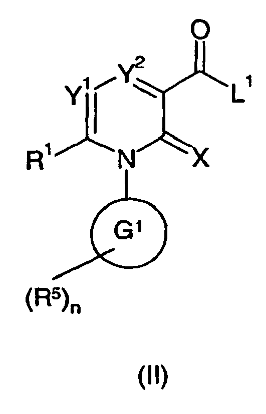
wherein R¹, R⁵, Y¹, Y², X, G¹ and n are as defined in Claim 1 and L¹ represents a leaving group,
with an amine of formula (III) or a salt thereof 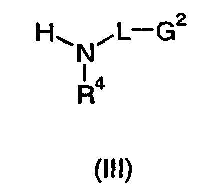 wherein R⁴, G² and L are as defined in Claim 1,
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Revendications

1. Composé de formule (I) 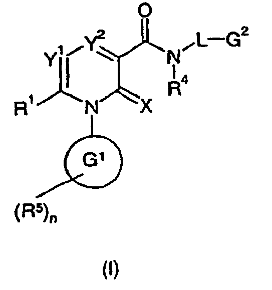 dans laquelle
**X** représente O ou S
**Y**^{**1**} représente N ou CR² ; et lorsque R¹ représente OH, Y¹ peut également, sous la forme tautomère, représenter NR⁶ ;
**Y**^{**2**} représente CR³ ; et lorsque Y¹ représente CR², alors Y² peut également représenter N ;
**R**^{**1**} représente H ou alkyle en C1 à 6 ; ledit alkyle étant éventuellement substitué par un ou plusieurs substituants choisis indépendamment parmi halogène, CN, CHO, OR⁷, NR⁸R⁹, S(O)ₘR¹⁰ et SO₂NR¹¹R¹² ;
et lorsque Y¹ représente N, **R**^{**1**} peut également représenter OH ;
**R**^{**7**} représente H, alkyle en C1 à 6 ou phényle ; ledit phényle étant éventuellement encore substitué par halogène, alkyle en C1 à 6 et alcoxy en C1 à 6 ;
**R**^{**2**} représente H, halogène ou alkyle en C1 à 6 ;
**R**^{**3**} représente H ou F ;
**G**^{**1**} représente phényle ou un cycle hétéroaromatique de cinq ou six chaînons contenant de 1 à 3 hétéroatomes choisis indépendamment parmi O, S et N ; ou **G**^{**1**} représente un cycle cycloalkyle saturé ou partiellement insaturé à cinq ou six chaînons ; ou **G**^{**1**} représente un cycle hétérocyclique saturé ou partiellement insaturé à cinq ou six chaînons contenant un hétéroatome choisi parmi O, S et NR¹³ où R¹³ représente H ou alkyle en C1 à 6 ;
**R**^{**5**} représente H, halogène, alkyle en C1 à 6, CN, alcoxy en C1 à 6, NO₂ NR¹⁴R¹⁵, alkyle en C1 à 3 substitué par un ou plusieurs atomes de F ou alcoxy en C1 à 3 substitué par un ou plusieurs atomes de F ;
**R**^{**14**} et **R**^{**15**} représentent indépendamment H ou alkyle en C1 à 3 ; ledit alkyle étant éventuellement encore substitué par un ou plusieurs atomes de F ;
**n** représente un entier 1, 2 ou 3 et lorsque n représente 2 ou 3, chaque groupement R⁵ est choisi indépendamment ;
**R**^{**4**} et **R**^{**6**} représentent indépendamment H ou alkyle en C1 à 6 ; ledit alkyle étant éventuellement encore substitué par OH ou alcoxy en C1 à 6 ;
ou **R**^{**4**} et **L** sont reliés ensemble de sorte que le groupement **-NR**^{**4**}**L** représente un cycle azacyclique à 5 à 7 chaînons comportant éventuellement un hétéroatome supplémentaire choisi parmi O, S et NR¹⁶ ;
**L** représente une liaison, O, NR²⁹ ou alkyle en C1 à 6 ; ledit alkyle comportant éventuellement un hétéroatome choisi parmi O, S et NR¹⁶ et ledit alkyle étant éventuellement encore substitué par OH ou OMe ;
**G**^{**2**} représente un système de cycle monocyclique choisi parmi :
i) phényle ou phénoxy,
ii) un cycle hétéroaromatique à 5 ou 6 chaînons contenant de un à trois hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant encore éventuellement un groupement carbonyle ; ou
**G**^{**2**} représente un système de cycle bicyclique dans lequel chacun des deux cycles est choisi indépendamment parmi :
i) phényle,
ii) un cycle hétéroaromatique à 5 ou 6 chaînons contenant de un à trois hétéroatomes choisis indépendamment parmi O, S et N,
iii) un cycloalkyle en C3 à 6 saturé ou partiellement insaturé, ou
iv) un cycle hétérocyclique en C4 à 7 saturé ou partiellement insaturé contenant un ou deux hétéroatomes choisis indépendamment parmi O, S(O)ₚ et NR¹⁷ et comportant encore éventuellement un groupement carbonyle ;
et les deux cycles sont fusionnés ensemble, liés directement ensemble ou séparés par un groupement lieur choisi parmi O, S(O)_{q} ou CH₂,
ledit système de cycle monocyclique ou bicyclique étant éventuellement encore substitué par un à trois substituants choisis indépendamment parmi CN, OH, alkyle en C1 à 6, alcoxy en C1 à 6, halogène, NR¹⁸R¹⁹, NO₂, OSO₂R³⁸, CO₂R²⁰, C(=NH)NH₂, C(O)NR²¹R²², C(S)NR²³R²⁴, SC(=NH)NH₂, NR³¹C(=NH)NH₂, S(O)ₛR²⁵, SO₂NR²⁶R²⁷, alcoxy en C1 à 3 substitué par un ou plusieurs atomes de F et alkyle en C1 à 3 substitué par SO₂R³⁹ ou par un ou plusieurs atomes de F ; ou
lorsque L ne représente pas une liaison **G**^{**2**} peut également représenter H ;
**m, p, q, s** et **t** représentent indépendamment un entier 0, 1 ou 2 ;
**R**^{**8**} et **R**^{**9**} représentent indépendamment H, alkyle en C1 à 6, formyle ou alcanoyle en C2 à 6 ; ledit alkyle étant éventuellement encore substitué par phényle éventuellement substitué par halogène, alkyle en C1 à 6, alcoxy en C1 à 6 ou SO₂R³⁰ ;
ou le groupement **NR**^{**8**}**R**^{**9**} représente ensemble un cycle azacyclique à 5 à 7 chaînons comportant éventuellement un hétéroatome supplémentaire choisi parmi O, S et NR²⁸ ;
**R**^{**18**} et **R**^{**19**} représentent indépendamment H, alkyle en C1 à 6, formyle, alcanoyle en C2 à 6, S(O)ₜR³² ou SO₂NR³³R³⁴ ; ledit groupement alkyle étant éventuellement encore substitué par halogène, CN, alcoxy en C1 à 4 ou CONR⁴¹R⁴² ;
**R**^{**25**} représente H, alkyle en C1 à 6 ou cycloalkyle en C3 à 6 ; ledit groupement alkyle étant éventuellement encore substitué par un ou plusieurs substituants choisis indépendamment parmi OH, CN, CONR³⁵R³⁶, CO₂R³⁷, OCOR⁴⁰, cycloalkyle en C3 à 6, un cycle hétérocyclique en C4 à 7 saturé contenant un ou deux hétéroatomes choisis indépendamment parmi O, S(O)ₚ et NR⁴³ et phényle ou un cycle hétéroaromatique à 5 ou 6 chaînons contenant de un à trois hétéroatomes choisis indépendamment parmi O, S et N ; ledit cycle aromatique étant éventuellement encore substitué par un ou plusieurs substituants choisis indépendamment parmi halogène, CN, alkyle en C1 à 4, alcoxy en C1 à 4, OH, CONR⁴⁴R⁴⁵, CO₂R⁴⁶, S(O)ₛR⁴⁷ et NHCOCH₃ ;
**R**^{**26**} et **R**^{**27**} représentent indépendamment H, alkyle en C1 à 6, formyle ou alcanoyle en C2 à 6 ;
**R**^{**32**} représente H, alkyle en C1 à 6 ou cycloalkyle en C3 à 6 ;
**R**^{**38**} représente H, alkyle en C1 à 6 ou phényle ; ledit phényle étant éventuellement encore substitué par halogène, alkyle en C1 à 6 ou alcoxy en C1 à 6 ;
**R**^{**10**}**, R**^{**11**}**, R**^{**12**}**, R**^{**16**}**, R**^{**17**}**, R**^{**20**}**, R**^{**21**}**, R**^{**22**}**, R**^{**23**}**, R**^{**24**}**, R**^{**28**}**, R**^{**29**}**, R**^{**30**}**, R**^{**31**}**, R**^{**33**}**, R**^{**34**}**, R**^{**35**}**, R**^{**36**}**, R**^{**37**}**, R**^{**39**}**, R**^{**40**}**, R**^{**41**}**, R**^{**42**}**, R**^{**43**}**, R**^{**44**}**, R**^{**45**}**, R**^{**46**} et **R**^{**47**} représentent indépendamment H ou alkyle en C1 à 6 ;
et les sels pharmaceutiquement acceptables de celui-ci, à condition que les composés suivants ne soient pas revendiqués :
le N-benzyl-5,6-diméthyl-2-oxo-1-phényl-1,2-dihydropyridine-3-carboxamide ;
le N-(2-phénéthyl)-5,6-diméthyl-2-oxo-1-phényl-1,2-dihydropyridine-3-carboxamide ;
le N-(2-hydroxyéthyl)-2,4-dioxo-3-phényl-1,2,3,4-tétrahydropyrimidine-5-carboxamide ;
le N-[2-(diméthylamino)éthyl]-2,4-dioxo-3-phényl-1,2,3,4-tétrahydropyrimidine-5-carboxamide ;
l'acide 4-[2-[[[1,2-dihydro-1-(4-méthylcyclohexyl)-2-oxo-3-pyridinyl]carbonyl]amino]éthyl]-benzoïque ;
l'acide 4-[2-[[(1-cyclohexyl-1,2-dihydro-2-oxo-3-pyridinyl]carbonyl]amino]éthyl]benzoïque ;
le N-benzyl-1-cyclohexyl-5,6-diméthyl-2-oxo-1,2-dihydropyridine-3-carboxamide ; et
le N-(2-phénéthyl)-1-cyclohexyl-5,6-diméthyl-2-oxo-1,2-dihydropyridine-3-carboxamide.

2. Composé selon la revendication 1, **caractérisé en ce que** X représente O.

3. Composé selon la revendication 1 ou la revendication 2, **caractérisé en ce que** R² ou R³ représente chacun H.

4. Composé de formule (I), selon l'une quelconque des revendications 1 à 3, **caractérisé en ce que** G¹ représente phényle ou pyridyle.

5. Composé de formule (I), selon la revendication 1, ou un sel pharmaceutiquement acceptable de celui-ci, destiné à être utilisé comme médicament.

6. Formulation pharmaceutique comprenant un composé de formule (I), tel que défini dans l'une quelconque des revendications 1 à 4, ou un sel pharmaceutiquement acceptable de celui-ci, éventuellement en mélange avec un diluant ou un support pharmaceutiquement acceptable.

7. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie de maladies ou d'affections humaines dans lesquelles l'inhibition de l'activité de l'élastase des neutrophiles est bénéfique.

8. Utilisation d'un composé de formule (I), tel que défini dans l'une quelconque des revendications 1 à 4, ou d'un sel pharmaceutiquement acceptable de celui-ci, dans la fabrication d'un médicament destiné au traitement ou à la prophylaxie de maladies ou d'affections inflammatoires.

9. Procédé de préparation d'un composé de formule (I), tel que défini dans l'une quelconque des revendications 1 à 4, et des isomères optiques, des racémates et des tautomères de celui-ci et des sels pharmaceutiquement acceptables de celui-ci, qui comprend :
la réaction d'un composé de formule (II) 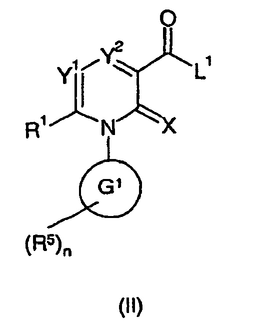
dans laquelle R¹, R⁵, Y¹, Y², X, G¹ et n sont tels que définis dans la revendication 1 et L¹ représente un groupement partant,
avec une amine de formule (III) ou un sel de celle-ci 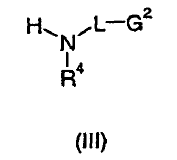 dans laquelle R⁴, G² et L sont tels que définis dans la revendication 1,
et si on le souhaite ou si nécessaire la transformation du composé résultant de formule (I), ou d'un autre sel de celui-ci, en un sel pharmaceutiquement acceptable de celui-ci ; ou la transformation d'un composé de formule (I) en un autre composé de formule (I) ; et si on le souhaite la transformation du composé résultant de formule (I) en un isomère optique de celui-ci.

## Patentansprüche

1. Verbindungen der Formel (I) 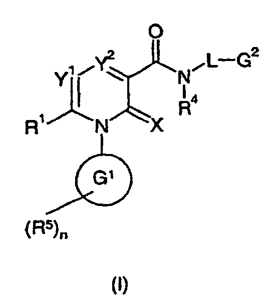 wobei:
X für O oder S steht;
Y¹ für N oder CR² steht; wobei, wenn R¹ für OH steht, Y¹ in der tautomeren Form auch für NR⁶ stehen kann;
Y² für CR³ steht; wobei, wenn Y¹ für CR² steht, Y² auch für N stehen kann;
R¹ für H oder C₁₋₆-Alkyl steht; wobei die Akylgruppe gegebenenfalls durch ein oder mehrere Substituenten unabhängig voneinander ausgewählt aus Halogen, CN, CHO, OR⁷, NR⁸R⁹, S(O)ₘR¹⁰ und SO₂NR¹¹R¹² substituiert ist;
und, wenn Y¹ für N steht, R¹ auch für OH stehen kann;
R⁷ für H, C₁₋₆-Alkyl oder Phenyl steht; wobei die Phenylgruppe gegebenenfalls weiter substituiert ist durch Halogen, C₁₋₆-Alkyl ond C₁₋₆-Alkoxy;
R² für H, Halogen oder C₁₋₆-Alkyl steht;
R³ für H oder F steht;
G¹ für Phenyl oder ein fünf- oder sechsgliedrigen heteroaromatischen Ring mit 1 bis 3 Heteroatomen unabhängig voneinander ausgewählt aus O, S und N steht; oder G¹ für einen fünf- oder sechsgliedrigen gesättigten oder teilweise ungesättigen Cycloalkylring steht; oder G¹ für einen fünf- oder sechsgliedrigen gesättigten oder teilweise ungesättigen heterocyclischen Ring mit einem Heteroatom ausgewählt aus O, S und NR¹³ steht; wobei R¹³ für H oder C₁₋₆-Alkyl steht;
R⁵ für H, Halogen, C₁₋₆-Alkyl, CN, C₁₋₆-Alkoxy, NO₂, NR¹⁴R¹⁵, C₁₋₃-Alkyl substituiert durch ein oder mehrere F-Atome oder C₁₋₃-Alkoxy substituiert durch ein oder mehrere F-Atome steht;
R¹⁴ und R¹⁵ unabhängig voneinander für H oder C₁₋₃₋Alkyl stehen; wobei die Alkylgruppe gegebenenfalls weiter substituiert ist durch ein oder mehrere F-Atome;
n für eine ganze Zahl 1, 2 oder 3 steht und, wenn n für 2 oder 3 steht, die R⁵-Gruppen jeweils unabhängig voneinander ausgewählt sind;
R⁴ und R⁶ unabhängig voneinander für H oder C₁₋₆₋Alkyl stehen; wobei die Alkylgruppe gegebenenfalls weiter substituiert ist durch OH oder C₁₋₆-Alkoxy;
oder R⁴ und L so miteinander verbunden sind, daß die Gruppe -NR⁴L für einen fünf- bis siebengliedrigen azacyclischen Ring steht, der gegebenenfalls ein weiteres Heteroatom ausgewählt aus O, S und NR¹⁶ enthält;
L für eine Bindung, O, NR²⁹ oder C₁₋₆-Alkyl steht; wobei die Alkylgruppe gegebenenfalls ein Heteroatom ausgewählt aus O, S und NR¹⁶ enthält; und wobei die Alkylgruppe gegebenenfalls weiter substituiert ist durch OH oder OMe;
G² für ein monocyclisches Ringsystem ausgewählt aus:
i) Phenyl oder Phenoxy,
ii) einem fünf- oder sechsgliedrigen heteroaromatischen Ring mit einem bis drei Heteroatomen unabhängig voneinander ausgewählt aus O, S und N,
iii) einem gesättigten oder ungesättigten C₃₋₆₋Cycloalkyl oder
iv) einem gesättigten oder teilweise ungesättigten heterocyclischen C₄₋₇-Ring mit einem oder zwei Heteroatomen unabhängig voneinander ausgewählt aus O, S(O)ₚ und NR¹⁷, der gegebenenfalls weiterhin eine Carbonylgruppe enthält, steht; oder
G² für ein bicyclisches Ringsystem steht, in welchem die beiden Ringe jeweils unabhängig voneinander ausgewählt sind aus:
i) Phenyl,
ii) einem fünf- oder sechsgliedrigen heteroaromatischen Ring mit einem bis drei Heteroatomen unabhängig voneinander ausgewählt aus O, S und N,
iii) einem gesättigten oder ungesättigten C₃₋₆₋Cycloalkyl oder
iv) einem gesättigten oder teilweise ungesättigten heterocyclischen C₄₋₇-Ring mit einem oder zwei Heteroatomen unabhängig voneinander ausgewählt aus O, S(O)ₚ und NR¹⁷, der gegebenenfalls weiterhin eine Carbonylgruppe enthält, steht;
und die beiden Ringe entweder miteinander kondensiert sind oder direkt aneinander gebunden sind oder durch eine Linkergruppe ausgwählt aus O, S(O)q oder CH₂, getrennt sind,
wobei das monocyclische oder bicyclische Ringsystem gegebenenfalls weiter substituiert ist durch einen bis drei Substituenten unabhängig voneinander ausgewählt aus CN, OH, C₁₋₆-Alkyl, C₁₋₆₋Alkoxy, Halogen, NR¹⁸R¹⁹, NO₂, OSO₂R³⁸, CO₂R²⁰, C(=NH)NH₂, C(O)NR²¹R²², C(S)NR²³R²⁴, SC(=NH)NH², NR³¹C(=NH)NH₂, S(O)_{S}R²⁵, SO₂NR²⁶R²⁷, C₁₋₃-Alkoxy substituiert durch ein oder mehrere F-Atome und C₁₋₃-Alkyl substituiert durch SO₂R³⁹ oder oder durch ein oder mehrere F-Atome;
oder
wenn L nicht für eine Bindung steht, G² auch für H stehen kann;
m, p, q, s und t unabhängig voneinander für eine ganze Zahl 0, 1 oder 2 stehen;
R⁸ und R⁹ unabhängig voneinander für H, C₁₋₆-Alkyl, Formyl oder C₂₋₆-Alkanoyl stehen; wobei die Alkylgruppen gegebenenfalls weiter substituiert sind durch Phenyl, das gegebenenfalls durch Halogen, C₁₋₆-Alkyl, C₁₋₆-Alkoxy oder SO₂R³⁰ substituiert ist;
oder die Gruppe NR⁸R⁹ zusammen für einen fünf- bis siebengliedrigen azacyclischen Ring steht, der gegebenenfalls ein weiteres Heteroatom ausgewählt aus O, S und NR²⁸;
R¹⁸ und R¹⁹ unabhängig voneinander für H, C₁₋₆-Alkyl, Formyl oder C₂₋₆-Alkanoyl, S(O)ₜR³² oder SO₂NR³³R³⁴ stehen; wobei die Alkylgruppe gegebenenfalls weiter substituiert ist durch Halogen, CN, C₁₋₄₋Alkoxy oder CONR⁴¹R⁴² ;
R²⁵ für H, C₁₋₆-Alkyl oder C₃₋₆-Cycloalkyl steht; wobei die Alkylgruppe gegebenenfalls weiter substituiert ist durch einen oder mehrere Substituenten unabhängig voneinander ausgewählt aus OH, CN, CONR³⁵R³⁶, CO₂R³⁷, OCOR⁴⁰, C₃₋₆₋Cycloalkyl, einen gesättigten heterocyclischen C₄₋₇-Ring mit einem oder zwei Heteroatomen unabhängig voneinander ausgewählt aus 0, S(O)ₚ und NR⁴³ und Phenyl oder einen fünf- oder sechsgliedrigen heteroaromatischen Ring mit einem bis drei Heteroatomen unabhängig voneinander ausgewählt O, S und N; wobei der aromatische Ring gegebenenfalls weiter substituiert ist durch einen oder mehrere Substituenten unabhängig voneinander ausgewählt aus Halogen, CN, C₁₋₄-Alkyl, C₁₋₄-Alkoxy, OH, CONR⁴⁴R⁴⁵, CO₂R⁴⁶, S(O)ₛR⁴⁷ und NHCOCH₃;
R²⁶ und R²⁷ unabhängig voneinander für H, C₁₋₆-Alkyl, Formyl oder C₂₋₆-Alkanoyl stehen;
R³² für H, C₁₋₆-Alkyl oder C₃₋₆-Cycloalkyl steht;
R³⁸ für H, C₁₋₆-Alkyl oder Phenyl steht, wobei die Phenylgruppe gegebenenfalls weiter substituiert ist durch Halogen, C₁₋₆-Alkyl oder C₁₋₆-Alkoxy steht;
R¹⁰, R¹¹, R¹², R¹⁶, R¹⁷, R²⁰, R²¹, R²², R²³, R²⁴, R²⁸, R²⁹, R³⁰, R³¹, R³³, R³⁴, R³⁵, R³⁶, R³⁷, R³⁹, R⁴⁰, R⁴¹, R⁴², R⁴³, R⁴⁴, R⁴⁵, R⁴⁶ und R⁴⁷ unabhängig voneinander für H oder C₁₋₆-Alkyl stehen;
und deren pharmazeutisch annehmbare Salze, mit der Maßgabe, daß die folgenden Verbindungen ausgenommen sind:
N-Benzyl-5,6-dimethyl-2-oxo-1-phenyl-1,2-dihydropyridin-3-carbonsäureamid;
N-(2-Phenethyl)-5,6-dimethyl-2-oxo-1-phenyl-1,2-dihydropyridin-3-carbonsäureamid;
N-(2-Hydroxyethyl)-2,4-dioxo-3-phenyl-1,2,3,4-tetrahydropyrimidin-5-carbonsäureamid;
N-[2-(Dimethylamino)ethyl]-2,4-dioxo-3-phenyl-1,2,3,4-tetrahydropyrimidin-5-carbonsäureamid;
4-(2-[[(1,2-Dihydro-1-(4-methylcyclohexyl)-2-oxo-3-pyridinyl]carbonyl]amino]ethyl]benzoesäure;
4-[2-[[(1-Cyclohexyl-1,2-dihydro-2-oxo-3-pyridinyl]carbonyl]amino]ethyl]benzoesäure;
N-Benzyl-1-cyclohexyl-5,6-dimethyl-2-oxo-1,2-dihydropyridin-3-carbonsäureamid; und
N-(2-Phenethyl)-1-cyclohexyl-5,6-dimethyl-2-oxo-1,2-dihydropyridin-3-carbonsäureamid.

2. Verbindungen nach Anspruch 1, wobei X für O steht.

3. Verbindungen nach Anspruch 1 oder 2, wobei R² und R³ jeweils für H stehen.

4. Verbindungen der Formel (I) nach einem der Ansprüche 1 bis 3, wobei G¹ für Phenyl oder Pyridyl steht.

5. Verbindungen der Formel (I) nach Anspruch 1 und deren pharmazeutisch annehmbare Salze zur Verwendung als Medikament.

6. Pharmazeutische Formulierung, enthaltend eine wie in einem der Ansprüche 1 bis 4 definierte Verbindung der Formel (I) oder ein pharmazeutisch annehmbares Salz davon, gegebenenfalls in einer Mischung mit einem pharmazeutisch annehmbaren Verdünnungsmittel bzw. Träger.

7. Verwendung einer wie in einem der Ansprüche 1 bis 4 definierten Verbindungen der Formel (I) oder eines pharmazeutisch annehmbaren Salzes davon bei der Herstellung eines Medikaments zur Behandlung oder Prophylaxe von humanen Krankheiten oder Leiden, bei denen die Inhibierung der Aktivität von neutrophiler Elastase von Nutzen ist.

8. Verwendung einer wie in einem der Ansprüche 1 bis 4 definierten Verbindung der Formel (I) oder eines pharmazeutisch annehmbaren Salzes davon bei der Herstellung eines Medikaments zur Behandlung oder Prophylaxe entzündlicher Krankheiten oder Leiden.

9. Verfahren zur Herstellung einer wie in einem der Ansprüche 1 bis 4 definierten Verbindungen der Formel (I) und von optischen Isomeren, Racematen und Tautomeren davon und pharmazeutisch annehmbaren Salzen davon, bei dem man:
eine Verbindung der Formel (II) 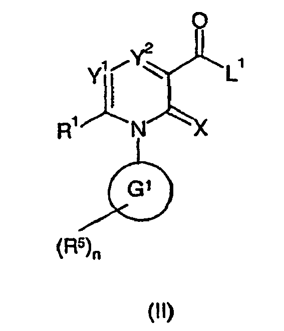
wobei R¹, R⁵, Y¹, Y², X, G¹ und n wie Anspruch 1 definiert sind und L¹ für eine Abgangsgruppe steht, mit einem Amin der Formel (III) oder einem Salz. davon 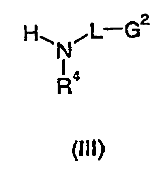 wobei R⁴, G² und L wie in Anspruch 1 definiert sind, umsetzt,
und, falls gewünscht oder erforderlich, die so erhaltene Verbindung der Formel (I) oder ein anderes Salz davon in ein pharmazeutisch annehmbares Salz davon umwandelt; oder eine Verbindung der Formel (I) in eine andere Verbindung der Formel (I) umwandelt; und, falls gewünscht, die so erhaltene Verbindung der Formel (I) in ein optisches Isomer davon umwandelt.
